# EUROPEAN PATENT APPLICATION

(11) **EP 4 434 987 A1**
(43) Date of publication of application: **25.09.2024**
(21) Application number: 23382280.8
(22) Date of filing: 24.03.2023
(51) Int. Cl.: C07D 471/04, A61P 35/00, A61K 31/437

(54) **NOVEL PD-L1 INHIBITORS**

(71) Applicant: Affirma Biotech S. L., 08025 Barcelona (ES)
(72) Inventor: BERGES, Maribel, 08025 Barcelona (ES); ALMANSA ROSALES, Carmen, 08022 Barcelona (ES); BASU, Sujay, 700075 Kolkata (IN)
(74) Representative: Hoffmann Eitle

(57) **Abstract**

The present invention relates to compounds for modulating PD-1/PD-L1 protein/protein interaction that are useful in the treatment of various diseases associated with over-expression of PD-1/PD-L1, including infectious diseases and cancer. The invention additionally relates to compositions comprising such compounds as well as to their uses.

## Description

### Technical field

The present invention relates to compounds for modulating PD-1/PD-L1 protein/protein interaction that are useful in the treatment of various diseases associated with over-expression of PD-1/PD-L1, including infectious diseases and cancer. The invention additionally relates to compositions comprising such compounds as well as to their uses.

### Background art

Immune checkpoint molecules are inhibitory receptors expressed on immune cells that trigger immunosuppressive signalling pathways. These molecules are crucial for maintaining self-tolerance and for modulating the length and magnitude of effector immune responses in peripheral tissues, in order to minimize collateral tissue damage.

The protein-protein interaction PD1/PD-L1 is an important immune checkpoint. PD-1 is expressed on all activated T cells and acts as an inhibitory brake to tamper T cell immune responses as an immune tolerance mechanism. PD-1 inhibits T cell function when bound to either of its binding partners, PD-L1 or PD-L2⁽¹⁾.

PD-L1 has been found to be overexpressed in various types of cancer cells. The up-regulation of PD-1 on immune cells also occur during acute and chronic infections, viral as well as bacterial or fungal. Accordingly, this immune checkpoint is believed to play a critical role in the suppression of antigen-specific T cell response in diseases like cancer and infections.

Targeting the programmed cell death protein 1/programmed cell death 1 ligand 1 (PD-1/PDL1) interaction has become an established strategy for cancer immunotherapy. Additionally, the fact that these inhibitory pathways are also exploited for immune evasion by pathogens suggests that blockade could be used for the prevention and treatment of infectious diseases, in either the acute or chronic phases of infection ⁽²⁾⁽³⁾.

The majority of clinically used PD-1/PD-L1 inhibitors are monoclonal antibodies but their applications are limited due to their PD-1/PD-L1 inhibitors.

However, the development of small molecule immunomodulators of the pathway has lagged far behind, and at present, only a limited number of drug candidates show good PD-1/PD-L1 blocking activity in cell-based assays ⁽²⁾. Therefore, the identification of chemical moieties, especially small molecule inhibitors, that facilitate this inhibition is necessary. In particular, it would be useful to have compounds for oral administration that could help overcoming the limitations of antibodies. Accordingly, the identification and development of new PD-1/PD-L1 inhibitor compounds for treating diseases or conditions associated with reversion of T-Cell exhaustion would open new opportunities in the realm of cancer and infections treatment.

### Non-patent literature

(1) M.N. Wikes, S. R. Lewin, Nat Rev Immunol. 2018, Vol. 18(2), 91-104
(2) C. Fang-Fang, L. Zheng et al. Oncoimmunology, 2020, Vol. 9 no. 1
(3) L. Chang, N.P. Seeram et al. Cancer Cell Int. 2021, 21-239

### Summary of the invention

The present invention provides a compound of Formula (I) or a pharmaceutically acceptable salt or a stereoisomer thereof 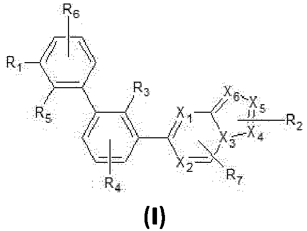 wherein the substituents are as defined herewith.

The present invention also relates to a pharmaceutical composition comprising a compound of the invention, or a pharmaceutically acceptable salt or a stereoisomer thereof, and one or more pharmaceutically acceptable carrier or excipient.

The present invention further provides compounds and compositions for use in a method of treatment of diseases or disorders associated with the reversion of T-Cell exhaustion, wherein such disease can be cancer or an infection.

The present invention additionally provides methods of treatment of diseases or disorders associated with the reversion of T-Cell exhaustion, wherein such disease can be cancer or an infection.

### Detailed description of the invention

### Definitions

Unless otherwise specified, all scientific, chemical names, and technical terms used herein have the same meaning as commonly understood by the skilled person. A dash (-) at the front or end of a chemical group is a matter of convenience to indicate the point of attachment. However, chemical groups may be depicted with or without one or more dashes without losing their ordinary meaning. If not otherwise specified, each linking substituent include both the forward and backward forms of the linking substituent. For example, -C₆-₁₀ aryl-C₁-₄ alkyl- includes both -C₆-₁₀ aryl-C₁-₄ alkyl- and -C₁-₄ alkyl-C₆-₁₀ aryl- and is intended to disclose each of the forms individually and can be attached on one side only (e.g., C₁-₄alkyl or C₆-₁₀ aryl-) or on both sides.

The term "substituted" means that an atom or group of atoms formally replaces hydrogen as a "substituent" attached to another group. The phrase "optionally substituted" means unsubstituted or substituted. The term "substituted", unless otherwise indicated, refers to any level of substitution, e.g., mono-, di-, tri-, or tetra- or penta-substitution, where such substitution is permitted. The substituents are independently selected, and substitution may be at any chemically accessible position. It is to be understood that substitution at a given atom is limited by valency, and by molecular stability. In the present disclosure letters as Xₙ, Yₙ, Rₙ can be used to indicate the substitution of a hydrogen atom with one or more substituents (e.g., Rₐ, R_{b}, R_{c}, R_{d}, Rₑ, R_{f}). The substituents are each independently selected, for example, if one Rₙ is selected to be a certain atom or group of atoms (e.g., C₁-₆ alkyl), the other Rₙₛ can be anything else from the lists of possible substituents.

The term "alkyl," as used herein refers to a saturated hydrocarbon group of general formula -CₙH₂ₙ₊₁ that may be straight-chained or branched. Non limiting examples of alkyl moieties include methyl, ethyl, n-propyl, isopropyl, n-butyl, tert-butyl, isobutyl, sec-butyl etc. The term "Cₙ₋ₘ alkyl" as used herein refers to an alkyl group having n to m carbon atoms. For example, the term "C₁-₆ alkyl" is intended to individually disclose (without limitation) methyl, ethyl, C₃ alkyl, C₄ alkyl, C₅ alkyl and C₆ alkyl.

The term "alkynyl," as used herein refers to a straight-chain or branched hydrocarbon group corresponding to an alkyl group having one or more triple carbon-carbon bonds. The term "Cₙ-ₘ alkynyl" refers to an alkynyl group having n to m carbons. Non limiting examples of alkynyl groups include ethynyl, propyn-1-yl, propyn-2-yl and the like.

The term "alkoxy," as used herein refers to a group of formula -O-alkyl, wherein the alkyl group is as defined above. The term "Cₙ-ₘ alkoxy" refers to an alkoxy group, the alkyl group of which has n to m carbons. Non limiting examples of alkoxy groups include methoxy, ethoxy, propoxy (e.g., n-propoxy and isopropoxy) and the like.

The terms "halogen" or "halo" as used herein refers to halogen atom selected from fluoro (F), chloro (CI), or bromo (Br). In some preferred embodiments of the present invention halogen groups are Cl.

The term nitrile" or "cyano" as used herein refers to a group of formula -CN, also written as (-C≡N).

The term "amino," as used herein refers to a group of formula -NH₂. The term Cₙ-Cₘ Alkyl-NHRₙ refers to an amino group linked to an alkyl group defined as above, wherein additionally one hydrogen atom is substituted with an atom or a group of atoms.

The term "carbamyl," as used herein refers to a group of formula -C(O)NH₂, -NH₂C(O), NHC(O)Rₙ, C(O)NHRₙ, also written NHCORₙ, CONHRₙ.

The term "carbonyl," as used herein refers to a -C(=O)- group, which also may be written as -C(O)-, or -CO-.

The term "oxo"(=O) refers to an oxygen atom as a divalent substituent, forming a carbonyl group when attached to carbon, or attached to a heteroatom forming a sulfoxide or sulfone group, or an N-oxide group. In some embodiments, aryl or heterocyclic groups may be optionally substituted by 1 or 2 oxo (=O) substituents.

The term "aryl," as used herein refers to an aromatic hydrocarbon group, which may be monocyclic or polycyclic (e.g., having 2 fused rings). The term "Cₙ-ₘ aryl" refers to an aryl group having from n to m ring carbon atoms. Aryl groups include, e.g., phenyl, naphthyl, indanyl, indenyl and the like.

The term "heteroaryl" as used herein refers to a monocyclic or polycyclic aromatic heterocycle having at least one heteroatom ring member selected from sulfur, oxygen and nitrogen. For example, the term 5-14 membered heteroaryl refers to an aromatic heterocycle having 5-14 ring atoms including carbon atoms and 1, 2, 3 or 4 heteroatom ring members independently selected from nitrogen, sulfur and oxygen, preferably nitrogen. Non-limiting examples of heteroaryl groups include pyridinyl, pyrimidinyl, pyrazinyl, pyridazinyl, pyrrolyl, pyrazolyl, azolyl, imidazolyl, furanyl, quinolinyl, isoquinolinyl, naphthyridinyl, indolyl, benzofuranyl, and the like.

The term "cycloalkyl," as used herein refers to a non-aromatic hydrocarbon ring system (monocyclic, bicyclic or polycyclic), including cyclized alkyl and alkenyl groups. The term "Cₙ-ₘ cycloalkyl" refers to a cycloalkyl that has n to m ring member carbon atoms. Cycloalkyl groups can include mono- or polycyclic (e.g., having 2, 3 or 4 fused rings) groups. Non limiting examples of cycloalkyl groups include cyclopropyl, cyclobutyl, cyclopentyl, cyclohexyl, cycloheptyl, cyclopentenyl, cyclohexenyl, cyclohexadienyl, cycloheptatrienyl, and the like.

The term "heterocycloalkyl," as used herein refers to a non-aromatic ring or ring system, which may optionally contain one or more alkenylene groups as part of the ring structure, which has at least one heteroatom ring member independently selected from nitrogen, sulfur and oxygen, and which can have for example, 4-14 ring members, 4-10 ring members, 4-7 ring members, or 4-6 ring members. Included within the term "heterocycloalkyl" are monocyclic 4-, 5-, 6- and 7-membered heterocycloalkyl groups. Non limiting examples of monocyclic heterocycloalkyl groups include piperidinyl, piperazinyl, pyrrolidinyl, azetidinyl, tetrahydropyranyl, tetrahydrofuranyl, and the like. Heterocycloalkyl groups can include also bicyclic or polycyclic (e.g., having two or three fused or bridged attached rings) or spirocyclic ring systems.

For example, 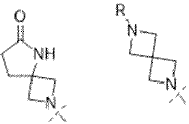 are to be considered as heterocycloalkyl substituted with an oxo group (=O) and with an R group respectively.

The term "cyclic-amide" as used herein refers to cyclic compound with the amide group - C(=O)N- in the ring. Cₙ-Cₘ-cyclic-amide refers to a cyclic-amide that has n to m ring member carbon atoms (e.g., C₃-C₆).

The terms described above, can be used alone or in combination with each other. For example, the terms C₆-₁₀ aryl-C₁-₄ alkyl-, 5-14 membered heteroary-C₁-₄ alkyl-, C₁-₆ alkyl-NHRₐ, or Cₙ-Cₘ-cyclic-amide-C₁-₄ alkyl- refer to an aryl, a 5-14 membered heteroaryl, an amino group or a Cₙ-Cₘ-cyclic-amide combined with an alkyl group as defined above. For example, 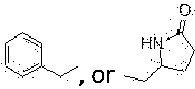 are to be considered within the above definition of C₆-₁₀ aryl-C₁-₄ alkyl-, and Cₙ-Cₘ-cyclic-amide-C₁-₄ alkyl-.

The term "pharmaceutically acceptable" herewith is intended to those compounds and materials, which are generally safe, non-toxic, and neither biologically nor otherwise undesirable and includes that which is acceptable for veterinary use as well as human pharmaceutical use.

The terms "subject" or "patient," or "recipient" used interchangeably, refer to any mammal, preferably mice, rats, other rodents, rabbits, dogs, cats, swine, or primates, and most preferably humans.

The phrase "therapeutically effective amount" refers to the amount of active compound or pharmaceutical agent that elicits the biological or medicinal response in a tissue, system, animal, individual or human.

As used herein, the term "treating" or "treatment" refers to the inhibition of the disease; condition or disorder in an individual who is experiencing or displaying the pathology or symptomatology of the disease, condition or disorder; and/or to ameliorating the disease; e.g., ameliorating a disease, condition or disorder in an individual who is experiencing or displaying the pathology or symptomatology of the disease, condition or disorder (i.e., reversing the pathology and/or symptomatology) such as decreasing the severity of disease.

### Compounds

The present invention relates to a compound of Formula (I) or a pharmaceutically acceptable salt or a stereoisomer thereof: 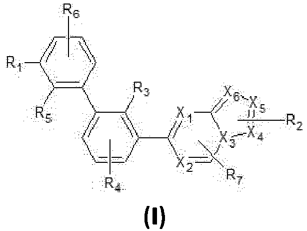 wherein:
X₁-X₆ are each independently C or N, with the proviso that at least two and at most five of X₁-X₆ are N;
R₃ is H, methyl, halogen, or CN;
R₄, and R₅ are each independently H, methyl, halogen, or CN;
R₇ is H, C₁-₆ alkyl, C₁-₆ alkoxy, halogen, or CN;
R₁ is selected from H, C₁-₆ alkyl, C₂-₆ alkynyl, C₁-₆ alkoxy, C₆-₁₀ aryl, 5-14 membered heteroaryl, C₆-₁₀ aryl- C₁-₄ alkyl-, C₃-₁₀ cycloalkyl-C₁-₄ alkyl-, (5-14 membered heteroaryl)-C₁-₄ alkyl-, (4-10 membered heterocycloalkyl)-C₁-₄ alkyl-, C₁-₆ alkyl-NHRₐ, NHORₐ, C(O)Rₐ, NHCORₐ, NHRₐ, CONHRₐ, NHSO₂Rₐ, SO₂NHRₐ, and OCH₂Rₐ, or R₁ and R₆ are linked together to form a 4-10 membered heterocycloalkyl, and wherein the C₁-₆ alkyl, C₂-₆ alkynyl, C₁-₆ alkoxy, C₆-₁₀ aryl, 5-14 membered heteroaryl, C₆-₁₀ aryl- C₁-₄ alkyl-, C₃-₁₀ cycloalkyl-C₁-₄ alkyl-, (5-14 membered heteroaryl)-C₁-₄ alkyl-, (4-10 membered heterocycloalkyl)-C₁-₄ alkyl- of R₁ are each optionally substituted with 1, 2, 3, or 4 independently selected R_{b} substituents;
R₂ is selected from C₁-₆ alkyl, C₂-₆ alkynyl, C₁-₆ alkoxy, C₆-₁₀ aryl, 5-14 membered heteroaryl, C₆-₁₀ aryl-C₁-₄ alkyl-, C₃-₁₀ cycloalkyl-C₁-₄ alkyl-, (5-14 membered heteroaryl)-C₁-₄ alkyl-, (4-10 membered heterocycloalkyl)-C₁-₄ alkyl-, C₃-C₆-cyclic-amide-C₁-₄ alkyl-, C₁-₆ alkyl-NHRₐ, - NHORₐ, C(O)Rₐ, NHCORₐ, CONHRₐ, NHSO₂Rₐ, SO₂NHRₐ, NHRₐ and OCH₂Rₐ, and wherein the C₁-₆ alkyl, C₂-₆ alkynyl, C₁-₆ alkoxy, C₆-₁₀ aryl, 5-14 membered heteroaryl, C₆-₁₀ aryl-C₁-₄ alkyl-, C₃-₁₀ cycloalkyl-C₁-₄ alkyl-, (5-14 membered heteroaryl)-C₁-₄ alkyl-, (4-10 membered heterocycloalkyl)-C₁-₄ alkyl-, C₃-C₆-cyclic-amide-C₁-₄ alkyl- of R₂ are each independently substituted with 1, 2, 3, or 4 independently selected R_{b} substituents;
R₆ is selected from H, C₁-₆ alkyl, C₂-₆ alkynyl, halogen, and OCH₂Rₐ or R₁ and R₆ are linked together to form a 4-10 membered heterocycloalkyl optionally substituted with 1, 2, 3, or 4 independently selected R_{b} substituents;
each Rₐ is independently selected from H, C₁-₆ alkyl, C₁-₆ alkoxy, C₆-₁₀ aryl, C₃-₁₀ cycloalkyl, 5-14 membered heteroaryl, 4-10 membered heterocycloalkyl, C₆-₁₀ aryl-C₁-₄ alkyl, C₃-₁₀ cycloalkyl-C₁-₄ alkyl-, (5-14 membered heteroaryl)-C₁-₄ alkyl-, (4-10 membered heterocycloalkyl)-C₁-₄ alkyl-, C₁-₆ alkyl-NHR_{c}, NHOR_{c}, NHCOR_{c}, NHR_{c}, and OCH₂R_{c}, and wherein the C₁-₆ alkyl, C₁-₆ alkoxy, C₆-₁₀ aryl, C₃-₁₀ cycloalkyl, 5-14 membered heteroaryl, 4-10 membered heterocycloalkyl, C₆-₁₀ aryl-C₁-₄ alkyl, C₃-₁₀ cycloalkyl-C₁-₄ alkyl-, (5-14 membered heteroaryl)-C₁-₄ alkyl-, (4-10 membered heterocycloalkyl)-C₁-₄ alkyl- of Rₐ are each independently substituted with 1, 2, or 3 independently selected R_{d} substituents;
each R_{b} substituent is independently selected from C₁-₆ alkyl, C₁-₆ alkoxy, C₆-₁₀ aryl, C₃-₁₀ cycloalkyl, 5-14 membered heteroaryl, 4-10 membered heterocycloalkyl, C₆-₁₀ aryl-C₁-₄ alkyl, C₃-₁₀ cycloalkyl-C₁-₄ alkyl-, (5-14 membered heteroaryl)-C₁-₄ alkyl-, (4-10 membered heterocycloalkyl)-C₁-₄ alkyl-, C₃-C₆ cyclic amide C₁-₄ alkyl-, OH, (=O), NH₂, NHOR_{c}, NHC(O)R_{c}, OR_{c}, C(O)R_{c}, C(O)NHR_{c}, C(O)NR_{c}R_{c}, C(O)OR_{c}, OC(O)R_{c}, C₁-₆ alkyl-NHR_{c}, NHR_{c}, NR_{c}R_{c}, S(O)₂NR_{c}R_{c}, and wherein the C₁-₆ alkyl, C₁-₆ alkoxy, C₆-₁₀ aryl, C₃-₁₀ cycloalkyl, 5-14 membered heteroaryl, 4-10 membered heterocycloalkyl, C₆-₁₀ aryl-C₁-₄ alkyl, C₃-₁₀ cycloalkyl-C₁-₄ alkyl-, (5-14 membered heteroaryl)-C₁-₄ alkyl-, (4-10 membered heterocycloalkyl)-C₁-₄ alkyl-, C₃-C₆ cyclic amide C₁-₄ alkyl- of R_{b} are each independently substituted with 1, 2, or 3 independently selected R_{d} substituents;
each R_{c} is independently selected from H, C₁-₆ alkyl, C₂-₆ alkenyl, C₁-₆ alkoxy, C₆-₁₀ aryl, C₃-₁₀ cycloalkyl, 5-14 membered heteroaryl, 4-10 membered heterocycloalkyl, C₆-₁₀ aryl-C₁-₄ alkyl, C₃-₁₀ cycloalkyl-C₁-₄ alkyl-, (5-14 membered heteroaryl)-C₁-₄ alkyl-, (4-10 membered heterocycloalkyl)-C₁-₄ alkyl-, C₃-C₆ cyclic amide C₁-₄ alkyl, C(O)R_{d}, C(O)NHR_{d}, and C(O)NR_{d}R_{d}, and wherein the C₁-₆ alkyl, C₂-₆ alkenyl, C₁-₆ alkoxy, C₆-₁₀ aryl, C₃-₁₀ cycloalkyl, 5-14 membered heteroaryl, 4-10 membered heterocycloalkyl, C₆-₁₀ aryl-C₁-₄ alkyl, C₃-₁₀ cycloalkyl-C₁-₄ alkyl-, (5-14 membered heteroaryl)-C₁-₄ alkyl-, (4-10 membered heterocycloalkyl)-C₁-₄ alkyl-, C₃-C₆ cyclic amide C₁-₄ alkyl of R_{c} are each independently substituted with 1, 2, or 3 independently selected R_{d} substituents;
each R_{d} is independently selected from C₁-₆ alkyl, C₁-₆ alkoxy, C₆-₁₀ aryl, C₃-₁₀ cycloalkyl, 5-14 membered heteroaryl, 4-10 membered heterocycloalkyl, C₆-₁₀ aryl-C₁-₄ alkyl-, C₃-₁₀ cycloalkyl-C₁-₄ alkyl-, (5-14 membered heteroaryl)-C₁-₄ alkyl-, (4-10 membered heterocycloalkyl)-C₁-₄ alkyl-, C₃-C₆ cyclic amide, C₃-C₆ cyclic amide C₁-₄ alkyl-, CN, NH₂, NHORₑ, C(O)Rₑ, C(O)NRₑRₑ, C(O)ORₑ, OC(O)Rₑ, OC(O)NRₑRₑ, (=O), NHRₑ, NRₑRₑ, NRₑC(O)Rₑ, NRₑC(O)ORₑ, ORₑ, S(O)₂Rₑ and S(O)₂NRₑRₑ, and wherein the C₁-₆ alkyl, C₁-₆ alkoxy, C₆-₁₀ aryl, C₃-₁₀ cycloalkyl, 5-14 membered heteroaryl, 4-10 membered heterocycloalkyl, C₆-₁₀ aryl-C₁-₄ alkyl, C₃-₁₀ cycloalkyl-C₁-₄ alkyl-, (5-14 membered heteroaryl)-C₁-₄ alkyl-, (4-10 membered heterocycloalkyl)-C₁-₄ alkyl-, C₃-C₆ cyclic amide, C₃-C₆ cyclic amide C₁-₄ alkyl- of R_{d} are each optionally substituted with 1, 2, or 3 independently selected R_{f} substituents;
each Rₑ is independently selected from H, OH, COOH, C(O)R_{f}, C₁-₆ alkyl, C₆-₁₀ aryl, C₃-₁₀ cycloalkyl, 5-14 membered heteroaryl, 4-10 membered heterocycloalkyl, C₃-C₆ cyclic amide C₁-₄ alkyl-, and wherein the C₁-₆ alkyl, C₆-₁₀ aryl, C₃-₁₀ cycloalkyl, 5-14 membered heteroaryl, 4-10 membered heterocycloalkyl, C₃-C₆ cyclic amide C₁-₄ alkyl of Rₑ are each optionally substituted with 1, 2 or 3 independently selected R_{f} substituents;
each R_{f} is independently selected from H, C₁-₄ alkyl, C₆-₁₀ aryl, C₃-₁₀ cycloalkyl, 5-14 membered heteroaryl, 4-10 membered heterocycloalkyl, C₆-₁₀ aryl-C₁-₄ alkyl-, C₃-₁₀ cycloalkyl-C₁-₄ alkyl-, (5-14 membered heteroaryl)-C₁-₄ alkyl-, (4-10 membered heterocycloalkyl)-C₁-₄ alkyl-, C₃-C₆ cyclic amide, halogen, CN, COOH, OH, (=O).

In the compound of Formula (I) described above,
preferably,
X₁-X₆ are each independently C or N, with the proviso that at least two and at most three of X₁-X₆ are N;
R₃ and R₅ are preferably each independently methyl or Cl. More preferably, R₃ and R₅ are both methyl. Alternatively, R₃ and R₅ are both Cl.
R₄ is preferably H.
R₇ is preferably H or C₁-₆ alkoxy, more preferably is H or OMe.
R₁ is preferably selected from from H, C₁-₆ alkyl, C₂-₆ alkynyl, C₁-₆ alkoxy, C₆-₁₀ aryl, 5-14 membered heteroaryl, C₆-₁₀ aryl- C₁-₄ alkyl-, C₃-₁₀ cycloalkyl-C₁-₄ alkyl-, (5-14 membered heteroaryl)-C₁-₄ alkyl-, (4-10 membered heterocycloalkyl)-C₁-₄ alkyl-, C₁-₆ alkyl-NHRₐ, NHCORₐ, NHRₐ, NHORₐ, C(O)Rₐ, CONHRₐ, NHSO₂Rₐ, SO₂NHRₐ, and OCH₂Rₐ, or R₁ and R₆ are linked together to form a 4-10 membered heterocycloalkyl, and wherein C₁-₆ alkyl, C₂-₆ alkynyl, C₁-₆ alkoxy, C₆-₁₀ aryl, 5-14 membered heteroaryl, C₆-₁₀ aryl- C₁-₄ alkyl-, C₃-₁₀ cycloalkyl-C₁-₄ alkyl-, (5-14 membered heteroaryl)-C₁-₄ alkyl-, (4-10 membered heterocycloalkyl)-C₁-₄ alkyl- of R₁ are each optionally substituted with 1, 2, or 3 independently selected R_{b} substituents;
R₂ is preferably selected from C₁-₆ alkyl, C₂-₆ alkynyl, C₁-₆ alkoxy, C₆-₁₀ aryl, 5-14 membered heteroaryl, C₆-₁₀ aryl-C₁-₄ alkyl-, (5-14 membered heteroaryl)-C₁-₄ alkyl-, (4-10 membered heterocycloalkyl)-C₁-₄ alkyl-, C₁-₆ alkyl-NHRₐ, and NHRₐ, and wherein the C₁-₆ alkyl, C₂-₆ alkynyl, C₁-₆ alkoxy, C₆-₁₀ aryl, 5-14 membered heteroaryl, C₆-₁₀ aryl-C₁-₄ alkyl-, (5-14 membered heteroaryl)-C₁-₄ alkyl-, (4-10 membered heterocycloalkyl)-C₁-₄ alkyl-, of R₂ are each independently substituted with 1, 2, or 3 independently selected R_{b} substituents;
R₆ is preferably selected from H, and OCH₂Rₐ or R₁ and R₆ are linked together to form a 4-10 membered heterocycloalkyl optionally substituted with 1, 2, 3, or 4 independently selected R_{b} substituents;
each Rₐ is preferably independently selected from H, C₁-₆ alkyl, C₁-₆ alkoxy, C₆-₁₀ aryl, 5-14 membered heteroaryl, 4-10 membered heterocycloalkyl, C₆-₁₀ aryl-C₁-₄ alkyl, (5-14 membered heteroaryl)-C₁-₄ alkyl-, (4-10 membered heterocycloalkyl)-C₁-₄ alkyl-, NHR_{c}, and OCH₂R_{c}, and wherein C₁-₆ alkyl, C₁-₆ alkoxy, C₆-₁₀ aryl, 5-14 membered heteroaryl, 4-10 membered heterocycloalkyl, C₆-₁₀ aryl-C₁-₄ alkyl, (5-14 membered heteroaryl)-C₁-₄ alkyl-, (4-10 membered heterocycloalkyl)-C₁-₄ alkyl- of Rₐ are each independently substituted with 1, or 2 independently selected R_{d} substituents;
each R_{b} substituent is preferably independently selected from C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, and wherein the C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, of R_{b} are each independently substituted with 1, 2, or 3 independently selected R_{d} substituents;
each R_{c} is preferably independently selected from H, C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl, C(O)R_{d}, C(O)NHR_{d}, and C(O)NR_{d}R_{d}, and wherein the C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl of R_{c} are each independently substituted with 1, 2, or 3 independently selected R_{d} substituents;
each R_{d} is preferably independently selected from C₁-₆ alkyl, C₁-₆ alkoxy, C₆-₁₀ aryl, C₃-₁₀ cycloalkyl, 5-14 membered heteroaryl, 4-10 membered heterocycloalkyl, C₃-C₆ cyclic amide, CN, NH₂, C(O)ORₑ, NHRₑ, NRₑRₑ, NRₑC(O)Rₑ, NRₑC(O)ORₑ, ORₑ, (=O), S(O)₂Rₑ and S(O)₂NRₑRₑ, and wherein the C₁-₆ alkyl, C₁-₆ alkoxy, C₆-₁₀ aryl, C₃-₁₀ cycloalkyl, 5-14 membered heteroaryl, 4-10 membered heterocycloalkyl, C₃-C₆ cyclic amide of R_{d} are each optionally substituted with 1, 2, or 3 independently selected R_{f} substituents;
each Rₑ is preferably independently selected from H, OH, COOH, C(O)R_{f}, C₁-₆ alkyl, and 5-14 membered heteroaryl, wherein the C₁-₆ alkyl, and 5-14 membered heteroaryl of Rₑ are each optionally substituted with 1, 2 or 3 independently selected R_{f} substituents;
each R_{f} is preferably independently selected from H, C₁-₄ alkyl, halogen, CN, COOH, OH, (=O).

In the compound of Formula (I) described above the heteroaryl group 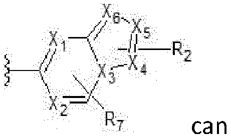 be selected from the group consisting of:

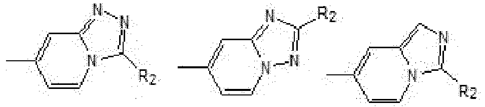

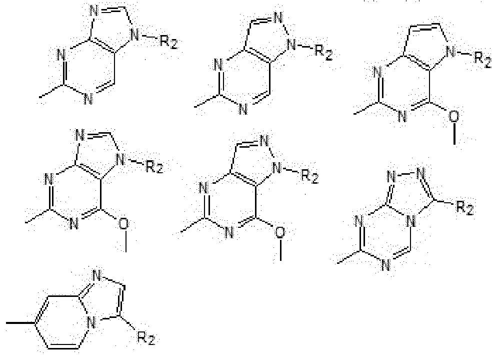

In the compound of Formula (I) described above the heteroaryl group 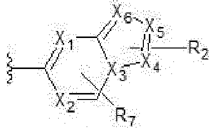 can be selected from the group consisting of:

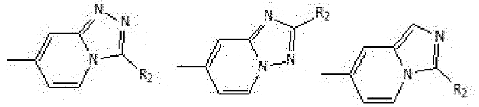

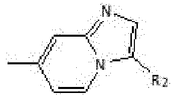

Preferably, the heteroaryl group 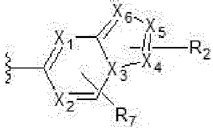 is selected from the group consisting of:

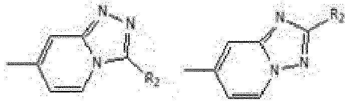

In the compound of Formula (I) described above, R₁ can be selected from the group consisting of: C₁-₃ alkoxy substituted with R_{b}, NHC(O)Rₐ, C₆-₁₀ aryl substituted with R_{b}, 5-14 membered heteroaryl substituted with R_{b}, NHRₐ, CONHRₐ, NHSO₂Rₐ, and SO₂NHRₐ. Rₐ, R_{b} and the other variables other variables of Formula (I) are as defined in any embodiment disclosed herein.

In the compound of Formula (I) described above, R₁ can be selected from the group consisting of: 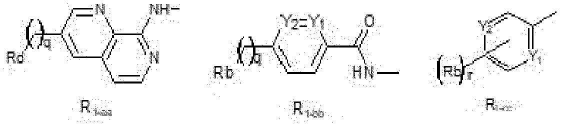 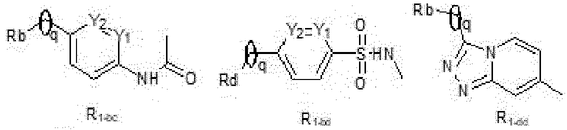 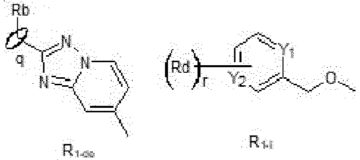 wherein, the subscripts r are each independently an integer of 0, 1, or 2, the subscripts q are each independently an integer of 0, 1, 2 or 3, Y₁ and Y₂ are each independently C, or N and R_{b} and R_{d} are as defined in any embodiment disclosed herein.

Preferably, R₁ is selected from the group consisting of: 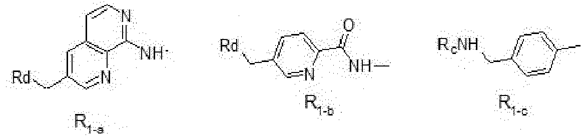 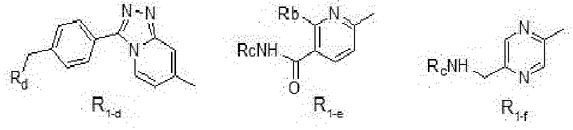 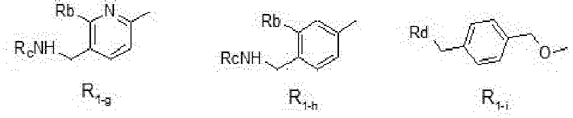 wherein, R_{c} and R_{d} are as defined in any embodiment disclosed herein.

In some embodiments, provided herein, are compounds of Formula (I) above, wherein R₁ is selected from: 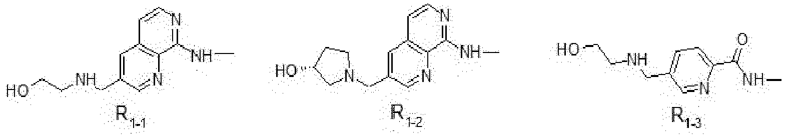 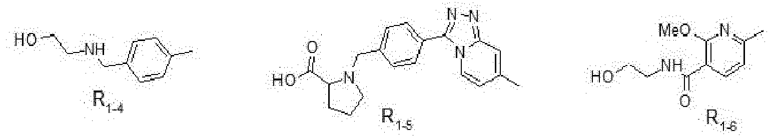 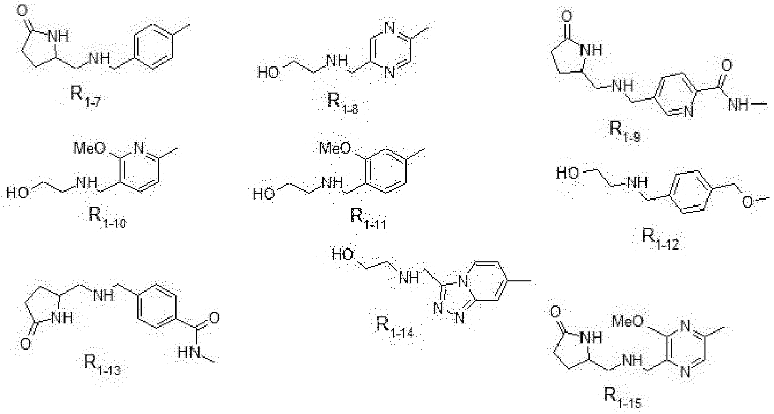 the other variables of Formula (I) are as defined in any embodiment disclosed herein.

In some embodiments, provided herewith is a compound of formula (I) wherein R₃ is methyl, CI, or CN, R₄, and R₇ are H, R₅ is H, methyl, or CI, the other variables of Formula (I) are as defined in any embodiment disclosed herein.

In some embodiments provided herewith is a compound of formula (I) wherein R₃ is methyl, R₄, R₅ and R₇ are H, the other variables of Formula (I) are as defined in any embodiment disclosed herein.

In some embodiments provided herewith is a compound of formula (I) wherein R₃ and R₅ are methyl, R₄ and R₇ are H, the other variables of Formula (I) are as defined in any embodiment disclosed herein.

In some embodiments provided herewith is a compound of formula (I) wherein R₃ and R₅ are Cl, R₄, and R₇ are H, the other variables of Formula (I) are as defined in any embodiment disclosed herein.

In some embodiments, provided herein, is a compound having Formula (II). Compound of Formula (II) of the present invention is a preferred embodiment of the compound of Formula (I) or a pharmaceutically acceptable salt or a stereoisomer thereof described above, and is specifically a compound or a pharmaceutically acceptable salt or a stereoisomer thereof represented by the following formula (II): 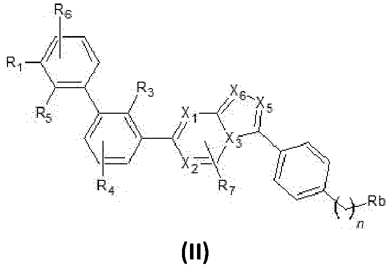 or a pharmaceutically acceptable salt or a stereoisomer thereof, wherein the subscript n is 1, 2, or 3, preferably the subscript n is 1, and wherein X₁-X₃, X₅ and X₆, R₁, R₃ -R₇, and R_{b} have the same meaning as in any embodiment of Formula (I) described herein.

In some embodiments, provided herein, is a compound having Formula (III). Compound of Formula (III) of the present invention is a preferred embodiment of the compound of Formula (I) or a pharmaceutically acceptable salt or a stereoisomer thereof described above, and is specifically a compound or a pharmaceutically acceptable salt or a stereoisomer thereof represented by the following formula (III): 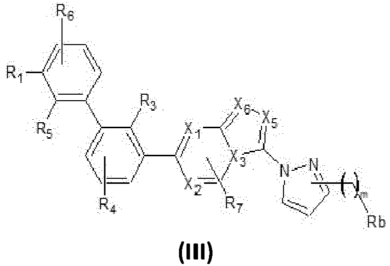 or a pharmaceutically acceptable salt or a stereoisomer thereof, wherein the subscript m is 0, 1, 2, or 3, preferably the subscript m is 0 or 1, and wherein X₁-X₃, X₅ and X₆, R₁, R₃ -R₇, and R_{b} have the same meaning as in any embodiment of Formula (I) described herein.

In some embodiments, provided herein, is a compound having Formula (IV) or (V). Compounds of Formula (IV) or (V) of the present invention is a preferred embodiment of the compound of Formula (I) or a pharmaceutically acceptable salt or a stereoisomer thereof described above, and is specifically a compound or a pharmaceutically acceptable salt or a stereoisomer thereof represented by the following Formula (IV) or (V): 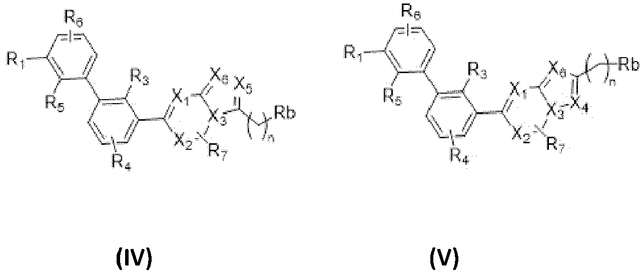 or a pharmaceutically acceptable salt or a stereoisomer thereof, wherein in Formula (IV) the subscript n is 1, 2, or 3, preferably the subscript n is 1 or 2, and wherein X₁-X₃, and X₅ and X₆, R₁, R₃-R₇, and R_{b} have the same meaning as in any embodiment of Formula (I) described herein;
wherein in Formula (V) the subscript n is 1, 2, or 3, preferably the subscript n is 1 or 2, and wherein X₁-X₄, and X₆ R₁, R₃--R₇, and R_{b} have the same meaning as in any embodiment of Formula (I) described herein.

In some embodiments, provided herein, is a compound having Formula (VI) or (VII). Compounds of Formula (VI) or (VII) of the present invention is a preferred embodiment of the compound of Formula (I) or a pharmaceutically acceptable salt or a stereoisomer thereof described above, and is specifically a compound or a pharmaceutically acceptable salt or a stereoisomer thereof represented by the following Formula (VI) or (VII): 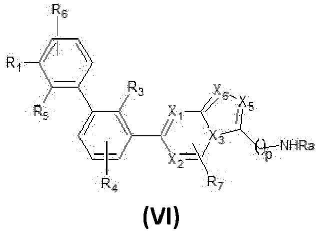 or a pharmaceutically acceptable salt or a stereoisomer thereof, wherein in Formula VI the subscript p is 0, or 1, and wherein X₁-X₃, X₅ and X₆, R₁, R₃, R₄-R₇, and Rₐ have the same meaning as in any embodiment of Formula (I) described herein. 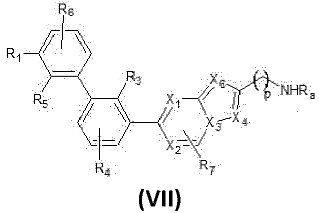 or a pharmaceutically acceptable salt or a stereoisomer thereof, wherein in Formula VII the subscript p is 0, or 1, and wherein X₁-X₄, and X₆, R₁, R₃, R₄-R₇, and Rₐ have the same meaning as in any embodiment of Formula (I) described herein.

In the compounds of formulae (II) to (VII) described above,
Preferably, X₁-X₆ are each independently C or N, with the proviso that at least two and at most three of X₁-X₆ are N;
R₃ and R₅ are preferably each independently methyl or Cl. More preferably, R₃ and R₅ are both methyl. Alternatively, R₃ and R₅ are both Cl.
R₄ is preferably H.
R₇ is preferably H or C₁-₆ alkoxy, more preferably is H or OMe.
R₁ is preferably selected from from H, C₁-₆ alkyl, C₂-₆ alkynyl, C₁-₆ alkoxy, C₆-₁₀ aryl, 5-14 membered heteroaryl, C₆-₁₀ aryl- C₁-₄ alkyl-, C₃-₁₀ cycloalkyl-C₁-₄ alkyl-, (5-14 membered heteroaryl)-C₁-₄ alkyl-, (4-10 membered heterocycloalkyl)-C₁-₄ alkyl-, C₁-₆ alkyl-NHRₐ, NHCORₐ, NHRₐ, NHORₐ, C(O)Rₐ, CONHRₐ, NHSO₂Rₐ, SO₂NHRₐ, and OCH₂Rₐ, or R₁ and R₆ are linked together to form a 4-10 membered heterocycloalkyl, and wherein the C₁-₆ alkyl, C₂-₆ alkynyl, C₁-₆ alkoxy, C₆-₁₀ aryl, 5-14 membered heteroaryl, C₆-₁₀ aryl- C₁-₄ alkyl-, C₃-₁₀ cycloalkyl-C₁-₄ alkyl-, (5-14 membered heteroaryl)-C₁-₄ alkyl-, (4-10 membered heterocycloalkyl)-C₁-₄ alkyl- of R₁ are each optionally substituted with 1, 2, or 3 independently selected R_{b} substituents;
R₂ is preferably selected from C₁-₆ alkyl, C₂-₆ alkynyl, C₁-₆ alkoxy, C₆-₁₀ aryl, 5-14 membered heteroaryl, C₆-₁₀ aryl-C₁-₄ alkyl-, (5-14 membered heteroaryl)-C₁-₄ alkyl-, (4-10 membered heterocycloalkyl)-C₁-₄ alkyl-, C₁-₆ alkyl-NHRₐ, and NHRₐ, and wherein the C₁-₆ alkyl, C₂-₆ alkynyl, C₁-₆ alkoxy, C₆-₁₀ aryl, 5-14 membered heteroaryl, C₆-₁₀ aryl-C₁-₄ alkyl-, (5-14 membered heteroaryl)-C₁-₄ alkyl-, (4-10 membered heterocycloalkyl)-C₁-₄ alkyl-, of R₂ are each independently substituted with 1, 2, or 3 independently selected R_{b} substituents;
R₆ is preferably selected from H, OCH₂Rₐ or R₁ and R₆ are linked together to form a 4-10 membered heterocycloalkyl optionally substituted with 1, 2, 3, or 4 independently selected R_{b} substituents;
each Rₐ is preferably independently selected from H, C₁-₆ alkyl, C₁-₆ alkoxy, C₆-₁₀ aryl, 5-14 membered heteroaryl, 4-10 membered heterocycloalkyl, C₆-₁₀ aryl-C₁-₄ alkyl, (5-14 membered heteroaryl)-C₁-₄ alkyl-, (4-10 membered heterocycloalkyl)-C₁-₄ alkyl-, NHR_{c}, and OCH₂R_{c}, and wherein the C₁-₆ alkyl, C₁-₆ alkoxy, C₆-₁₀ aryl, 5-14 membered heteroaryl, 4-10 membered heterocycloalkyl, C₆-₁₀ aryl-C₁-₄ alkyl, (5-14 membered heteroaryl)-C₁-₄ alkyl-, (4-10 membered heterocycloalkyl)-C₁-₄ alkyl- of Rₐ are each independently substituted with 1, or 2 independently selected R_{d} substituents;
each R_{b} substituent is preferably independently selected from C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, and wherein the C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, of R_{b} are each independently substituted with 1, 2, or 3 independently selected R_{d} substituents;
each R_{c} is preferably independently selected from H, C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl, C(O)R_{d}, C(O)NHR_{d}, and C(O)NR_{d}R_{d}, and wherein the C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl of R_{c} are each independently substituted with 1, 2, or 3 independently selected R_{d} substituents;
each R_{d} is preferably independently selected from C₁-₆ alkyl, C₁-₆ alkoxy, C₆-₁₀ aryl, C₃-₁₀ cycloalkyl, 5-14 membered heteroaryl, 4-10 membered heterocycloalkyl, C₃-C₆ cyclic amide, CN, NH₂, C(O)ORₑ, NHRₑ, NRₑRₑ, NRₑC(O)Rₑ, NRₑC(O)ORₑ, ORₑ, (=O), S(O)₂Rₑ and S(O)₂NRₑRₑ, and wherein the C₁-₆ alkyl, C₁-₆ alkoxy, C₆-₁₀ aryl, C₃-₁₀ cycloalkyl, 5-14 membered heteroaryl, 4-10 membered heterocycloalkyl, C₃-C₆ cyclic amide of R_{d} are each optionally substituted with 1, 2, or 3 independently selected R_{f} substituents;
each Rₑ is preferably independently selected from H, OH, COOH, C(O)R_{f}, C₁-₆ alkyl, and 5-14 membered heteroaryl, wherein the C₁-₆ alkyl, and 5-14 membered heteroaryl of Rₑ are each optionally substituted with 1, 2 or 3 independently selected R_{f} substituents;
each R_{f} is independently selected from H, C₁-₄ alkyl, halogen, CN, COOH, OH, (=O).

In the compounds of Formulae (II), (III), (IV) or Formula (VI) described above the heteroaryl group 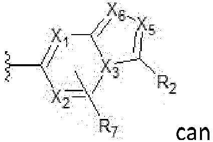 be selected from the group consisting of:

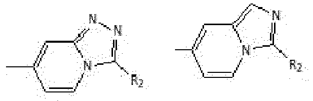

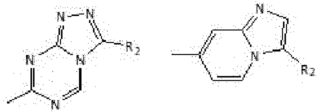

Preferably is:

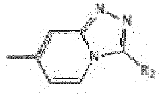

In the compounds of Formula (V) or Formula (VII) described above, the heteroaryl group 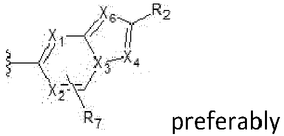 is:

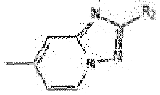

In the compounds of Formulae (II)-(VII) described above, R₁ can be selected from the group consisting of: C₁-₃ alkoxy substituted with R_{b}, C₆-₁₀ aryl substituted with R_{b}, 5-14 membered heteroaryl substituted with R_{b}, NHRₐ, CONHRₐ, NHSO₂Rₐ, and SO₂NHRₐ, Rₐ and R_{b} are as defined in any embodiment defined herewith. The other variables of Formulae (II)-(VII) are as defined in any embodiment disclosed herein.

In the compounds of Formulae (II)-(VII) described above, R₁ can be selected from the group consisting of: 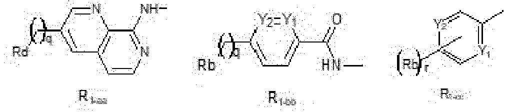 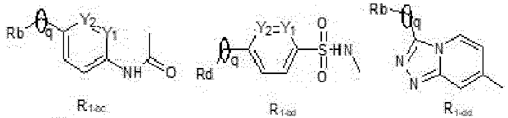 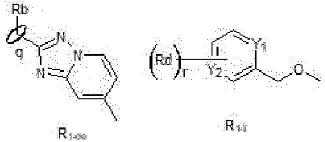 wherein, the subscripts r are each independently an integer of 0, 1, or 2, the subscripts q are each independently an integer of 0, 1, 2 or 3, Y₁ and Y₂ are each independently C or N and R_{b} and R_{d} are as defined in any embodiment disclosed herein. The other variables of Formulae (II)-(VII) are as defined in any embodiment disclosed herein.

In the compounds of Formulae (II)-(VII) described above, preferably, R₁ is selected from the group consisting of: 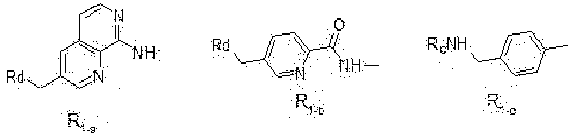 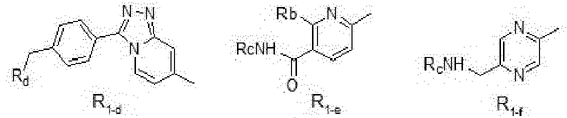 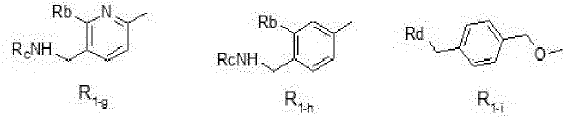 wherein, R_{c} and R_{d} are as defined in any embodiment disclosed herein. The other variables of Formulae (II)-(VII) are as defined in any embodiment disclosed herein.

In some embodiments, provided herein, are compounds of Formulae (II)-(VII) above, wherein R₁ is selected from: 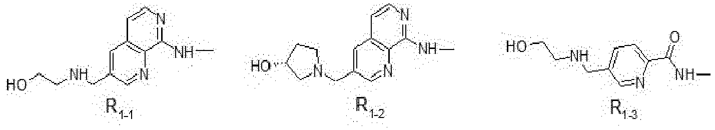 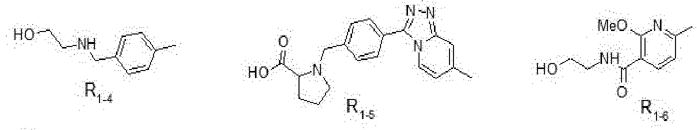 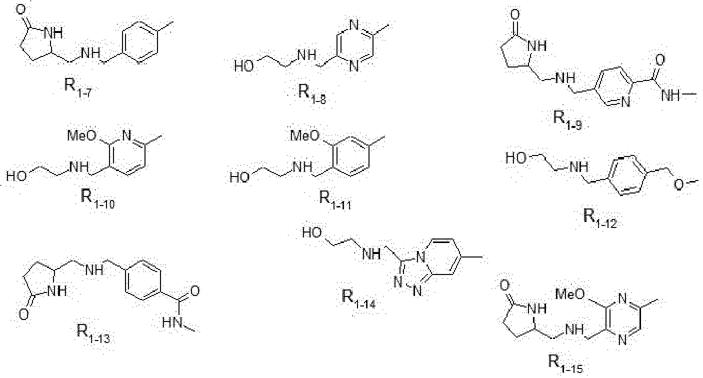 the other variables of Formulae (II)-(VII) are as defined in any embodiment disclosed herein.

In some embodiments, provided herewith is a compound of Formulae (I), (II), (III), (IV), (V), (VI), (VII), wherein R₃ is methyl, CI, or CN, R₄, and R₇ are H, R₅ is H, methyl, or CI, the other variables are as defined in any embodiment of Formula (I) disclosed herein.

In some embodiments provided herewith is a compound of Formulae (I), (II), (III), (IV), (V), (VI) (VII) wherein R₃ is methyl, R₄, R₅ and R₇ are H, the other variables are as defined in any embodiment of Formula (I) disclosed herein.

In some embodiments provided herewith is a compound of Formulae (I), (II), (III), (IV), (V), (VI), (VII) wherein R₃ and R₅ are methyl, R₄ and R₇ are H, the other variables are as defined in any embodiment of Formula (I) disclosed herein.

In some embodiments provided herewith is a compound of Formulae (I), (II), (III), (IV), (V), (VI), (VII) wherein R₃ and R₅ are Cl, R₄, and R₇ are H, the other variables are as defined in any embodiment of Formula (I) disclosed herein.

In some embodiments provided herewith is a compound of Formula (I) wherein R₃ is methyl or Cl, R₁, R₄, R₅, R₆ and R₇ are H, R₂ is C₆-₁₀ aryl-C₁-₄ alkyl- substituted with R_{b}, R_{b} is 4-10 membered heterocycloalkyl substituted with R_{d}, R_{d} is C(O)ORₑ, and Rₑ is H. The other variables are as defined in any embodiment of Formula (I) disclosed herein.

In some embodiments provided herewith is a compound of Formula (I) wherein R₃ and R₅ are methyl or Cl, R₁, R₄, R₆ and R₇ are H, R₂ is C₆-₁₀ aryl-C₁-₄ alkyl- substituted with R_{b}, R_{b} is 4-10 membered heterocycloalkyl substituted with R_{d}, R_{d} is C(O)ORₑ, and Rₑ is H. The other variables are as defined in any embodiment of Formula (I) disclosed herein.

In some embodiments provided herewith is a compound of Formula (II) wherein R₃ and R₅ are methyl or CI, R₁, R₄, R₆ and R₇ are H, R_{b} is 4-10 membered heterocycloalkyl substituted with R_{d}, R_{d} is C(O)ORₑ, and Rₑ is H. The other variables are as defined in any embodiment of Formula (II) disclosed herein.

In some embodiments provided herewith is a compound of Formula (II) wherein R₃ is methyl or CI, R₁, R₄, R₅, R₆ and R₇ are H, R_{b} is 4-10 membered heterocycloalkyl substituted with R_{d}, R_{d} is C(O)ORₑ, and Rₑ is H. The other variables are as defined in any embodiment of Formula (II) disclosed herein.

In some embodiments provided herewith is a compound of Formula (I) wherein R₃ is methyl, R₄, R₅, and R₇ are H, R₁ and R₆ are H, or are linked together to form a 4-10 membered heterocycloalkyl, R₂ is C₆-₁₀ aryl-C₁-₄ alkyl- substituted with R_{b}, R_{b} is NHR_{c}, R_{c} is C₁-₆ alkyl substituted with 1, or 2 R_{ds}, R_{d} is C(O)ORₑ, S(O)₂Rₑ, or ORₑ, and Rₑ is H, or OH. The other variables of Formula (I) are as defined in any embodiment disclosed herein.

In some embodiments provided herewith is a compound of Formula (II) wherein R₃ is methyl, R₄, R₅, and R₇ are H, R₁ and R₆ are H, or are linked together to form a 4-10 membered heterocycloalkyl, R_{b} is NHR_{c}, R_{c} is C₁-₆ alkyl substituted with 1, or 2 R_{ds}, R_{d} is C(O)ORₑ, S(O)₂Rₑ, or ORₑ, and Rₑ is H, or OH. The other variables of Formula (II) are as defined in any embodiment disclosed herein.

In some embodiments provided herewith is a compound of Formula (I) wherein R₃ is methyl or Cl, R₄, R₅, and R₇ are H, R₁ and R₆ are linked together to form a 4-10 membered heterocycloalkyl, R₂ is C₆-₁₀ aryl-C₁-₄ alkyl- substituted with R_{b}, R_{b} is 4-10 membered heterocycloalkyl substituted with 1, or 2 R_{ds}, R_{d} is C(O)ORₑ, or ORₑ, and Rₑ is H. The other variables of Formula (I) are as defined in any embodiment disclosed herein.

In some embodiments provided herewith is a compound of Formula (II) wherein R₃ is methyl or Cl, R₄, R₅, and R₇ are H, R₁ and R₆ are linked together to form a 4-10 membered heterocycloalkyl, R_{b} is 4-10 membered heterocycloalkyl substituted with 1, or 2 R_{ds}, R_{d} is C(O)ORₑ, or ORₑ, and Rₑ is H. The other variables of Formula (II) are as defined in any embodiment disclosed herein.

In some embodiments provided herewith is a compound of Formula (I) wherein R₃ is methyl, R₄, R₅, and R₇ are H, R₁ and R₆ are linked together to form a 4-10 membered heterocycloalkyl, R₂ is C₆-₁₀ aryl-C₁-₄ alkyl- substituted with 3 R_{bs}, R_{b1} is 4-10 membered heterocycloalkyl substituted with 1 or 2 R_{ds}, R_{d} is C(O)ORₑ, or ORₑ, and Rₑ is H, R_{b2} and R_{b3} are C₁-₆ alkoxy. The other variables of Formula (I) are as defined in any embodiment disclosed herein.

In some embodiments provided herewith is a compound of Formula (I) wherein R₃ is CN, R₁, R₄, R₅, R₆ and R₇ are H, R₂ is C₆-₁₀ aryl-C₁-₄ alkyl- substituted with R_{b}, R_{b} is 4-10 membered heterocycloalkyl substituted with R_{d}, R_{d} is C(O)ORₑ, and Rₑ is H. The other variables of Formula (I) are as defined in any embodiment disclosed herein.

In some embodiments provided herewith is a compound of Formula (I) wherein R₃ is methyl, R₁, R₄, R₅, R₆ and R₇ are H, R₂ is C₂-₆ alkynyl- substituted with R_{b}, R_{b} is 4-10 membered heterocycloalkyl substituted with R_{d}, R_{d} is C(O)ORₑ, and Rₑ is H. The other variables of Formula (I) are as defined in any embodiment disclosed herein.

In some embodiments provided herewith is a compound of Formula (I) wherein R₃ is methyl, R₁, R₄, R₅, R₆ and R₇ are H, R₂ is 5-14 membered heteroaryl substituted with R_{b}, R_{b} is C(O)NHR_{c}, R_{c} is C₁-₆ alkyl substituted with R_{d}, R_{d} is C(O)ORₑ, and Rₑ is H. The other variables of Formula (I) are as defined in any embodiment disclosed herein.

In some embodiments provided herewith is a compound of Formula (III) wherein R₃ is methyl, R₁, R₄, R₅, R₆ and R₇ are H, m is 0, R_{b} is C(O)NHR_{c}, R_{c} is C₁-₆ alkyl substituted with R_{d}, R_{d} is C(O)ORₑ, and Rₑ is H. The other variables of Formula (III) are as defined in any embodiment disclosed herein.

In some embodiments provided herewith is a compound of Formula (I) wherein R₃ is methyl, R₄, R₅, and R₇ are H, R₁ and R₆ are linked together to form a 4-10 membered heterocycloalkyl, R₂ is 5-14 membered heteroaryl substituted with R_{b}, R_{b} is C(O)NHR_{c}, R_{c} is C₁-₆ alkyl substituted with R_{d}, R_{d} is C(O)ORₑ, and Rₑ is H. The other variables of Formula (I) are as defined in any embodiment disclosed herein.

In some embodiments provided herewith is a compound of Formula (III) wherein R₃ is methyl, R₄, R₅, and R₇ are H, R₁ and R₆ are linked together to form a 4-10 membered heterocycloalkyl, m is 0, R_{b} is C(O)NHR_{c}, R_{c} is C₁-₆ alkyl substituted with R_{d}, R_{d} is C(O)ORₑ, and Rₑ is H. The other variables of Formula (I) are as defined in any embodiment disclosed herein.

In some embodiments provided herewith is a compound of Formula (I) wherein R₁ is selected from: 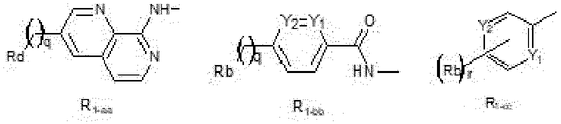 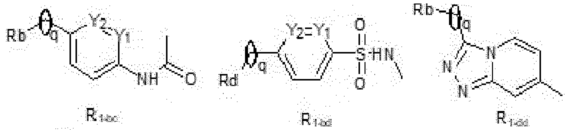 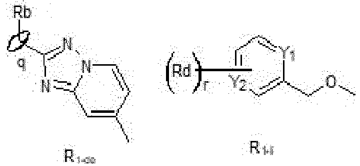 the subscripts r are each independently an integer of 0, 1, or 2, the subscripts q are each independently an integer of 0, 1, 2 or 3, Y₁ and Y₂ are each independently C or N
X₁-X₆ are each independently C or N, with the proviso that at least two and at most five of X₁-X₆ are N;
R₃ is methyl, or Cl;
R₅ is methyl, or Cl
R₄, is H;
R₇ is H, C₁-₆ alkyl, or C₁-₆ alkoxy;
R₂ is selected from C₁-₆ alkyl, C₂-₆ alkynyl, C₁-₆ alkoxy, C₆-₁₀ aryl, 5-14 membered heteroaryl, C₆-₁₀ aryl-C₁-₄ alkyl-, (5-14 membered heteroaryl)-C₁-₄ alkyl-, (4-10 membered heterocycloalkyl)-C₁-₄ alkyl-, C₁-₆ alkyl-NHRₐ, and NHRₐ, and wherein the C₁-₆ alkyl, C₂-₆ alkynyl, C₁-₆ alkoxy, C₆-₁₀ aryl, 5-14 membered heteroaryl, C₆-₁₀ aryl-C₁-₄ alkyl-, (5-14 membered heteroaryl)-C₁-₄ alkyl-, (4-10 membered heterocycloalkyl)-C₁-₄ alkyl-, of R₂ are each independently substituted with 1, 2, or 3 independently selected R_{b} substituents;
R₆ is selected from H, C₁-₆ alkyl, halogen, and OCH₂Rₐ;
each Rₐ substituent is independently selected from H, C₁-₆ alkyl, C₁-₆ alkoxy, C₆-₁₀ aryl, 5-14 membered heteroaryl, 4-10 membered heterocycloalkyl, C₆-₁₀ aryl-C₁-₄ alkyl, (5-14 membered heteroaryl)-C₁-₄ alkyl-, (4-10 membered heterocycloalkyl)-C₁-₄ alkyl-, NHR_{c}, and OCH₂R_{c}, and wherein C₁-₆ alkyl, C₁-₆ alkoxy, C₆-₁₀ aryl, 5-14 membered heteroaryl, 4-10 membered heterocycloalkyl, C₆-₁₀ aryl-C₁-₄ alkyl, (5-14 membered heteroaryl)-C₁-₄ alkyl-, (4-10 membered heterocycloalkyl)-C₁-₄ alkyl- of Rₐ are each independently substituted with 1, or 2 independently selected R_{d} substituents;
each R_{b} substituent is independently selected from C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, and wherein the C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, of R_{b} are each independently substituted with 1, 2, or 3 independently selected R_{d} substituents;
each R_{c} is independently selected from H, C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl, C(O)R_{d}, C(O)NHR_{d}, and C(O)NR_{d}R_{d}, and wherein the C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl of R_{c} are each independently substituted with 1, 2, or 3 independently selected R_{d} substituents;
each R_{d} is independently selected from C₁-₆ alkyl, C₁-₆ alkoxy, C₆-₁₀ aryl, C₃-₁₀ cycloalkyl, 5-14 membered heteroaryl, 4-10 membered heterocycloalkyl, C₃-C₆ cyclic amide, CN, NH₂, C(O)ORₑ, NHRₑ, NRₑRₑ, NRₑC(O)Rₑ, NRₑC(O)ORₑ, ORₑ, (=O), S(O)₂Rₑ and S(O)₂NRₑRₑ, and wherein the C₁-₆ alkyl, C₁-₆ alkoxy, C₆-₁₀aryl, C₃-₁₀ cycloalkyl, 5-14 membered heteroaryl, 4-10 membered heterocycloalkyl, C₃-C₆ cyclic amide of R_{d} are each optionally substituted with 1, 2, or 3 independently selected R_{f} substituents;
each Rₑ is independently selected from H, OH, COOH, C(O)R_{f}, C₁-₆ alkyl, and 5-14 membered heteroaryl wherein the C₁-₆ alkyl, and 5-14 membered heteroaryl of Rₑ are each optionally substituted with 1, 2 or 3 independently selected R_{f} substituents;
each R_{f} is independently selected from H, C₁-₄ alkyl, halogen, CN, COOH, OH, (=O).

In some embodiments provided herewith is a compound of Formula (I) wherein R₁ is selected from: 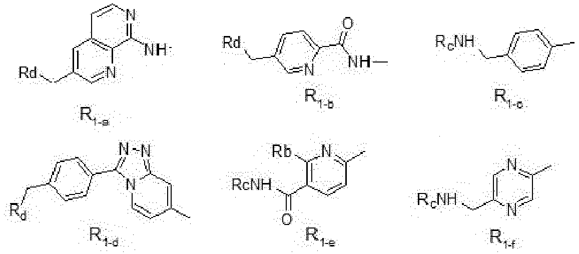 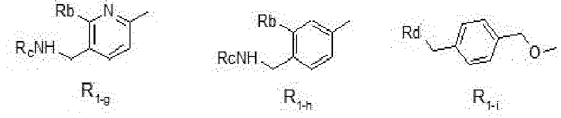
X₁-X₆ are each independently C or N, with the proviso that at least two and at most five of X₁-X₆ are N;
R₃ is methyl, or Cl;
R₅ is methyl, or CI
R₄, is H;
R₇ is H, C₁-₆ alkyl, or C₁-₆ alkoxy;
R₂ is selected from C₁-₆ alkyl, C₂-₆ alkynyl, C₁-₆ alkoxy, C₆-₁₀ aryl, 5-14 membered heteroaryl, C₆-₁₀ aryl-C₁-₄ alkyl-, (5-14 membered heteroaryl)-C₁-₄ alkyl-, (4-10 membered heterocycloalkyl)-C₁-₄ alkyl-, C₁-₆ alkyl-NHRₐ, and NHRₐ, and wherein the C₁-₆ alkyl, C₂-₆ alkynyl, C₁-₆ alkoxy, C₆-₁₀ aryl, 5-14 membered heteroaryl, C₆-₁₀ aryl-C₁-₄ alkyl-, (5-14 membered heteroaryl)-C₁-₄ alkyl-, (4-10 membered heterocycloalkyl)-C₁-₄ alkyl-, of R₂ are each independently substituted with 1, 2, or 3 independently selected R_{b} substituents;
R₆ is selected from H, C₁-₆ alkyl, halogen, and OCH₂Rₐ;
each Rₐ is independently selected from H, C₁-₆ alkyl, C₁-₆ alkoxy, C₆-₁₀ aryl, 5-14 membered heteroaryl, 4-10 membered heterocycloalkyl, C₆-₁₀ aryl-C₁-₄ alkyl, (5-14 membered heteroaryl)-C₁-₄ alkyl-, (4-10 membered heterocycloalkyl)-C₁-₄ alkyl-, NHR_{c}, and OCH₂R_{c}, and wherein C₁-₆ alkyl, C₁-₆ alkoxy, C₆-₁₀ aryl, 5-14 membered heteroaryl, 4-10 membered heterocycloalkyl, C₆-₁₀ aryl-C₁-₄ alkyl, (5-14 membered heteroaryl)-C₁-₄ alkyl-, (4-10 membered heterocycloalkyl)-C₁-₄ alkyl- of Rₐ are each independently substituted with 1, or 2 independently selected R_{d} substituents;
each R_{b} substituent is independently selected from C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, and wherein the C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, of R_{b} are each independently substituted with 1, 2, or 3 independently selected R_{d} substituents;
each R_{c} is independently selected from H, C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl, C(O)R_{d}, C(O)NHR_{d}, and C(O)NR_{d}R_{d}, and wherein the C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl of R_{c} are each independently substituted with 1, 2, or 3 independently selected R_{d} substituents;
each R_{d} is independently selected from C₁-₆ alkyl, C₁-₆ alkoxy, C₆-₁₀ aryl, C₃-₁₀ cycloalkyl, 5-14 membered heteroaryl, 4-10 membered heterocycloalkyl, C₃-C₆ cyclic amide, CN, NH₂, C(O)ORₑ, NHRₑ, NRₑRₑ, NRₑC(O)Rₑ, NRₑC(O)ORₑ, ORₑ, (=O), S(O)₂Rₑ and S(O)₂NRₑRₑ, and wherein the C₁-₆ alkyl, C₁-₆ alkoxy, C₆-₁₀aryl, C₃-₁₀ cycloalkyl, 5-14 membered heteroaryl, 4-10 membered heterocycloalkyl, C₃-C₆ cyclic amide of R_{d} are each optionally substituted with 1, 2, or 3 independently selected R_{f} substituents;
each Rₑ is independently selected from H, OH, COOH, C(O)R_{f}, C₁-₆ alkyl, and 5-14 membered heteroaryl wherein the C₁-₆ alkyl, and 5-14 membered heteroaryl of Rₑ are each optionally substituted with 1, 2 or 3 independently selected R_{f} substituents;
each R_{f} is independently selected from H, C₁-₄ alkyl, halogen, CN, COOH, OH, (=O).

In some embodiments provided herewith is a compound of Formula (I) wherein R₁ is selected from: 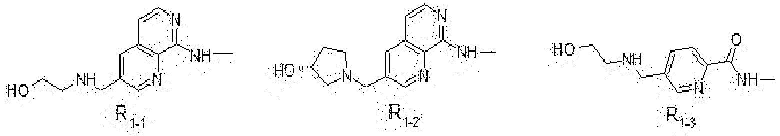 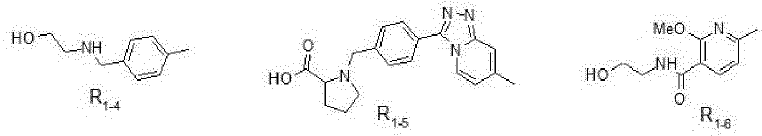 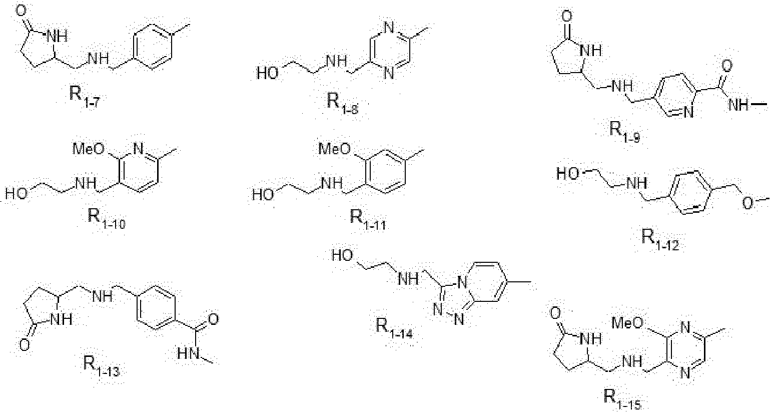
X₁-X₆ are each independently C or N, with the proviso that at least two and at most five of X₁-X₆ are N;
R₃ is methyl, or Cl;
R₅ is methyl, or Cl
R₄, is H;
R₇ is H, C₁-₆ alkyl, or C₁-₆ alkoxy;
R₂ is selected from C₁-₆ alkyl, C₂-₆ alkynyl, C₁-₆ alkoxy, C₆-₁₀ aryl, 5-14 membered heteroaryl, C₆-₁₀ aryl-C₁-₄ alkyl-, (5-14 membered heteroaryl)-C₁-₄ alkyl-, (4-10 membered heterocycloalkyl)-C₁-₄ alkyl-, C₁-₆ alkyl-NHRₐ, and NHRₐ and wherein the C₁-₆ alkyl, C₂-₆ alkynyl, C₁-₆ alkoxy, C₆-₁₀ aryl, 5-14 membered heteroaryl, C₆-₁₀ aryl-C₁-₄ alkyl-, (5-14 membered heteroaryl)-C₁-₄ alkyl-, (4-10 membered heterocycloalkyl)-C₁-₄ alkyl-, of R₂ are each independently substituted with 1, 2, or 3 independently selected R_{b} substituents;
R₆ is selected from H, C₁-₆ alkyl, halogen, and OCH₂Rₐ;
each Rₐ is independently selected from H, C₁-₆ alkyl, C₁-₆ alkoxy, C₆-₁₀ aryl, 5-14 membered heteroaryl, 4-10 membered heterocycloalkyl, C₆-₁₀ aryl-C₁-₄ alkyl, (5-14 membered heteroaryl)-C₁-₄ alkyl-, (4-10 membered heterocycloalkyl)-C₁-₄ alkyl-, NHR_{c}, and OCH₂R_{c}, and wherein C₁-₆ alkyl, C₁-₆ alkoxy, C₆-₁₀ aryl, 5-14 membered heteroaryl, 4-10 membered heterocycloalkyl, C₆-₁₀ aryl-C₁-₄ alkyl, (5-14 membered heteroaryl)-C₁-₄ alkyl-, (4-10 membered heterocycloalkyl)-C₁-₄ alkyl- of Rₐ are each independently substituted with 1, or 2 independently selected R_{d} substituents;
each R_{b} substituent is independently selected from C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, and wherein the C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, of R_{b} are each independently substituted with 1, 2, or 3 independently selected R_{d} substituents;
each R_{c} is independently selected from H, C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl, C(O)R_{d}, C(O)NHR_{d}, and C(O)NR_{d}R_{d}, and wherein the C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl of R_{c} are each independently substituted with 1, 2, or 3 independently selected R_{d} substituents;
each R_{d} is independently selected from C₁-₆ alkyl, C₁-₆ alkoxy, C₆-₁₀ aryl, C₃-₁₀ cycloalkyl, 5-14 membered heteroaryl, 4-10 membered heterocycloalkyl, C₃-C₆ cyclic amide, CN, NH₂, C(O)ORₑ, NHRₑ, NRₑRₑ, NRₑC(O)Rₑ, NRₑC(O)ORₑ, ORₑ, (=O), S(O)₂Rₑ and S(O)₂NRₑRₑ, and wherein the C₁-₆ alkyl, C₁-₆ alkoxy, C₆-₁₀aryl, C₃-₁₀ cycloalkyl, 5-14 membered heteroaryl, 4-10 membered heterocycloalkyl, C₃-C₆ cyclic amide of R_{d} are each optionally substituted with 1, 2, or 3 independently selected R_{f} substituents;
each Rₑ is independently selected from H, OH, COOH, C(O)R_{f}, C₁-₆ alkyl, and 5-14 membered heteroaryl, and wherein the C₁-₆ alkyl, and 5-14 membered heteroaryl of Rₑ are each optionally substituted with 1, 2 or 3 independently selected R_{f} substituents;
each R_{f} is independently selected from H, C₁-₄ alkyl, halogen, CN, COOH, OH, (=O).
In some embodiments provided herewith is a compound of Formula (I) wherein R₃ and Rsare each independently methyl or CI, R₄, R₆ and R₇ are H, R₂ is C₆-₁₀ aryl-C₁-₄ alkyl- substituted with R_{b}, R_{b} is 4-10 membered heterocycloalkyl substituted with R_{d}, R_{d} is C(O)ORₑ, and Rₑ is H, X₁-X₆ are each independently C or N, with the proviso that at least two and at most five of X₁-X₆ are N, and R₁ is selected from R₁₋ₐₐ, R_{1-bb}, R_{1-cc}, R_{1-dd}, R_{1-bc}, R_{1-bd}, R_{1-dd}, R_{1-de}, and R₁₋ᵢᵢ,
wherein in R₁₋ₐₐ, R_{1-bb}, R_{1-cc}, R_{1-dd}, R_{1-bc}, R_{1-bd}, R_{1-dd}, R_{1-de}, and R₁₋ᵢᵢ, the subscripts r are each independently an integer of 0, 1, or 2, the subscripts q are each independently an integer of 0, 1, 2 or 3, Y₁ and Y₂ are each independently C or N;
each R_{b} substituent is independently selected from C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, and wherein the C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, of R_{b} are each independently substituted with 1, 2, or 3 independently selected R_{d} substituents;
each R_{c} is independently selected from H, C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl, C(O)R_{d}, C(O)NHR_{d}, and C(O)NR_{d}R_{d}, and wherein the C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl of R_{c} are each independently substituted with 1, 2, or 3 independently selected R_{d} substituents;
each R_{d} is independently selected from C₁-₆ alkyl, C₁-₆ alkoxy, C₆-₁₀ aryl, C₃-₁₀ cycloalkyl, 5-14 membered heteroaryl, 4-10 membered heterocycloalkyl, C₃-C₆ cyclic amide, CN, NH₂, C(O)ORₑ, NHRₑ, NRₑRₑ, NRₑC(O)Rₑ, NRₑC(O)ORₑ, ORₑ, (=O), S(O)₂Rₑ and S(O)₂NRₑRₑ, and wherein the C₁-₆ alkyl, C₁-₆ alkoxy, C₆-₁₀aryl, C₃-₁₀ cycloalkyl, 5-14 membered heteroaryl, 4-10 membered heterocycloalkyl, C₃-C₆ cyclic amide of R_{d} are each optionally substituted with 1, 2, or 3 independently selected R_{f} substituents;
each Rₑ is independently selected from H, OH, COOH, C(O)R_{f}, C₁-₆ alkyl, and 5-14 membered heteroaryl wherein the C₁-₆ alkyl, and 5-14 membered heteroaryl of Rₑ are each optionally substituted with 1, 2 or 3 independently selected R_{f} substituents;
each R_{f} is independently selected from H, C₁-₄ alkyl, halogen, CN, COOH, OH, (=O). The other variables of Formula (I) are as defined in any embodiment disclosed herein.

In some embodiments provided herewith is a compound of Formula (I) wherein R₃ and Rsare each independently methyl or CI, R₄, R₆ and R₇ are H, R₂ is C₆-₁₀ aryl-C₁-₄ alkyl- substituted with R_{b}, R_{b} is 4-10 membered heterocycloalkyl substituted with R_{d}, R_{d} is C(O)ORₑ, and Rₑ is H, X₁-X₆ are each independently C or N, with the proviso that at least two and at most five of X₁-X₆ are N, and R₁ is selected from R₁₋ₐ, R_{1-b}, R_{1-c}, R_{1-d}, R₁₋ₑ, R_{1-f}, R_{1-g}, R₁₋ₕ, and R₁₋ᵢ,
wherein in R₁₋ₐ, R_{1-b}, R_{1-c}, R_{1-d}, R₁₋ₑ, R_{1-f}, R_{1-g}, R₁₋ₕ, and R₁₋ᵢ each R_{c} is independently selected from H, C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl, C(O)R_{d}, C(O)NHR_{d}, and C(O)NR_{d}R_{d}, wherein the C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl of R_{c} are each independently substituted with 1, 2, or 3 independently selected R_{d} substituents;
each R_{d} is independently selected from C₁-₆ alkyl, C₁-₆ alkoxy, C₆-₁₀ aryl, C₃-₁₀ cycloalkyl, 5-14 membered heteroaryl, 4-10 membered heterocycloalkyl, C₃-C₆ cyclic amide, CN, NH₂, C(O)ORₑ, NHRₑ, NRₑRₑ, NRₑC(O)Rₑ, NRₑC(O)ORₑ, ORₑ, (=O), S(O)₂Rₑ and S(O)₂NRₑRₑ, and wherein the C₁-₆ alkyl, C₁-₆ alkoxy, C₆-₁₀aryl, C₃-₁₀ cycloalkyl, 5-14 membered heteroaryl, 4-10 membered heterocycloalkyl, C₃-C₆ cyclic amide of R_{d} are each optionally substituted with 1, 2, or 3 independently selected R_{f} substituents;
each Rₑ is independently selected from H, OH, COOH, C(O)R_{f}, C₁-₆ alkyl, and 5-14 membered heteroaryl, and wherein the C₁-₆ alkyl, and 5-14 membered heteroaryl of Rₑ are each optionally substituted with 1, 2 or 3 independently selected R_{f} substituents;
each R_{f} is independently selected from H, C₁-₄ alkyl, halogen, CN, COOH, OH, (=O). The other variables of Formula (I) are as defined in any embodiment disclosed herein.

In some embodiments provided herewith is a compound of Formula (I) wherein R₃ and Rsare each independently methyl or CI, R₄, R₆ and R₇ are H, R₂ is C₆-₁₀ aryl-C₁-₄ alkyl- substituted with R_{b}, R_{b} is 4-10 membered heterocycloalkyl substituted with R_{d}, R_{d} is C(O)ORₑ, and Rₑ is H, and R₁ is selected from R₁-₁, R₁-₂, R₁-₃, R₁-₄, R₁-₅, R₁-₆, R₁-₇, R₁-₈, R₁-₉, R₁-₁₀, R₁-₁₁, R₁-₁₂, R₁-₁₃, and R₁-₁₄. The other variables of Formula (I) are as defined in any embodiment disclosed herein.

In some embodiments provided herewith is a compound of Formula (I) wherein R₃ and Rsare each independently methyl or CI, R₄, R₆ and R₇ are H, R₂ is C₆-₁₀ aryl-C₁-₄ alkyl- substituted with R_{b}, R_{b} is NHR_{c}, R_{c} is C₁-₆ alkyl substituted with R_{d}, R_{d} is ORₑ or C₃-C₆ cyclic amide with or without a C₁-₄ alkyl, Rₑ is H or C₁-₆ alkyl, X₁-X₆ are each independently C or N, with the proviso that at least two and at most five of X₁-X₆ are N and R₁ is selected from R₁₋ₐₐ, R_{1-bb}, R_{1-cc}, R_{1-dd}, R_{1-bc}, R_{1-bd}, R_{1-dd}, R_{1-de}, and R₁₋ᵢᵢ,
wherein in R₁₋ₐₐ, R_{1-bb}, R_{1-cc}, R_{1-dd}, R_{1-bc}, R_{1-bd}, R_{1-dd}, R_{1-de}, and R₁₋ᵢᵢ, the subscripts r are each independently an integer of 0, 1, or 2, the subscripts q are each independently an integer of 0, 1, 2 or 3, Y₁ and Y₂ are each independently C or N;
each R_{b} substituent is independently selected from C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, wherein the C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, of R_{b} are each independently substituted with 1, 2, or 3 independently selected R_{d} substituents;
each R_{c} is independently selected from H, C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl, C(O)R_{d}, C(O)NHR_{d}, and C(O)NR_{d}R_{d}, and wherein the C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl of R_{c} are each independently substituted with 1, 2, or 3 independently selected R_{d} substituents;
each R_{d} is independently selected from C₁-₆ alkyl, C₁-₆ alkoxy, C₆-₁₀ aryl, C₃-₁₀ cycloalkyl, 5-14 membered heteroaryl, 4-10 membered heterocycloalkyl, C₃-C₆ cyclic amide, CN, NH₂, C(O)ORₑ, NHRₑ, NRₑRₑ, NRₑC(O)Rₑ, NRₑC(O)ORₑ, ORₑ, (=O), S(O)₂Rₑ and S(O)₂NRₑRₑ, and wherein the C₁-₆ alkyl, C₁-₆ alkoxy, C₆-₁₀aryl, C₃-₁₀ cycloalkyl, 5-14 membered heteroaryl, 4-10 membered heterocycloalkyl, C₃-C₆ cyclic amide of R_{d} are each optionally substituted with 1, 2, or 3 independently selected R_{f} substituents;
each Rₑ is independently selected from H, OH, COOH, C(O)R_{f}, C₁-₆ alkyl, and 5-14 membered heteroaryl, and wherein the C₁-₆ alkyl, and 5-14 membered heteroaryl of Rₑ are each optionally substituted with 1, 2 or 3 independently selected R_{f} substituents;
each R_{f} is independently selected from H, C₁-₄ alkyl, halogen, CN, COOH, OH, (=O). The other variables of Formula (I) are as defined in any embodiment disclosed herein.

In some embodiments provided herewith is a compound of Formula (I) wherein R₃ and Rsare each independently methyl or CI, R₄, R₆ and R₇ are H, R₂ is C₆-₁₀ aryl-C₁-₄ alkyl- substituted with R_{b}, R_{b} is NHR_{c}, R_{c} is C₁-₆ alkyl substituted with R_{d}, R_{d} is ORₑ or C₃-C₆ cyclic amide with or without a C₁-₄ alkyl, Rₑ is H or C₁-₆ alkyl, X₁-X₆ are each independently C or N, with the proviso that at least two and at most five of X₁-X₆ are N and R₁ is selected from R₁₋ₐ, R_{1-b}, R1_{-c}, R_{1-d}, R₁₋ₑ, R_{1-f}, R_{1-g}, R₁₋ₕ, and R₁₋ᵢ,
wherein in R₁₋ₐ, R_{1-b}, R1_{-c}, R₁₋ₐ, R₁₋ₑ, R_{1-f}, R_{1-g}, R₁₋ₕ, and R₁₋ᵢ each R_{c} is independently selected from H, C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl, C(O)R_{d}, C(O)NHR_{d}, and C(O)NR_{d}R_{d}, and wherein the C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl of R_{c} are each independently substituted with 1, 2, or 3 independently selected R_{d} substituents;
each R_{d} is independently selected from C₁-₆ alkyl, C₁-₆ alkoxy, C₆-₁₀ aryl, C₃-₁₀ cycloalkyl, 5-14 membered heteroaryl, 4-10 membered heterocycloalkyl, C₃-C₆ cyclic amide, CN, NH₂, C(O)ORₑ, NHRₑ, NRₑRₑ, NRₑC(O)Rₑ, NRₑC(O)ORₑ, ORₑ, (=O), S(O)₂Rₑ and S(O)₂NRₑRₑ, and wherein the C₁-₆ alkyl, C₁-₆ alkoxy, C₆-₁₀aryl, C₃-₁₀ cycloalkyl, 5-14 membered heteroaryl, 4-10 membered heterocycloalkyl, C₃-C₆ cyclic amide of R_{d} are each optionally substituted with 1, 2, or 3 independently selected R_{f} substituents;
each Rₑ is independently selected from H, OH, COOH, C(O)R_{f}, C₁-₆ alkyl, and 5-14 membered heteroaryl, and wherein the C₁-₆ alkyl, and 5-14 membered heteroaryl of Rₑ are each optionally substituted with 1, 2 or 3 independently selected R_{f} substituents;
each R_{f} is independently selected from H, C₁-₄ alkyl, halogen, CN, COOH, OH, (=O). The other variables of Formula (I) are as defined in any embodiment disclosed herein.

In some embodiments provided herewith is a compound of Formula (I) wherein R₃ and Rsare each independently methyl or CI, R₄, R₆ and R₇ are H, R₂ is C₆-₁₀ aryl-C₁-₄ alkyl- substituted with R_{b}, R_{b} is NHR_{c}, R_{c} is C₁-₆ alkyl substituted with R_{d}, R_{d} is ORₑ or C₃-C₆ cyclic amide with or without a C₁-₄ alkyl, Rₑ is H or C₁-₆ alkyl, and R₁ is selected from R₁-₁, R₁-₂, R₁-₃, R₁₋ₐ, R₁-₅, R-₆, R₁-₇, R₁-₈, R₁-₉, R₁-₁₀, R₁-₁₁, R₁-₁₂, R₁-₁₃, and R₁-₁₄. The other variables of Formula (I) are as defined in any embodiment disclosed herein.

In some embodiments provided herewith is a compound of Formula (I) wherein R₃ and Rsare each independently methyl or CI, R₄, R₆ and R₇ are H, R₂ is C₁-₆ alkyl-NHRₐ, Rₐ is C₁-₆ alkyl substituted with R_{d}, R_{d} is ORₑ or C₃-C₆ cyclic amide, Rₑ is H or C₁-₆ alkyl, X₁-X₆ are each independently C or N, with the proviso that at least two and at most five of X₁-X₆ are N and R₁ is selected from R₁₋ₐₐ, R_{1-bb}, R_{1-cc}, R_{1-dd}, R_{1-bc}, R_{1-bd}, R_{1-dd}, R_{1-de}, and R₁₋ᵢᵢ,
wherein in R₁₋ₐₐ, R_{1-bb}, R_{1-cc}, R_{1-dd}, R_{1-bc}, R_{1-bd}, R_{1-dd}, R_{1-de}, and R₁₋ᵢᵢ, the subscripts r are each independently an integer of 0, 1, or 2, the subscripts q are each independently an integer of 0, 1, 2 or 3, Y₁ and Y₂ are each independently C or N;
each R_{b} substituent is independently selected from C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, and wherein the C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, of R_{b} are each independently substituted with 1, 2, or 3 independently selected R_{d} substituents;
each R_{c} is independently selected from H, C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl, C(O)R_{d}, C(O)NHR_{d}, and C(O)NR_{d}R_{d}, and wherein the C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl of R_{c} are each independently substituted with 1, 2, or 3 independently selected R_{d} substituents;
each R_{d} is independently selected from C₁-₆ alkyl, C₁-₆ alkoxy, C₆-₁₀ aryl, C₃-₁₀ cycloalkyl, 5-14 membered heteroaryl, 4-10 membered heterocycloalkyl, C₃-C₆ cyclic amide, CN, NH₂, C(O)ORₑ, NHRₑ, NRₑRₑ, NRₑC(O)Rₑ, NRₑC(O)ORₑ, ORₑ, (=O), S(O)₂Rₑ and S(O)₂NRₑRₑ, and wherein the C₁-₆ alkyl, C₁-₆ alkoxy, C₆-₁₀aryl, C₃-₁₀ cycloalkyl, 5-14 membered heteroaryl, 4-10 membered heterocycloalkyl, C₃-C₆ cyclic amide of R_{d} are each optionally substituted with 1, 2, or 3 independently selected R_{f} substituents;
each Rₑ is independently selected from H, OH, COOH, C(O)R_{f}, C₁-₆ alkyl, and 5-14 membered heteroaryl, and wherein the C₁-₆ alkyl, and 5-14 membered heteroaryl of Rₑ are each optionally substituted with 1, 2 or 3 independently selected R_{f} substituents;
each R_{f} is independently selected from H, C₁-₄ alkyl, halogen, CN, COOH, OH, (=O). The other variables of Formula (I) are as defined in any embodiment disclosed herein.

In some embodiments provided herewith is a compound of Formula (I) wherein R₃ and R₅ are methyl or Cl, R₄, R₆ and R₇ are H, R₂ is C₁-₆ alkyl-NHRₐ, Rₐ is C₁-₆ alkyl substituted with R_{d}, R_{d} is ORₑ or C₃-C₆ cyclic amide, Rₑ is H or C₁-₆ alkyl, and R₁ is selected from R₁₋ₐ, R_{1-b}, R_{1-c}, R_{1-d}, R₁₋ₑ, R_{1-f}, R_{1-g}, R₁₋ₕ, and R₁₋ᵢ,
wherein in R₁₋ₐ, R_{1-b}, R_{1-c}, R_{1-d}, R₁₋ₑ, R_{1-f}, R_{1-g}, R₁₋ₕ, and R₁₋ₗ each R_{c} is independently selected from H, C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl, C(O)R_{d}, C(O)NHR_{d}, and C(O)NR_{d}R_{d}, and wherein the C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl of R_{c} are each independently substituted with 1, 2, or 3 independently selected R_{d} substituents;
each R_{d} is independently selected from C₁-₆ alkyl, C₁-₆ alkoxy, C₆-₁₀ aryl, C₃-₁₀ cycloalkyl, 5-14 membered heteroaryl, 4-10 membered heterocycloalkyl, C₃-C₆ cyclic amide, CN, NH₂, C(O)ORₑ, NHRₑ, NRₑRₑ, NRₑC(O)Rₑ, NRₑC(O)ORₑ, ORₑ, (=O), S(O)₂Rₑ and S(O)₂NRₑRₑ, and wherein the C₁-₆ alkyl, C₁-₆ alkoxy, C₆-₁₀ aryl, C₃-₁₀ cycloalkyl, 5-14 membered heteroaryl, 4-10 membered heterocycloalkyl, C₃-C₆ cyclic amide of R_{d} are each optionally substituted with 1, 2, or 3 independently selected R_{f} substituents;
each Rₑ is independently selected from H, OH, COOH, C(O)R_{f}, C₁-₆ alkyl, and 5-14 membered heteroaryl, and wherein the C₁-₆ alkyl, and 5-14 membered heteroaryl of Rₑ are each optionally substituted with 1, 2 or 3 independently selected R_{f} substituents;
each R_{f} is independently selected from H, C₁-₄ alkyl, halogen, CN, COOH, OH, (=O). The other variables of Formula (I) are as defined in any embodiment disclosed herein.

In some embodiments provided herewith is a compound of Formula (I) wherein R₃ and R₅ are each independently methyl or CI, R₄, R₆ and R₇ are H, R₂ is C₁-₆ alkyl-NHRₐ, Rₐ is C₁-₆ alkyl substituted with R_{d}, R_{d} is ORₑ or C₃-C₆ cyclic amide, Rₑ is H or C₁-₆ alkyl, and R₁ is selected from R₁-₁, R₁-₂, R₁-₃, R₁-₄, R₁-₅, R-₆, R₁-₇, R₁-₈, R₁-₉, R₁-₁₀, R₁-₁₁, R₁-₁₂, R₁-₁₃, and R₁-₁₄. The other variables of Formula (I) are as defined in any embodiment disclosed herein.

In some embodiments provided herewith is a compound having Formula (II) 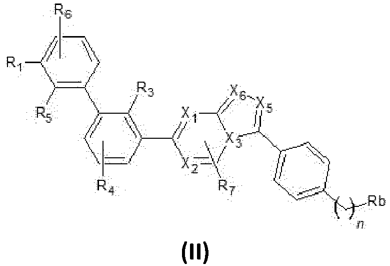
or a pharmaceutically acceptable salt or a stereoisomer thereof, wherein the subscript n is 1, 2, or 3;
X₁-X₃, X₅ and X₆ are each independently C or N, with the proviso that at least two and at most five of X₁-X₃, X₅ and X₆ are N;
R₃ is methyl, or Cl;
R₅ is methyl, or Cl
R₄, is H;
R₇ is H, C₁-₆ alkyl, or C₁-₆ alkoxy;
R₁ is selected from:

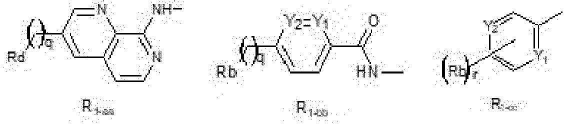

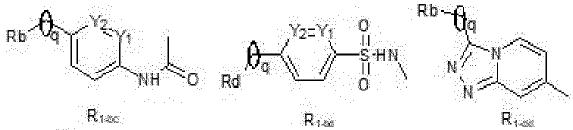

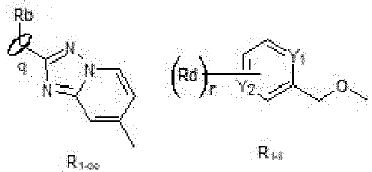

In R₁ the subscripts r are each independently an integer of 0, 1, or 2, the subscripts q are each independently an integer of 0, 1, 2 or 3, Y₁ and Y₂ are each independently C or N
preferably R₁ is selected from R₁₋ₐ, R_{1-b}, R1_{-c}, R_{1-d}, R₁₋ₑ, R_{1-f}, R_{1-g}, R₁₋ₕ, and R₁₋ᵢ, more preferably is selected from R₁-₁, R₁-₂, R₁-₃, R₁-₄, R₁-₅, R-₆, R₁-₇, R₁-₈, R₁-₉, R₁-₁₀, R₁-₁₁, R₁-₁₂, R₁-₁₃, and R₁-₁₄,
R₆ is selected from H, C₁-₆ alkyl, halogen, and OCH₂Rₐ;
each R_{b} substituent is independently selected from C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, and wherein the C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, of R_{b} are each independently substituted with 1, 2, or 3 independently selected R_{d} substituents;
each R_{c} is independently selected from H, C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl, C(O)R_{d}, C(O)NHR_{d}, and C(O)NR_{d}R_{d}, and wherein the C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl of R_{c} are each independently substituted with 1, 2, or 3 independently selected R_{d} substituents;
each R_{d} is independently selected from C₁-₆ alkyl, C₁-₆ alkoxy, C₆-₁₀ aryl, C₃-₁₀ cycloalkyl, 5-14 membered heteroaryl, 4-10 membered heterocycloalkyl, C₃-C₆ cyclic amide, CN, NH₂, C(O)ORₑ, NHRₑ, NRₑRₑ, NRₑC(O)Rₑ, NRₑC(O)ORₑ, ORₑ, (=O), S(O)₂Rₑ and S(O)₂NRₑRₑ, and wherein the C₁-₆ alkyl, C₁-₆ alkoxy, C₆-₁₀ aryl, C₃-₁₀ cycloalkyl, 5-14 membered heteroaryl, 4-10 membered heterocycloalkyl, C₃-C₆ cyclic amide of R_{d} are each optionally substituted with 1, 2, or 3 independently selected R_{f} substituents;
each Rₑ is independently selected from H, OH, COOH, C(O)R_{f}, C₁-₆ alkyl, and 5-14 membered heteroaryl, and wherein the C₁-₆ alkyl, and 5-14 membered heteroaryl of Rₑ are each optionally substituted with 1, 2 or 3 independently selected R_{f} substituents;
each R_{f} is independently selected from H, C₁-₄ alkyl, halogen, CN, COOH, OH, (=O).

In some embodiments provided herewith is a compound having Formula (III) 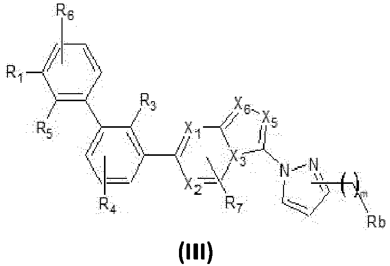
or a pharmaceutically acceptable salt or a stereoisomer thereof, wherein the subscript m is 0, 1, 2, or 3;
X₁-X₃, X₅ and X₆ are each independently C or N, with the proviso that at least two and at most five of X₁-X₃, X₅ and X₆ are N;
R₃ is methyl, or Cl;
R₅ is methyl, or Cl
R₄, is H;
R₇ is H, C₁-₆ alkyl, or C₁-₆ alkoxy;
R₁ is selected from: 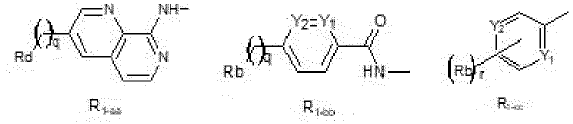 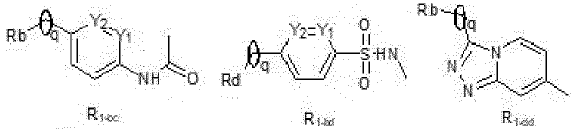 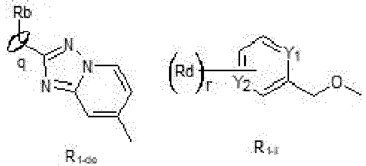
the subscripts r are each independently an integer of 0, 1, or 2, the subscripts q are each independently an integer of 0, 1, 2 or 3, Y₁ and Y₂ are each independently C or N
preferably R₁ is selected from R₁₋ₐ, R_{1-b}, R_{1-c}, R₁₋ₐ, R₁₋ₑ, R_{1-f}, R_{1-g}, R₁₋ₕ, and R₁₋ᵢ, more preferably is selected from R₁-₁, R₁-₂, R₁-₃, R₁-₄, R₁-₅, R-₆, R₁-₇, R₁-₈, R₁-₉, R₁-₁₀, R₁-₁₁, R₁-₁₂, R₁-₁₃, and R₁-₁₄,
R₆ is selected from H, C₁-₆ alkyl, halogen, and OCH₂Rₐ;
each R_{b} substituent is independently selected from C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, and wherein the C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, of R_{b} are each independently substituted with 1, 2, or 3 independently selected R_{d} substituents;
each R_{c} is independently selected from H, C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl, C(O)R_{d}, C(O)NHR_{d}, and C(O)NR_{d}R_{d}, and wherein the C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl of R_{c} are each independently substituted with 1, 2, or 3 independently selected R_{d} substituents;
each R_{d} is independently selected from C₁-₆ alkyl, C₁-₆ alkoxy, C₆-₁₀ aryl, C₃-₁₀ cycloalkyl, 5-14 membered heteroaryl, 4-10 membered heterocycloalkyl, C₃-C₆ cyclic amide, CN, NH₂, C(O)ORₑ, NHRₑ, NRₑRₑ, NRₑC(O)Rₑ, NRₑC(O)ORₑ, ORₑ, (=O), S(O)₂Rₑ and S(O)₂NRₑRₑ, and wherein the C₁-₆ alkyl, C₁-₆ alkoxy, C₆-₁₀aryl, C₃-₁₀ cycloalkyl, 5-14 membered heteroaryl, 4-10 membered heterocycloalkyl, C₃-C₆ cyclic amide of R_{d} are each optionally substituted with 1, 2, or 3 independently selected R_{f} substituents;
each Rₑ is independently selected from H, OH, COOH, C(O)R_{f}, C₁-₆ alkyl, and 5-14 membered heteroaryl, and wherein the C₁-₆ alkyl, and 5-14 membered heteroaryl of Rₑ are each optionally substituted with 1, 2 or 3 independently selected R_{f} substituents;
each R_{f} is independently selected from H, C₁-₄ alkyl, halogen, CN, COOH, OH, (=O).

In some embodiments provided herewith is a compound having Formulae (IV) or (V) 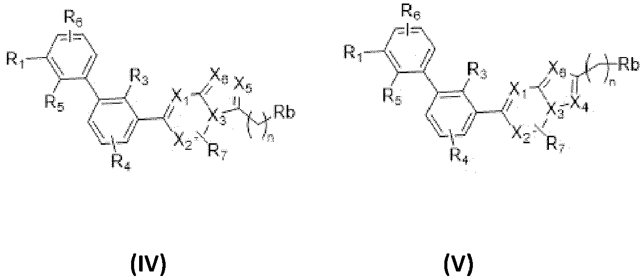 or a pharmaceutically acceptable salt or a stereoisomer thereof, wherein in Formula (IV) the subscript n is 1, 2, or 3;
X₁-X₃, X₅ and X₆ are each independently C or N, with the proviso that at least two and at most five of X₁-X₃, X₅ and X₆ are N;
R₃ is methyl, or Cl;
R₅ is methyl, or Cl
R₄, is H;
R₇ is H, C₁-₆ alkyl, or C₁-₆ alkoxy;
R₁ is selected from: 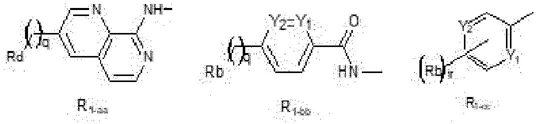 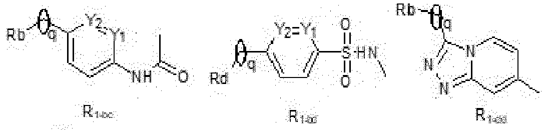 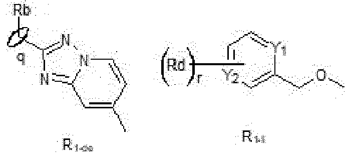
in R₁ the subscripts r are each independently an integer of 0, 1, or 2, the subscripts q are each independently an integer of 0, 1, 2 or 3, Y₁ and Y₂ are each independently C or N
preferably, R₁ is selected from R₁₋ₐ, R_{1-b}, R1_{-c}, R₁₋ₐ, R₁₋ₑ, R_{1-f}, R_{1-g}, R₁₋ₙ, and R₁₋ᵢ, more preferably is selected from R₁-₁, R₁-₂, R₁-₃, R₁-₄, R₁-₅, R-₆, R₁-₇, R₁-₈, R₁-₉, R₁-₁₀, R₁-₁₁, R₁-₁₂, R₁-₁₃,and R₁-₁₄
R₆ is selected from H, C₁-₆ alkyl, halogen, and OCH₂Rₐ;
each R_{b} substituent is independently selected from C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, and wherein the C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, of R_{b} are each independently substituted with 1, 2, or 3 independently selected R_{d} substituents;
each R_{c} is independently selected from H, C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl, C(O)R_{d}, C(O)NHR_{d}, and C(O)NR_{d}R_{d}, and wherein the C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl of R_{c} are each independently substituted with 1, 2, or 3 independently selected R_{d} substituents;
each R_{d} is independently selected from C₁-₆ alkyl, C₁-₆ alkoxy, C₆-₁₀ aryl, C₃-₁₀ cycloalkyl, 5-14 membered heteroaryl, 4-10 membered heterocycloalkyl, C₃-C₆ cyclic amide, CN, NH₂, C(O)ORₑ, NHRₑ, NRₑRₑ, NRₑC(O)Rₑ, NRₑC(O)ORₑ, ORₑ, (=O), S(O)₂Rₑ and S(O)₂NRₑRₑ, and wherein the C₁-₆ alkyl, C₁-₆ alkoxy, C₆-₁₀ aryl, C₃-₁₀ cycloalkyl, 5-14 membered heteroaryl, 4-10 membered heterocycloalkyl, C₃-C₆ cyclic amide of R_{d} are each optionally substituted with 1, 2, or 3 independently selected R_{f} substituents;
each Rₑ is independently selected from H, OH, COOH, C(O)R_{f}, C₁-₆ alkyl, and 5-14 membered heteroaryl, and wherein the C₁-₆ alkyl, and 5-14 membered heteroaryl of Rₑ are each optionally substituted with 1, 2 or 3 independently selected R_{f} substituents;
each R_{f} is independently selected from H, C₁-₄ alkyl, halogen, CN, COOH, OH, (=O).
wherein, in Formula (V) the subscript m is 1, 2, or 3;
X₁-X₄, and X₆ are each independently C or N, with the proviso that at least two and at most five of X₁-X₄, and X₆ are N;
R₃ is methyl, or Cl;
R₅ is methyl, or Cl
R₄, is H;
R₇ is H, C₁-₆ alkyl, or C₁-₆ alkoxy;
R₁ is selected from: 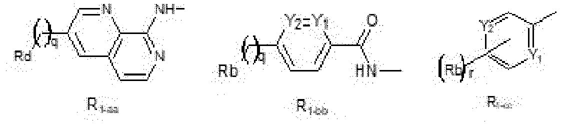 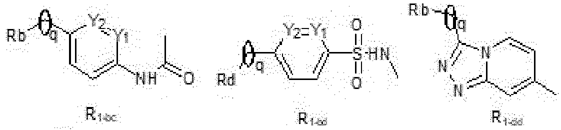 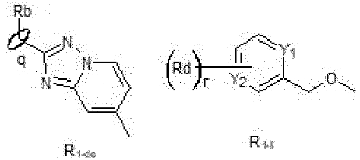
in R₁ the subscripts r are each independently an integer of 0, 1, or 2, the subscripts q are each independently an integer of 0, 1, 2 or 3, Y₁ and Y₂ are each independently C or N,
preferably, R₁ is selected from R₁₋ₐ, R_{1-b}, R1_{-c}, R₁₋ₐ, R₁₋ₑ, R_{1-f}, R_{1-g}, R₁₋ₕ, and R₁₋ᵢ, more preferably is selected from R₁-₁, R₁-₂, R₁-₃, R₁-₄, R₁-₅, R-₆, R₁-₇, R₁-₈, R₁-₉, R₁-₁₀, R₁-₁₁, R₁-₁₂, R₁-₁₃,and R₁-₁₄
R₆ is selected from H, C₁-₆ alkyl, halogen, and OCH₂Rₐ;
each R_{b} substituent is independently selected from C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, and wherein the C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, of R_{b} are each independently substituted with 1, 2, or 3 independently selected R_{d} substituents;
each R_{c} is independently selected from H, C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl, C(O)R_{d}, C(O)NHR_{d}, and C(O)NR_{d}R_{d}, and wherein the C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl of R_{c} are each independently substituted with 1, 2, or 3 independently selected R_{d} substituents;
each R_{d} is independently selected from C₁-₆ alkyl, C₁-₆ alkoxy, C₆-₁₀ aryl, C₃-₁₀ cycloalkyl, 5-14 membered heteroaryl, 4-10 membered heterocycloalkyl, C₃-C₆ cyclic amide, CN, NH₂, C(O)ORₑ, NHRₑ, NRₑRₑ, NRₑC(O)Rₑ, NRₑC(O)ORₑ, ORₑ, (=O), S(O)₂Rₑ and S(O)₂NRₑRₑ, and wherein the C₁-₆ alkyl, C₁-₆ alkoxy, C₆-₁₀ aryl, C₃-₁₀ cycloalkyl, 5-14 membered heteroaryl, 4-10 membered heterocycloalkyl, C₃-C₆ cyclic amide of R_{d} are each optionally substituted with 1, 2, or 3 independently selected R_{f} substituents;
each Rₑ is independently selected from H, OH, COOH, C(O)R_{f}, C₁-₆ alkyl, and 5-14 membered heteroaryl, and wherein the C₁-₆ alkyl, and 5-14 membered heteroaryl of Rₑ are each optionally substituted with 1, 2 or 3 independently selected R_{f} substituents;
each R_{f} is independently selected from H, C₁-₄ alkyl, halogen, CN, COOH, OH, (=O).

In some embodiments provided herewith is a compound having Formula (VI) 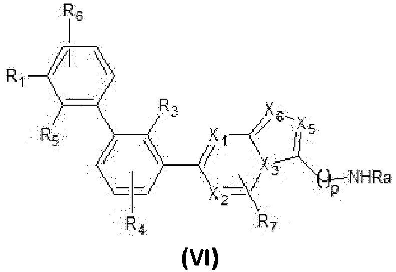
or a pharmaceutically acceptable salt or a stereoisomer thereof, wherein subscript p is 0, or 1;
R₁ is selected from: 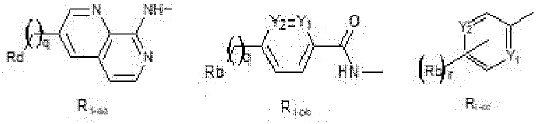 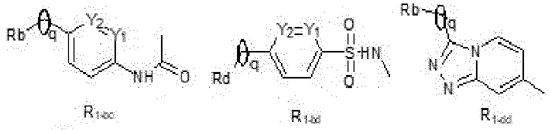 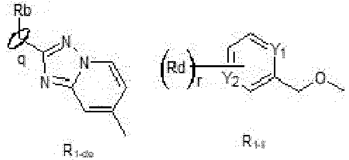
the subscripts r are each independently an integer of 0, 1, or 2, the subscripts q are each independently an integer of 0, 1, 2 or 3, Y₁ and Y₂ are each independently C or N
X₁-X₃, X₅ and X₆ are each independently C or N, with the proviso that at least two and at most five of X₁-X₃, X₅ and X₆ are N;
R₃ is methyl, or Cl;
R₅ is methyl, or Cl
R₄, is H;
R₇ is H, C₁-₆ alkyl, or C₁-₆ alkoxy;
R₆ is selected from H, C₁-₆ alkyl, halogen, and OCH₂Rₐ;
each Rₐ is independently selected from H, C₁-₆ alkyl, C₁-₆ alkoxy, C₆-₁₀ aryl, 5-14 membered heteroaryl, 4-10 membered heterocycloalkyl, C₆-₁₀ aryl-C₁-₄ alkyl, (5-14 membered heteroaryl)-C₁-₄ alkyl-, (4-10 membered heterocycloalkyl)-C₁-₄ alkyl-, NHR_{c}, and OCH₂R_{c}, and wherein C₁-₆ alkyl, C₁-₆ alkoxy, C₆-₁₀ aryl, 5-14 membered heteroaryl, 4-10 membered heterocycloalkyl, C₆-₁₀ aryl-C₁-ₐ alkyl, (5-14 membered heteroaryl)-C₁-₄ alkyl-, (4-10 membered heterocycloalkyl)-C₁-₄ alkyl- of Rₐ are each independently substituted with 1, or 2 independently selected R_{d} substituents;
each R_{b} substituent is independently selected from C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, and wherein the C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, of R_{b} are each independently substituted with 1, 2, or 3 independently selected R_{d} substituents;
each R_{c} is independently selected from H, C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl, C(O)R_{d}, C(O)NHR_{d}, and C(O)NR_{d}R_{d}, and wherein the C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl of R_{c} are each independently substituted with 1, 2, or 3 independently selected R_{d} substituents;
each R_{d} is independently selected from C₁-₆ alkyl, C₁-₆ alkoxy, C₆-₁₀ aryl, C₃-₁₀ cycloalkyl, 5-14 membered heteroaryl, 4-10 membered heterocycloalkyl, C₃-C₆ cyclic amide, CN, NH₂, C(O)ORₑ, NHRₑ, NRₑRₑ, NRₑC(O)Rₑ, NRₑC(O)ORₑ, ORₑ, (=O), S(O)₂Rₑ and S(O)₂NRₑRₑ, and wherein the C₁-₆ alkyl, C₁-₆ alkoxy, C₆-₁₀ aryl, C₃-₁₀ cycloalkyl, 5-14 membered heteroaryl, 4-10 membered heterocycloalkyl, C₃-C₆ cyclic amide of R_{d} are each optionally substituted with 1, 2, or 3 independently selected R_{f} substituents;
each Rₑ is independently selected from H, OH, COOH, C(O)R_{f}, C₁-₆ alkyl, and 5-14 membered heteroaryl, and wherein the C₁-₆ alkyl, and 5-14 membered heteroaryl of Rₑ are each optionally substituted with 1, 2 or 3 independently selected R_{f} substituents;
each R_{f} is independently selected from H, C₁-₄ alkyl, halogen, CN, COOH, OH, (=O).

In some embodiments provided herewith is a compound having Formula (VI) 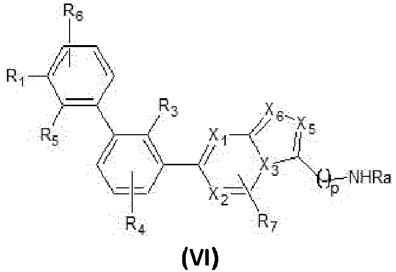
or a pharmaceutically acceptable salt or a stereoisomer thereof, wherein subscript p is 0, or 1;
R₁ is selected from: 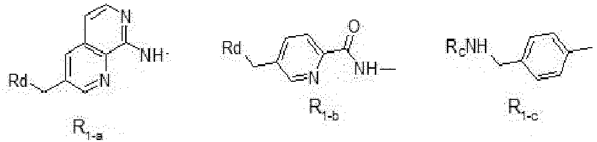 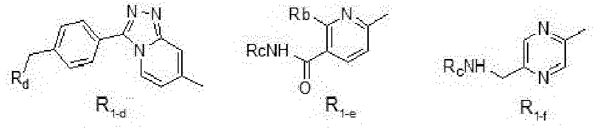 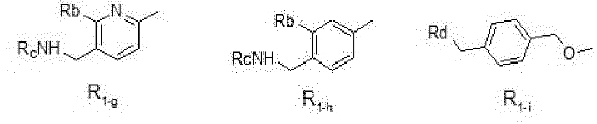
X₁-X₃, X₅ and X₆ are each independently C or N, with the proviso that at least two and at most five of X₁-X₃, X₅ and X₆ are N;
R₃ is methyl, or Cl;
R₅ is methyl, or Cl
R₄, is H;
R₇ is H, C₁-₆ alkyl, or C₁-₆ alkoxy;
R₆ is selected from H, C₁-₆ alkyl, halogen, and OCH₂Rₐ;
each Rₐ is independently selected from H, C₁-₆ alkyl, C₁-₆ alkoxy, C₆-₁₀ aryl, 5-14 membered heteroaryl, 4-10 membered heterocycloalkyl, C₆-₁₀ aryl-C₁-₄ alkyl, (5-14 membered heteroaryl)-C₁-₄ alkyl-, (4-10 membered heterocycloalkyl)-C₁-₄ alkyl-, NHR_{c}, and OCH₂R_{c}, and wherein C₁-₆ alkyl, C₁-₆ alkoxy, C₆-₁₀ aryl, 5-14 membered heteroaryl, 4-10 membered heterocycloalkyl, C₆-₁₀ aryl-C₁-ₐ alkyl, (5-14 membered heteroaryl)-C₁-₄ alkyl-, (4-10 membered heterocycloalkyl)-C₁-₄ alkyl- of Rₐ are each independently substituted with 1, or 2 independently selected R_{d} substituents;
each R_{b} substituent is independently selected from C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, and wherein the C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, of R_{b} are each independently substituted with 1, 2, or 3 independently selected R_{d} substituents;
each R_{c} is independently selected from H, C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl, C(O)R_{d}, C(O)NHR_{d}, and C(O)NR_{d}R_{d}, and wherein the C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl of R_{c} are each independently substituted with 1, 2, or 3 independently selected R_{d} substituents;
each R_{d} is independently selected from C₁-₆ alkyl, C₁-₆ alkoxy, C₆-₁₀ aryl, C₃-₁₀ cycloalkyl, 5-14 membered heteroaryl, 4-10 membered heterocycloalkyl, C₃-C₆ cyclic amide, CN, NH₂, C(O)ORₑ, NHRₑ, NRₑRₑ, NRₑC(O)Rₑ, NRₑC(O)ORₑ, ORₑ, (=O), S(O)₂Rₑ and S(O)₂NRₑRₑ, and wherein the C₁-₆ alkyl, C₁-₆ alkoxy, C₆-₁₀ aryl, C₃-₁₀ cycloalkyl, 5-14 membered heteroaryl, 4-10 membered heterocycloalkyl, C₃-C₆ cyclic amide of R_{d} are each optionally substituted with 1, 2, or 3 independently selected R_{f} substituents;
each Rₑ is independently selected from H, OH, COOH, C(O)R_{f}, C₁-₆ alkyl, and 5-14 membered heteroaryl wherein the C₁-₆ alkyl, and 5-14 membered heteroaryl of Rₑ are each optionally substituted with 1, 2 or 3 independently selected R_{f} substituents;
each R_{f} is independently selected from H, C₁-₄ alkyl, halogen, CN, COOH, OH, (=O).

In some embodiments provided herewith is a compound having Formula (VI) 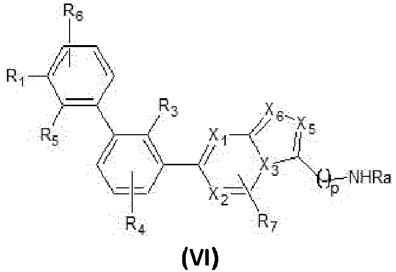
or a pharmaceutically acceptable salt or a stereoisomer thereof, wherein subscript p is 0, or 1;
R₁ is selected from: 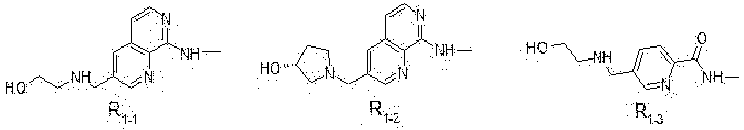 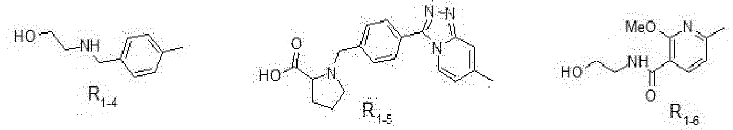 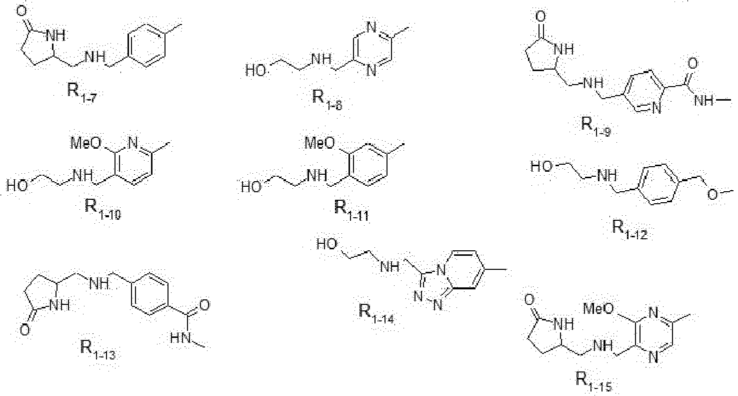
X₁-X₃, X₅ and X₆ are each independently C or N, with the proviso that at least two and at most five of X₁-X₃, X₅ and X₆ are N;
R₃ is methyl, or Cl;
R₅ is methyl, or Cl
R₄, is H;
R₇ is H, C₁-₆ alkyl, or C₁-₆ alkoxy;
R₆ is selected from H, C₁-₆ alkyl, halogen, and OCH₂Rₐ;
each Rₐ is independently selected from H, C₁-₆ alkyl, C₁-₆ alkoxy, C₆-₁₀ aryl, 5-14 membered heteroaryl, 4-10 membered heterocycloalkyl, C₆-₁₀ aryl-C₁-₄ alkyl, (5-14 membered heteroaryl)-C₁-₄ alkyl-, (4-10 membered heterocycloalkyl)-C₁-₄ alkyl-, NHR_{c}, and OCH₂R_{c}, and wherein C₁-₆ alkyl, C₁-₆ alkoxy, C₆-₁₀ aryl, 5-14 membered heteroaryl, 4-10 membered heterocycloalkyl, C₆-₁₀ aryl-C₁-ₐ alkyl, (5-14 membered heteroaryl)-C₁-₄ alkyl-, (4-10 membered heterocycloalkyl)-C₁-₄ alkyl- of Rₐ are each independently substituted with 1, or 2 independently selected R_{d} substituents;
each R_{b} substituent is independently selected from C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, wherein the C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, ORₑ, C(O)R_{c}, C(O)NHR_{c}, C(O)OR_{c}, C₁-₆ alkyl-NHR_{c}, NHR_{c}, of R_{b} are each independently substituted with 1, 2, or 3 independently selected R_{d} substituents;
each R_{c} is independently selected from H, C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl, C(O)R_{d}, C(O)NHR_{d}, and C(O)NR_{d}R_{d}, and wherein the C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl of R_{c} are each independently substituted with 1, 2, or 3 independently selected R_{d} substituents;
each R_{d} is independently selected from C₁-₆ alkyl, C₁-₆ alkoxy, C₆-₁₀ aryl, C₃-₁₀ cycloalkyl, 5-14 membered heteroaryl, 4-10 membered heterocycloalkyl, C₃-C₆ cyclic amide, CN, NH₂, C(O)ORₑ, NHRₑ, NRₑRₑ, NRₑC(O)Rₑ, NRₑC(O)ORₑ, ORₑ, (=O), S(O)₂Rₑ and S(O)₂NRₑRₑ, and wherein the C₁-₆ alkyl, C₁-₆ alkoxy, C₆-₁₀ aryl, C₃-₁₀ cycloalkyl, 5-14 membered heteroaryl, 4-10 membered heterocycloalkyl, C₃-C₆ cyclic amide of R_{d} are each optionally substituted with 1, 2, or 3 independently selected R_{f} substituents;
each Rₑ is independently selected from H, OH, COOH, C(O)R_{f}, C₁-₆ alkyl, and 5-14 membered heteroaryl, and wherein the C₁-₆ alkyl, and 5-14 membered heteroaryl of Rₑ are each optionally substituted with 1, 2 or 3 independently selected R_{f} substituents;
each R_{f} is independently selected from H, C₁-₄ alkyl, halogen, CN, COOH, OH, (=O).

In some embodiments provided herewith is a compound having Formula (VII) 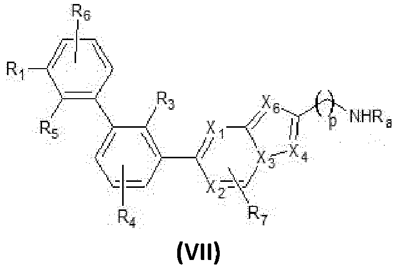
or a pharmaceutically acceptable salt or a stereoisomer thereof, wherein subscript p is 0, or 1;
R₁ is selected from: 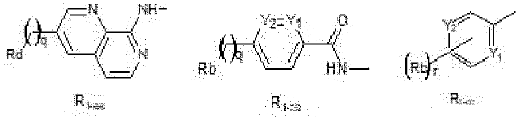 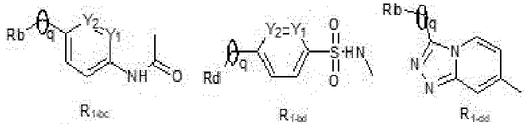 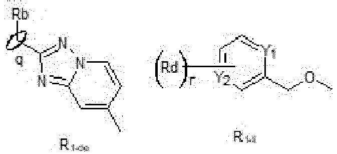
the subscripts r are each independently an integer of 0, 1, or 2, the subscripts q are each independently an integer of 0, 1, 2 or 3, Y₁ and Y₂ are each independently C or N
X₁-X₄, and X₆ are each independently C or N, with the proviso that at least two and at most five of X₁-X₄, and X₆ are N;
R₃ is methyl, or Cl;
R₅ is methyl, or Cl
R₄, is H;
R₇ is H, C₁-₆ alkyl, or C₁-₆ alkoxy;
R₆ is selected from H, C₁-₆ alkyl, halogen, and OCH₂Rₐ;
each Rₐ is independently selected from H, C₁-₆ alkyl, C₁-₆ alkoxy, C₆-₁₀ aryl, 5-14 membered heteroaryl, 4-10 membered heterocycloalkyl, C₆-₁₀ aryl-C₁-₄ alkyl, (5-14 membered heteroaryl)-C₁-₄ alkyl-, (4-10 membered heterocycloalkyl)-C₁-₄ alkyl-, NHR_{c}, and OCH₂R_{c}, and wherein C₁-₆ alkyl, C₁-₆ alkoxy, C₆-₁₀ aryl, 5-14 membered heteroaryl, 4-10 membered heterocycloalkyl, C₆-₁₀ aryl-C₁-₄ alkyl, (5-14 membered heteroaryl)-C₁-₄ alkyl-, (4-10 membered heterocycloalkyl)-C₁-₄ alkyl- of Rₐ are each independently substituted with 1, or 2 independently selected R_{d} substituents;
each R_{b} substituent is independently selected from C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, and wherein the C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, of R_{b} are each independently substituted with 1, 2, or 3 independently selected R_{d} substituents;
each R_{c} is independently selected from H, C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl, C(O)R_{d}, C(O)NHR_{d}, and C(O)NR_{d}R_{d}, and wherein the C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl of R_{c} are each independently substituted with 1, 2, or 3 independently selected R_{d} substituents;
each R_{d} is independently selected from C₁-₆ alkyl, C₁-₆ alkoxy, C₆-₁₀ aryl, C₃-₁₀ cycloalkyl, 5-14 membered heteroaryl, 4-10 membered heterocycloalkyl, C₃-C₆ cyclic amide, CN, NH₂, C(O)ORₑ, NHRₑ, NRₑRₑ, NRₑC(O)Rₑ, NRₑC(O)ORₑ, ORₑ, (=O), S(O)₂Rₑ and S(O)₂NRₑRₑ, and wherein the C₁-₆ alkyl, C₁-₆ alkoxy, C₆-₁₀ aryl, C₃-₁₀ cycloalkyl, 5-14 membered heteroaryl, 4-10 membered heterocycloalkyl, C₃-C₆ cyclic amide of R_{d} are each optionally substituted with 1, 2, or 3 independently selected R_{f} substituents;
each Rₑ is independently selected from H, OH, COOH, C(O)R_{f}, C₁-₆ alkyl, and 5-14 membered heteroaryl, and wherein the C₁-₆ alkyl, and 5-14 membered heteroaryl of Rₑ are each optionally substituted with 1, 2 or 3 independently selected R_{f} substituents;
each R_{f} is independently selected from H, C₁-₄ alkyl, halogen, CN, COOH, OH, (=O).

In some embodiments provided herewith is a compound having Formula (VII) 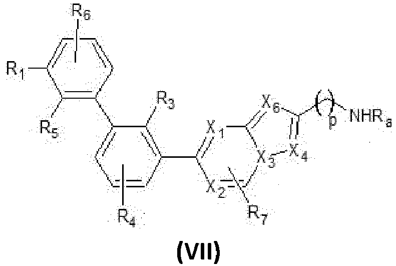
or a pharmaceutically acceptable salt or a stereoisomer thereof, wherein subscript p is 0, or 1;
R₁ is selected from: 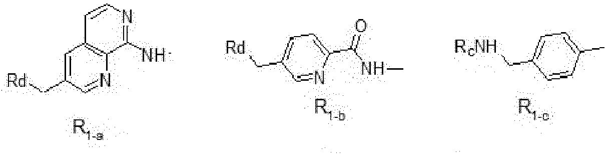 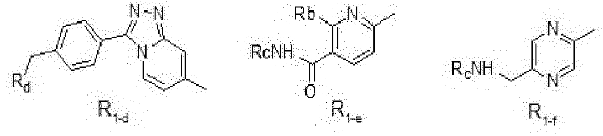 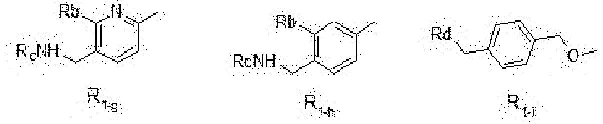
X₁-X₄, and X₆ are each independently C or N, with the proviso that at least two and at most five of X₁-X₄, and X₆ are N;
R₃ is methyl, or Cl;
R₅ is methyl, or Cl
R₄, is H;
R₇ is H, C₁-₆ alkyl, or C₁-₆ alkoxy;
R₆ is selected from H, C₁-₆ alkyl, halogen, and OCH₂Rₐ;
each Rₐ is independently selected from H, C₁-₆ alkyl, C₁-₆ alkoxy, C₆-₁₀ aryl, 5-14 membered heteroaryl, 4-10 membered heterocycloalkyl, C₆-₁₀ aryl-C₁-₄ alkyl, (5-14 membered heteroaryl)-C₁-₄ alkyl-, (4-10 membered heterocycloalkyl)-C₁-₄ alkyl-, NHR_{c}, and OCH₂R_{c}, and wherein C₁-₆ alkyl, C₁-₆ alkoxy, C₆-₁₀ aryl, 5-14 membered heteroaryl, 4-10 membered heterocycloalkyl, C₆-₁₀ aryl-C₁-₄ alkyl, (5-14 membered heteroaryl)-C₁-₄ alkyl-, (4-10 membered heterocycloalkyl)-C₁-₄ alkyl- of Rₐ are each independently substituted with 1, or 2 independently selected R_{d} substituents;
each R_{b} substituent is independently selected from C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, and wherein the C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, of R_{b} are each independently substituted with 1, 2, or 3 independently selected R_{d} substituents;
each R_{c} is independently selected from H, C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl, C(O)R_{d}, C(O)NHR_{d}, and C(O)NR_{d}R_{d}, and wherein the C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl of R_{c} are each independently substituted with 1, 2, or 3 independently selected R_{d} substituents;
each R_{d} is independently selected from C₁-₆ alkyl, C₁-₆ alkoxy, C₆-₁₀ aryl, C₃-₁₀ cycloalkyl, 5-14 membered heteroaryl, 4-10 membered heterocycloalkyl, C₃-C₆ cyclic amide, CN, NH₂, C(O)ORₑ, NHRₑ, NRₑRₑ, NRₑC(O)Rₑ, NRₑC(O)ORₑ, ORₑ, (=O), S(O)₂Rₑ and S(O)₂NRₑRₑ, and wherein the C₁-₆ alkyl, C₁-₆ alkoxy, C₆-₁₀ aryl, C₃-₁₀ cycloalkyl, 5-14 membered heteroaryl, 4-10 membered heterocycloalkyl, C₃-C₆ cyclic amide of R_{d} are each optionally substituted with 1, 2, or 3 independently selected R_{f} substituents;
each Rₑ is independently selected from H, OH, COOH, C(O)R_{f}, C₁-₆ alkyl, and 5-14 membered heteroaryl, and wherein the C₁-₆ alkyl, and 5-14 membered heteroaryl of Rₑ are each optionally substituted with 1, 2 or 3 independently selected R_{f} substituents;
each R_{f} is independently selected from H, C₁-₄ alkyl, halogen, CN, COOH, OH, (=O).

In some embodiments provided herewith is a compound having Formula (VII) 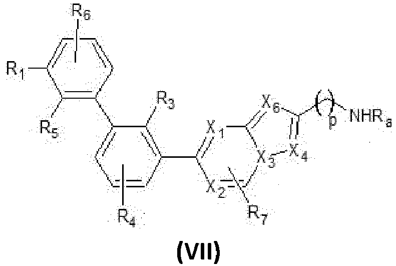
or a pharmaceutically acceptable salt or a stereoisomer thereof, wherein subscript p is 0, or 1;
R₁ is selected from: 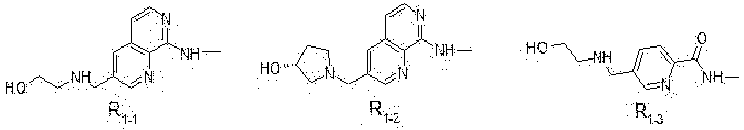 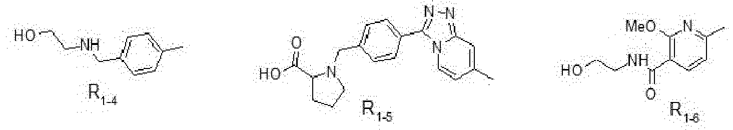 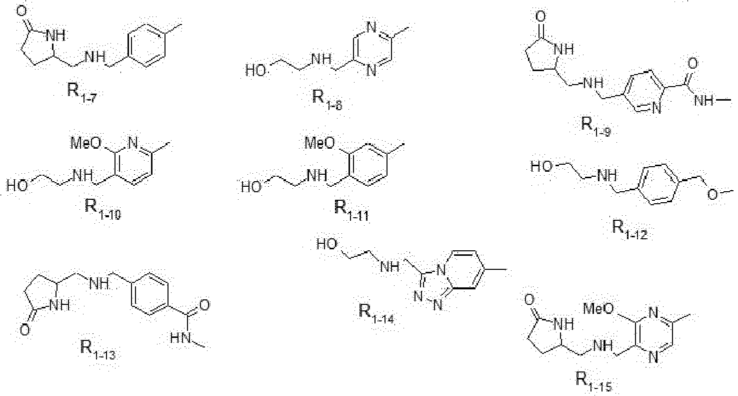
X₁-X₄, and X₆ are each independently C or N, with the proviso that at least two and at most five of X₁-X₄, and X₆ are N;
R₃ is methyl, or Cl;
R₅ is methyl, or Cl
R₄, is H;
R₇ is H, C₁-₆ alkyl, or C₁-₆ alkoxy;
R₆ is selected from H, C₁-₆ alkyl, halogen, and OCH₂Rₐ;
each Rₐ is independently selected from H, C₁-₆ alkyl, C₁-₆ alkoxy, C₆-₁₀ aryl, 5-14 membered heteroaryl, 4-10 membered heterocycloalkyl, C₆-₁₀ aryl-C₁-₄ alkyl, (5-14 membered heteroaryl)-C₁-₄ alkyl-, (4-10 membered heterocycloalkyl)-C₁-₄ alkyl-, NHR_{c}, and OCH₂R_{c}, and wherein C₁-₆ alkyl, C₁-₆ alkoxy, C₆-₁₀ aryl, 5-14 membered heteroaryl, 4-10 membered heterocycloalkyl, C₆-₁₀ aryl-C₁-₄ alkyl, (5-14 membered heteroaryl)-C₁-₄ alkyl-, (4-10 membered heterocycloalkyl)-C₁-₄ alkyl- of Rₐ are each independently substituted with 1, or 2 independently selected R_{d} substituents;
each R_{b} substituent is independently selected from C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, and wherein the C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, of R_{b} are each independently substituted with 1, 2, or 3 independently selected R_{d} substituents;
each R_{c} is independently selected from H, C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl, C(O)R_{d}, C(O)NHR_{d}, and C(O)NR_{d}R_{d}, and wherein the C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl of R_{c} are each independently substituted with 1, 2, or 3 independently selected R_{d} substituents;
each R_{d} is independently selected from C₁-₆ alkyl, C₁-₆ alkoxy, C₆-₁₀ aryl, C₃-₁₀ cycloalkyl, 5-14 membered heteroaryl, 4-10 membered heterocycloalkyl, C₃-C₆ cyclic amide, CN, NH₂, C(O)ORₑ, NHRₑ, NRₑRₑ, NRₑC(O)Rₑ, NRₑC(O)ORₑ, ORₑ, (=O), S(O)₂Rₑ and S(O)₂NRₑRₑ, and wherein the C₁-₆ alkyl, C₁-₆ alkoxy, C₆-₁₀ aryl, C₃-₁₀ cycloalkyl, 5-14 membered heteroaryl, 4-10 membered heterocycloalkyl, C₃-C₆ cyclic amide of R_{d} are each optionally substituted with 1, 2, or 3 independently selected R_{f} substituents;
each Rₑ is independently selected from H, OH, COOH, C(O)R_{f}, C₁-₆ alkyl, and 5-14 membered heteroaryl wherein the C₁-₆ alkyl, and 5-14 membered heteroaryl of Rₑ are each optionally substituted with 1, 2 or 3 independently selected R_{f} substituents;
each R_{f} is independently selected from H, C₁-₄ alkyl, halogen, CN, COOH, OH, (=O).

### Some embodiments of the present invention disclose a compound having Formula I wherein the compound is selected from:

1-(4-(7-(2-methyl-[1,1'-biphenyl]-3-yl)imidazo[1,2-a]pyridin-3-yl)benzyl)piperidine-2-carboxylic acid;
(4-(7-(2-methyl-[1,1'-biphenyl]-3-yl)imidazo[1,2-*a*]pyridin-3-yl)benzyl)proline;
3-((4-(7-(2-methyl-[1,1'-biphenyl]-3-yl)imidazo[1,2-*a*]pyridin-3-yl)benzyl)amino)oxetane-3-carboxylic acid;
(2*S*,4*R*)-4-hydroxy-1-(4-(7-(2-methyl-[1,1'-biphenyl]-3-yl)imidazo[1,2-*α*]pyridin-3-yl)benzyl)pyrrolidine-2-carboxylic acid;
1-((4-(7-(2-methyl-[1,1'-biphenyl]-3-yl)imidazo[1,2-*a*]pyridin-3 yl)benzyl)amino)cyclobutane-1-carboxylic acid;
4-(4-(7-(2-methyl-[1,1'-biphenyl]-3-yl)imidazo[1,2-*a*]pyridin-3-yl)benzyl)morpholine-3-carboxylic acid;
1-(2,6-dimethoxy-4-(7-(2-methyl-[1,1'-biphenyl]-3-yl)imidazo[1,2-*α*]pyridin-3-yl)benzyl)piperidine-2-carboxylic acid;
1-(4-(7-(2-methyl-[1,1'-biphenyl]-3-yl)imidazo[1,2-*α*]pyridin-3-yl)benzyl)azetidine-3-carboxylic acid;
*N*-(2-hydroxyethyl)-1-(7-(2-methyl-[1,1'-biphenyl]-3-yl)imidazo[1,2-α]pyridin-3-yl)-1H-pyrazole-3-carboxamide;
1-(4-(7-(2-methyl-[1,1'-biphenyl]-3-yl)-[1,2,4]triazolo[4,3-*α*]pyridin-3-yl)benzyl)piperidine-2-carboxylic acid;
1-(4-(7-(3-(2,3-dihydrobenzo[*b*][1,4]dioxin-6-yl)-2-methylphenyl)imidazo[1,2-*α*]pyridin-3-yl)benzyl)piperidine-2-carboxylic acid;
(4-(7-(3-(2,3-dihydrobenzo[*b*][1,4]dioxin-6-yl)-2-methylphenyl)imidazo[1,2-*α*]pyridin-3-yl)benzyl)proline;
3-((4-(7-(2-methyl-[1,1'-biphenyl]-3-yl)imidazo[1,2-*α*]pyridin-3-yl)benzyl)amino)propan-1-ol;
(2,6-dimethoxy-4-(7-(2-methyl-[1,1'-biphenyl]-3-yl)imidazo[1,2-*α*]pyridin-3-yl)benzyl)proline;
(2,6-dimethoxy-4-(7-(2-methyl-[1,1'-biphenyl]-3-yl)imidazo[1,2-*α*]pyridin-3-yl)benzyl)proline;
(4-(7-(2-methyl-[1,1'-biphenyl]-3-yl)imidazo[1,2-*α*]pyridin-3-yl)benzyl)valine;
(4-(7-(2-methyl-[1,1'-biphenyl]-3-yl)imidazo[1,2-*α*]pyridin-3-yl)benzyl)-*L*-proline;
(4-(7-(2-methyl-[1,1'-biphenyl]-3-yl)imidazo[1,2-*α*]pyridin-3-yl)benzyl)-*D*-proline;
(4-(7-(2-methyl-[1,1'-biphenyl]-3-yl)imidazo[1,2-*α*]pyridin-3-yl)benzyl)glycine;
(*S*)-1-(4-(7-(2-methyl-[1,1'-biphenyl]-3-yl)imidazo[1,2-*α*]pyridin-3-yl)benzyl)azetidine-2-carboxylic acid;
(4-(7-(2-methyl-[1,1'-biphenyl]-3-yl)-[1,2,4]triazolo[4,3-*α*]pyridin-3-yl)benzyl)proline;
3-hydroxy-4-((4-(7-(2-methyl-[1,1'-biphenyl]-3-yl)imidazo[1,2-*α*]pyridin-3-yl)benzyl)amino)butanoic acid;
(1-(7-(2-methyl-[1,1'-biphenyl]-3-yl)imidazo[1,2-*α*]pyridin-3-yl)-1*H*-pyrazole-3-carbonyl)glycine;
4-(4-(7-(3-(2,3-dihydrobenzo[*b*][1,4]dioxin-6-yl)-2-methylphenyl)imidazo[1,2-*α*]pyridin-3-yl)benzyl)morpholine-3-carboxylic acid;
2-((4-(7-(2-methyl-[1,1'-biphenyl]-3-yl)-[1,2,4]triazolo[4,3-*α*]pyridin-3-yl)benzyl)amino)ethan-1-ol;
(3*S*,5*R*)-1-(4-(7-(3-(2,3-dihydrobenzo[*b*][1,4]dioxin-6-yl)-2-methylphenyl)imidazo[1,2-*α*]pyridin-3-yl)benzyl)-5-hydroxypyrrolidine-3-carboxylic acid;
3-((4-(7-(2-methyl-[1,1'-biphenyl]-3-yl)-[1,2,4]triazolo[4,3-*α*]pyridin-3-yl)benzyl)amino)propan-1-ol;
1-(4-(7-(3-(2,3-dihydrobenzo[*b*][1,4]dioxin-6-yl)-2-methylphenyl)imidazo[1,2-*α*]pyridin-3-yl)benzyl)azetidine-3-carboxylic acid;
(*S*)-1-(4-(7-(3-(2,3-dihydrobenzo[*b*][1,4]dioxin-6-yl)-2-methylphenyl)imidazo[1,2-*α*]pyridin-3-yl)benzyl)azetidine-2-carboxylic acid;
((4-(7-(3-(2,3-dihydrobenzo[*b*][1,4]dioxin-6-yl)-2-methylphenyl)imidazo[1,2-*α*]pyridin-3-yl)-2,6-dimethoxybenzyl)-*D*-proline;
(4-(7-(3-(2,3-dihydrobenzo[*b*][1,4]dioxin-6-yl)-2-methylphenyl)imidazo[1,2-*α*]pyridin-3-yl)-2,6-dimethoxybenzyl)-*L*-proline;
(4-(7-(3-(2,3-dihydrobenzo[*b*][1,4]dioxin-6-yl)-2-methylphenyl)imidazo[1,2-*α*]pyridin-3-yl)benzyl)-*D*-proline;
(4-(7-(3-(2,3-dihydrobenzo[*b*][1,4]dioxin-6-yl)-2-methylphenyl)imidazo[1,2-*α*]pyridin-3-yl)benzyl)-*L*-proline;
1-(1-(1-(4-(7-(2-methyl-[1,1'-biphenyl]-3-yl)imidazo[1,2-*α*]pyridin-3-yl)phenyl)ethyl)azetidin-3-yl)ethan-1-one;
2-methyl-2-((4-(7-(2-methyl-[1,1'-biphenyl]-3-yl)imidazo[1,2-*α*]pyridin-3-yl)benzyl)amino)propan-1-ol;
3-hydroxy-4-((4-(7-(2-methyl-[1,1'-biphenyl]-3-yl)-[1,2,4]triazolo[4,3-*α*]pyridin-3-yl)benzyl)amino)butanoic acid;
(3-(7-(2-methyl-[1,1'-biphenyl]-3-yl)imidazo[1,2-*α*]pyridin-3-yl)prop-2-yn-1-yl)-*D-*proline;
(3-(7-(2-methyl-[1,1'-biphenyl]-3-yl)imidazo[1,2-*α*]pyridin-3-yl)prop-2-yn-1-yl)-*L-*proline;
(*R*)-(1-(4-(7-(2-methyl-[1,1'-biphenyl]-3-yl)imidazo[1,2-*α*]pyridin-3-yl)benzyl)pyrrolidin-2-yl)methanol;
(*S*)-(1-(4-(7-(2-methyl-[1,1'-biphenyl]-3-yl)imidazo[1,2-*α*]pyridin-3-yl)benzyl)pyrrolidin-2-yl)methanol;
4-((4-(7-(3-(2,3-dihydrobenzo[*b*][1,4]dioxin-6-yl)-2-methylphenyl)imidazo[1,2-*α*]pyridin-3-yl)benzyl)amino)-3-hydroxybutanoic acid;
(*R*)-3-((4-(7-(3-(2,3-dihydrobenzo[*b*][1,4]dioxin-6-yl)-2-methylphenyl)imidazo[1,2-*α*]pyridin-3-yl)benzyl)amino)propane-1,2-diol;
(S)-3-((4-(7-(3-(2,3-dihydrobenzo[b][1,4]dioxin-6-yl)-2-methylphenyl)imidazo[1,2-*α*]pyridin-3-yl)benzyl)amino)propane-1,2-diol;
(4-(7-(3-(2,3-dihydrobenzo[*b*][1,4]dioxin-6-yl)-2-methylphenyl)-[1,2,4]triazolo[4,3-*α*]pyridin-3-yl)benzyl)-*D*-proline;
(4-(7-(3-(2,3-dihydrobenzo[*b*][1,4]dioxin-6-yl)-2-methylphenyl)-[1,2,4]triazolo[4,3-*α*]pyridin-3-yl)benzyl)-*L*-proline;
(4-(7-(3-(2,3-dihydrobenzo[*b*][1,4]dioxin-6-yl)-2-methylphenyl)imidazo[1,2-*α*]pyridin-3-yl)benzoyl)glycine;
*N*-(2-acetamidoethyl)-1-(7-(2-methyl-[1,1'-biphenyl]-3-yl)imidazo[1,2-*α*]pyridin-3-yl)-1*H*-pyrazole-3-carboxamide;
(4-(7-(3-(2,3-dihydrobenzo[*b*][1,4]dioxin-6-yl)-2-methylphenyl)imidazo[1,2-*α*]pyridin-3-yl)benzoyl)-*D*-proline;
(4-(7-(3-(2,3-dihydrobenzo[*b*][1,4]dioxin-6-yl)-2-methylphenyl)imidazo[1,2-*α*]pyridin-3-yl)benzoyl)-*L*-proline;
3-((4-(7-(2-methyl-[1,1'-biphenyl]-3-yl)imidazo[1,2-*α*]pyridin-3-yl)benzyl)amino)propanoic acid;
3-(1-(7-(2-methyl-[1,1'-biphenyl]-3-yl)imidazo[1,2-*α*]pyridin-3-yl)-1*H*-pyrazole-3-carboxamido)propanoic acid;
(1-(7-(3-(2,3-dihydrobenzo[*b*][1,4]dioxin-6-yl)-2-methylphenyl)imidazo[1,2-*α*]pyridin-3-yl)-1H-pyrazole-3-carbonyl)glycine;
(4-(7-(2-methyl-[1,1'-biphenyl]-3-yl)-[1,2,4]triazolo[4,3-*α*]pyridin-3-yl)benzyl)-*D-*proline;
(4-(7-(2-methyl-[1,1'-biphenyl]-3-yl)-[1,2,4]triazolo[4,3-*α*]pyridin-3-yl)benzyl)-*L-*proline;
2-((4-(7-(2-methyl-[1,1'-biphenyl]-3-yl)imidazo[1,2-*α*]pyridin-3-yl)benzyl)amino)ethane-1-sulfonic acid;
(2*S*,4*R*)-1-(4-(7-(3-(2,3-dihydrobenzo[*b*][1,4]dioxin-6-yl)-2-methylphenyl)-[1,2,4]triazolo[4,3-*α*]pyridin-3-yl)benzyl)-4-hydroxypyrrolidine-2-carboxylic acid;
3-((4-(7-(3-(2,3-dihydrobenzo[*b*][1,4]dioxin-6-yl)-2-methylphenyl)-[1,2,4]triazolo[4,3-*α*]pyridin-3-yl)benzyl)amino)propanoic acid;
(1-(7-(3-(2,3-dihydrobenzo[*b*][1,4]dioxin-6-yl)-2-methylphenyl)-[1,2,4]triazolo[4,3-*α*]pyridin-3-yl)-1*H*-pyrazole-3-carbonyl)glycine;
(4-(7-(3'-(5-(((2-hydroxyethyl)amino)methyl)picolinamido)-2,2'-dimethyl-[1,1'-biphenyl]-3-yl)-[1,2,4]triazolo[4,3-*α*]pyridin-3-yl)benzyl)-*D*-proline;
(2'*R*)-((((2,2'-dimethyl-[1,1'-biphenyl]-3,3'-diyl)bis([1,2,4]triazolo[4,3-*α*]pyridine-7,3-diyl))bis(4,1-phenylene))bis(methylene))di-*D*-proline;
(4-(7-(4"-(((2-hydroxyethyl)amino)methyl)-2,2'-dimethyl-[1,1':3',1"-terphenyl]-3-yl)-[1,2,4]triazolo[4,3-*α*]pyridin-3-yl)benzyl)-*D*-proline;
tert-butyl (4-(7-(3'-(5-((2-hydroxyethyl)carbamoyl)-6-methoxypyridin-2-yl)-2,2'-dimethyl-[1,1'-biphenyl]-3-yl)-[1,2,4]triazolo[4,3-*α*]pyridin-3-yl)benzyl)-*D*-prolinate;
(4-(7-(3'-(5-(((2-hydroxyethyl)amino)methyl)pyrazin-2-yl)-2,2'-dimethyl-[1,1'-biphenyl]-3-yl)-[1,2,4]triazolo[4,3-*α*]pyridin-3-yl)benzyl)-*D*-proline;
5-(((2-hydroxyethyl)amino)methyl)-*N*-(3'-(3-(4-(((2-hydroxyethyl)amino)methyl)phenyl)-[1,2,4]triazolo[4,3-*α*]pyridin-7-yl)-2,2'-dimethyl-[1,1'-biphenyl]-3-yl)picolinamide;
(4-(7-(3'-(5-(((2-hydroxyethyl)amino)methyl)picolinamido)-2,2'-dimethyl-[1,1'-biphenyl]-3-yl)-[1,2,4]triazolo[4,3-*α*]pyridin-3-yl)benzyl)-*L*-proline;
2-((4-(7-(4"-(((2-hydroxyethyl)amino)methyl)-2,2'-dimethyl-[1,1':3',1"-terphenyl]-3-yl)-[1,2,4]triazolo[4,3-a]pyridin-3-yl)benzyl)amino)ethan-1-ol;
*N*-(2,2'-dimethyl-3'-(3-(4-(((((*R*)-5-oxopyrrolidin-2-yl)methyl)amino)methyl)phenyl)-[1,2,4]triazolo[4,3-*α*]pyridin-7-yl)-[1,1'-biphenyl]-3-yl)-5-(((((*R*)-5-oxopyrrolidin-2-yl)methyl)amino)methyl)picolinamide;
(4-(7-(4"-(((2-hydroxyethyl)amino)methyl)-2,2'-dimethyl-[1,1':3',1"-terphenyl]-3-yl)-[1,2,4]triazolo[4,3-*α*]pyridin-3-yl)benzyl)-*L*-proline;
(R)-5-((((2',2"-dimethyl-3"-(3-(4-(((((*R*)-5-oxopyrrolidin-2-yl)methyl)amino)methyl)phenyl)-[1,2,4]triazolo[4,3-*α*]pyridin-7-yl)-[1,1':3',1"-terphenyl]-4-yl)methyl)amino)methyl)pyrrolidin-2-one;
2-((4-(7-(4"-(((2-hydroxyethyl)amino)methyl)-3"-methoxy-2,2'-dimethyl-[1,1':3',1"-terphenyl]-3-yl)-[1,2,4]triazolo[4,3-*α*]pyridin-3-yl)benzyl)amino)ethan-1-ol;
2-((4-(7-(3'-(5-(((2-hydroxyethyl)amino)methyl)-6-methoxypyridin-2-yl)-2,2'-dimethyl-[1,1'-biphenyl]-3-yl)-[1,2,4]triazolo[4,3-*α*]pyridin-3-yl)benzyl) amino)ethan-1-ol;
2-((4-(7-(3'-((3-(((2-hydroxyethyl)amino)methyl)-1,7-naphthyridin-8-yl)amino)-2,2'-dimethyl-[1,1'-biphenyl]-3-yl)-[1,2,4]triazolo[4,3-*α*]pyridin-3-yl)benzyl)amino)ethan-1-ol;
5-(((2-hydroxyethyl)amino)methyl)-*N*-(3'-(3-(4-(((2-methoxyethyl)amino)methyl)phenyl)-[1,2,4]triazolo[4,3-*α*]pyridin-7-yl)-2,2'-dimethyl-[1,1'-biphenyl]-3-yl)picolinamide;
(4-(7-(3'-((3-(((R)-3-hydroxypyrrolidin-1-yl)methyl)-1,7-naphthyridin-8-yl)amino)-2,2'-dimethyl-[1,1'-biphenyl]-3-yl)-[1,2,4]triazolo[4,3-*α*]pyridin-3-yl)benzyl)-*D*-proline;
ethyl (1-(7-(4"-(((2-hydroxyethyl)amino)methyl)-2,2'-dimethyl-[1,1':3',1"-terphenyl]-3-yl)-[1,2,4]triazolo[4,3-a]pyridin-3-yl)-1*H*-pyrazole-3-carbonyl)glycinate;
2-(((7-(4"-(((2-hydroxyethyl)amino)methyl)-2,2'-dimethyl-[1,1':3',1"-terphenyl]-3-yl)-[1,2,4]triazolo[1,5-*a*]pyridin-2-yl)methyl)amino)ethan-1-ol;
2-(((7-(4"-(((2-hydroxyethyl)amino)methyl)-2,2'-dimethyl-[1,1':3',1"-terphenyl]-3-yl)-[1,2,4]triazolo[4,3-a]pyridin-3-yl)methyl)amino)ethan-1-ol;
(*S*)-5-((((2',2"-dimethyl-3"-(3-(4-(((((*S*)-5-oxopyrrolidin-2-yl)methyl)amino)methyl)phenyl)-[1,2,4]triazolo[4,3-*α*]pyridin-7-yl)-[1,1':3',1"-terphenyl]-4-yl)methyl)amino)methyl)pyrrolidin-2-one;
2-(((3"-(3-((2-hydroxyethyl)amino)-[1,2,4]triazolo[4,3-*α*]pyridin-7-yl)-2',2"-dimethyl-[1,1':3',1"-terphenyl]-4-yl)methyl)amino)ethan-1-ol;
2-(((2',2"-dichloro-3"-(3-(4-(((2-hydroxyethyl)amino)methyl)phenyl)-[1,2,4]triazolo[4,3-*α*]pyridin-7-yl)-[1,1':3',1"-terphenyl]-4-yl)methyl)amino)ethan-1-ol;
(4-(7-(2,2'-dichloro-4"-(((2-hydroxyethyl)amino)methyl)-[1,1':3',1"-terphenyl]-3-yl)-[1,2,4]triazolo[4,3-*α*]pyridin-3-yl)benzyl)-*D*-proline;
2-(((2',2"-dichloro-3"-(3-(((2-hydroxyethyl)amino)methyl)-[1,2,4]triazolo[4,3-*α*]pyridin-7-yl)-[1,1':3',1"-terphenyl]-4-yl)methyl)amino)ethan-1-ol;
(*S*)-5-(((4-(7-(3'-((3-(((*R*)-3-hydroxypyrrolidin-1-yl)methyl)-1,7-naphthyridin-8-yl)amino)-2,2'-dimethyl-[1,1'-biphenyl]-3-yl)-[1,2,4]triazolo[4,3-*α*]pyridin-3-yl)benzyl) amino)methyl)pyrrolidin-2-one;
5-(((2-hydroxyethyl)amino)methyl)-N-(3'-(3-(((2-hydroxyethyl)amino)methyl)-[1,2,4]triazolo[4,3-*α*]pyridin-7-yl)-2,2'-dimethyl-[1,1'-biphenyl]-3-yl)picolinamide;
(*S*)-5-((((7-(4"-(((2-hydroxyethyl)amino)methyl)-2,2'-dimethyl-[1,1':3',1"-terphenyl]-3-yl)-[1,2,4]triazolo[4,3-*α*]pyridin-3-yl)methyl)amino)methyl)pyrrolidin-2-one;
(*S*)-*N*-(2,2'-dimethyl-3'-(3-((((5-oxopyrrolidin-2-yl)methyl)amino)methyl)[1,2,4]triazolo[4,3-*α*]pyridin-7-yl)-[1,1'-biphenyl]-3-yl)-5-(((2-hydroxyethyl)amino)methyl)picolinamide;
(*S*)-5-((((2',2"-dimethyl-3"-(3-(((((*S*)-5-oxopyrrolidin-2-yl)methyl)amino)methyl)-[1,2,4]triazolo[4,3-*α*]pyridin-7-yl)-[1,1':3',1"-terphenyl]-4-yl)methyl)amino)methyl)pyrrolidin-2-one;
(*R*)-1-((8-((3'-(3-(4-(((2-hydroxyethyl)amino)methyl)phenyl)-[1,2,4]triazolo[4,3-*α*]pyridin-7-yl)-2,2'-dimethyl-[1,1'-biphenyl]-3-yl)amino)-1,7-naphthyridin-3-yl)methyl)pyrrolidin-3-ol;
(*R*)-1-(4-(7-(4"-(((2-hydroxyethyl)amino)methyl)-2,2'-dimethyl-[1,1':3',1"-terphenyl]-3-yl)-[1,2,4]triazolo[4,3-*α*]pyridin-3-yl)benzyl)pyrrolidine-3-carboxylic acid;
5-(((2-hydroxyethyl)amino)methyl)-*N*-(3'-(2-(((2-hydroxyethyl)amino)methyl)-[1,2,4]triazolo[1,5-*α*]pyridin-7-yl)-2,2'-dimethyl-[1,1'-biphenyl]-3-yl)picolinamide;
(*R*)-1-((7-(4"-(((2-hydroxyethyl)amino)methyl)-2,2'-dimethyl-[1,1':3',1"-terphenyl]-3-yl)-[1,2,4]triazolo[4,3-*α*]pyridin-3-yl)methyl)pyrrolidine-3-carboxylic acid;
(*S*)-*N*-(2,2'-dimethyl-3'-(2-((((5-oxopyrrolidin-2-yl)methyl)amino)methyl)-[1,2,4]triazolo[1,5-*α*]pyridin-7-yl)-[1,1'-biphenyl]-3-yl)-4-(((2-hydroxyethyl)amino)methyl)benzamide;
(*S*)-5-((((7-(4"-(((2-hydroxyethyl)amino)methyl)-2,2'-dimethyl-[1,1':3',1"-terphenyl]-3-yl)-[1,2,4]triazolo[1,5-a]pyridin-2-yl)methyl) amino) methyl) pyrrolidin-2-one
4-(((2-hydroxyethyl)amino)methyl)-*N*-(3'-(3-(((2-hydroxyethyl)amino)methyl)-[1,2,4]triazolo[4,3-*α*]pyridin-7-yl)-2,2'-dimethyl-[1,1'-biphenyl]-3-yl)benzamide;
2,2'-((((2,2'-dimethyl-[1,1'-biphenyl]-3,3'-diyl)bis([1,2,4]triazolo[4,3-*α*]pyridine-7,3-diyl))bis(methylene))bis(azanediyl))bis(ethan-1-ol);
4-(((2-hydroxyethyl)amino)methyl)-*N*-(3'-(2-(((2-hydroxyethyl)amino)methyl)-[1,2,4]triazolo[1,5-*α*]pyridin-7-yl)-2,2'-dimethyl-[1,1'-biphenyl]-3-yl)benzamide;
(*S*)-5-((((2',2"-dimethyl-3"-(2-(((((*S*)-5-oxopyrrolidin-2-yl)methyl)amino)methyl)-[1,2,4]triazolo[1,5-*α*]pyridin-7-yl)-[1,1':3',1"-terphenyl]-4-yl)methyl)amino)methyl)pyrrolidin-2-one;
2-(((2',2"-dichloro-3"-(2-(((2-hydroxyethyl)amino)methyl)-[1,2,4]triazolo[1,5-*α*]pyridin-7-yl)-[1,1':3',1"-terphenyl]-4-yl)methyl)amino)ethan-1-ol;
(*S*)-5-((((5-(2,2'-dimethyl-3'-(3-(((((*S*)-5-oxopyrrolidin-2-yl)methyl)amino)methyl)-[1,2,4]triazolo[4,3-*α*]pyridin-6-yl)-[1,1'-biphenyl]-3-yl)-3-methoxypyrazin-2-yl)methyl)amino)methyl)pyrrolidin-2-one;
((7-(4"-(((2-hydroxyethyl)amino)methyl)-2,2'-dimethyl-[1,1':3',1"-terphenyl]-3-yl)-[1,2,4]triazolo[4,3-*α*]pyridin-3-yl)methyl)-*D*-proline;
(*S*)-5-((((2',2"-dichloro-3"-(2-(((((*S*)-5-oxopyrrolidin-2-yl)methyl)amino)methyl)-[1,2,4]triazolo[1,5-*α*]pyridin-7-yl)-[1,1':3',1"-terphenyl]-4-yl)methyl)amino)methyl)pyrrolidin-2-one;
(S)-5-((((2',2"-dichloro-3"-(3-(((((S)-5-oxopyrrolidin-2-yl)methyl)amino)methyl)-[1,2,4]triazolo[4,3-a]pyridin-7-yl)-[1,1':3',1"-terphenyl]-4-yl)methyl)amino)methyl)pyrrolidin-2-one.

In one embodiment, the compound of Formula (I) is
(*S*)-5-((((2',2"-dimethyl-3"-(2-(((((*S*)-5-oxopyrrolidin-2-yl)methyl)amino)methyl)-[1,2,4]triazolo[1,5-*α*]pyridin-7-yl)-[1,1':3',1"-terphenyl]-4-yl)methyl)amino)methyl)pyrrolidin-2-one;

Certain features of the invention that are described in separate embodiments, can also be provided in combination in a single embodiment. Thus, it is contemplated that features described as embodiments of the compounds of Formula (I) can be combined in any suitable combination, and with compounds of Formulae (II)-(VII).

The compounds described and claimed herein can have one or more stereocenters and can be isolated in enantiopure form (optically active) or as stereomeric mixtures, such as racemic forms. Methods on how to obtain enantiopure compounds with enantioselective synthesis and/or from optically inactive starting materials are known in the art. Cis and trans geometric isomers of the compounds of the present invention may be isolated as a mixture of isomers or as separated isomeric forms.

In some embodiments, the compounds of the invention have the (*R*)-configuration. In other embodiments, the compounds have the (*S*)-configuration. In compounds with more than one chiral center, each of the chiral centers may be independently (*R*) or (*S*).

The term "compound" as used herein, includes all geometric isomers, and stereoisomers, of the structures depicted. The present invention also includes pharmaceutically acceptable salts of the compounds described herein. The pharmaceutically acceptable salts of the present invention include the generally regarded as safe (GRAS) salts of the parent compound formed, e.g., from GRAS inorganic or organic acids.

All compounds, and pharmaceutically acceptable salts thereof, can be found as such or with other substances such as water and solvents. This in turn means that in the solid state, the compounds described herewith, and salts thereof may occur in various forms, e.g., taking the form of solvates, including hydrates. So, unless indicated otherwise, reference in the specification to compounds and salts thereof should be understood as encompassing any solid state form of the compound (e.g., polymorph or solvate).

The compounds of the present inventions or salts thereof can be substantially or partially isolated/purified from the environment in which they were formed or detected. This can be done via a purification process, according to methods known in the art. The term substantially purified compound refers to compositions containing at least 50%, at least about 60%, at least about 70%, at least about 80%, at least about 90%, at least about 95%, or at least about 99% by weight of the compounds of the invention, or salt thereof.

### Medical uses

For brevity, the uses and methods will be described simply referring to "the compound of the invention". It is understood that for the uses described herewith, any of the compounds disclosed, including any of the embodiments thereof, may be used.

The compounds of the invention are for use as a medicament.

The compound of the invention, or a pharmaceutically acceptable salt or a stereoisomer thereof, or a composition of the invention, can be used in methods of treating a disease or disorder associated with inhibition of PD-1/PD-L1 interaction wherein the disease or disorder is cancer or an infection.

Compounds of the present disclosure can inhibit the activity of PD-1/PD-L1 protein/protein interaction and are therefore useful in treating diseases and disorders associated with activity of PD-1/PD-L1 interaction. Accordingly, the present invention provides compounds and compositions for use in methods of inhibiting PD-1/PD-L1 interaction, said methods comprising administering to a patient in need thereof a compound of the invention, or a pharmaceutically acceptable salt or a stereoisomer thereof.

The compound of the invention, or a pharmaceutically acceptable salt or a stereoisomer thereof, or a composition of the invention, can further be used to enhance, stimulate and/or increase the immune response in a patient, wherein the patient has cancer or an acute or chronic infection. The compounds of the present disclosure can be used alone, in combination with other agents or therapies or as an adjuvant for the treatment of diseases or disorders, including cancer or infection diseases.

The compound of the invention induces a **reversion of T-Cell exhaustion,** that may occur in cancer or infections. The experiments produced by the applicant have shown that the compound of the invention inhibits the PD-1/PD-L1 interaction in vitro and/or vivo, in cancerous cells and during infections. The blockade of PD-1 enhances the immune response to cancerous cells and infectious diseases in mammals, humans included.

In some embodiments, provided herein, are compounds and compositions for use in a method for treating cancer.

In one embodiment, the present disclosure provides compounds and compositions for use in a method for inhibiting growth of tumor cells.

In some embodiments provided herein, is a method for treating cancer, said method including administering the compounds and compositions of the invention to a subject in need thereof.

In some embodiments provided herein, is a method for inhibiting growth of tumor cells, said method including administering the compounds and compositions of the invention to a subject in need thereof.

The compound of the invention can be used for all types of cancers where there is an over-expression of PD-L1. Non-limiting examples of cancers that are treatable using the compound of the present invention are MSI-H/dMMR colorectal cancer. PM, pleural mesothelioma, TNBC, triple-negative breast cancer, CSCC, cutaneous squamous cell carcinoma, TMB-H, tumor mutation burden high, CRC, colorectal cancer, BCG-BC, Bacillus Calmette-Guérin bladder cancer, EC, endometrial carcinoma, ESCC, esophageal squamous cell carcinoma, SCLC, small cell lung cancer, RCC, renal cell carcinoma, MCC, Merkel cell carcinoma, HCC, hepatocellular carcinoma, PMBCL, primary mediastinal large B cell lymphoma, CC, cervical cancer, GC, gastric cancer, MSI-H, microsatellite instability high, dMMR, mismatch repair-deficient, UC, urothelial carcinoma, cHL, classical Hodgkin's lymphoma, HNSCC, head and neck squamous cell carcinoma, NSCLC, non-small cell lung cancer.

PD-1 pathway blockade with compounds of the present disclosure can also be useful for treating infections. Accordingly, the compound of the invention can be used in a method for treating infections. The compound of the invention can be used for all types of infections where there is an over-expression of PD-1/PD-L1. These can be viral, bacteria, fungus and parasite infections. Non-limiting examples of infections that are treatable with the compound of the invention are chronic hepatitis B, chronic hepatitis C, chronic hepatitis D, HIV and SIV, Covid and other coronaviruses, herpes simplex virus, cytomegalovirus, Kaposi's sarcoma-associated herpesvirus, varicella zoster virus and other herpesviruses, influenza virus, lymphocytic choriomeningitis virus, Japanese encephalitis virus, Ebola virus, hantavirus, Friend retrovirus, respiratory syncytial virus, rabies virus, tuberculosis, malaria, candida albicans, helicobacter pylori, staphylococcus aureus, pseudomonas aeruginosa, borrelia, burgdorferi, sepsis.

In one embodiment the present disclosure provides compounds and compositions for use in a method for treating bacterial infections.

In one embodiment the present disclosure provides compounds and compositions for use in a method for treating virus infections.

In one embodiment the present disclosure provides compounds and compositions for use in a method for treating chronic infections.

In one embodiment the present disclosure provides compounds and compositions for use in a method for treating acute infections.

In one embodiment the present disclosure provides a method for treating virus infections, said method including administering the compounds or compositions of the invention to a subject in need thereof.

In one embodiment the present disclosure provides a method for treating bacterial infections, said method including administering the compounds or compositions of the invention to a subject in need thereof.

In one embodiment the present disclosure provides a method for treating chronic infections, said method including administering the compounds or compositions of the invention to a subject in need thereof.

In one embodiment the present disclosure provides a method for treating acute infections, said method including administering the compounds or compositions of the invention to a subject in need thereof.

### Formulation, Dosage Forms and Administration

This invention also relates to pharmaceutical compositions comprising the compound of the present disclosure or a pharmaceutically acceptable salt thereof, in combination with one or more pharmaceutically acceptable carriers or excipients. In these compositions, the compound of the invention is used as an active ingredient.

Thus, the present disclosure provides a composition comprising a compound of Formula (I) or any of the formulas as described herein, or a pharmaceutically acceptable salt thereof, or any of the embodiments thereof, and at least one pharmaceutically acceptable carrier or excipient.

These compositions can be prepared according to methods known in the art and can be administered by any route appropriate to the condition to be treated. For example, suitable routes include topical, transdermal, parenteral, nasal, oral, rectal, and the like. It will be appreciated that the preferred route may vary with, for example, the condition of the recipient. Particularly preferred and advantageous is the oral administration. The compounds according to the invention can be easily and safely administered by oral administration.

In making the compositions of the invention, the active ingredient is typically mixed with an excipient, diluted by an excipient or enclosed within such a carrier in the form of, e.g., a tablet, capsule, sachet, paper, or other container (liquid, solid, spray).

Tablets, capsules and other formulation suitable for oral administration are particularly preferred.

Some examples of suitable excipients include microcrystalline cellulose, polyvinylpyrrolidone, cellulose, lactose, dextrose, sucrose, alginates, gelatin, calcium silicate, water, sorbitol, mannitol, starches, gum acacia, calcium phosphate, syrup and methyl cellulose. The formulations can include emulsifying and suspending agents; preserving agents such as methyl- and propylhydroxy-benzoates; sweetening agents; and flavoring agents. The compositions can also include lubricating agents such as talc, magnesium stearate and mineral oil; wetting agents; The compositions of the invention can be formulated by employing procedures known in the art.

**General synthesis.** In another aspect, the present invention refers to processes for obtaining the compounds according to the invention. Examples of the general procedures for obtaining the compounds of the invention, and of the synthetic routes for preparing them as well as their intermediate compounds are explained below.

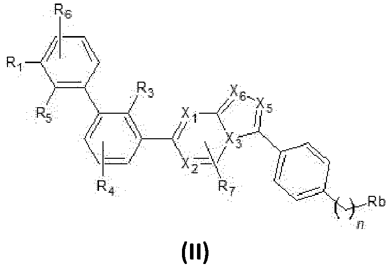

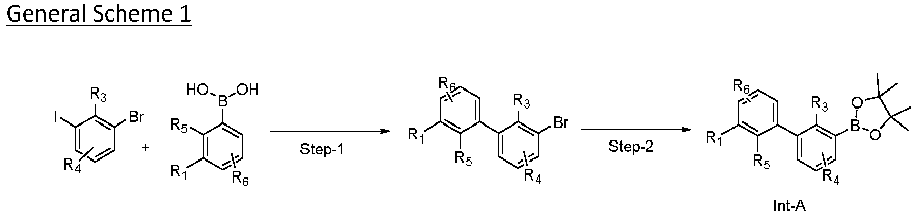

In General Scheme 1, the following reaction conditions typically apply: (Step-1) synthesized by any metal cross coupling reactions (such as Suzuki coupling reaction; in the presence of suitable catalyst (for example, Pd(PPh₃)₄, Pd(dppf)Cl₂) and a suitable base (for example, Na₂CO₃,K₂CO₃,Cs₂CO₃) using suitable solvent (1,4-dioxane, 1,2-dimethoxyethane), at a suitable temperature (for example, 110 °C); (Step-2) converted to the corresponding boron derivative (Int-A) using bis(pinacolato)diboron and base (for example, KOAc) and palladium catalyst (for example, Pd(dppf)Cl₂.DCM complex) in presence of inert solvents like dioxane, DMF, THF and the like and at a suitable temperature (for example, 90 to 110 °C). This compound further used for another cross metal coupling reaction using above mentioned conditions.

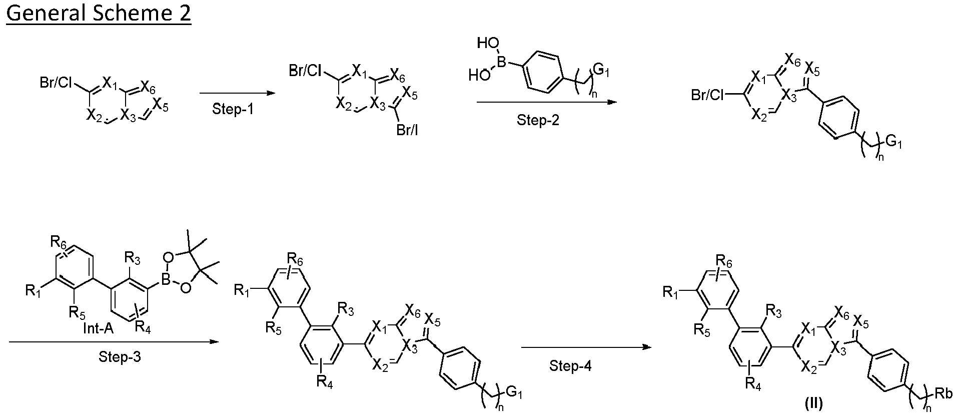

In General Scheme 2, the following reaction conditions typically apply: (Step-1) synthesized using suitable halogenating reagents (for example, NBS or NIS) to make corresponding bromo or lodo derivatives; (Step-2 & Step-3) In the presence of suitable catalyst (for example, Pd(PPh₃)₄, Pd(dppf)Cl₂.DCM complex) with a suitable base (for example, Na₂CO₃,K₂CO₃,) in a suitable solvent (for example 1,4-dioxane, 1,2-dimethoxy ethane) at a suitable temperature (for example, 90 to 110 °C); (Step-4) Compound (II) derivatives prepared by alkylation using suitable base (for example, K₂CO₃) or reductive amination using suitable reducing reagent (for example, NaBH₃CN, NaBH(OAc)₃) or by amide coupling using suitable reagents (T₃P, EDC, HOBt, HATU) in a suitable solvent (for example, DCM or MeOH or DMF or mixture of solvents) at a suitable temperature (for example, 25 °C); and other different sets of reaction conditions dependent on the G₁ for introducing R_{b}.

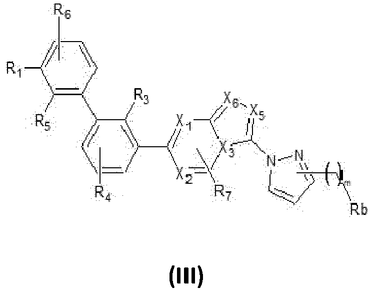

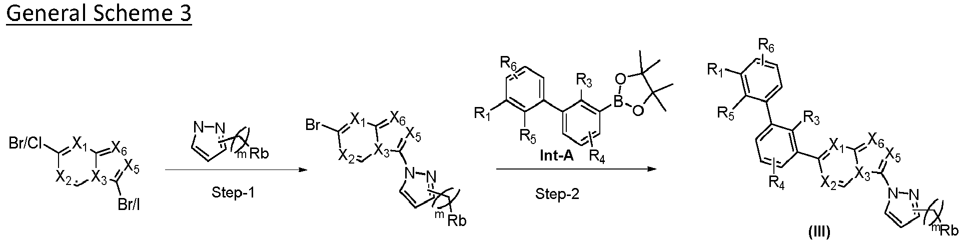

In General Scheme 3, the following reaction conditions typically apply: (Step-1) synthesized by copper-catalysed coupling using suitable reagent (for example, Cul) and suitable ligands (for example, TEMDA) and suitable base (for example, Cs₂CO₃) in a suitable solvent (for example, acetonitrile) at a suitable temperature (for example, 80 °C); (step-2) In the presence of suitable catalyst (for example, Pd(dppf)Cl₂) in a suitable solvent, such as 1,4-dioxane, with a suitable base (for example, Na₂CO₃) at a suitable temperature (for example, 110 °C).

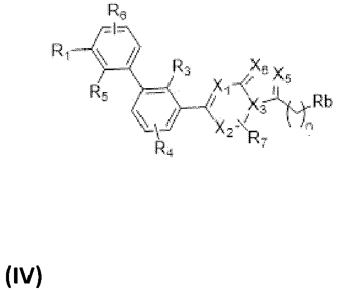

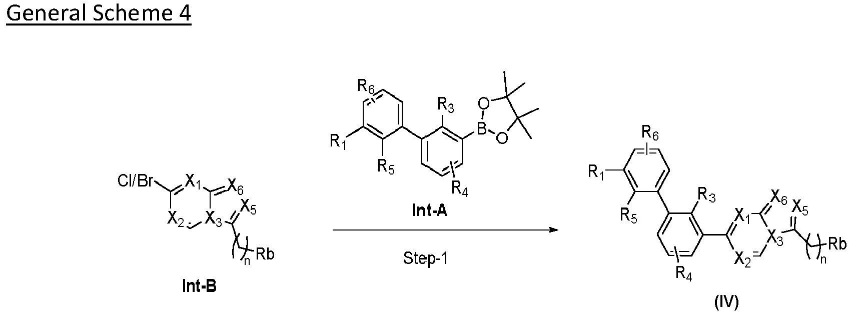

In General Scheme 4, the following reaction conditions typically apply: Int-B prepared from the commercially available material, different sets of reaction conditions used depending on the coupling reagents for introducing R_{b} (for example, alkylation using suitable base like K₂CO₃ or reductive amination using suitable reducing reagents like NaBH₃CN, NaBH(OAc)₃ or by amide coupling using suitable reagents like T₃P, EDC, HOBt, HATU in a suitable solvent like DCM or MeOH or DMF at a suitable temperature. (Step-1) synthesized in the presence of suitable catalyst (for example, Pd(PPh₃)₄, Pd(dppf)Cl₂, Pd(dppf)Cl₂.DCM complex) in a suitable solvent (for example, 1,4-Dioxane, 1,2-Dimethoxy ethane) with a suitable base (for example, Na₂CO₃, K₂CO₃, Cs₂CO₃) at a suitable temperature (for example, 90 or 110 °C).

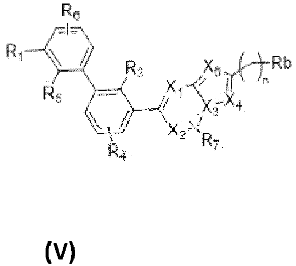

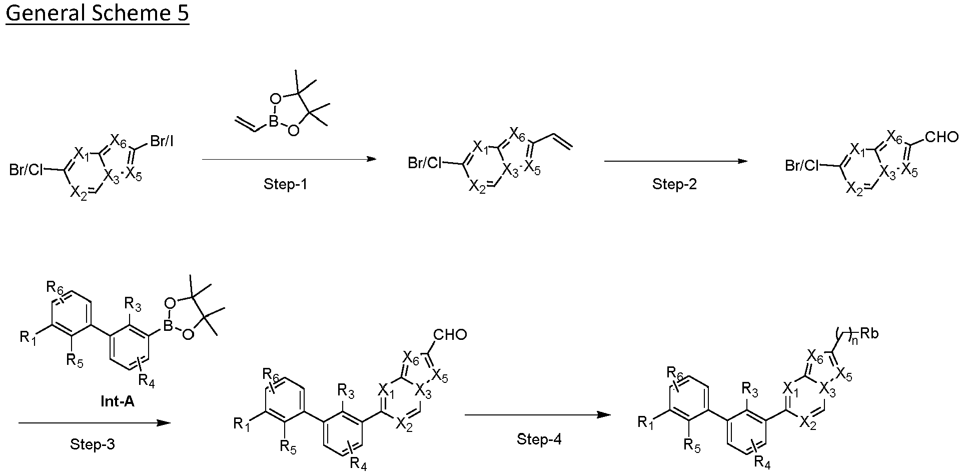

In General Scheme 4, the following reaction conditions typically apply: (Step-1) synthesized by commercially available starting materials by using suitable catalyst (for example, Pd(dppf)Cl₂) with a suitable base (for example, Na₂CO₃) in a suitable solvent (for example 1,4-dioxane) at a suitable temperature (for example, 90 °C); (Step-2) prepared aldehyde using suitable reagents (for example, OSO₄, NaIO₄) or alkyl halide using suitable reagents (for example, OSO₄, NaIO₄, NaBH₄,PBr₃) and other different sets of reaction conditions for introducing Rb.(Step-3) synthesized in the presence of suitable catalyst (for example, Pd(PPh₃)₄, Pd(dppf)Cl₂, Pd(dppf)Cl₂.DCM complex) in a suitable solvent (for example, 1,4-dioxane, 1,2-dimethoxyethane) with a suitable base (for example, Na₂CO₃, K₂CO₃, Cs₂CO₃) at a suitable temperature (for example, 90 or 110 °C).

### EXAMPLES

Compounds of the invention can be prepared using known organic synthesis techniques. The skilled person can easily select the suitable solvents, temperatures and starting materials, as well as the suitable synthetic routes.

### Abbreviations:

ACN - Acetonitrile
AcOH - Acetic acid
BOC₂O - Di-tert-butyl dicarbonate
^{t}BuXphos - 2-Di-tert-butylphosphino-2',4',6'-triisopropylbiphenyl
B₂Pin₂ - 4,4,4',4 ,,5,5,5',5 ,-Octamethyl-2,2'-bi- ,3,2-dioxaborolane
DCM - Dichloromethane
DEAD - Diethyl azodicarboxylate
Dess-martin - Dess-Martin periodinane
DME - 1,2 -Dimethoxyethane
DMF- N, N'-Dimethylformamide
Dioxane - 1,4-Dioxane
EA - Ethyl acetate
EtOH - Ethanol
Ex - Example
FA - Formic acid
h - Hours
HPLC - High performance liquid chromatography
KOAc - Potassium acetate
LAH - Lithium aluminum hydride
LC-MS - Liquid chromatography - Mass spectrometry
MeOH - Methanol
MW - Micro oven
MS - Molecular sieves
NBS - N-Bromosuccinimide
NIS - N-lodosuccinimide NMR - Nuclear Magnetic Resonance
Pd₂(dba)₃ - Tris(dibenzylideneacetone)dipalladium(0)
Pd(PPh₃)₄ - Tetrakis(triphenylphosphine)palladium(0)
PdCl₂(dppf) -[1,1 '-Bis (diphenylphosphino)ferrocene]dichloropalladium(II)
PdCl₂(dppf).dcm - [1,1 '-Bis (diphenylphosphino)ferrocene]dichloropalladium(II) dichloromethane complex
Prep-HPLC - Preparative high pressure liquid chromatography
Py - Pyridine
RT - Room temperature
Sat. - Saturated
SFC - Supercritical fluid chromatography
SM - Starting material
TEA - Triethylamine
TFA - Trifluoroacetic acid
THF - TetrahydrofuranTLC - Thin layer chromatography
TMEDA - Tetramethyl ethylenediamine
TPP-Triphenyl phosphine
T₃P - Propane phosphonic acid anhydride

### General considerations and Analytical Methods:

The compounds used in the reaction processes, if not mentioned otherwise, were commercially available. All compounds were characterized by NMR, HPLC and LC-MS (ESI+APCI). NMR data were obtained on Varian 400 MHz spectrometer, all chemical shifts were reported in parts per million (ppm) and were measured relative to TMS. LCMS (ESI+APCI) measurements were performed on Shimadzu LCMS 2020 with N- Series mass spectrometer. The yields of the compounds provided refer to isolated compounds.

All compounds were purified or enantiomers separation by using one of the below mentioned methods.

### Reverse phase HPLC Purification methods:

1. The crude compound was subjected purification by reverse phase HPLC, Inertsil ODS 3V column (250 x 20) mm, 5.0 µm; mobile phase (A: B), A = 0.1% FA in H₂O, B = ACN 100%.
2. The crude compound was subjected purification by reverse phase HPLC, Inertsil ODS column (250 x 20) mm, 5.0 µm; mobile phase (A: B), A = 0.1% FA in H₂O, B = ACN:MeOH (1:1) 100%.
3. The crude compound was subjected purification by reverse phase HPLC, X-Bridge C18 column (250 x 19) mm, 5.0 µm; mobile phase (A: B), A = 0.1% FA in H₂O, B = ACN 100%.
4. The crude compound was subjected purification by reverse phase HPLC, X-Bridge C18 column (250 x 20) mm, 5.0 µm; mobile phase (A: B), A = 0.1% FA in H₂O, B = ACN:MeOH (1:1) 100%.
5. The crude compound was subjected purification by reverse phase HPLC, Triart C18 column (250 x 21) mm, 5.0 µm; mobile phase (A: B), A = 10 mmol ABC in H₂O, B = ACN:MeOH (1:1) 100%.
6. The crude compound was subjected purification by reverse phase HPLC, X-Bridge C18 column (250 x 20) mm, 5.0 µm; mobile phase (A: B), A = 10 mmol ABC in H₂O, B = ACN:MeOH (1:1) 100%.
7. The crude compound was subjected purification by reverse phase HPLC, X-Bridge C18 column (250 x 20) mm, 5.0 µm; mobile phase (A:B), A = 10 mmol ABC in H₂O, B = ACN 100%.

### SFC Purification methods:

1. The racemic compound was subjected to chiral separation by SFC, dissolved in tetrahydrofuran: methanol (1:1)}, column chiral Pak IG (250 x 21) mm, 5.0 µm; mobile phase 55:45 (A:B).A = Liquid CO₂, B = Acetonitrile: Methanol(1:1), flow rate: 45 mL/min; Wavelength 233 nm.
2. The racemic compound was subjected to chiral separation by SFC, in tetrahydrofuran: methanol (1:1)}, column chiral Pak IC (250 x 21) mm, 5.0 µm; mobile phase 60:40(A:B). A = Liquid CO₂, B = 0.2% triethyl amine in methanol, flow rate: 40 mL/min; Wavelength 254 nm

### Chiral HPLC Purification methods:

1. The racemic compound was subjected to chiral separation by Normal phase HPLC, Column chiral Pak IG (250 * 21) mm, 5.0 µm; mobile phase 60:40 (A: B).A=0.1%TFA in n-Hexane, B= Ethanol(1:1).

### Example 1: 1-(4-(7-(2-methyl-[1,1'-biphenyl]-3-yl)imidazo[1,2-α]pyridin-3-yl)benzyl) piperidine-2-carboxylic acid

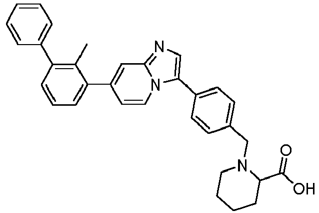

The compound of Example 1 was synthesized via intermediate 4, the route shown in the scheme below,

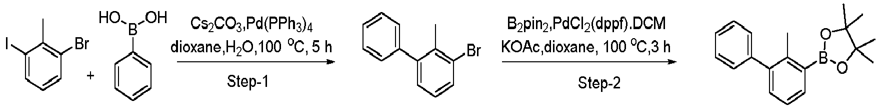

The preparation of Intermediate (4): 4,4,5,5-tetramethyl-2-(2-methyl-[1,1'-biphenyl]-3-yl)-1,3,2-dioxaborolane

### Step-1: Synthesis of 3-bromo-2-methyl-1,1'-biphenyl

A mixture of 1-bromo-3-iodo-2-methylbenzene (4.0 g, 13.4 mmol), phenyl boronic acid (1.72 g, 14.1 mmol), in 1,4-dioxane (20 mL) was added Cs₂CO₃ (10.97 g, 33.6 mmol) in water (4.0 mL), Pd(PPh₃)₄ (467 mg, 0.4 mmol) and mixture was degassed with N₂ for 30 minutes then heated the reaction mixture at 100 °C for 5 h. The reaction mixture was cooled down to room temperature, diluted with EA (10 mL) and water (10 mL). Organic layer was separated, and aqueous layer was extracted with ethyl acetate (3 x 30 mL). The combined organic layer was washed with brine (20 mL), dried over Na₂SO₄, and concentrated under vacuo to obtain a crude product, which was purified by Biotage flash column chromatography using 0-10% ethyl acetate in hexane as an eluent to obtain the title compound (1.5 g, 45%) as a colorless liquid. LC-MS calculated for C32H29N3O2 (M+H)+: m/z = 487.6; found: 488.2.

### Step-2: Synthesis of 4,4,5,5-tetramethyl-2-(2-methyl-[1,1'-biphenyl]-3-yl)-1,3,2-dioxaborolane

A mixture of 3-bromo-2-methyl-1,1'-biphenyl (1.5 g, 6.0 mmol), B₂Pin₂(pinacolato)diborane (1.84 g, 7.2 mmol), in 1,4-dioxane (15 mL) was added KOAc (1.48 g, 15.1 mmol), Pd(dppf)Cl₂.DCM (247 mg, 0.3 mmol) and the mixture was degassed with N₂ for 30 min, then heated the reaction mixture at 100 °C for 3 h. The reaction mixture was cooled down to room temperature, water was added to the reaction mixture and extracted with EA (3 x 25 mL). The combined organic layer was washed with brine (20 mL), dried over Na₂SO₄ and concentrated under vacuo to obtain a crude. Crude material was purified by Biotage flash column chromatography using 0-10% ethyl acetate in hexane to obtain the title compound (1.0 g, 56 %) as an off-white solid and used in next step as such.

The preparation of Example 1: 1-(4-(7-(2-methyl-[1,1'-biphenyl]-3-yl)imidazo[1,2-*a*]pyridin-3-yl)benzyl) piperidine-2-carboxylic acid

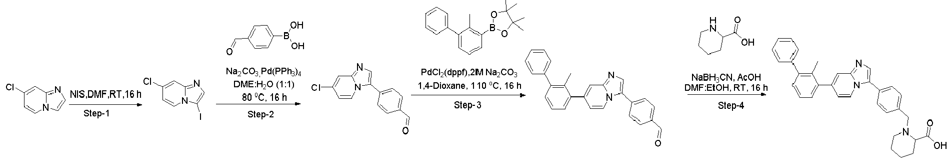

### Step-1: Synthesis of 7-chloro-3-iodoimidazo[1,2-α]pyridine

To a stirred solution of 7-chloroimidazo[1,2-a]pyridine (5.0 g, 32.7 mmol) in DMF (50 mL) was added NIS (7.74g, 34.4 mmol) in portions and stirred the reaction mixture at room temperature for 16 h. The reaction mixture was slowly poured into ice cold water with vigorous stirring. Solid material precipitated out was filtered off, washed with water, dried in vacuum to obtain the title compound (8.8 g, 96%) as a pale-yellow solid. Crude compound was taken in next step without purification.

### Step2: Synthesis of 4-(7-chloroimidazo[1,2-α]pyridin-3-yl)benzaldehyde

A mixture of 7-chloro-3-iodoimidazo[1,2-a]pyridine (2.59 g, 9.8 mmol), (4-formylphenyl) boronic acid (1.67 g, 11.1 mmol), Na₂CO₃ (6.89 g, 6.51 mmol), Pd(PPh₃)₄ (538 mg, 0.04 mmol), water (65 mL) and DME (130 mL) and the mixture was degassed with N₂ for 30 minutes and stirred the reaction mixture at 80 °C for 16 h. The reaction mixture was cooled down to room temperature, diluted with EA. The organic layer was separated, and aqueous layer was extracted with EA (2 x 25 mL). The combined organic layer was washed with brine (50 mL), dried over Na₂SO₄ and concentrated under vacuo to obtain a crude. The crude compound was purified by combi flash column chromatography using 10-50 % ethyl acetate in hexane as a mobile phase to obtain the title compound (2.0 g, 84%) as an off-white solid.

### Step-3: Synthesis of 4-(7-(2-methyl-[1,1'-biphenyl]-3-yl)imidazo[1,2-α]pyridin-3-yl)benzaldehyde

To a stirred solution 4-(7-chloroimidazo[1,2-a]pyridin-3-yl)benzaldehyde (440 mg, 1.74 mmol) and 4,4,5,5-tetramethyl-2-(2-methyl-[1,1'-biphenyl]-3-yl)-1,3,2-dioxaborolane (900 mg, 3.08 mmol), in 1,4-dioxane (10 mL) was added Na₂CO₃ (545 mg, 5.14 mmol) in water (2.5 mL), Pd(dppf)Cl₂ (62 mg, 0.085 mmol) and the mixture was degassed with N₂ for 30 min then heated the reaction mixture at 110 °C for 16 h. Then cooled down to room temperature, EA (20 mL) and water (20 mL) were added to the mixture, separated, the aqueous layer extracted with EA (3 x 20 mL). The combined organic layer was dried over Na₂SO₄, filtered, and evaporated. Crude obtained was purified by combiflash column chromatography using (5-30%) ethyl acetate in hexane to obtain the title compound (470 mg, 70%) as an off-white solid.

### Step-4: Synthesis of 1-(4-(7-(2-methyl-[1,1'-biphenyl]-3-yl)imidazo[1,2-α]pyridin-3-yl)benzyl)piperidine-2-carboxylic acid

A mixture of 4-(7-(2-methyl-[1,1'-biphenyl]-3-yl)imidazo[1,2-a]pyridin-3-yl)benzaldehyde (200 mg, 0.51 mmol), piperidine-2-carboxylic acid (51.2 mg, 0.44 mmol) and one drop of AcOH in ethanol and DMF (1:1) (4.0 mL) was stirred at room temperature for 3 h. NaBH₃CN (97 mg, 1.54 mmol) was added to the reaction mixture and continued the stirring at room temperature for 16 h. Organic volatiles were removed under reduce pressure and the residue dissolved in water and extracted with DCM (3 x 20 mL). Combined organic layer was washed with brine, dried over Na₂SO₄, filtered, and evaporated the solvent under reduced pressure. The obtained crude material was purified by reverse phase HPLC (X-Bridge C18 column (250 x 19) mm, 5.0 µm; mobile phase (A: B), A = 0.1% FA in B = ACN 100%) to obtain the title compound 1, (20 mg, 8 %) as a pale-yellow solid. **¹H NMR (400 MHz, DMSO-d₆) *δ*:** 8.61 (d, *J =*7.2 Hz 1H), 7.82 (s, 1H), 7.66 (t, *J* = 8Hz, 3H), 7.53 (d, *J* = 8 Hz, 2H), 7.47(t, *J* = 7.6Hz, 2H), 7.42-7.35(m, 4H), 7.27(d, *J* = 5.6Hz, 1H), 7.04(d, *J* = 7.2Hz, 1H), 3.94(d *,J* = 13.6Hz,, 1H),3.57 (d, *J* = 13.6 Hz, 1H), 3.15-3.13 (m, 1H), 2.91(m, 1H), 2.32(m, 1H),2.17(s,3H), 1.51(m,3H) , LC-MS calculated for C33H31N3O2 (M+H)+: m/z = 501.63; found: 502.25.

The racemic compound 1 was subjected to chiral separation by Normal phase HPLC, Column chiral Pak IG (250 * 21) mm, 5.0 µm; mobile phase 60:40 (A:B). A=0.1%TFA in n-Hexane, B= Ethanol(1:1), to afforded Peak 1 and Peak 2.

Peak 1: **¹H NMR (400 MHz, DMSO-*d*₆) *δ*:** 8.65 (d, *J* = 7.2 Hz, 1H), 7.92 (s, 1H), 7.76 (d, *J* = 7.2 Hz, 2H), 7.69 (s, 1H), 7.59 (d, *J* = 7.2 Hz, 2H), 7.49-7.34 (m, 7H), 7.29 (d, *J* = 7.2 Hz, 1H), 7.12 (d, *J* = 6.8 Hz, 1H), 4.22 (m,1H), 3.91 (m, 1H), 2.67 (s, 1H), 2.3 (d, *J* = 7.6 Hz, 1H), 2.17 (s, 3H), 2.0-1.98 (m, 2H), 1.77 (m, 1H), 1.62-1.47 (m, 4H).

LC-MS calculated for C33H31N3O2 (M+H)+: m/z = 501.63; found: 502.2

Peak 2: **¹H NMR (400 MHz, DMSO-*d*₆) *δ*:** 8.65(d, J = 7.2 Hz, 1H), 7.92 (s, 1H), 7.76 (d, J = 7.2 Hz, 2H), 7.69 (s, 1H), 7.50-7.53 (m, 2H), 7.81(t, J = 7.6 Hz, 2H), 7.42-7.34 (m, 5H), 7.28(d, J = 7.2 Hz, 1H), 7.13-7.08 (m, 1H), 4.17 (s, 3H), 3.78 (d, J =12.8Hz, 1H), 3.11-3.09 (m, 1H), 2.63-2.58 (m, 1H), 2.17-2.10 (m, 4H), 1.90-1.73 (m,3H), 1.47 (m, 1H).

LC-MS calculated for C33H31N3O2 (M+H)+: m/z = 501.63; found: 502.2

Following Examples 2 to 14 as shown in Table 1 were prepared according to similar procedure described for Example 1 with appropriate amines. Example 15 in Table 1 was prepared according to similar procedure described for Example 52 with appropriate amines:

**Table 1:**

| **Ex.No** | **Structure IUPAC Name** | **LC-MS(ESI)m/z:** |
|---|---|---|
| 2 | 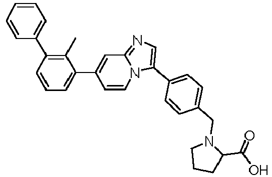 (4-(7-(2-methyl-[1,1'-biphenyl]-3-yl)imidazo[1,2-*a*]pyridin-3-yl)benzyl)proline | LC-MS calculated for C32H29N3O2 (M+H)+: m/z = 487.6; found: 488.2 |
| 3 | 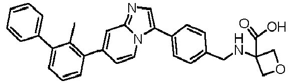 3-((4-(7-(2-methyl-[1,1'-biphenyl]-3-yl)imidazo[1,2-*α*]pyridin-3-yl)benzyl)amino)oxetane-3-carboxylic acid | LC-MS calculated for C31H27N3O3 (M+H)+: m/z = 489.58; found: 490.3 |
| 4 | 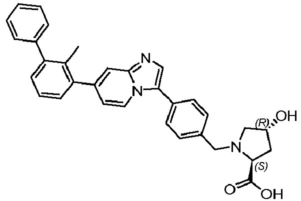 (25,4R)-4-hydroxy-1-(4-(7-(2-methyl-[1,1'-biphenyl]-3-yl)imidazo[1,2-*α*]pyridin-3-yl)benzyl)pyrrolidine-2-carboxylic acid | LC-MS calculated for C32H29N3O3 (M+H)+: m/z = 503.6; found: 504.6 |
| 5 | 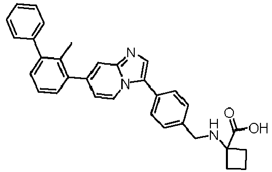 1-((4-(7-(2-methyl-[1,1'-biphenyl]-3-yl)imidazo[1,2-o]pyridin-3-yl)benzyl)amino)cyclobutane-1-carboxylic acid | LC-MS calculated for C32H29N3O2 (M+H)+: m/z = 487.60; found: 488.3 |
| 6 | 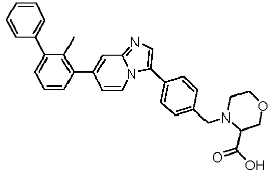 4-(4-(7-(2-methyl-[1,1'-biphenyl]-3-yl)imidazo[1,2-*a*]pyridin-3-yl)benzyl)morpholine-3-carboxylic acid | LC-MS calculated for C32H29N3O3 (M+H)+: m/z = 503.6; found: 488.2 |
| 7 | 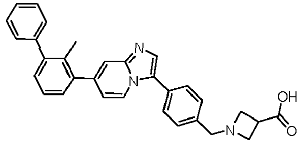 1-(4-(7-(2-methyl-[1,1'-biphenyl]-3-yl)imidazo[1,2-*α*]pyridin-3-yl)benzyl)azetidine-3-carboxylic acid | LC-MS calculated for C31H27N3O2 (M+H)+: m/z = 473.58; found: 474.5 |
| 8 | 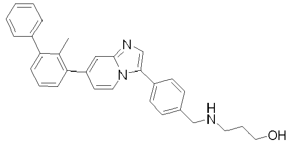 3-((4-(7-(2-methyl-[1,1'-biphenyl]-3-yl)imidazo[1,2-*α*]pyridin-3-yl)benzyl)amino)propan-1-ol | LC-MS calculated for C30H29N3O (M+H)+: m/z = 447.58; found: 448.4 |
| 9 | 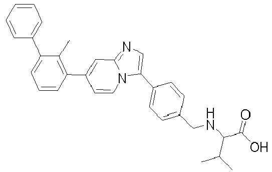 (4-(7-(2-methyl-[1,1'-biphenyl]-3-yl)imidazo[1,2-*a*]pyridin-3-yl)benzyl)valine | LC-MS calculated for C32H31N3O2 (M+H)+: m/z = 489.62; found: 490.4 |
| 10 | 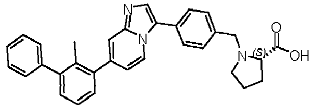 (4-(7-(2-methyl-[1,1'-biphenyl]-3-yl)imidazo[1,2-*a*]pyridin-3-yl)benzyl)-*L-*proline | LC-MS calculated for C32H29N3O2 (M+H)+: m/z = 487.6; found: 488.4 |
| 11 | 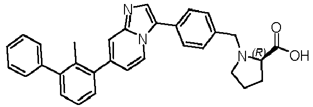 (4-(7-(2-methyl-[1,1'-biphenyl]-3-yl)imidazo[1,2-*a*]pyridin-3-yl)benzyl)-*D-*proline | LC-MS calculated for C32H29N3O2 (M+H)+: m/z = 487.6; found: 488.4 |
| 12 | 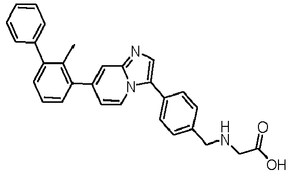 (4-(7-(2-methyl-[1,1'-biphenyl]-3-yl)imidazo[1,2-a]pyridin-3-yl)benzyl)glycine | LC-MS calculated for C29H25N3O2 (M+H)+: m/z = 447.54; found: 448.2 |
| 13 | 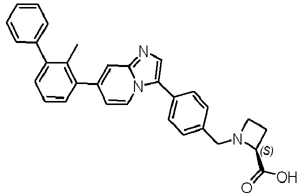 (*S*)-1-(4-(7-(2-methyl-[1,1'-biphenyl]-3-yl)imidazo[1,2-*α*]pyridin-3-yl)benzyl)azetidine-2-carboxylic acid | LC-MS calculated for C31H27N3O2 (M+H)+: m/z = 473.58; found: 474.3 |
| 14 | 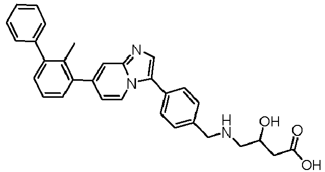 3-hydroxy-4-((4-(7-(2-methyl-[1,1'-biphenyl]-3-yl)imidazo[1,2-*α*]pyridin-3-yl)benzyl)amino)butanoic acid | LC-MS calculated for C31H29N3O3 (M+H)+: m/z = 491.59; found: 492.3 |
| 15 | 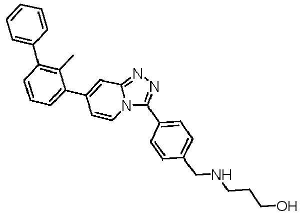 3-((4-(7-(2-methyl-[1,1'-biphenyl]-3-yl)-[1,2,4]triazolo[4,3-*a*]pyridin-3-yl)benzyl)amino)propan-1-ol | LC-MS calculated for C29H28N4O (M+H)+: m/z = 448.57; found: 449.4 |
| 16 | 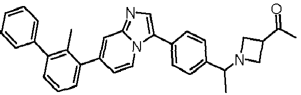 1-(1-(1-(4-(7-(2-methyl-[1,1'-biphenyl]-3-yl)imidazo[1,2-*α*]pyridin-3-yl)phenyl)ethyl)azetidin-3-yl)ethan-1-one | LC-MS calculated for C33H31N3O (M+H)+: m/z = 485.63; found: 488.2 |
| 17 | 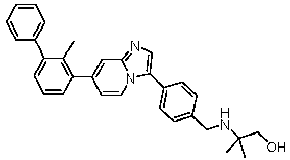 2-methyl-2-((4-(7-(2-methyl-[1,1'-biphenyl]-3-yl)imidazo[1,2-*α*]pyridin-3-yl)benzyl)amino)propan-1-ol | LC-MS calculated for C33H33N3O5 (M+H)+: m/z = 551.64; found: 552.3 |
| 18 | 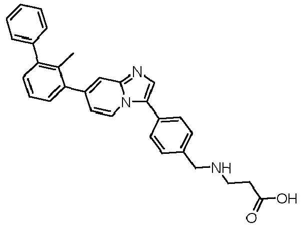 3-((4-(7-(2-methyl-[1,1'-biphenyl]-3-yl)imidazo[1,2-*α*]pyridin-3-yl)benzyl)amino)propanoic acid | LC-MS calculated for C30H27N3O2 (M+H)+: m/z = 461.57; found: 462.4 |
| 19 | 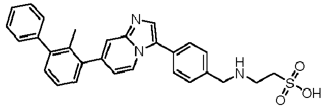 2-((4-(7-(2-methyl-[1,1'-biphenyl]-3-yl)imidazo[1,2-*α*]pyridin-3-yl)benzyl)amino)ethane-1-sulfonic acid | LC-MS calculated for C29H29N3O3S (M+H)+: m/z = 497.6; found: 498.4 |

### Example 20: 1-(2,6-dimethoxy-4-(7-(2-methyl-[1,1'-biphenyl]-3-yl)imidazo[1,2-α]pyridin-3-yl)benzyl)piperidine-2-carboxylic acid

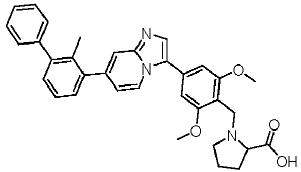

The compound of Example 20 was synthesized via the route shown in the scheme below,

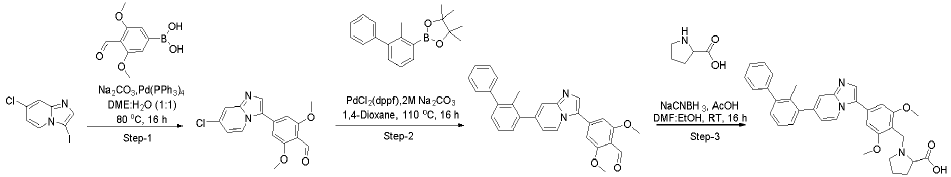

This was prepared in the same manner as the procedure described for Example 1 (Step 1 and step 2)

### Step-3: Synthesis of (2,6-dimethoxy-4-(7-(2-methyl-[1,1'-biphenyl]-3-yl)imidazo[1,2-a]pyridin-3-yl)benzyl)proline

To a stirred solution of 2,6-dimethoxy-4-(7-(2-methyl-[1,1'-biphenyl]-3-yl)imidazo[1,2-a]pyridin-3-yl)benzaldehyde (150 mg, 0.44 mmol) in ethanol and DMF (1:1) (3.0 mL) was added proline 7 (51.2 mg, 0.44 mmol) and acetic acid (1 drop) and stirred the reaction at room temperature for 3 hours. To this reaction mixture NaCNBH3 (83.8 mg, 1.33 mmol) was added and continued the stirring at room temperature for 16 h. Completion of the reaction confirmed by TLC. The organic volatiles were evaporated under vacuo to obtain a crude residue and the residue dissolved in water and extracted with DCM (3 x 10 mL) and the combined organic layer was washed with water, dried over Na₂SO₄, filtered and evaporated. The crude material was purified by preparative HPLC to obtain the title compound (30 mg, 15 %) as pale-yellow solid.

**¹H NMR (400 MHz, DMSO-*d*₆) *δ*:** 8.7 (d, *J* = 6.8 Hz, 1H), 7.94 (s, 1H), 7.66 (s, 1H), 7.27-7.49 (m, 8H), 7.08-7.04 (m, 3H), 4.3 (s, 2H), 3.93 (s, 6H), 3.79 (s, 1H), 3.45(s,1H), 2.98 (s, 3H), 2.27-2.32 (m, 1H), 2.18 (s, 3H), 1.95 (s, 2H), 1.76 (s, 1H). LC-MS calculated for C34H33N3O4 (M+H)+: m/z = 547.66; found: 548.5

Chiral Separation:
The racemic compound was subjected to chiral separation by Normal phase HPLC, Column chiral Pak IG (250 ^{∗} 21) mm, 5.0 µm; mobile phase 60:40 (A:B).A=0.1%TFA in n-Hexane, B= Ethanol(1:1), to afforded Peak 1 and Peak 2.

Peak 1: **¹H NMR (400 MHz, DMSO-*d*₆)** 8.7 (d, *J* = 6.8 Hz, 1H), 7.94 (s, 1H), 7.66 (s, 1H), 7.27-7.49 (m, 8H), 7.08-7.04 (m, 3H), 4.3 (s, 2H), 3.93 (s, 6H), 3.79 (s, 1H), 3.45(s,1H), 2.98 (s, 3H), 2.27-2.32 (m, 1H), 2.18 (s, 3H), 1.95 (s, 2H), 1.76 (s, 1H).

LC-MS calculated for C33H31N3O2 (M+H)+: m/z = 547.66.63; found: 548.5

Peak 2: **¹H NMR (400 MHz, DMSO-*d*₆) *δ*:** 8.7 (d, *J* = 7.6 Hz, 1H), 7.92 (s, 1H), 7.65 (s, 1H), 7.35-7.47 (m, 7H), 7.27 (d, *J* = 7.2 Hz, 1H), 7.06 (d, *J* = 7.26 Hz, 1H), 7.01 (s, 2H), 4.2 (s, 2H), 3.92 (s, 6H), 3.48 (s, 2H), 2.83 (s, 1H), 2.18 (s, 4H), 1.97(s, 1H), 1.83 (s, 1H), 1.67 (s, 1H).

LC-MS calculated for C34H33N3O4 (M+H)+: m/z = 547.66; found: 548.4

Following examples as shown in Table 2 were prepared according to similar procedure described for Example 20 with appropriate amines:

**Table 2:**

| **Ex.No** | **Structure IUPAC Name** | **LC-MS(ESI)m/z: (M+1)** |
|---|---|---|
| 21 | 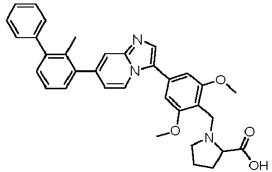 (2,6-dimethoxy-4-(7-(2-methyl-[1,1'-biphe nyl]-3-yl)imidazo[1,2-*α*]pyridin-3-yl)benzyl)proline | LC-MS calculated for C34H33N3O4 (M+H)+: m/z = 547.66; found: 548.4 |
| 22 | 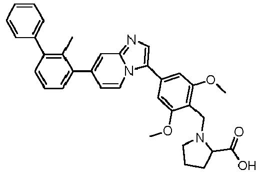 (2,6-dimethoxy-4-(7-(2-methyl-[1,1'-biphenyl]-3-yl)imidazo[1,2-*α*]pyridin-3-yl)benzyl)proline | LC-MS calculated for C34H33N3O4 (M+H)+: m/z = 547.66; found: 548.4 |

### Example 23: (R)-(1-(4-(7-(2-methyl-[1,1'-biphenyl]-3-yl)imidazo[1,2-α]pyridin-3-yl)benzyl)pyrrolidin-2-yl)methanol

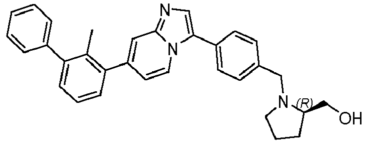

The compound of **Example 23** was synthesized via the route shown in the scheme below,

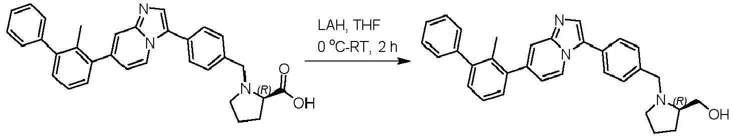

To a solution of (4-(7-(2-methyl-[1,1'-biphenyl]-3-yl)imidazo[1,2-*a*]pyridin-3-yl)benzyl)-D-proline (1 g, 23 mmol) in THF (20 mL) was added LAH (180 mg, 47.3 mmol) at 0 °C under nitrogen atmosphere. After further stirring at room temperature for 2 h, the reaction mixture was quenched with 1N NaOH, filtered, washed with ethyl acetate, and dried over Na₂SO₄ and concentrated to give title compound (450 mg, 50%) as a yellow solid.

**¹H NMR (400 MHz, DMSO-*d*₆) *δ*:** 8.59 (d, *J* = 7.2 Hz, 1H), 7.8 (s, 1H), 7.64 (d, *J* = 8.0 Hz, 3H), 7.51-7.45 (m, 4H), 7.42-7.34 (m, 5H), 7.27 (dd, *J* = 6.8 Hz, 1H), 7.04 (dd, *J* = 7.2 Hz, 1H), 4.46 (s, 1H), 4.13 (d, *J* = 13.2 Hz, 1H), 3.49 (d, *J* = 7.6 Hz, 1H), 3.41 (d, 2H), 2.83 (t, *J* = 2.4 Hz, 1H), 2.67-2.57 (m, 2H), 2.2 (s, 3H), 1.87 (q, *J* = 8.4 Hz, 1H), 1.64-1.56 (m, 3H). LC-MS calculated for C32H31N3O (M+H)+: m/z = 473.62; found: 474.4

Following examples as shown in Table 3 were prepared according to similar procedure described for Example 23 with appropriate amines:

**Table 3:**

| **Ex.No** | **Structure IUPAC Name** | **LC-MS(ESI)m/z:** |
|---|---|---|
| 24 | 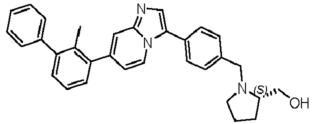 (*S*)-(1-(4-(7-(2-methyl-[1,1'-biphenyl]-3-yl)imidazo[1,2-*α*]pyridin-3-yl)benzyl)pyrrolidin-2-yl)methanol | LC-MS calculated for C32H31N3O (M+H)+: m/z = 473.62; found: 474.2 |

### Example 25: 1-(4-(7-(3-(2,3-dihydrobenzo[b][1,4]dioxin-6-yl)-2-methylphenyl)imidazo[1,2-α]pyridin-3-yl)benzyl)azetidine-3-carboxylic acid

The compound of Example 25 was synthesized via the route shown in the scheme below,

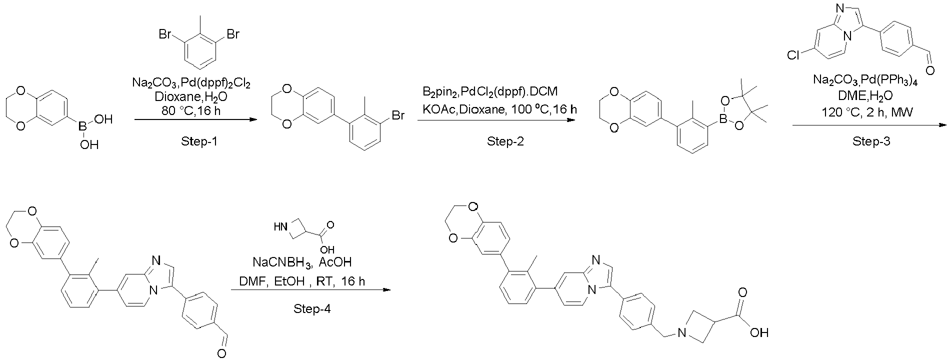

### Step-1: Synthesis of 6-(3-bromo-2-methylphenyl)-2,3-dihydrobenzo[b][1,4]dioxine

The title compound was prepared in a similar way as Example 1, to give the title compound (5.2 g, 56%) as a viscous liquid.

### Step-2: Synthesis of 2-(3-(2,3-dihydrobenzo[b][1,4]dioxin-6-yl)-2-methylphenyl)-4,4,5,5-tetramethyl-1,3,2-dioxaborolane

The title compound was prepared in a similar way as Example 1 to give the title compound (5.2 g, 60%) as a white solid.

### Step-3: Synthesis of 4-(7-(3-(2,3-dihydrobenzo[b][1,4]dioxin-6-yl)-2-methylphenyl) imidazo[1,2-a]pyridin-3-yl)benzaldehyde

The title compound was prepared in a similar way as Example 1 to give the title compound (1.87 g, 90%) as a pale-yellow solid. LC-MS calculated for C29H22N2O3 (M+H)+: m/z = 446.51; found: 447.25.

### Step-4 : Synthesis of 1-(4-(7-(3-(2,3-dihydrobenzo[b][1,4]dioxin-6-yl)-2-methylphenyl)imidazo[1,2-α]pyridin-3-yl)benzyl) azetidine-3-carboxylic acid

The title compound was prepared in a similar way as Example 1 the crude material was purified by prep-HPLC to give the title compound (45 mg, 29%as a white solid.

### ¹H NMR (400 MHz, DMSO-d₆) δ:

8.58 (d, J = 6.8 Hz, 1H), 7.80 (s, 1H), 7.63 (t, J = 7.6 Hz, 3H), 7.45 (d, J = 6.8 Hz, 2H), 7.33-7.31 (m, 2H), 7.24 (m, 1H), 7.03 (d, J = 6 Hz, 1H), 6.94-6.84 (m, 3H), 4.28 (s, 4H), 3.62 (s, 2H), 3.5 (m, 1H), 3.42 (s, 2H), 3.23 (s, 2H), 2.17 (s, 3H). LC-MS calculated for C33H28N3O4 (M+H)+: m/z = 531.61; found: 532.2

Following examples as shown in Table 4 were prepared according to similar procedure described for Example 25 with appropriate amines:

**Table 4:**

| **Ex.No** | **Structure IUPAC Name** | **LC-MS(ESI)m/z:** |
|---|---|---|
| 26 | 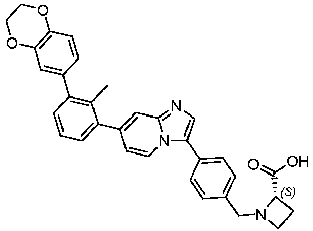 (*S*)-1-(4-(7-(3-(2,3-dihydrobenzo[*b*][1,4]dioxin-6-yl)-2-methylphenyl)imidazo[1,2-*α*]pyridin-3-yl)benzyl)azetidine-2-carboxylic acid | LC-MS calculated for C33H29N3O4 (M+H)+: m/z = 531.61; found: 532.2 |
| 27 | 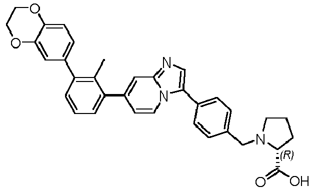 (4-(7-(3-(2,3-dihydrobenzo[*b*][1,4]dioxin-6-yl)-2-methylphenyl)imidazo[1,2-*a*]pyridin-3-yl)benzyl)-*D-*proline | LC-MS calculated for C34H31N3O4 (M+H)+: m/z = 545.64; found: 546.4 |
| 28 | 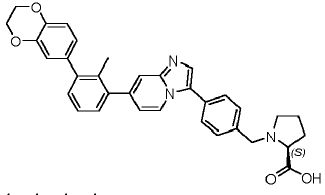 (4-(7-(3-(2,3-dihydrobenzo[*b*][1,4]dioxin-6-yl)-2-methylphenyl)imidazo[1,2-*α*]pyridin-3-yl)benzyl)-*L-*proline | LC-MS calculated for C34H31N3O4 (M+H)+: m/z = 545.64; found: 546.4 |
| 29 | 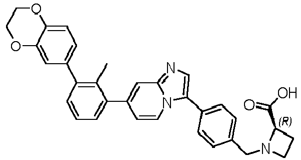 (R)-1-(4-(7-(3-(2,3-dihydrobenzo[*b*][1,4]dioxin-6-yl)-2-methylphenyl)imidazo[1,2-*α*]pyridin-3-yl)benzyl)azetidine-2-carboxylic acid | LC-MS calculated for C33H29N3O4 (M+H)+: m/z = 531.61; found: 532.2 |
| 30 | 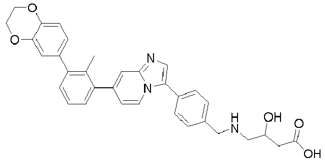 4-((4-(7-(3-(2,3-dihydrobenzo[*b*][1,4]dioxin-6-yl)-2-methylphenyl)imidazo[1,2-*a*]pyridin-3-yl)benzyl)amino)-3-hydroxybutanoic acid | LC-MS calculated for C33H31N3O8 (M+H)+: m/z = 549.63; found: 550.2 |
| 31 | 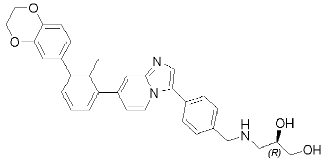 (*R*)-3-((4-(7-(3-(2,3-dihydrobenzo[*b*][1,4]dioxin-6-yl)-2-methylphenyl)imidazo[1,2-*α*]pyridin-3-yl)benzyl)amino)propane-1,2-diol | LC-MS calculated for C32H31N3O4 (M+H)+: m/z = 521.62; found: 522.25 |
| 32 | 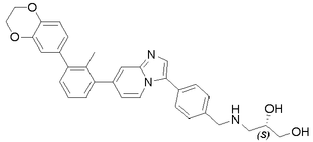 (*S*)-3-((4-(7-(3-(2,3-dihydrobenzo[*b*][1,4]dioxin-6-yl)-2-methylphenyl)imidazo[1,2-*α*]pyridin-3-yl)benzyl)amino)propane-1,2-diol | LC-MS calculated for C32H31N3O4 (M+H)+: m/z = 521.62; found: 522.4 |
| 33 Racemic | 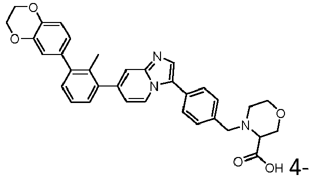 (4-(7-(3-(2,3-dihydrobenzo[*b*][1,4]dioxin-6-yl)-2-methylphenyl)imidazo[1,2-*α*]pyridin-3-yl)benzyl)morpholine-3-carboxylic acid | LC-MS calculated for C34H31N3O5 (M+H)+: m/z = 561.64; found: 562.4 |
| 34 Peak 1 | 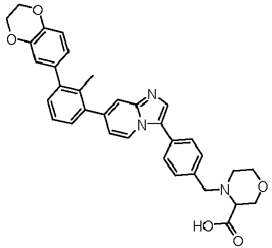 4-(4-(7-(3-(2,3-dihydrobenzo[*b*][1,4]dioxin-6-yl)-2-methylphenyl)imidazo[1,2-*α*]pyridin-3-yl)benzyl)morpholine-3-carboxylic acid | LC-MS calculated for C34H31N3O5 (M+H)+: m/z = 561.64; found: 562.4 |
| 35 Peak 2 Chirally Pure | 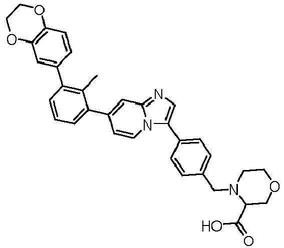 4-(4-(7-(3-(2,3-dihydrobenzo[*b*][1,4]dioxin-6-yl)-2-methylphenyl)imidazo[1,2-*α*]pyridin-3-yl)benzyl)morpholine-3-carboxylic acid | LC-MS calculated for C34H31N3O5 (M+H)+: m/z = 561.64; found: 562.4 |
| 36 | 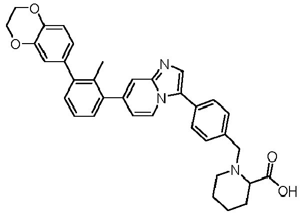 1-(4-(7-(3-(2,3-dihydrobenzo[*b*][1,4]dioxin-6-yl)-2-methylphenyl)imidazo[1,2-*α*]pyridin-3-yl)benzyl)piperidine-2-carboxylic acid | LC-MS calculated for C35H33N3O4 (M+H)+: m/z = 559.67; found: 560.4 |
| 37 | 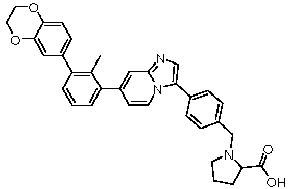 (4-(7-(3-(2,3-dihydrobenzo[*b*][1,4]dioxin-6-yl)-2-methylphenyl)imidazo[1,2-*a*]pyridin-3-yl)benzyl)proline | LC-MS calculated for C34H31N3O4 (M+H)+: m/z = 545.64; found: 546.45 |
| 38 | 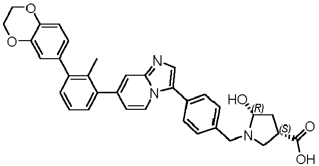 (3*S*,5*R*)-1-(4-(7-(3-(2,3-dihydrobenzo[*b*][1,4]dioxin-6-yl)-2-methylphenyl)imidazo[1,2-*a*]pyridin-3-yl)benzyl)-5-hydroxypyrrolidine-3-carboxylic acid | LC-MS calculated for C34H31N3O5 (M+H)+: m/z = 561.64; found: 562.4 |

### Example 39: ((4-(7-(3-(2,3-dihydrobenzo[b][1,4]dioxin-6-yl)-2-methylphenyl)imidazo[1,2-α]pyridin-3-yl)-2,6-dimethoxybenzyl)-D-proline

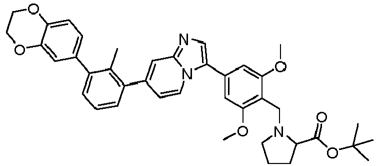

The compound of Example 39 was synthesized via the route shown in the scheme below,

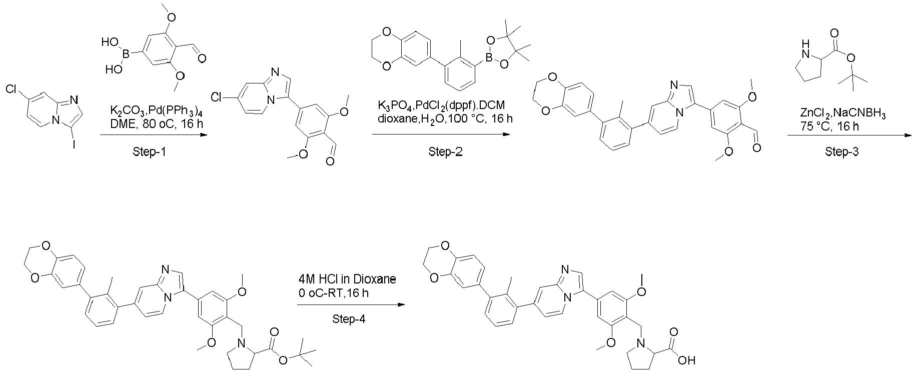

### Step-1: Synthesis of 4-(7-chloroimidazo[1,2-a]pyridin-3-yl)-2,6-dimethoxybenzaldehyde

This was prepared in the same manner as the procedure described for Example 1 to obtain the title compound (1.15 g, 37 %) as a white solid. LC-MS calculated for C16H13CIN2O3 (M+H)+: m/z = 316.74; found: 317.25

### Step-2: Synthesis of 4-(7-(3-(2,3-dihydrobenzo[b][1,4]dioxin-6-yl)-2-methylphenyl)imidazo[1,2-α]pyridin-3-yl)-2,6-dimethoxybenzaldehyde.

This was prepared in the same manner as the procedure described for Example 1 to obtain the title compound (1 g, 100 %) as a white solid. LC-MS calculated for C31H26N2O5 (M+H)+: m/z = 506.56; found: 507.25.

### Step-3: Synthesis of tert-butyl (4-(7-(3-(2,3-dihydrobenzo[b][1,4]dioxin-6-yl)-2-methylphenyl)imidazo[1,2-α]pyridin-3-yl)-2,6-dimethoxybenzyl)prolinate

This was prepared in the same manner as the procedure described for Example 1 to obtain the title compound (250 mg) as a brown sticky mass as crude, which has used for next step without purification. LC-MS calculated for C40H43N3O6 (M+H)+: m/z = 661.8; found: 662.5

### Step-4: Synthesis of (4-(7-(3-(2,3-dihydrobenzo[b][1,4]dioxin-6-yl)-2-methylphenyl)imidazo[1,2-α]pyridin-3-yl)-2,6-dimethoxybenzyl)proline

To a stirred solution of tert-butyl (4-(7-(3-(2,3-dihydrobenzo[*b*][1,4]dioxin-6-yl)-2-methylphenyl)imidazo[1,2-*a*]pyridin-3-yl)-2,6-dimethoxybenzyl)prolinate (250 mg, 377 mmol) in DCM (5 mL) in Dioxane (2 mL), was added 4M HCl in Dioxane (5 mL) at 0 °C. The reaction mixture then stirred at room temperature for 16 h. The organic volatiles were evaporated to get the crude which was purified by prep-HPLC (X-Bridge C18 column (250 x 19) mm, 5.0 µm; mobile phase (A: B), A = 0.1% FA in B = ACN 100%.) to obtain the title compound (55 mg, 24 %) as a white solid.

**¹H NMR (400 MHz, DMSO-*d*₆) *δ*:** 8.73 (d, *J* = 6.8 Hz, 1H), 7.93 (s, 1H), 7.64 (s, 1H), 7.32 (dd, *J* = 7.6 Hz, 2H), 7.24 (d, *J* = 15.6 Hz, 1H), 7.06-7.02 (m, 3H), 6.94-6.84 (m, 3H), 4.28 (s, 4H), 4.23 (s, 2H), 3.93 (s, 6H), 3.56 (d, *J* = 5.2 Hz, 2H), 2.87 (d, *J* = 6.8 Hz, 2H), 2.18 (s, 3H), 1.99-1.87 (m, 2H), 1.67 (s, 1H). LC-MS calculated for C36H35N3O8 (M+H)+: m/z = 605.69; found: 606.4.

Following example as shown in Table 5 was prepared according to similar procedure described for Example 39 with appropriate amines:

**Table 5:**

| **Ex.No** | **Structure IUPAC Name** | **LC-MS(ESI)m/z:** |
|---|---|---|
| 40 | 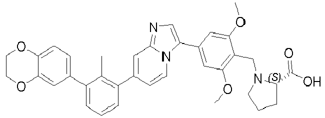 (4-(7-(3-(2,3-dihydrobenzo[*b*][1,4]dioxin-6-yl)-2-methylphenyl)imidazo[1,2-*α*]pyridin-3-yl)-2,6-dimethoxybenzyl)-*L*-proline | LC-MS calculated for C36H35N3O6 (M+H)+: m/z = 605.69; found: 606.4 |

### Example 41: (4-(7-(3-(2,3-dihydrobenzo[b][1,4]dioxin-6-yl)-2-methylphenyl)imidazo[1,2-α]pyridin-3-yl)benzoyl)glycine

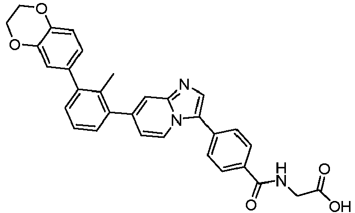

The compound of Example 41 was synthesized via the route shown in the scheme below,

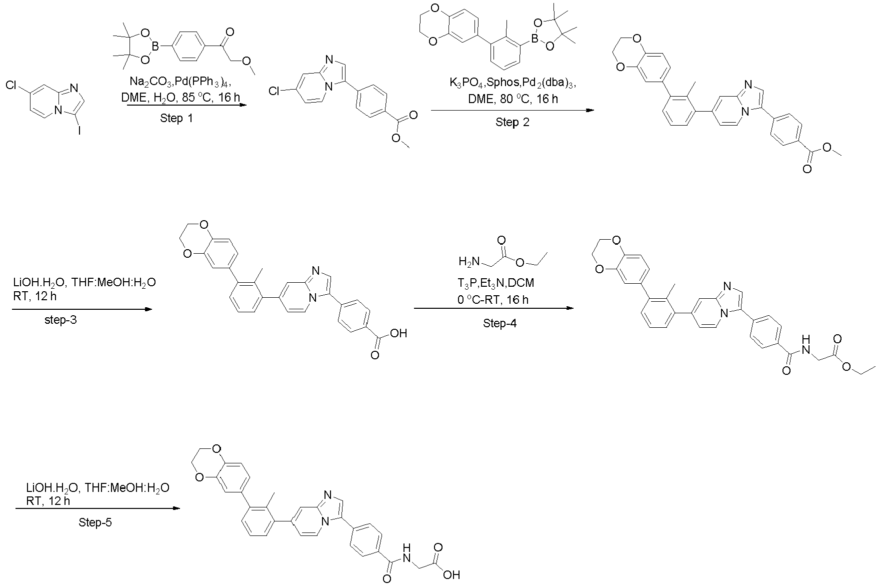

### Step-1: Synthesis of methyl 4-(7-chloroimidazo[1,2-α]pyridin-3-yl)benzoate

The title compound was prepared in a similar way as Example 1 to give the title compound (1.1 g, 35%) as an off-white solid. LC-MS calculated for C15H11CIN2O2 (M+H)+: m/z = 286.72; found: 287.2.

### Step-2: Synthesis of methyl 4-(7-(3-(2,3-dihydrobenzo[b][1,4]dioxin-6-yl)-2-methylphenyl)imidazo[1,2-α]pyridin-3-yl)benzoate

The title compound was prepared in a similar way as Example 1 to give the title compound (800 mg, 45%) as a pale-yellow solid. LC-MS calculated for C30H24N2O4 (M+H)+: m/z = 476.53; found: 477.

### Step-3: Synthesis of 4-(7-(3-(2,3-dihydrobenzo[b][1,4]dioxin-6-yl)-2-methylphenyl)imidazo[1,2-α]pyridin-3-yl)benzoic acid

To a stirred solution of methyl 4-(7-(3-(2,3-dihydrobenzo[b][1,4]dioxin-6-yl)-2-methylphenyl)imidazo[1,2-a]pyridin-3-yl)benzoate (800 mg, 1.67 mmol) in THF:MeOH (14 mL:4mL) was added LiOH.H₂O (121 mg, 5.042 mmol) in water (2 mL) and stirred the reaction at room temperature for 16 h. Solvent was removed under reduced pressure and the residue dissolved in the water and washed with diethyl ether (2 x 10 mL) and aqueous layer was acidified with 1N HCl and extracted with DCM (3 x 10 mL). The combined organic layer was dried over Na₂SO₄, filtered, and evaporated under reduced pressure to give the crude, which was washed with pentane, filtered, and dried under vacuum to obtain the title compound (600 mg, 77 %) as a white solid.LC-MS calculated for C29H22N2O4 (M+H)+: m/z = 462.51; found: 463.

### Step-4: Synthesis of ethyl (4-(7-(3-(2,3-dihydrobenzo[b][1,4]dioxin-6-yl)-2-methylphenyl)imidazo[1,2-α]pyridin-3-yl)benzoyl)glycinate.

To a stirred solution of 4-(7-(3-(2,3-dihydrobenzo[*b*][1,4]dioxin-6-yl)-2-methylphenyl)imidazo[1,2-*a*]pyridin-3-yl)benzoic acid (200 mg, 0.43 mmol), ethyl glycinate (71 mg, 0.51 mmol), TEA (241 mL, 1.73 mmol) in DCM (10 mL), T3P (193 mL, 0.64 mmol) was added at 0 °C and stirred the reaction mixture at room temperature for 16 h. Diluted the reaction with water and DCM, separated organic layer. The aqueous layer was extracted with DCM (3 × 10 mL) and the combined organic layer was dried over Na₂SO₄, filtered, and evaporated to obtain the title compound (190 mg, 80.23 %) as a brown sticky mass which was taken to next step without purification. LC-MS calculated for C33H29N3O5 (M)+: m/z = 547.61; found: 547.2

### Step-5: Synthesis of (4-(7-(3-(2,3-dihydrobenzo[b][1,4]dioxin-6-yl)-2-methylphenyl)imidazo[1,2-α]pyridin-3-yl)benzoyl)glycine

To a stirred solution of ethyl (4-(7-(3-(2,3-dihydrobenzo[*b*][1,4]dioxin-6-yl)-2-methylphenyl)imidazo[1,2-*α*]pyridin-3-yl)benzoyl)glycinate (190 mg, 0.34 mmol) in THF:MeOH (7.0 mL:2mL) was added LiOH.H₂O (43 mg, 1.042 mmol) in water (1 mL) and stirred the reaction at room temperature for 16 h. Solvent was removed under reduced pressure and the residue dissolved in the water and washed with diethyl ether (2 x 10 mL) and aqueous layer was acidified with 1N HCl and extracted with DCM (3 x 10 mL). The combined organic layer was dried over Na₂SO₄, filtered, and evaporated under reduced pressure to give the crude, which was washed with pentane, filtered, and dried under vacuum to obtain the title compound (8 mg, 15 %) as a white solid.

**¹H NMR (400 MHz, DMSO-*d*₆) *δ*:** 8.70 (d, *J* = 7.2 Hz, 1H), 8.05 (d, *J* = 8 Hz, 2H), 7.95 (s, 1H), 7.85 (d, *J* = 8 Hz, 2H), 7.65 (s, 1H), 7.33 (d, *J* = 7.6 Hz, 2H), 7.24 (d, *J* = 7.2 Hz, 1H), 7.08 (d, *J =* 6.8 Hz, 1H), 6.94-6.84 (m, 3H), 4.28 (s, 4H), 3.91 (d, *J* = 4.4 Hz, 2H), 2.18 (s, 3H). LC-MS calculated for C31H25N3O5 (M+H)+: m/z = 519.56; found: 518.2.

Following examples as shown in Table 6 were prepared according to similar procedure described for Example 41 with appropriate amines:

**Table 6:**

| **Ex.No** | **Structure IUPAC Name** | **LC-MS(ESI)m/z:** |
|---|---|---|
| 42 | 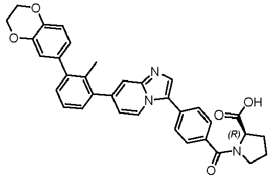 (4-(7-(3-(2,3-dihydrobenzo[*b*][1,4]dioxin-6-yl)-2-methylphenyl)imidazo[1,2-a]pyridin-3-yl)benzoyl)-*D-*proline | LC-MS calculated for C34H29N3O5 (M+H)+: m/z = 559.62; found: 560.4 |
| 43 | 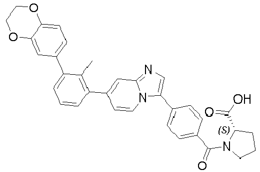 (4-(7-(3-(2,3-dihydrobenzo[*b*][1,4]dioxin-6-yl)-2-methylphenyl)imidazo[1,2-*α*]pyridin-3-yl)benzoyl)-*L-*proline | LC-MS calculated for C34H29N3O5 (M+H)+: m/z = 559.62; found: 560.4 |

### Example 44: N-(2-hydroxyethyl)-1-(7-(2-methyl-[1,1'-biphenyl]-3-yl)imidazo[1,2-α]pyridin-3-yl)-1H-pyrazole-3-carboxamide

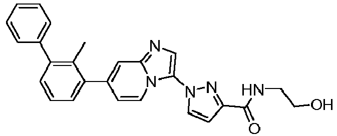

The compound of Example 44 was synthesized via the route shown in the scheme below,

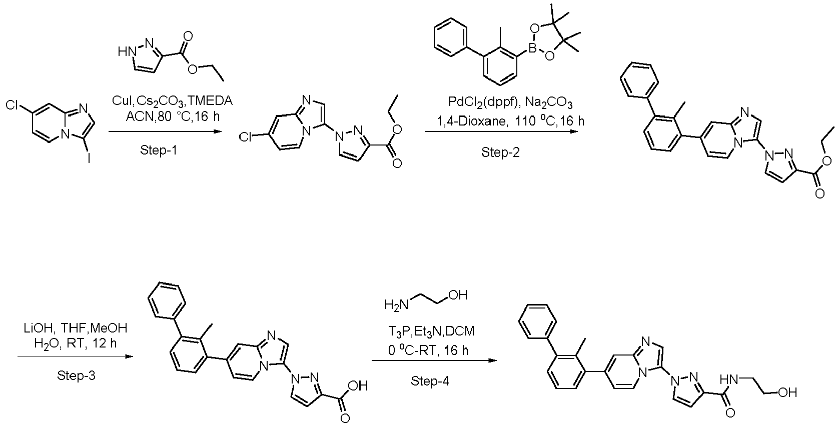

### Step-1: Synthesis of ethyl 1-(7-chloroimidazo[1,2-α]pyridin-3-yl)-1H-pyrazole-3-carboxylate

To a stirred solution of 7-chloro-3-iodoimidazo[1,2-*α*]pyridine (500 mg, 1.79 mmol), ethyl 1*H*-pyrazole-3-carboxylate (189 mg, 1.34 mmol), Cul (52 mg, 0.26 mmol), Cs₂CO₃ (880 mg, 2.69 mmol) in acetonitrile (5 mL) was added TMEDA (0.08 mL, 0.53 mmol) and reaction mixture was refluxed for 16 h. Reaction mixture was filtered through celite bed, filtrate was evaporated. The crude material was purified by combiflash column chromatography using 0-25% ethyl acetate in hexanes to obtain the title compound (220 mg, 42 %) as an off-white solid. LC-MS calculated for C13H11CIN4O2 (M+H)+: m/z = 290.71; found: 291.2.

### Step-2: Synthesis of ethyl 1-(7-(2-methyl-[1,1'-biphenyl]-3-yl)imidazo[1,2-α]pyridine-3-yl)-1H-pyrazole-3-carboxylate

The title compound was prepared in a similar way as Example 1, to give the title compound (150 mg, 46%) as an off-white solid. LC-MS calculated for C26H22N4O2 (M+H)+: m/z = 422.49; found: 423.4.

### Step-3: Synthesis of 1-(7-(2-methyl-[1,1'-biphenyl]-3-yl)imidazo[1,2-α]pyridin-3-yl)-1H-pyrazole-3-carboxylic acid

To a stirred solution of ethyl 1-(7-(2-methyl-[1,1'-biphenyl]-3-yl)imidazo[1,2-a]pyridin-3-yl)-1*H*-pyrazole-3-carboxylate (250 mg, 0.5924 mmol) in THF:MeOH (7.0 mL:2mL) was added LiOH.H₂O (75 mg, 1.77 mmol) in water (1 mL) and stirred the reaction at room temperature for 16 h. Solvent was removed under reduced pressure and the residue dissolved in the water and washed with diethyl ether (2 × 10 mL) and aqueous layer was acidified with 1N HCl and extracted with DCM (3 × 10 mL). The combined organic layer was dried over Na₂SO₄, filtered, and evaporated under reduced pressure to give the crude, which was washed with pentane, filtered, and dried under vacuum to obtain the title compound (220 mg, 94 %) as an off-white solid. LC-MS calculated for C24H18N4O2 (M+H)+: m/z = 394.43; found: 395.2.

### Step-4: Synthesis of N-(2-hydroxyethyl)-1-(7-(2-methyl-[1,1'-biphenyl]-3-yl)imidazo[1,2-α]pyridin-3-yl)-1H-pyrazole-3-carboxamide:

The title compound was prepared in a similar way as Example 41 The crude material was purified by prep-HPLC (X-Bridge C18 column (250 x 19) mm, 5.0 µm; mobile phase (A: B), A = 0.1% FA in B = ACN 100%.) to obtain the title compound (29.6 mg, 27 %) as a white solid.

**¹H NMR (400 MHz, DMSO-*d*₆) *δ*:** 8.42 (d, *J* = 7.2 Hz, 1H), 8.34 (d, *J* = 5.6 Hz, 2H), 7.98 (s, 1H), 7.69 (s, 1H), 7.33-7.49 (m, 7H), 7.28 (d, *J* = 7.2 Hz, 1H), 7.13 (d, *J* = 6.8 Hz, 1H), 6.98 (s, 1H), 4.74 (t, *J* = 5.2 Hz, 1H), 3.5 (d, *J* = 6.0 Hz, 2H), 3.32 (s, 2H), 2.14 (s, 3H). LC-MS calculated for C26H23N5O2 (M+H)+: m/z = 437.52; found: 438.4.

Following examples as shown in Table 7 were prepared according to similar procedure described for Example 44 with appropriate amines:

**Table 7:**

| **Ex.No** | **Structure IUPAC Name** | **LC-MS (ESI)m/z:** |
|---|---|---|
| 45 | 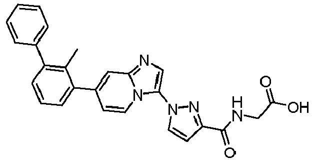 (1-(7-(2-methyl-[1,1'-biphenyl]-3-yl)imidazo[1,2-*α*]pyridin-3-yl)-1*H*-pyrazole-3-carbonyl)glycine | LC-MS calculated for C20H21N5O3 (M+H)+: m/z = 451.49; found: 452.3 |
| 46 | | LC-MS calculated for |
| | 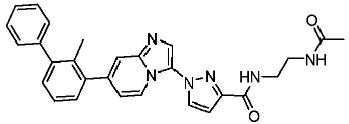 *N*-(2-acetamidoethyl)-1-(7-(2-methyl-[1,1'-biphenyl]-3-yl)imidazo[1,2-*α*]pyridin-3-yl)-1*H-*pyrazole-3-carboxamide | C28H28N5O2 (M+H)+: m/z = 478.56; found: 479.4 |
| 47 | 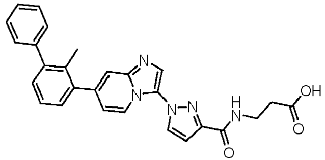 3-(1-(7-(2-methyl-[1,1'-biphenyl]-3-yl)imidazo[1,2-*α*]pyridin-3-yl)-1*H*-pyrazole-3-carboxamido)propanoic acid | LC-MS calculated for C27H23N5O3 (M+H)+: m/z = 465.51; found: 466.3 |

### Example 48: (1-(7-(3-(2,3-dihydrobenzo[b][1,4]dioxin-6-yl)-2-methylphenyl)imidazo[1,2-α]pyridin-3-yl)-1H-pyrazole-3-carbonyl)glycine

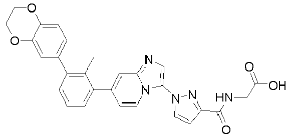

The compound of Example 48 was synthesized via the route shown in the scheme below,

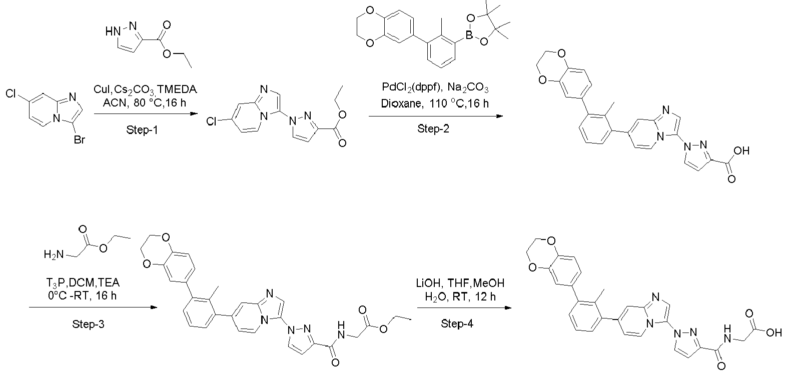

### Step-1: Ethyl 1-(7-chloro-[1,2,4]triazolo[4,3-α]pyridin-3-yl)-1H-pyrazole-3-carboxylate

The title compound was prepared in a similar way as Example 41 to give the title compound (880 mg, 45%) as an off-white solid. LC-MS calculated for C13H11CIN4O2 (M+H)+: m/z = 290.71; found: 291.25

### Step-2: Synthesis of 1-(7-(3-(2,3-dihydrobenzo[b][1,4]dioxin-6-yl)-2-methylphenyl)-[1,2,4]triazolo[4,3-α]pyridin-3-yl)-1H-pyrazole-3-carboxylic acid

The title compound was prepared in a similar way as Example 1 to give the title compound (90 mg, 12%) as a white solid. LC-MS calculated for C26H20N4O4 (M+H)+: m/z = 452.47; found: 453.

### Step-3: Synthesis of ethyl (1-(7-(2-methyl-[1,1'-biphenyl]-3-yl)-[1,2,4]triazolo[4,3-α]pyridin-3-yl)-1H-pyrazole-3-carbonyl)glycinate

The title compound was prepared in a similar way as Example 41 to give the title compound (120 mg, crude) as a brown sticky solid. LC-MS calculated for C30H27NSOS (M+H)+: m/z = 537.58; found: 538.

### Step-4: Synthesis of (1-(7-(3-(2,3-dihydrobenzo[b][1,4]dioxin-6-yl)-2-methylphenyl)-[1,2,4]triazolo[4,3-α]pyridin-3-yl)-1H-pyrazole-3-carbonyl)glycine

The title compound was prepared in a similar way as Example 41, to give the title compound (70 mg, 61%) as a white solid. **¹H NMR (400 MHz, DMSO-*d*₆) *δ*:** 12.6 (s, 1H), 8.73 (t, J = 1.2 Hz, 1H), 8.52 (d, J = 6.8 Hz, 1H), 8.39 (d, J = 2.4 Hz, 1H), 8.08 (s, 1H), 7.72 (s, 1H), 7.34 (m, 2H), 7.26 (d, J = 7.2 Hz, 1H), 7.18 (d, J = 6.8 Hz, 1H), 7.02 (d, J = 2.4 Hz, 1H), 6.93 (d, J = 8.4 Hz, 1H), 6.89 (s, 1H), 6.85 (d, J = 8.0 Hz, 1H), 4.28 (s, 4H), 3.93 (d, J = 6.0 Hz, 2H), 2.16 (s, 3H). LC-MS calculated for C28H23NSOS (M+H)+: m/z = 509.52; found: 510.3

### Example 49: (1-(7-(3-(2,3-dihydrobenzo[b][1,4]dioxin-6-yl)-2-methylphenyl)-[1,2,4]triazolo[ 4,3-α]pyridin-3-yl)-1H-pyrazole-3-carbonyl)glycine

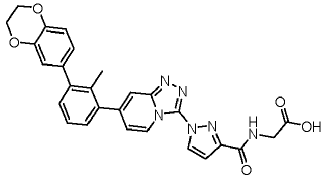

The compound of Example 49 was synthesized via the route shown in the scheme below,

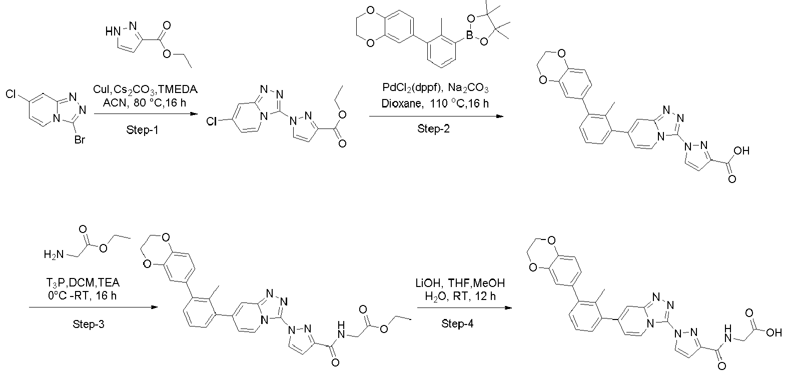

### Step-1: Ethyl 1-(7-chloro-[1,2,4]triazolo[4,3-α]pyridin-3-yl)-1H-pyrazole-3-carboxylate

The title compound was prepared in a similar way as example 41 to give the title compound (880 mg, 45%) as an off-white solid. LC-MS calculated for C12H10CIN5O2 (M+H)+: m/z = 291.7; found: 292

### Step-2: Synthesis of 1-(7-(3-(2,3-dihydrobenzo[b][1,4]dioxin-6-yl)-2-methylphenyl)-[1,2,4]triazolo[4,3-α]pyridin-3-yl)-1H-pyrazole-3-carboxylic acid

The title compound was prepared in a similar way as example 1 to give the title compound (90 mg, 12%) as a white solid. LC-MS calculated for C25H19N5O4 (M+H)+: m/z = 453.46; found: 454.30 (M+1).

### Step-3: Synthesis of ethyl (1-(7-(2-methyl-[1,1'-biphenyl]-3-yl)-[1,2,4]triazolo[4,3-α]pyridin-3-yl)-1H-pyrazole-3-carbonyl)glycinate

The title compound was prepared in a similar way as Example 41 to give the title compound (120 mg, crude) as a brown sticky solid. LC-MS calculated for C29H26N6O5 (M+H)+: m/z = 538.56; found: 539.35 (M+1)

### Step-4: Synthesis of (1-(7-(3-(2,3-dihydrobenzo[b][1,4]dioxin-6-yl)-2-methylphenyl)-[1,2,4]triazolo[4,3-α]pyridin-3-yl)-1H-pyrazole-3-carbonyl)glycine

The title compound was prepared in a similar way as Example 41, to give the title compound (70 mg, 61%) as a white solid. **¹H NMR (400 MHz, DMSO-*d*₆) *δ*:**12.75 (br s, 1H), 9.12 (d, *J =* 7.2 Hz, 1H), 8.94 (s, 1H), 8.69 (d, *J* = 2 Hz, 1H), 7.86 (s, 1H), 7.40-7.31 (m, 2H), 7.28 (d, *J* = 6.4 Hz, 1H), 7.20 (d, *J* = 7.2 Hz, 1H), 7.07 (d, *J* = 2 Hz, 1H), 6.94 (d, *J* = 8 Hz, 1H), 6.90 (s, 1H), 6.85 (d, *J* = 8.4 Hz, 1H), 4.29 (s, 4H), 3.94 (d, *J* = 5.2 Hz, 2H), 2.18 (s, 3H).LC-MS calculated for C27H22N6O5 (M+H)+: m/z = 510.51; found: 511.35.

### Example 50: (3-(7-(2-methyl-[1,1'-biphenyl]-3-yl)imidazo[1,2-α]pyridin-3-yl)prop-2-yn-1-yl)-D-proline

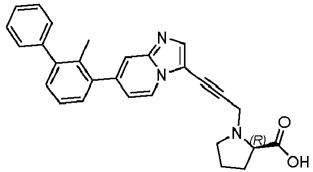

The compound of Example 50 was synthesized via the route shown in the scheme below,

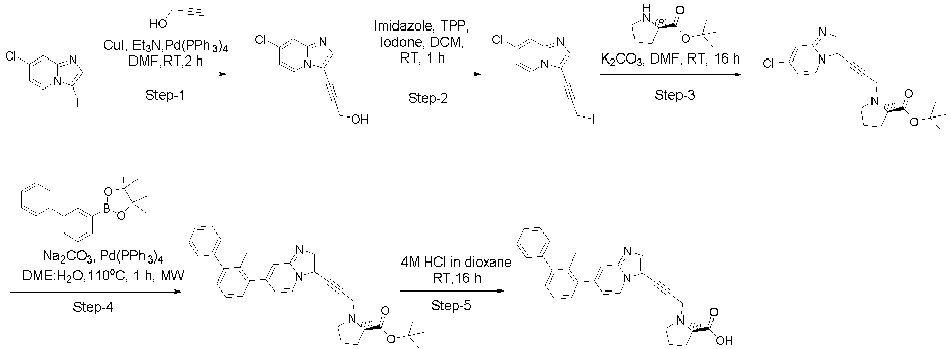

### Step-1: Synthesis of 3-(7-chloroimidazo[1,2-α]pyridin-3-yl)prop-2-yn-1-ol

A mixture of 7-chloro-3-iodoimidazo[1,2-*α*]pyridine (1.0 g, 3.59 mmol), propargyl alcohol (0.26 g, 4.3 mmol), TEA (1.25 mL, 8.9 mmol), Cul (40 mg, 0.18 mmol), Pd(PPh₃)₄ (200 mg, 0.18 mmol), and DMF (10 mL) was degassed with N₂ for 30 minutes and stirred at room temperature for 2 h. The reaction mixture was diluted with water and extracted with ethyl acetate (3 x 15 mL), combined organic layer was washed with water, brine, dried over Na₂SO₄, filtered, and evaporated. The crude compound was purified by combiflash column chromatography using 10-50 % ethyl acetate in hexane as a mobile phase to obtain the title compound (720 mg, 97%) as a colorless liquid. LC-MS calculated for C10H7CIN2O (M+H)+: m/z = 206.63; found: 207.20

### Step-2: Synthesis of 7-chloro-3-(3-iodoprop-1-yn-1-yl)imidazo[1,2-α]pyridine

A mixture of 3-(7-chloroimidazo[1,2-a]pyridin-3-yl)prop-2-yn-1-ol (620 mg, 3.09 mmol), TPP (1.2 g, 4.5 mmol), imidazole (300 mg, 4.5 mmol), I₂ (1.15 g, 4.5 mmol), in DCM (10 mL) was stirred at room temperature for 1 h. The reaction mixture was diluted with water and extracted with DCM (3 × 10 mL), combined organic layer was dried over Na₂SO₄, filtered, and evaporated. The crude compound was purified by combi lash column chromatography using 0-40 % ethyl acetate in hexane as a mobile phase to obtain the title compound (500 mg, 52 %) as an off-white solid. LC-MS calculated for C10H6CIIN2 (M+H)+: m/z = 316.53; found: 317.10

### Step-3: Synthesis of tert-butyl (3-(7-chloroimidazo[1,2-α]pyridin-3-yl)prop-2-yn-1-yl)-D-prolinate

A mixture of 7-chloro-3-(3-iodoprop-1-yn-1-yl)imidazo[1,2-a]pyridine (250 mg, 0.79 mmol), tert-butyl *D*-prolinate (200 mg, 0.95 mmol), K₂CO₃ (330 mg, 2.37 mmol), in DMF (5 mL) was stirred at room temperature for 16 h. The reaction mixture was diluted with water and extracted with ethyl acetate (3 × 10 mL), combined organic layer was washed with water, brine, dried over Na₂SO₄, filtered, and evaporated to obtain the title compound (310 mg). The crude material was taken for the next step without further purification.

### Step-4: Synthesis of tert-butyl (3-(7-(2-methyl-[1,1'-biphenyl]-3-yl)imidazo[1,2-α]pyridin-3-yl)prop-2-yn-1-yl)-D-prolinate

The title compound was prepared in a similar way as Example 1 at 110 °C in Microwave reactor for 1 h, to give the title compound 30 (300 mg, 71%) as a white solid. LC-MS calculated for C32H33N3O2 (M+H)+: m/z = 491.64; found: 492.40.

### Step-5: Synthesis of (3-(7-(2-methyl-[1,1'-biphenyl]-3-yl)imidazo[1,2-α]pyridin-3-yl)prop-2-yn-1-yl)-D-proline:

The title compound was prepared in a similar way as example 39 to give the title compound (20 mg, 8%) as a white solid. LC-MS calculated for C28H25N3O2 (M+H)+: m/z = 435.53; found: 436.35.

Following example as shown in Table 8 was prepared according to similar procedure described for Example 50 with appropriate amines:

**Table 8:**

| **S.No** | **Ex.No** | **Structure IUPAC Name** | **LC-MS(ESI)m/z:** |
|---|---|---|---|
| | 51 | 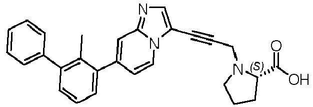 (3-(7-(2-methyl-[1,1'-biphenyl]-3-yl)imidazo[1,2-*a*]pyridin-3-yl)prop-2-yn-1-yl)-*L*-proline | LC-MS calculated for C28H25N3O2 (M+H)+: m/z = 435.53; found: 436.35 |

### Example 52: 1-(4-(7-(2-methyl-[1,1'-biphenyl]-3-yl)-[1,2,4]triazolo[4,3-α]pyridin-3-yl)benzyl)piperidine-2-carboxylic acid

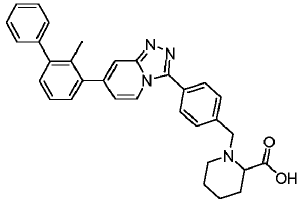

The compound of Example 52 was synthesized via the route shown in the scheme below,

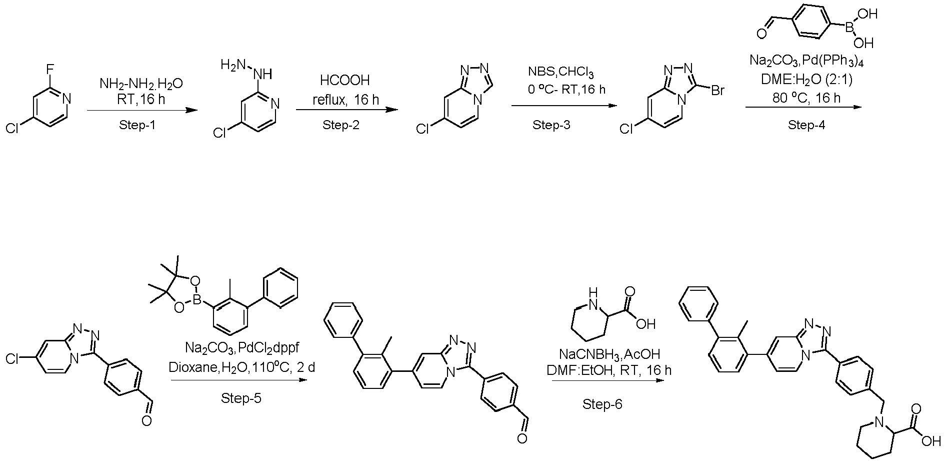

### Step-1: Synthesis of 4-chloro-2-hydrazineylpyridine

A mixture of 4-chloro-2-fluoropyridine (25 g, 190 mmol) and hydrazine monohydrate (67 mL, 209 mmol) was stirred at room temperature of 16 h. The reaction mixture was quenched by addition of 4M NaOH (63 mL), water (125 mL) and stirred for 15 minutes. Solid material precipitated out was filtered off, washed with water, dried in vacuum to obtain the title compound (27 g, 98%) as a white solid.

LC-MS calculated for C5H6CIN3 (M+H)+: m/z = 143.57; found: 144.57.

### Step-2: Synthesis of 7-chloro-[1,2,4]triazolo[4,3-α]pyridine

A mixture of 4-chloro-2-hydrazineylpyridine (27 g, 188 mmol) and formic acid (27 mL, 10 vol) was refluxed for 16 h. The organic volatiles were removed under reduced pressure, residue dissolved in the water and extracted with 5% MeOH in DCM (3 x 200 ml) and the combined organic layer was dried over Na₂SO₄, filtered, and evaporated to obtain the title compound (26 g, 90%) as a white solid. LC-MS calculated for C6H4CIN3 (M+H)+: m/z = 153.57; found: 154.20.

### Step-3: Synthesis of 3-bromo-7-chloro-[1,2,4]triazolo[4,3-a]pyridine

NBS (10.4 g, 58.6 mmol) was added in portions to a stirred solution of 7-chloro-[1,2,4]triazolo[4,3-*α*]pyridine (5 g, 32.5 mmol) in chloroform (250 mL) at 0 °C and stirred the reaction mixture at room temperature for 16 h. Saturated solution of NaHCOs was added to the reaction mixture and extracted with DCM (2 x 50 mL).The combined organic layer was washed with water, dried over Na₂SO₄, filtered, and evaporated. The crude material was purified by combi flash column chromatography using 0-80% ethyl acetate in hexane to obtain the title compound (6.3 g, 83 %) as an off-white solid. LC-MS calculated for C6H3BrCIN3 (M+H)+: m/z = 232.47; found: 234.15.

### Step-4: Synthesis of 4-(7-chloro-[1,2,4]triazolo[4,3-α]pyridin-3-yl)benzaldehyde

The title compound was prepared in a similar way as example 1 to give the title compound (228 mg, 41%) as an off-white solid. LC-MS calculated for C13H8CIN3O (M+H)+: m/z = 257.68; found: 257.95.

### Step-5: Synthesis of 4-(7-(2-methyl-[1,1'-biphenyl]-3-yl)-[1,2,4]triazolo[4,3-a]pyridin-3-yl)benzaldehyde

The title compound was prepared in a similar way as example 1 to give the title compound (95 mg, 27%) as an off-white solid. LC-MS calculated for C26H19N3O (M+H)+: m/z = 389.46; found: 390.25.

### Step-6: Synthesis of 1-(4-(7-(2-methyl-[1,1'-biphenyl]-3-yl)-[1,2,4]triazolo[4,3-α]pyridin-3-yl)benzyl)piperidine-2-carboxylic acid

The title compound was prepared in a similar way as example 1 to get crude which was purified by prep-HPLC to give the title compound (7.6 mg, 7%) as an off-white solid.**¹H NMR (400 MHz, DMSO-*d*₆) *δ*:**8.62 (d, *J* = 6.8 Hz, 1H), 7.91 (d, *J* = 8.0 Hz, 2H), 7.84 (s, 1H), 7.60 (d, *J* = 8 Hz, 3H), 7.5-7.46 (m, 2H), 7.46-7.34 (m, 4H), 7.34-7.28 (m, 1H), 7.10 (d, *J* = 6.4 Hz, 1H), 3.95 (d, *J* = 13.2 Hz, 1H), 3.58 (d, *J* = 14 Hz, 1H), 3.2-3.15 (m, 1H), 2.95-2.85 (m, 1H), 2.3-2.1 (m, 1H), 2.17 (s, 3H), 1.9-1.3 (m, 2H), 1.6-1.45 (m, 4H). LC-MS calculated for C32H30N4O2 (M+H)+: m/z = 502.62; found: 503.45.

Following example as shown in Table 9 was prepared according to similar procedure described for Example 52 with appropriate amines:

**Table 9:**

| **Ex.No** | **Structure IUPAC Name** | **LC-MS(ESI)m/z:** |
|---|---|---|
| 53 | 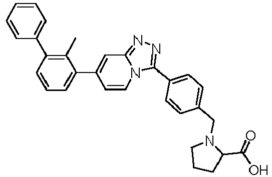 (4-(7-(2-methyl-[1,1'-biphenyl]-3-yl)-[1,2,4]triazolo[4,3-a]pyridin-3-yl)benzyl)proline | LC-MS calculated for C31H28N4O2 (M+H)+: m/z = 488.59; found: 489.4 |
| 54 | 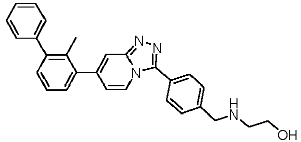 2-((4-(7-(2-methyl-[1,1'-biphenyl]-3-yl)-[1,2,4]triazolo[4,3-a]pyridin-3-yl)benzyl)amino)ethan-1-ol | LC-MS calculated for C28H26N4O (M+H)+: m/z = 434.54; found: 435.3 |
| 55 | 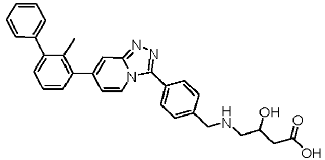 3-hydroxy-4-((4-(7-(2-methyl-[1,1'-biphenyl]-3-yl)-[1,2,4]triazolo[4,3-*a*]pyridin-3-yl)benzyl)amino)butanoic acid | LC-MS calculated for C30H28N4O3 (M+H)+: m/z = 492.58; found: 493.2 |
| 56 | 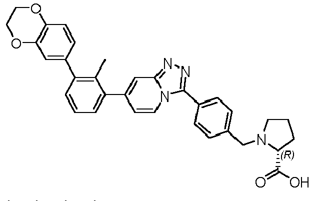 (4-(7-(3-(2,3-dihydrobenzo[b][1,4]dioxin-6-yl)-2-methylphenyl)-[1,2,4]triazolo[4,3-*a*]pyridin-3-yl)benzyl)-D-proline | LC-MS calculated for C33H30N4O4 (M+H)+: m/z = 546.63; found: 547.3 |
| 57 | 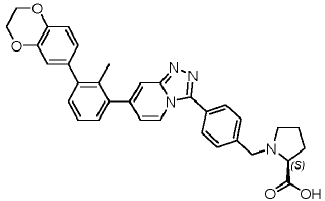 (4-(7-(3-(2,3-dihydrobenzo[*b*][1,4]dioxin-6-yl)-2-methylphenyl)-[1,2,4]triazolo[4,3-*a*]pyridin-3-yl)benzyl)-L-proline | LC-MS calculated for C33H30N4O4 (M+H)+: m/z = 546.63; found: 547.3 |
| 58 | 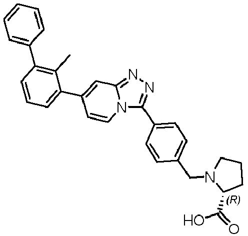 (4-(7-(2-methyl-[1,1'-biphenyl]-3-yl)-[1,2,4]triazolo[4,3-a]pyridin-3-yl)benzyl)-D-proline | LC-MS calculated for C31H28N4O2 (M+H)+: m/z = 488.59; found: 489.4 |
| 59 | 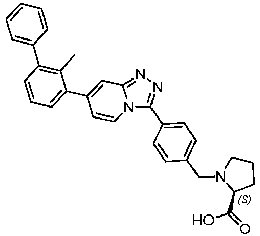 (4-(7-(2-methyl-[1,1'-biphenyl]-3-yl)-[1,2,4]triazolo[4,3-a]pyridin-3-yl)benzyl)-L-proline | LC-MS calculated for C31H28N4O2 (M+H)+: m/z = 488.59; found: 489.4 |
| 60 | 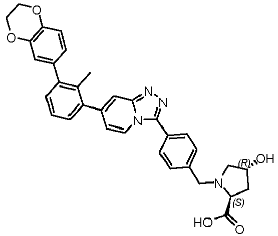 (25,4R)-1-(4-(7-(3-(2,3-dihydrobenzo[*b*][1,4]dioxin-6-yl)-2-methylphenyl)-[1,2,4]triazolo[4,3-*a*]pyridin-3-yl)benzyl)-4-hydroxypyrrolidine-2-carboxylic acid | LC-MS calculated for C33H30N4O5 (M+H)+: m/z = 562.63; found: 563.4 |
| 61 | 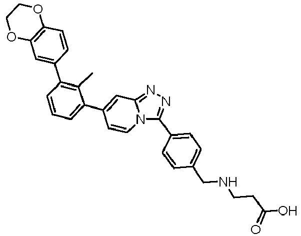 3-((4-(7-(3-(2,3-dihydrobenzo[*b*][1,4]dioxin-6-yl)-2-methylphenyl)-[1,2,4]triazolo[4,3-*a*]pyridin-3-yl)benzyl)amino)propanoic acid | LC-MS calculated for C31H28N4O4 (M+H)+: m/z = 520.59; found: 521.4 |

### Example 62: (4-(7-(4"-(((2-hydroxyethyl)amino)methyl)-2,2'-dimethyl-[1,1':3',1"-terphenyl]-3-yl)-[1,2,4]triazolo[4,3-a]pyridin-3-yl)benzyl)-D-proline

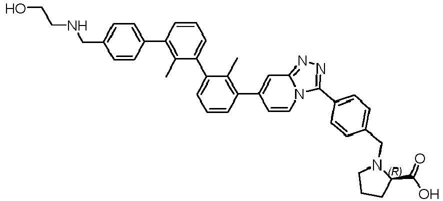

The compound of Example 62 was synthesized via the route shown in the scheme below,

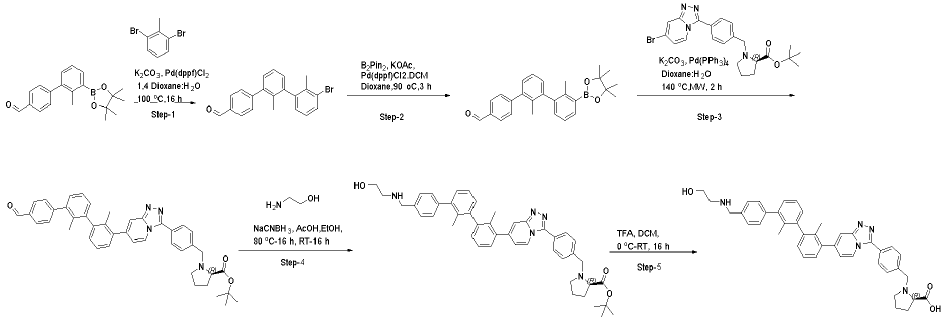

### Step-1: Synthesis of 3"-bromo-2',2"-dimethyl-[1,1':3',1"-terphenyl]-4-carbaldehyde

The title compound was prepared in a similar way as example 1 to give the title compound (1.76 g, 57%) as an off-white solid. LC-MS calculated for C21H17BrO (M+H)+: m/z = 365.27; found: 366.

### Step-2: Synthesis of 2',2"-dimethyl-3"-(4,4,5,5-tetramethyl-1,3,2-dioxaborolan-2-yl)-[1,1':3',1"-terphenyl]-4-carbaldehyde.

The title compound was prepared in a similar way as example 1 to give the title compound (1.54 g, 79%) as a light brown solid. LC-MS calculated for C27H29BO3 (M+H)+: m/z = 412.34; found: 413.

### Step-3: Synthesis of tert-butyl (4-(7-(4"-formyl-2,2'-dimethyl-[1,1':3',1"-terphenyl]-3-yl)-[1,2,4]triazolo[4,3-a]pyridin-3-yl)benzyl)-D-prolinate

The title compound was prepared in a similar way as example 1 to give the title compound (278 mg, 86%) as a light brown solid. LC-MS calculated for C43H42N4O3 (M+H)+: m/z = 662.83; found: 663.

### Step-4: Synthesis of tert-butyl (4-(7-(4"-(((2-hydroxyethyl)amino)methyl)-2,2'-dimethyl-[1,1':3',1"-terphenyl]-3-yl)-[1,2,4]triazolo[4,3-a]pyridin-3-yl)benzyl)-D-prolinate

To a stirred solution of tert-butyl (4-(7-(4"-formyl-2,2'-dimethyl-[1,1':3',1"-terphenyl]-3-yl)-[1,2,4]triazolo[4,3-*a*]pyridin-3-yl)benzyl)-D-prolinate (270 mg, 0.40 mmol) in ethanol (10.0 mL) was added ethanolamine (0.04 mL, 0.61 mmol) and acetic acid (0.05 mL, 0.816 mmol) and stirred the reaction at 80 °C for 16 h. Cooled the reaction mixture to room temperature, NaBH₃CN (77 mg, 1.22 mmol) was added and continued stirring at room temperature for another 16 h. The organic volatiles were evaporated under vacuo, water was added to the residue, stirred for 30 min, and filtered, dried under vacuum to give the title compound (224 mg, 77 %) as an off-white solid. LC-MS calculated for C45H49N5O3 (M+H)+: m/z = 707.92; found: 708.40.

### Step-5: Synthesis of (4-(7-(4"-(((2-hydroxyethyl)amino)methyl)-2,2'-dimethyl-[1,1':3',1"-terphenyl]-3-yl)-[1,2,4]triazolo[4,3-a]pyridin-3-yl)benzyl)-D-proline

To a stirred solution of tert-butyl (4-(7-(4"-(((2-hydroxyethyl)amino)methyl)-2,2'-dimethyl-[1,1':3',1"-terphenyl]-3-yl)-[1,2,4]triazolo[4,3-a]pyridin-3-yl)benzyl)-D-prolinate (147 mg, 0.207 mmol) in DCM (3.0 mL), was added TFA (0.75 mL) at 0 °C. The reaction mixture then stirred at room temperature for 16 h. The organic volatiles were evaporated, and water was added to the residue. The aqueous layer was extracted with DCM (3 X 15 mL) and the combined organic layer was dried over Na₂SO₄, filtered, and removed the solvent under reduced pressure. The crude material was purified by prep-HPLC (X-Bridge C18 column (250 x 19) mm, 5.0 µm; mobile phase (A: B), A = 0.1% FA in H₂O, B = ACN 100%.) to obtain the title compound (25 mg, 19 %) as a white solid. **¹H NMR (400 MHz, DMSO-*d₆*) *δ*:** 8.60 (d, *J* = 7.6 Hz, 1H), 7.92 (d, *J* = 6.8 Hz, 2H), 7.85 (s, 1H), 7.63 (d, *J* = 7.6 Hz, 2H), 7.41-7.35 (m, 4H), 7.33 (d, *J* = 7.2 Hz, 2H), 7.24 (d, *J* = 8.8 Hz, 2H), 7.16 (d, *J* = 7.6 Hz, 1H), 7.09 (d, *J* = 6.8 Hz, 1H), 4.5 (s, 1H), 4.14 (d, *J* = 13.2 Hz, 1H), 3.77-3.72 (m, 3H), 3.49 (t, *J* = 5.6 Hz, 2H),3.49-3.33 (m, 3H), 3.05-3.48 (m, 1H), 2.61 (t, *J* = 5.6 Hz, 2H), 2.14-2.07 (m, 1H), 2.03 (s, 3H), 1.95 (s, 3H), 1.9-1.88 (m, 1H), 1.78-1.65 (m, 2H). LC-MS calculated for C41H41N5O3 (M+H)+: m/z = 651.81; found: 652.7.

Following examples as shown in Table 10 were prepared according to similar procedure described for Example 62 with appropriate amines:

**Table 10:**

| **Ex.No** | **Structure IUPAC Name** | **LC-MS(ESI)m/z:** |
|---|---|---|
| **63** | 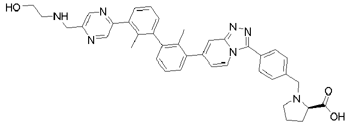 (4-(7-(3'-(5-(((2-hydroxyethyl)amino)methyl)pyraz in-2-yl)-2,2'-dimethyl-[1,1'-biphenyl]-3-yl)-[1,2,4]triazolo[4,3-a]pyridin-3-yl)benzyl)-*D*-proline | LC-MS calculated for C39H39N7O3 (M+H)+: m/z = 653.79; found: 654.4. |
| **64** | 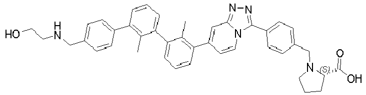 (4-(7-(4"-(((2-hydroxyethyl)amino)methyl)-2,2'-dimethyl-[1,1':3',1"-terphenyl]-3-yl)-[1,2,4]triazolo[4,3-*a*]pyridin-3-yl)benzyl)-*L*-proline | LC-MS calculated for C41H41N5O3 (M+H)+: m/z = 651.81; found: 650.3 |
| **65** | 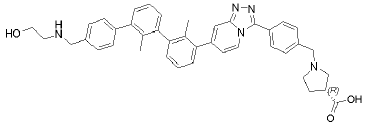 (*R*)-1-(4-(7-(4"-(((2-hydroxyethyl)amino)methyl)-2,2'-dimethyl-[1,1':3',1"-terphenyl]-3-yl)-[1,2,4]triazolo[4,3-*a*]pyridin-3-yl)benzyl)pyrrolidine-3-carboxylic acid | LC-MS calculated for C41H41N5O3 (M+H)+: m/z = 651.81; found: 652.1 |

### Example 66: 2-(((2',2"-dichloro-3"-(3-(4-(((2-hydroxyethyl)amino)methyl)phenyl)-[1,2,4]triazolo[4,3-a]pyridin-7-yl)-[1,1':3',1"-terphenyl]-4-yl)methyl)amino)ethan-1-ol

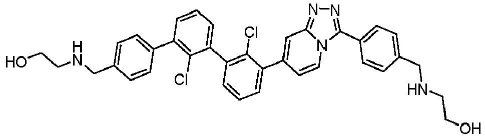

The compound of Example 66 was synthesized via the route shown in the scheme below,

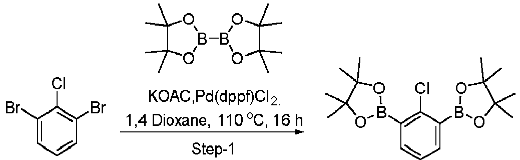

### 2,2'-(2-chloro-1,3-phenylene)bis(4,4,5,5-tetramethyl-1,3,2-dioxaborolane)

To a stirred solution of 1,3-dibromo-2-chlorobenzene (5 g, 20.08 mmol), 2,2'-(2-chloro-1,3-phenylene)bis(4,4,5,5-tetramethyl-1,3,2-dioxaboolane) (2.4 g, 40.8 mmol), in 1,4-dioxane (75 mL) was added KOAc (11.78 g, 120 mmol), Pd(dppf)Cl₂ (1.02 g, 0.14 mmol) and the mixture was degassed for 30 minutes, then the reaction mixture was heated in a microwave at 140 °C for 2 h. Reaction mixture was cooled to room temperature, diluted with water, extracted with ethyl acetate (3 x 25 mL).The combined organic layer was washed with brine, dried over Na₂SO₄, filtered and evaporated. The crude material was purified by Biotage flash column chromatography using 0-10% ethyl acetate in hexane to obtain the title compound (3.0 g, 48 %) as a white solid. LC-MS calculated for C18H27B2ClO4 (M+H)+: m/z = 364.48; found: 365.

### Example 66: 2-(((2',2"-dichloro-3"-(3-(4-(((2-hydroxyethyl)amino)methyl) phenyl)-[1,2,4]triazolo[4,3-a]pyridin-7-yl)-[1,1':3',1"-terphenyl]-4-yl)methyl)amino)ethan-1-ol

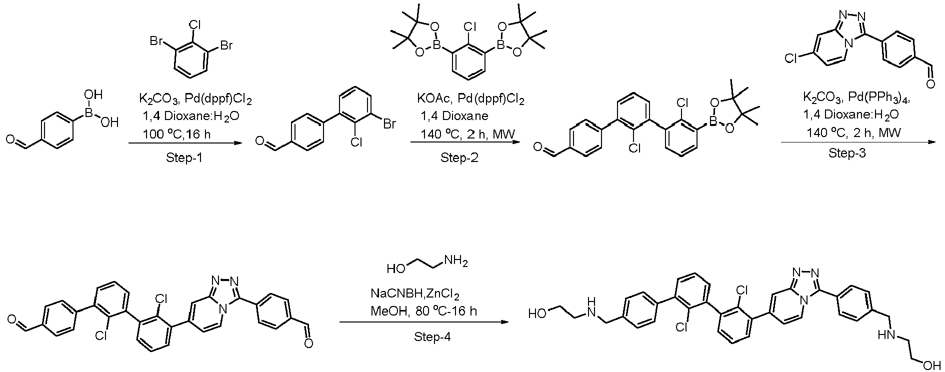

### Step-1 : Synthesis of 3'-bromo-2'-chloro-[1,1'-biphenyl]-4-carbaldehyde

The title compound was prepared in a similar way as example 1 to give the title compound (1.7 g, 43%) as an off-white solid.

### Step-2: 2',2"-dichloro-3"-(4,4,5,5-tetramethyl-1,3,2-dioxaborolan-2-yl)-[1,1':3',1"-terphenyl]-4-carbaldehyde

To a stirred solution of 3'-bromo-2'-chloro-[1,1'-biphenyl]-4-carbaldehyde (500 mg, 1.65 mmol), 2,2'-(2-chloro-1,3-phenylene)bis(4,4,5,5-tetramethyl-1,3,2-dioxaboolane) (470 mg, 1.86 mmol, reference), in 1,4-dioxane (3.5 mL) was added KOAc (498 mg, 5.07 mmol), Pd(dppf)Cl₂ (138 mg, 0.16 mmol) and the mixture was degassed for 30 minutes, then the reaction mixture was heated in a microwave at 140 °C for 2 h. Reaction mixture was cooled to room temperature, diluted with water, extracted with ethyl acetate (3 x 25 mL).The combined organic layer was washed with brine, dried over Na₂SO₄, filtered and evaporated. The crude material was purified by Biotage flash column chromatography using 0-10% ethyl acetate in hexane to obtain the title compound (460 mg, 60 %) as a white solid.

### Step-3: 2',2"-dichloro-3"-(3-(4-formylphenyl)-[1,2,4]triazolo[4,3-a]pyridin-7-yl)-[1,1':3',1"-terphenyl]-4-carbaldehyde:

A mixture of 2',2"-dichloro-3"-(4,4,5,5-tetramethyl-1,3,2-dioxaborolan-2-yl)-[1,1':3',1"-terphenyl]-4-carbaldehyde (120 mg, 26.48 mmol), 4-(7-chloro-[1,2,4]triazolo[4,3-a]pyridin-3-yl)benzaldehyde (68 mg, 26.48 mmol), in 1,4-dioxane (4 mL) was added K₂CO₃ (109 mg, 79.4 mmol) in water (0.4 mL), Pd(PPh₃)₄ (15.2 mg, 1.3 mmol) and the mixture was degassed with N₂ for 30 minutes then heated the reaction mixture in MW at 140 °C for 2 h. Diluted the reaction mixture with ethyl acetate (10 mL) and water (10 mL). The organic layer was separated, and aqueous layer extracted with ethyl acetate (3 x 30 mL). The combined organic layer was dried over Na₂SO₄, filtered, and evaporated. Crude material was purified by Biotage flash column chromatography using (0-10%) methanol in DCM to obtain the title compound (64.6 g, 44%) as an off-white solid. LC-MS calculated for C32H19Cl2N3O2 (M+H)+: m/z = 548.42; found: 548.30.

### Step-4 : Synthesis of 2-(((2',2"-dichloro-3"-(3-(4-(((2-hydroxyethyl)amino)methyl)phenyl)-[1,2,4]triazolo[4,3-a]pyridin-7-yl)-[1,1':3',1"-terphenyl]-4-yl)methyl)amino)ethan-1-ol

The title compound was prepared in a similar way as in example 1 to give the title compound (7.1 mg, 10%) as an off-white solid.

1H NMR (400 MHz, DMSO-d6): δ: 8.62 (d, J= 6.8 Hz, 1H), 7.96 (s, 1H), 7.89 (d, J = 7.6 Hz, 2H), 7.65-7.58 ( m, 4H), 7.53 ( t, J = 4Hz, 2H), 7.51-7.42 ( m, 6H), 7.14-7.12 ( m, 1H), 4.49 (q, J = 5.6 Hz, 2H), 3.83 (s, 2H), 3.76 (s, 2H), 3.5 ( q, J = 5.2 Hz, 4H), 2.6 (q, J = 6 Hz, 4H). LC-MS calculated for C36H33Cl2N5O2 (M+H)+: m/z = 638.59; found: 639.

### Example 67: (4-(7-(2,2'-dichloro-4"-(((2-hydroxyethyl)amino)methyl)-[1,1':3',1"-terphenyl]-3-yl)-[1,2,4]triazolo[4,3-a]pyridin-3-yl)benzyl)-D-proline

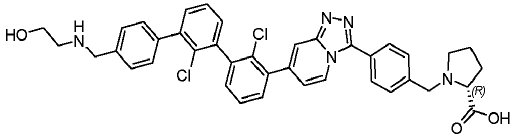

The compound of Example 67 was synthesized via the route shown in the scheme below,

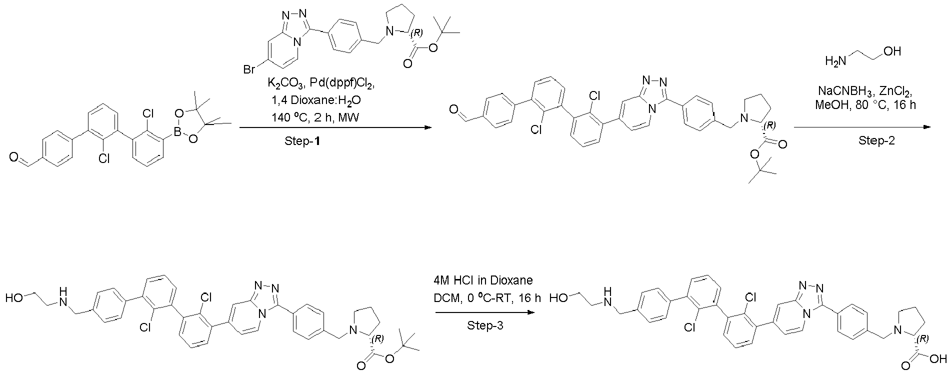

### Step-1: Synthesis of tert-butyl (4-(7-(2,2'-dichloro-4"-formyl-[1,1':3',1"-terphenyl]-3-yl)-[1,2,4]triazolo[4,3-a]pyridin-3-yl)benzyl)-D-prolinate

The title compound was prepared in a similar way as example 1 to give the title compound (348 mg, 80%) as off-white solid. LC-MS calculated for C41H36Cl2N4O3 (M+H)+: m/z = 703.66; found: 703.45.

### Step-2: Synthesis of tert-butyl (4-(7-(2,2'-dichloro-4"-(((2-hydroxyethyl)amino)methyl)-[1,1':3',1"-terphenyl]-3-yl)-[1,2,4]triazolo[4,3-a]pyridin-3-yl)benzyl)-D-prolinate

The title compound was prepared in a similar way as in example 1 to give the title compound (188 mg, 72%) as an off-white solid. LC-MS calculated for C43H43Cl2N5O3 (M+H)+: m/z = 748.75; found: 749.45

### Step-3: Synthesis of (4-(7-(2,2'-dichloro-4"-(((2-hydroxyethyl)amino)methyl)-[1,1':3',1"-terphenyl]-3-yl)-[1,2,4]triazolo[4,3-a]pyridin-3-yl)benzyl)-D-proline

The title compound was prepared in a similar way as example 39 to give the title compound (35 mg, 20%) as an off-white solid.

1H NMR (400 MHz, DMSO-d6) δ:8.64 (d, J =7.2 Hz 1H), 8.19 (s, 1H), 4.5 (s, 2H), 4.15 (d, J = 13.6 Hz, 1H), 3.81(s, 2H), 3.75(d, J = 13.6 Hz, 1H), 3.51 (d, J = 5.6Hz, 2H), 3.1-3.0 (m, 2H), 2.2-2.09 (m, 2H), 2.0-1.85 (m, 2H), 1.85-1.7 (m, 2H). LC-MS calculated for C39H35Cl2N5O3 (M+H)+: m/z = 692.64; found: 693.

### Example 68: (4-(7-(3'-(5-(((2-hydroxyethyl)amino)methyl)picolinamido)-2,2'-dimethyl-[1,1'-biphenyl]-3-yl)-[1,2,4]triazolo[4,3-a]pyridin-3-yl)benzyl)-D-proline

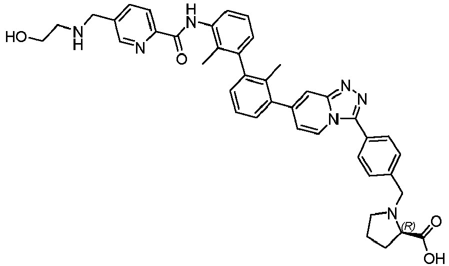

The compound of Example 68 was synthesized via the route shown in the scheme below,

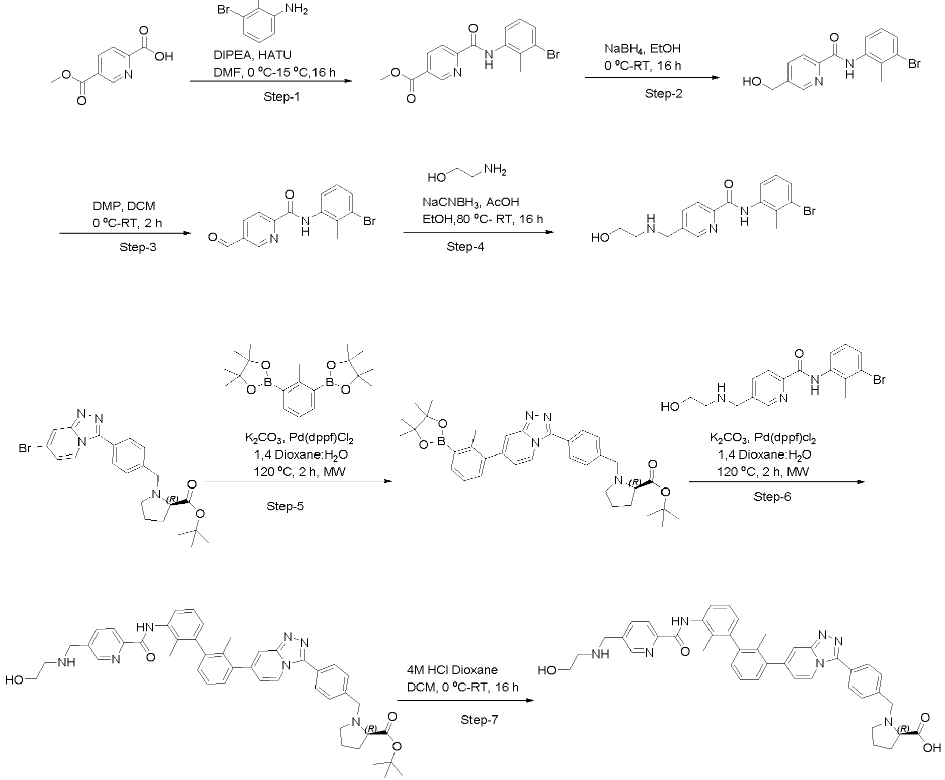

### Step-1: Synthesis of methyl 6-((3-bromo-2-methylphenyl)carbamoyl)nicotinate

To a stirred solution of 5-(methoxycarbonyl)picolinic acid (1 g, 5.52 mmol) and 3-bromo-2-methylaniline (1.027 g, 5.52 mmol) in DMF (10 mL) was added DIPEA (2.35 mL, 1.38 mmol) and HATU (3.14 g, 8.2 mmol) at 0 °C. The reaction mass was stirred at room temperature for 16 h. The reaction mixture was diluted with water and extracted with ethyl acetate (2 x 50 mL). Organic layer was washed with sodium bicarbonate solution (1 × 50 mL), water and brine. The organic layer was dried over Na₂SO₄, filtered, and evaporated. The crude residue was purified by combi flash NextGen 300+ eluting with 0-30% EtOAc in n-hexanes to obtain the title compound (1.76 g, 92 %) as an off-white solid. LC-MS calculated for C15H13BrN2O3 (M+H)+: m/z = 349.18; found: 350.45.

### Step-2: Synthesis of N-(3-bromo-2-methylphenyl)-5-(hydroxymethyl)picolinamide

Sodium borohydride (381 mg, 0.01 mmol) was added to a stirred solution of methyl 6-((3-bromo-2-methylphenyl)carbamoyl)nicotinate (1.76 g, 0.005 mmol) in THF :MeOH (7 mL : 1 mL) at 0 °C and stirred the reaction mixture at room temperature about 16 h. Ammonium chloride solution was added to the reaction mixture and aqueous layer was extracted with ethyl acetate (3 x 25 mL).The combined organic layer was washed with brine, dried over Na₂SO₄, filtered, and evaporated the solvent under reduced pressure. Crude material was purified by Biotage flash column chromatography using 0-30% ethyl acetate in hexane to obtain the title compound (0.979 g, 60 %) as a white solid. LC-MS calculated for C14H13BrN2O2 (M+H)+: m/z = 321.17; found: 322.45.

### Step-3: Synthesis of N-(3-bromo-2-methylphenyl)-5-formylpicolinamide

DMP (381 mg, 898 mmol) was added to a stirred solution of *N*-(3-bromo-2-methylphenyl)-5-(hydroxymethyl)picolinamide (500 mg, 1.5568 mmol), sodium bicarbonate (326.9 mg, 3891 mmol) in DCM (12.5 mL) at 0 °C and stirred the reaction mixture at room temperature about 2 h. Filtered the reaction mixture over celite bed, washed with DCM (20 mL), evaporated the solvent under reduced pressure. Crude material was purified by Biotage flash column chromatography using 0-20% ethyl acetate in hexane to obtain the title compound xx (530 mg, 60 %) as a white solid. LC-MS calculated for C14H118rN2O2 (M+H)+: m/z = 319.16; found: 320.2.

### Step-4: Synthesis of N-(3-bromo-2-methylphenyl)-5-(((2-hydroxyethyl)amino)methyl) picolinamide

The title compound was prepared in a similar way as in example 1 in a microwave to give the title compound (648 mg, 60%) as an off-white solid. LC-MS calculated for C16H18BrN3O2 (M+H)+: m/z = 364.24; found: 365.

### Step-5: Synthesis of tert-butyl (4-(7-(2-methyl-3-(4,4,5,5-tetramethyl-1,3,2-dioxaborolan-2-yl)phenyl)-[1,2,4]triazolo[4,3-a]pyridin-3-yl)benzyl)-D-prolinate

The title compound was prepared in a similar way as example 1 to give the title compound (2.2 g, 53.6%) as a brown solid. LC-MS calculated for C35H43BN4O4 (M+H)+: m/z = 594.56; found: 595.

### Step-6: Synthesis of tert-butyl (4-(7-(3'-(5-(((2-hydroxyethyl)amino)methyl)picolinamido)-2,2'-dimethyl-[1,1'-biphenyl]-3-yl)-[1,2,4]triazolo[4,3-a]pyridin-3-yl)benzyl)-D-prolinate

The title compound was prepared in a similar way as example 1 in a microwave, to give the title compound (1.5 g, 45%) as a colourless liquid. LC-MS calculated for C45H49N7O4 (M+H)+: m/z = 751.93; found: 752.

### Step-7: Synthesis of (4-(7-(3'-(5-(((2-hydroxyethyl)amino)methyl)picolinamido)-2,2'-dimethyl-[1,1'-biphenyl]-3-yl)-[1,2,4]triazolo[4,3-a]pyridin-3-yl)benzyl)-D-proline

The title compound was prepared in a similar way as example 39 to give the title compound (2.2 g, 53.6%) as crude. The crude material was purified by prep-HPLC (X-Bridge C18 column (250 x 19) mm, 5.0 µm; mobile phase (A: B), A = 0.1% FA in H₂O, B = ACN 100%.) to obtain the title compound (25 mg, 17 %) as a white solid. 1H NMR (400 MHz, DMSO-d6) δ:10.36 (s, 1H), 8.69 (s, 1H), 8.61 (d, J = 7.2Hz, 1H), 8.15 (d, J = 8 Hz, 1H), 8.04(d, J = 8Hz, 1H), 7.93(d, J = 7.6 Hz, 2H), 7.87 ( d, J = 8 Hz, 2H), 7.63 (d, J = 8 Hz, 2H), 7.4 (t, J = 6.4 Hz, 2H), 7.34(t, J = 8Hz, 1H), 7.22 (d, J = 4.4 Hz, 1H), 7.08 (dd, J = 7.2Hz, 2H), 4.14 (d, J = 12.4 Hz, 1H), 3.92 (s, 2H), 3.77(d ,J = 13.2Hz,, 1H), 3.50 (s, 2H), 2.65 (d, J =12.4 Hz, 1H), 2.59-2.54 (m, 2H), 2.16-2.11 (m, 1H), 2.07(s, 3H), 1.9 (m, 2H), 1.80-1.74 (m, 2H), LC-MS calculated for C41H41N7O4 (M+H)+: m/z = 695.82; found: 694.4.

Following example as shown in Table 11 was prepared according to similar procedure described for Example 68 with appropriate amines:

**Table 11:**

| **Ex.No** | **Structure IUPAC Name** | **LC-MS(ESI)m/z:** |
|---|---|---|
| **69** | 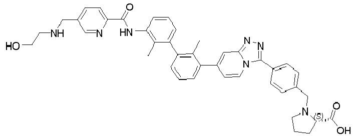 (4-(7-(3'-(5-(((2-hydroxyethyl)amino)methyl)picoli namido)-2,2'-dimethyl-[1,1'-biphenyl]-3-yl)-[1,2,4]triazolo[4,3-*a*]pyridin-3-yl)benzyl)-*L*-proline | LC-MS calculated for C41H41N7O4 (M+H)+: m/z = 695.82; found: 696.35 |

### Example 70: 5-(((2-hydroxyethyl)amino)methyl)-N-(3'-(3-(4-(((2-hydroxyethyl)amino)methyl)phenyl)-[1,2,4]triazolo[4,3-a]pyridin-7-yl)-2,2'-dimethyl-[1,1'-biphenyl]-3-yl)picolinamide

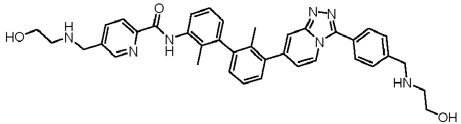

The compound of Example 70 was synthesized via the route shown in the scheme below,

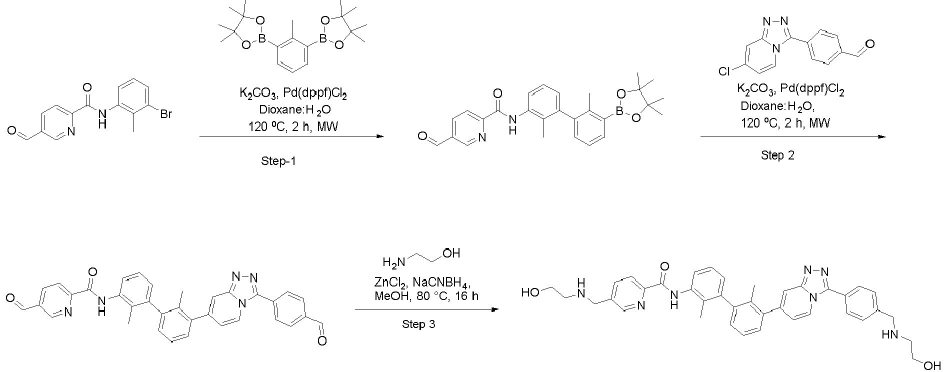

### Step-1: Synthesis of N-(2,2'-dimethyl-3'-(4,4,5,5-tetramethyl-1,3,2-dioxaborolan-2-yl)-[1,1'-biphenyl]-3-yl)-5-formylpicolinamide.

The title compound was prepared in a similar way as example 1 to give the title compound (550 mg, 36%) as a pale-yellow solid. LC-MS calculated for C27H29BN2O4 (M+H)+: m/z = 456.35; found: 457.35.

### Step-2: Synthesis of 5-formyl-N-(3'-(3-(4-formylphenyl)-[1,2,4]triazolo[4,3-a]pyridin-7-yl)-2,2'-dimethyl-[1,1'-biphenyl]-3-yl)picolinamide

The title compound was prepared in a similar way as example 1 to give the title compound (85 mg, 70%) as a colorless sticky mass. LC-MS calculated for C34H25N5O3 (M+H)+: m/z = 551.61; found: 552.15.

### Step-3: Synthesis of 5-(((2-hydroxyethyl)amino)methyl)-N-(3'-(3-(4-(((2-hydroxyethyl)amino)methyl)phenyl)-[1,2,4]triazolo[4,3-a]pyridin-7-yl)-2,2'-dimethyl-[1,1'-biphenyl]-3-yl)picolinamide

The title compound was prepared in a similar way as example 1the obtained crude material was purified by prep-HPLC (X-Bridge C18 column (250 x 19) mm, 5.0 µm; mobile phase (A: B), A = 0.1% FA in H₂O, B = ACN 100%.) to obtain the title compound (15 mg, 15 %) as a white solid. 1H NMR (400 MHz, DMSO-d6) δ:10.84 (s, 1H), 8.68 (s, 1H), 8.59 (d, J = 6.8Hz, 1H), 8.17 (s, 2H), 8.14(d, J = 8Hz, 1H), 8.02 (d, J = 8 Hz, 1H), 7.85-7.92 ( m, 4H), 7.62 (d, J = 8 Hz, 2H), 7.32-7.44 (m, 3H), 7.22(d, J = 5.2Hz, 1H), 7.08 (dd, J = 6 Hz, 2H), 4.61 (s, 2H), 3.90 (d, J = 13.2 Hz, 4H), 3.53 (t, J = 5.6 Hz, 2H), 3.48 (t ,J = 6 Hz,, 2H), 2.69 (t, J = 6 Hz, 2H), 2.6 (t, J =5.6 Hz, 2H), 2.07 (s, 3H), 2.01 (s, 3H), LC-MS calculated for C38H39N7O3 (M+H)+: m/z = 641.78; found: 642.5.

Following examples as shown in Table 12 were prepared according to similar procedure described for Example 70 with appropriate amines:

**Table 12:**

| **Ex.No** | **Structure IUPAC Name** | **LC-MS(ESI)m/z:** |
|---|---|---|
| 71 | 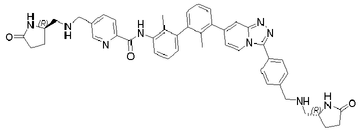 *N*-(2,2'-dimethyl-3'-(3-(4-(((((*R*)-5-oxopyrrolidin-2-yl)methyl)amino)methyl)phenyl)-[1,2,4]triazolo[4,3-*a*]pyridin-7-yl)-[1,1'-biphenyl]-3-yl)-5-(((((*R*)-5-oxopyrrolidin-2-yl)methyl)amino)methyl)picolina mide | LC-MS calculated for C44H45N9O3 (M+H)+: m/z = 747.9; found: 748.5 |
| 72 | 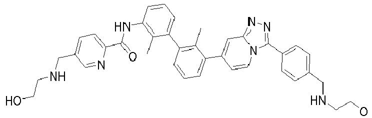 5-(((2-hydroxyethyl)amino)methyl)-*N-*(3'-(3-(4-(((2-methoxyethyl)amino)methyl)phe nyl)-[1,2,4]triazolo[4,3-*a*]pyridin-7-yl)-2,2'-dimethyl-[1,1'-biphenyl]-3-yl)picolinamide | LC-MS calculated for C39H41N7O3 (M+H)+: m/z = 655.8; found: 656.3 |

### Example 73: (2'R)-((((2,2'-dimethyl-[1,1'-biphenyl]-3,3'-diyl)bis([1,2,4]triazolo[4,3-a]pyridine-7,3-diyl))bis(4,1-phenylene))bis(methylene))di-D-proline

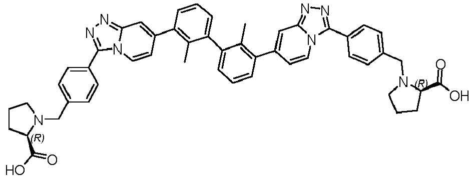

The compound of Example 73 was synthesized via the route shown in the scheme below,

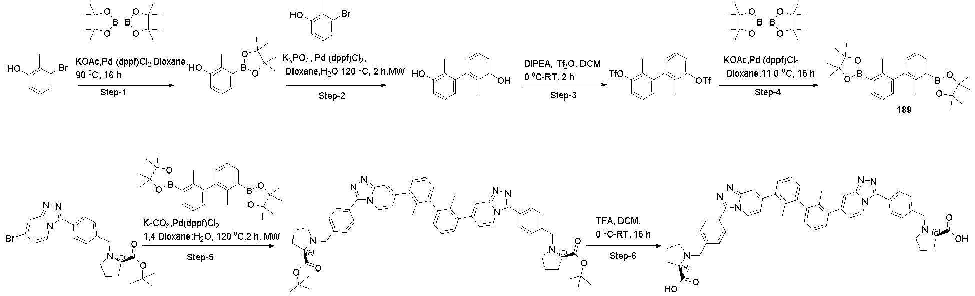

### Step-1: Synthesis of 2-methyl-3-(4,4,5,5-tetramethyl-1,3,2-dioxaborolan-2-yl)phenol

The title compound was prepared in a similar way as example 1 to give the title compound (825 mg, 76%) as an off-white solid.

### Step-2: Synthesis of 2,2'-dimethyl-[1,1'-biphenyl]-3,3'-diol

To a stirred solution of 2-methyl-3-(4,4,5,5-tetramethyl-1,3,2-dioxaborolan-2-yl)phenol (369.1 mg, 1.71 mmol) and 3-bromo-2-methylphenol (400.0 mg, 1.71 mmol) in 1,4-dioxane (5 ml) and water (1.25 ml) was added K₃PO₄ (335.4 mg, 3.42 mmol) under N₂ atmosphere. The mixture was purged with N₂ for 30 minutes and Pd(dppf)Cl₂ (62.5 mg, 0.09 mmol) was added. The reaction mixture was heated in a MW at 120 °C for 2 h. Cooled to room temperature, diluted with water, and extracted with ethyl acetate (2 x 50 mL). The combined organic layer was washed with brine, dried over Na₂SO₄, filtered, and concentrated under reduced pressure. The residue was adsorbed to silica gel and purified by combi flash column chromatography using (0-25%) Ethyl acetate in hexane to afford the title compound (181 mg, 49.4%) as an off-white solid.

### Step-3: Synthesis of 2,2'-dimethyl-[1,1'-biphenyl]-3,3'-diyl bis(trifluoromethanesulfonate)

Trifluoromethanesulfonic anhydride (0.798 mL, 4.74 mmol) was added via syringe to a stirred mixture of 2,2'-dimethyl-[1,1'-biphenyl]-3,3'-diol (1.00 g, 3.16 mmol) and DIPEA (1.38 mL, 7.90 mmol) in dichloromethane (10 mL) at 0 °C. After 30 min, ethanol (0.5 mL) was added. Reaction stirred at 0 °C for 30 min. The reaction mixture was concentrated under reduced pressure. The residue was purified by combi flash column chromatography using (0-10%) Ethyl acetate in hexane to afford the title compound (130 mg, 22%) as a brown solid.

### Step-4: Synthesis of 2,2'-(2,2'-dimethyl-[1,1'-biphenyl]-3,3'-diyl)bis(4,4,5,5-tetramethyl-1,3,2-dioxaborolane)

The title compound was prepared in a similar way as example 1 to give the title compound (130 mg, 65%) as a white solid.

### Step-5: Synthesis of di-tert-butyl ((((2,2'-dimethyl-[1,1'-biphenyl]-3,3'-diyl)bis([1,2,4]triazolo[4,3-a]pyridine-7,3-diyl))bis(4,1-phenylene))bis(methylene))(R)-di-D-prolinate

The title compound was prepared in a similar way as example 1 to get the crude compound (506 mg) as a brown semi solid. LC-MS calculated for C58H62N8O4 (M+H)+: m/z = 935.19; found: 936.50.

### Step-6: Synthesis of (2'R)-((((2,2'-dimethyl-[1,1'-biphenyl]-3,3'-diyl)bis([1,2,4]triazolo[4,3-a]pyridine-7,3-diyl))bis(4,1-phenylene))bis(methylene))di-D-proline

The title compound was prepared in a similar way as example 62 to give the title compound (35 mg, 14%) as an off-white solid.

1H NMR (400 MHz, DMSO-d6) δ: 8.60 (d, J=7.2 Hz, 2H), 7.92 (d, J = 8Hz, 4H), 7.86 (s, 2H), 7.63(d, J = 8Hz, 4H), 7.43-7.41 (m, 4H), 7.28 ( d, J = 6.8 Hz, 2H), 7.10 (d, J = 7.2 Hz, 2H), 4.14 (d, J = 13.6 Hz, 2H), 3.76(d, J = 13.6Hz, 2H), 3.05 (s, 2H), 2.6 (s, 2H), 2.13-2.06 (m, 8H), 1.92-1.76 (m, 8H), LC-MS calculated for C50H46N8O4 (M+H)+: m/z = 822.97; found: 821.6.

### Example 74: Synthesis of (S)-5-((((2',2"-dimethyl-3"-(3-(4-(((((S)-5-oxopyrrolidin-2-yl)methyl)amino)methyl)phenyl)-[1,2,4]triazolo[4,3-a]pyridin-7-yl)-[1,1':3',1"-terphenyl]-4-yl)methyl)amino)methyl)pyrrolidin-2-one

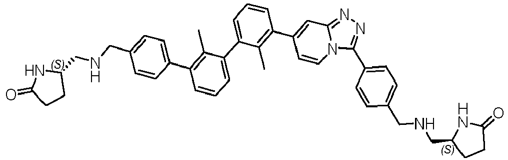

The compound of Example 74 was synthesized via the route shown in the scheme below,

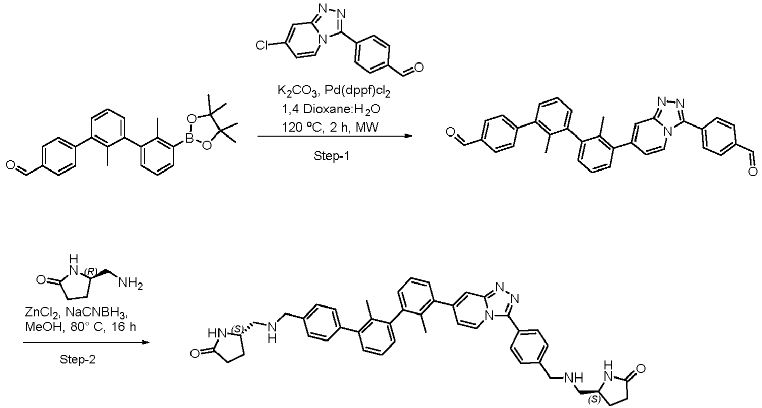

### Step-1: Synthesis of 3"-(3-(4-formylphenyl)-[1,2,4]triazolo[4,3-a]pyridin-7-yl)-2',2"-dimethyl-[1,1':3',1"-terphenyl]-4-carbaldehyde

The title compound was prepared in a similar way as example 1 to give the title compound (170 mg, 70%) as a pale brown solid. LC-MS calculated for C34H25N3O2 (M+H)+: m/z = 507.59; found: 508.20.

### Step-2: Synthesis of (S)-5-((((2',2"-dimethyl-3"-(3-(4-(((((S)-5-oxopyrrolidin-2-yl)methyl)amino)methyl)phenyl)-[1,2,4]triazolo[4,3-a]pyridin-7-yl)-[1,1':3',1"-terphenyl]-4-yl)methyl)amino)methyl)pyrrolidin-2-one

The title compound was prepared in a similar way as in example 1 to give the title compound (20 mg, 20%) as an off-white solid.

1H NMR (400 MHz, DMSO-d6) δ: 8.59 (d, J = 7.2 Hz, 1H), 7.709 (s, 1H), 7.314-7.412 (m, 7H), 7.235 (t, J = 5.6Hz, 2H), 7.15(d, J = 7.6 Hz, 1H), 4.48-4.52 (m, 2H), 4.29 (s, 2H), 3.76 (s, 2H), 3.44-3.49 (m, 4H), 2.609 (t, J = 5.2 Hz, 4H), 2.007 (s, 3H), 1.945 (s, 3H), LC-MS calculated for C44H45N7O2 (M+H)+: m/z = 703.89 found:704.6.

Following example as shown in Table 13 was prepared according to similar procedure described for Example 74 with appropriate amines:

**Table 13:**

| **Ex.No** | **Structure IUPAC Name** | **LC-MS(ESI)m/z:** |
|---|---|---|
| 75 | 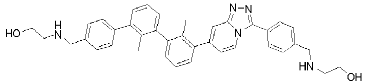 2-((4-(7-(4"-(((2-hydroxyethyl)amino)methyl)-2,2'-dimethyl-[1,1':3',1"-terphenyl]-3-yl)-[1,2,4]triazolo[4,3-*a*]pyridin-3-yl)benzyl)amino)ethan-1-ol | LC-MS calculated for C38H39N5O2 (M+H)+: m/z = 597.76; found: 598.3 |
| 76 | 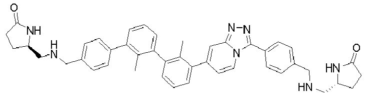 (*R*)-5-((((2',2"-dimethyl-3"-(3-(4-(((((*R*)-5-oxopyrrolidin-2-yl)methyl)amino)methyl)phenyl)-[1,2,4]triazolo[4,3-*a*]pyridin-7-yl)-[1,1':3',1"-terphenyl]-4-yl)methyl)amino)methyl)pyrrolidi n-2-one | LC-MS calculated for C44H45N7O2 (M+H)+: m/z = 703.89; found: 704.3 |

### Example 77: 2-((4-(7-(3'-(5-(((2-hydroxyethyl)amino)methyl)-6-methoxypyridin-2-yl)-2,2'-dimethyl-[1,1'-biphenyl]-3-yl)-[1,2,4]triazolo[4,3-a]pyridin-3-yl)benzyl) amino)ethan-1-ol

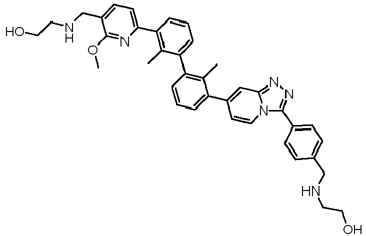

The compound of Example 77 was synthesized via the route shown in the scheme below,

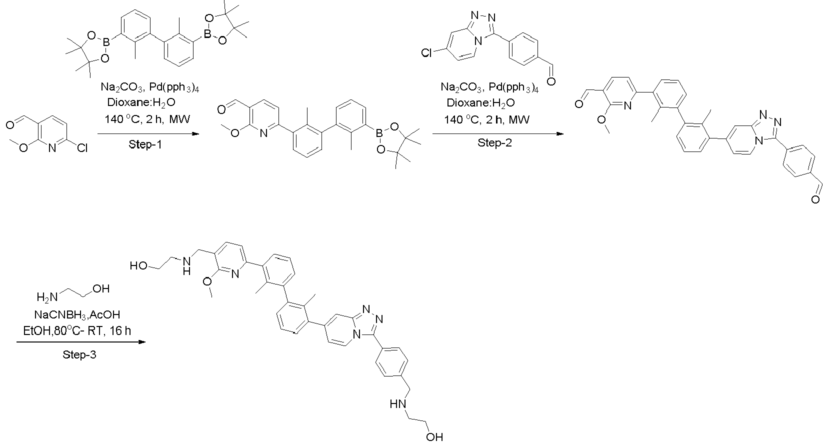

### Step-1: Synthesis of 6-(2,2'-dimethyl-3'-(4,4,5,5-tetramethyl-1,3,2-dioxaborolan-2-yl)-[1,1'-biphenyl]-3-yl)-2-methoxynicotinaldehyde

The title compound was prepared in a similar way as example 1 to give the title compound (125 mg, 24%) as a white solid. LC-MS calculated for C27H30BNO4 (M+H)+: m/z = 443.35; found: 444.15.

### Step-2: Synthesis of 6-(3'-(3-(4-formylphenyl)-[1,2,4]triazolo[4,3-a]pyridin-7-yl)-2,2'-dimethyl-[1,1'-biphenyl]-3-yl)-2-methoxynicotinaldehyde

The title compound was prepared in a similar way as example 1 to give the title compound (107 mg, 74%) as an off-white solid. LC-MS calculated for C34H26N4O3 (M+H)+: m/z = 538.61; found: 539.25.

### Step-3: Synthesis of 2-((4-(7-(3'-(5-(((2-hydroxyethyl)amino)methyl)-6-methoxypyridin-2-yl)-2,2'-dimethyl-[1,1'-biphenyl]-3-yl)-[1,2,4]triazolo[4,3-a]pyridin-3-yl)benzyl)amino)ethan-1-ol

The title compound was prepared in a similar way as in example 1 to give the title compound (19 mg, 16%) as an off-white solid.

1H NMR (400 MHz, DMSO-d6) δ: 8.59 (d, J = 7.2 Hz, 1H), 8.2 (s, 2H), 7.91 (d, J = 8 Hz, 2H), 7.85(s, 1H), 7.80 (d, J = 7.2 Hz, 1H), 7.62 ( d, J = 8 Hz, 2H), 7.45-7.35 (m, 4H), 7.23 (q, J = 6.8 Hz, 2H), 7.15(d, J = 7.2Hz, 1H), 7.09 (d, J = 7.2 Hz, 1H), 3.9 (s, 5H), 3.7 (s, 2H), 3.5 (q, J = 5.2 Hz, 4H), 2.69 (t ,J = 5.2Hz,, 4H), 2.08 (s, 3H), LC-MS calculated for C38H40N6O3 (M+H)+: m/z = 628.78; found: 629.6.

Following example as shown in Table 14 was prepared according to similar procedure described for Example 77 with appropriate amines:

**Table 14:**

| **Ex.No** | **Structure IUPAC Name** | **LC-MS(ESI)m/z:** |
|---|---|---|
| 78 | 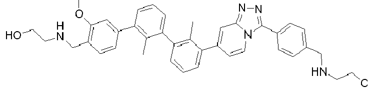 2-((4-(7-(4"-(((2-hydroxyethyl)amino)methyl)-3"-methoxy-2,2'-dimethyl-[1,1':3',1"-terphenyl]-3-yl)-[1,2,4]triazolo[4,3-*a*]pyridin-3-yl)benzyl)amino)ethan-1-ol | LC-MS calculated for C39H41N5O3 (M+H)+: m/z = 627.79; found: 628.6 |

### Example 79: (S)-5-(((4-(7-(3'-((3-(((R)-3-hydroxypyrrolidin-1-yl)methyl)-1,7-naphthyridin-8-yl)amino)-2,2'-dimethyl-[1,1'-biphenyl]-3-yl)-[1,2,4]triazolo[4,3-a]pyridin-3-yl)benzyl) amino)methyl)pyrrolidin-2-one

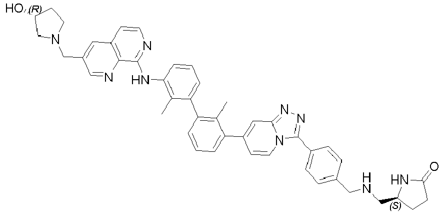

The compound of Example 79 was synthesized via the route shown in the scheme below,

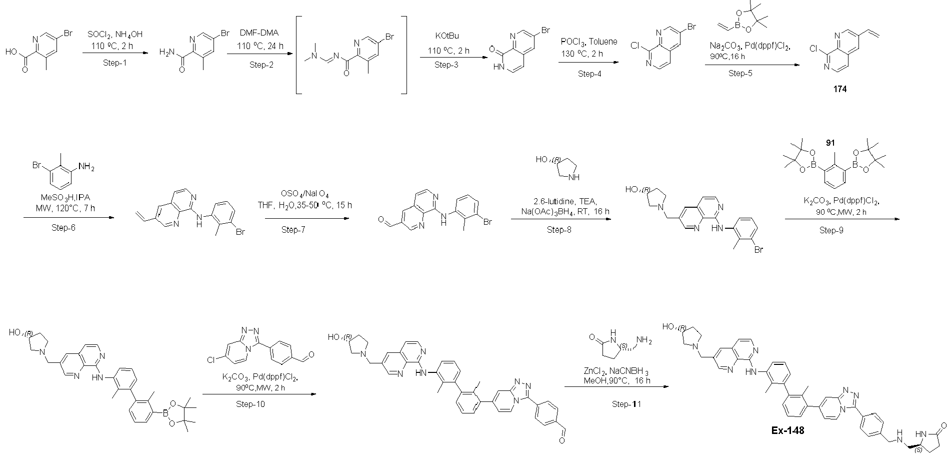

### Step-1: Synthesis of 5-bromo-3-methylpicolinamide

Thionyl chloride (4.05 mL, 55.5 mmol) was added to a stirred mixture of 5-bromo-3-methylpicolinic acid (10 g, 46.2 mmol) in Toluene (100 mL) and DMF (0.025 mL) at room temperature. The reaction mixture was heated 0 °C for 2 h. The reaction mixture was cooled to RT and ammonium hydroxide solution (60 mL) was added to the reaction mixture, solid product separated, filtered, washed with water dried under vacuum to give the title compound (9 g, 90 %) as an off-white solid.

### Steps-2 and 3: Synthesis of 3-bromo-1,7-naphthyridin-8(7H)-one

DMF-DMA (7.2 mL, 54.4 mmol) was added to a stirred mixture of 5-bromo-3-methylpicolinamide (9 g, 41.8mmol) in toluene (80 mL) at room temperature. The reaction mixture was heated at 110 °C for 24 h. The reaction mixture was cooled down to room temperature and added dropwise to a stirred solution of tBuOK (7.04 g 62.7 mmol) in toluene (80 mL). The reaction mixture again heated at 110 °C for 3 h. The organic volatiles were removed under reduce pressure and the reaction mixture adjusted to pH 7 by adding 1N HCl solution to obtain the solid, which was filtered, washed with water dried under vacuum to give the title compound (4.5 g, 48%) as a brown solid. LC-MS calculated for C8H5BrN2O (M+H)+: m/z = 225.05 found:224.95.

### Step-4: Synthesis of 3-bromo-8-chloro-1,7-naphthyridine

Phosphorous oxychloride (3.05 mL, 33.3 mmol) was added to a stirred mixture of 3-bromo-1,7-naphthyridin-8(7H)-one (2.5 g, 11.1mmol) toluene (100 mL) followed by N, N-diethyl aniline (5.2 mL, 33 mmol) at room temperature. The reaction mixture was heated at 110 °C for 2 h. Organic volatiles were removed under reduce pressure and the residue was diluted with cold water. The mixture was stirred tat room temperature for 1 h and solid separated which was filtered, washed with water dried under vacuum to obtain the title compound (2.3 g, 85%) as a brown solid. LC-MS calculated for C8H4BrCIN2 (M+H)+: m/z = 243.49 found:244.90.

### Step-5: Synthesis of 8-chloro-3-vinyl-1,7-naphthyridine

A mixture of 3-bromo-8-chloro-1,7-naphthyridine (1.7 g, 6.9 mmol), 4,4,5,5-tetramethyl-2-vinyl-1,3,2-dioxaborolane (860 mg, 5.5 mmol), in 1,4-dioxane (20 mL) was added Na₂CO₃ (1.47 g, 13.8 mmol) in water (5 mL), Pd(dppf)Cl₂ (260 mg, 0.3 mmol) and degassed with N₂ for 30 minutes then heated the reaction at 90 °C for 16 h. Diluted the reaction mixture with ethyl acetate, filtered over celite bed, washed with ethyl acetate and filtrate was evaporated. Crude material was purified by Biotage flash column chromatography using (0-30%) ethyl acetate in Hexane to obtain the title compound (700 mg, 60%) as a brown gummy mass. LC-MS calculated for C10H7CIN2 (M+H)+: m/z = 190.63 found:191.0.

### Step-6: Synthesis of N-(3-bromo-2-methylphenyl)-3-vinyl-1,7-naphthyridin-8-amine

To a solution of 8-chloro-3-vinyl-1,7-naphthyridine (200 mg, 0.1 mmol) and 3-bromo-2-methylaniline (195 mg, 0.1 mmol) in IPA (10 mL) was added methane sulfonic acid (105 mg, 0.1 mmol) at ambient temperature. The resulting reaction mixture was warmed to 60 °C and stirred at 60 °C for 7 h. Cooled to room temperature before the pH was adjusted to 8 - 9 with a 20% aqueous sodium hydroxide (NaOH) solution. The neutralized reaction mixture was stirred at ambient temperature for 30 minutes and the solids were collected by filtration. The wet cake was then purified by re-slurring in a mixture of MTBE (20 mL) and water (10 mL) at room temperature for 2 h. The solids were collected by filtration and dried under vacuum to obtain the title compound (300 mg, 85%), as a yellow solid, which was used in the subsequent reaction without further purification. LC-MS calculated for C17H14BrN3 (M+H)+: m/z 340.22= found:340.20.

### Step-7: Synthesis of 8-((3-bromo-2-methylphenyl)amino)-1,7-naphthyridine-3-carbaldehyde

To a stirred solution of *N*-(3-bromo-2-methylphenyl)-3-vinyl-1,7-naphthyridin-8-amine (260 mg, 0.7 mmol) and 2,6-dimethylpyridine (184 mg, 1.4 mmol) in THF (20 mL) and water (6 mL) was charged OsO₄ (300 mg, 1.18 mmol) at room temperature. The mixture was stirred at room temperature for 1 h before NalO₄ (330 mg, 1.5 mmol) was added. The resulting reaction mixture was then warmed to 30-35 °C and stirred at that temperature for 15 h. Water (14.0 mL) was added into the reaction mixture. The resulting mixture was stirred at room temperature for 1 h and concentrated under the reduced pressure. The solids were collected by filtration and residue was suspended in water (20.0 mL). The suspension was heated to 50 °C and stirred at 50 °C for 1 h. The solids were then collected by filtration and the wet cake was further purified by reslurrying in MTBE (6.2 mL). The suspension was heated at 50 °C for 1 h before being filtered. The collected solids were dried under vacuum to obtain the title compound (300 mg) as crude which has taken for next step without purification. LC-MS calculated for C16H12BrN3O (M+H)+: m/z = 342.20 found:343.95.

### Step-8: Synthesis of (R)-1-((8-((3-bromo-2-methylphenyl)amino)-1,7-naphthyridin-3-yl)methyl)pyrrolidin-3-ol

A mixture of 8-((3-bromo-2-methylphenyl)amino)-1,7-naphthyridine-3-carbaldehyde (220 mg, 0.6 mmol), (A)-pyrrolidin-3-ol hydrochloride (140 mg, 1.13 mmol) and triethylamine (0.150 mL, 1.13 moles) in DCM (15 mL) was stirred at room temperature for 2 h. Sodium triacetoxyborohydride (280 mg, 1.2 mmol) was added portion-wise to the mixture at room temperature over 30 min. The reaction mixture was stirred at 25-30 °C for 1 h. The reaction mixture was cooled to 0-5 °C and quenched with a 10% aqueous sodium bicarbonate solution (10 mL). The reaction mixture was then gradually warmed to room temperature and stirred at room temperature for overnight. The resulting solids were collected by filtration and washed with dichloromethane (10 mL), water (5 mL) and heptane (5 mL). The wet solid was dried under vacuum to obtain the title compound (300 mg, 115%) as a pale-yellow solid as crude which has taken for next step without purification. LC-MS calculated for C20H21BrN4O (M+H)+: m/z = 413.32, found:413.05.

### Step-9: Synthesis of (R)-1-((8-((2,2'-dimethyl-3'-(4,4,5,5-tetramethyl-1,3,2-dioxaborolan-2-yl)-[1,1'-biphenyl]-3-yl)amino)-1,7-naphthyridin-3-yl)methyl)pyrrolidin-3-ol (209)

The title compound was prepared in a similar way as example 1 to give the title compound (130 mg, 35%) as a pale-yellow solid. LC-MS calculated for C33H39BN4O3 (M+H)+: m/z = 550.51 found:551.25.

### Step-10: Synthesis of (R)-4-(7-(3'-((3-((3-hydroxypyrrolidin-1-yl)methyl)-1,7-naphthyridin-8-yl)amino)-2,2'-dimethyl-[1,1'-biphenyl]-3-yl)-[1,2,4]triazolo[4,3-a]pyridin-3-yl)benzaldehyde

The title compound was prepared in a similar way as example 1 to give the title compound (90 mg, 60%) as a pale-yellow solid. LC-MS calculated for C40H35N7O2 (M+H)+: m/z = 645.77 found:642.20.

### Step-11: Synthesis (S)-5-(((4-(7-(3'-((3-(((R)-3-hydroxypyrrolidin-1-yl)methyl)-1,7-naphthyridin-8-yl)amino)-2,2'-dimethyl-[1,1'-biphenyl]-3-yl)-[1,2,4]triazolo[4,3-a]pyridin-3-yl)benzyl)amino)methyl)pyrrolidin-2-one

The title compound was prepared in a similar way as example 1 to give the title compound as crude which was purified by prep-HPLC (X-Bridge C18 column (250 x 19) mm, 5.0 µm; mobile phase (A: B), A = 0.1% FA in H₂O, B = ACN 100%.) to give title compound (25 mg, 48%) as a pale-yellow solid.

1H NMR (400 MHz, DMSO-d6) δ:9.321 (s, 1H), 8.86 (s, 1H), 8.59 (d, J = 6.8Hz, 1H), 8.44 (d, J = 7.6 Hz, 1H), 8.18 (s, 1H), 8.05 (d, J = 6 Hz, 1H), 7.9 ( d, J = 7.6 Hz, 2H), 7.86 (s, 1H), 7.68 (s, 1H), 7.60 (d, J = 8.4Hz, 2H), 7.4 (t, J = 6.8 Hz, 2H), 7.339 (t, J = 8Hz, 1H), 7.24 (d, J = 6.8 Hz, 1H), 7.175 (d, J = 5.6 Hz, 1H), 7.095 (d ,J = 6.8Hz, 1H), 6.94 (d, J = 7.6 Hz, 1H), 4.720 (d, J =4.4 Hz, 1H), 4.220 (s, 1H), 3.8-3.86 (m, 4H), 3.64 (s, 1H), 2.72-2.76 (m, 1H),2.66 (d, J = 8 Hz, 2H), 2.63 (s, 1H), 2.38 (dd, J = 9.6 Hz, J =9.6 Hz, 2H), 2.125 (d, J = 13.2 Hz, 6H) 2.04 (s, 3H), 1.709 (d, J = 4.8 Hz, 1H), LC-MS calculated for C45H45N9O2 (M+H)+: m/z = 743.92; found: 744.3.

Following examples as shown in Table 15 were prepared according to similar procedure described for Example 79 with appropriate amines:

**Table 15:**

| **Ex.No** | **Structure IUPAC Name** | **LC-MS(ESI)m/z:** |
|---|---|---|
| **80** | 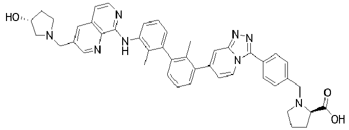 (4-(7-(3'-((3-(((*R*)-3-hydroxypyrrolidin-1-yl)methyl)-1,7-naphthyridin-8-yl)amino)-2,2'-dimethyl-[1,1'-biphenyl]-3-yl)-[1,2,4]triazolo[4,3-*a*]pyridin-3-yl)benzyl)-*D*-proline | LC-MS calculated for C45H44N8O3 (M+H)+: m/z = 744.90; found: 745.35 |
| **81** | 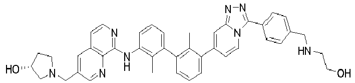 (*R*)-1-((8-((3'-(3-(4-(((2-hydroxyethyl)amino)methyl)phen yl)-[1,2,4]triazolo[4,3-*a*]pyridin-7-yl)-2,2'-dimethyl-[1,1'-biphenyl]-3-yl)amino)-1,7-naphthyridin-3-yl)methyl)pyrrolidin-3-ol | LC-MS calculated for C42H42N8O2 (M+H)+: m/z = 690.85; found: 691.2 |

### Example 82: tert-butyl (4-(7-(3'-(5-((2-hydroxyethyl)carbamoyl)-6-methoxypyridin-2-yl)-2,2'-dimethyl-[1,1'-biphenyl]-3-yl)-[1,2,4]triazolo[4,3-a]pyridin-3-yl)benzyl)-D-prolinate

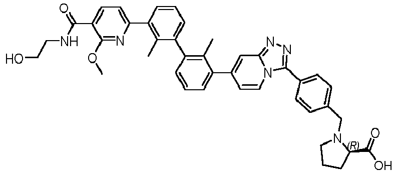

The compound of Example 82 was synthesized via the route shown in the scheme below,

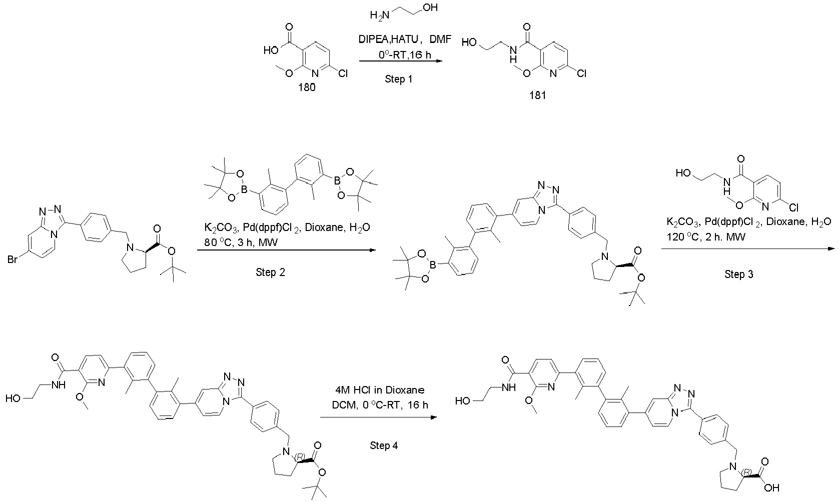

### Step-1 : Synthesis of 6-chloro-N-(2-hydroxyethyl)-2-methoxynicotinamide

The title compound was prepared in a similar way as Example 68 to give the title compound (446 mg, 80%) as an off-white solid. LC-MS calculated for C9H11CIN2O3 (M+H)+: m/z = 230.65 found:231.

### Step-2: Synthesis of tert-butyl (4-(7-(2,2'-dimethyl-3'-(4,4,5,5-tetramethyl-1,3,2-dioxaborolan-2-yl)-[1,1'-biphenyl]-3-yl)-[1,2,4]triazolo[4,3-a]pyridin-3-yl)benzyl)-D-prolinate

The title compound was prepared in a similar way as Example 1 to give the title compound (56 mg, 76%) as an off-white solid. LC-MS calculated for C42H49BN4O4 (M+H)+: m/z = 684.69; found: 685.30.

### Step-3: Synthesis of tert-butyl (4-(7-(3'-(5-((2-hydroxyethyl)carbamoyl)-6-methoxypyridin-2-yl)-2,2'-dimethyl-[1,1'-biphenyl]-3-yl)-[1,2,4]triazolo[4,3-a]pyridin-3-yl)benzyl)-D-prolinate

The title compound was prepared in a similar way as Example 1 to give the title compound (56 mg, 76%) as an off-white solid. LC-MS calculated for C45H48N63O5 (M+H)+: m/z = 752.92; found: 753.65.**Step-4**: **Synthesis of (4-(7-(3'-(5-((2-hydroxyethyl)carbamoyl)-6-methoxypyridin-2-yl)-2,2'-dimethyl-[1,1'-biphenyl]-3-yl)-[1,2,4]triazolo[4,3-*a*]pyridin-3-yl)benzyl)-*D*-proline**

The title compound was prepared in a similar way as Example 39 to give the title compound (25 mg, 10%) as a white solid.

1H NMR (400 MHz, DMSO-d6) δ:8.61 (d, J =7.2 Hz 1H), 8.34-8.28 (m, 2H), 7.92 (d, J = 8Hz, 2H), 7.86 (s, 1H), 7.6(d, J = 8Hz, 2H), 7.49(d, J = 7.2 Hz, 1H), 7.44-7.39 (m, 3H), 7.33(d, J = 8.1Hz, 1H), 7.26(d ,J = 4.4Hz,, 2H), 7.1 (d, J = 7.2 Hz, 1H), 4.8 (s, 1H), 4.14(d, J = 13.2 Hz, 1H), 4.02(s, 3H), 3.79 (d, J = 12.8 Hz, 1H), 3.53(s, 3H), 3.42-.32 (m, 2H), 3.05 (s, 1H), 2.4-2.55 (m, 2H), 2.16-1.75 (m, 10 H). LC-MS calculated for C41H40N6O5 (M+H)+: m/z = 696.81; found: 697.5.

### Example 83: ethyl (1-(7-(4"-(((2-hydroxyethyl)amino)methyl)-2,2'-dimethyl-[1,1':3',1"-terphenyl]-3-yl)-[1,2,4]triazolo[4,3-a]pyridin-3-yl)-1H-pyrazole-3-carbonyl)glycinate

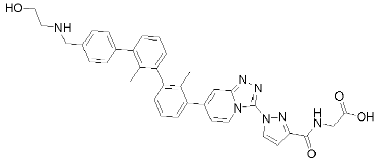

The compound of Example 83 was synthesized via the route shown in the scheme below,

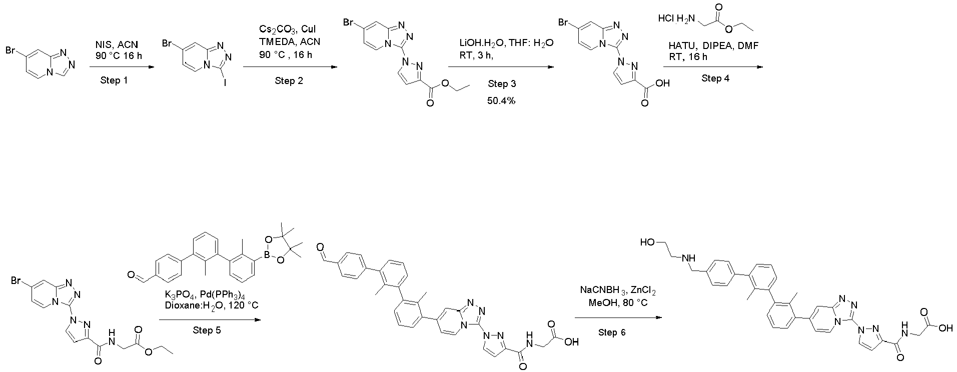

### Step-1: Synthesis of 7-bromo-3-iodo-[1,2,4]triazolo[4,3-a]pyridine

The title compound was prepared in a similar way as Example 1 to give the title compound (5 g, 55.5%) as a brown solid.

### Step-2: Synthesis of ethyl 1-(7-bromo-[1,2,4]triazolo[4,3-a]pyridin-3-yl)-1H-pyrazole-3-carboxylate

The title compound was prepared in a similar way as Example 1 to give the title compound (1.2 g, 57.6%) as a yellow solid.

### Step-3: Synthesis of 1-(7-bromo-[1,2,4]triazolo[4,3-a]pyridin-3-yl)-1H-pyrazole-3-carboxylic acid

The title compound was prepared in a similar way as Example 41 to give the title compound (260 mg, 43.6%) as a yellow gummy liquid.

### Step-4: Synthesis of ethyl (1-(7-bromo-[1,2,4]triazolo[4,3-a]pyridin-3-yl)-1H-pyrazole-3-carbonyl)glycinate

The title compound was prepared in a similar way as Example 68 to give the title compound (250 mg, 75.1%) as a yellow gummy solid.

### Step-5: Synthesis of (1-(7-(4"-formyl-2,2'-dimethyl-[1,1':3',1"-terphenyl]-3-yl)-[1,2,4]triazolo[4,3-a]pyridin-3-yl)-1H-pyrazole-3-carbonyl)glycine

The title compound was prepared in a similar way as Example 1 to give the title compound (250 mg, 61.5%) as a white solid.

### Step-6: Synthesis of (1-(7-(4"-(((2-hydroxyethyl)amino)methyl)-2,2'-dimethyl-[1,1':3',1"-terphenyl]-3-yl)-[1,2,4]triazolo[4,3-a]pyridin-3-yl)-1H-pyrazole-3-carbonyl)glycine

The title compound was prepared in a similar way as Example 1 to give the title compound (16 mg, 5.9%) as a white solid.

¹H NMR (400 MHz, DMSO-*d*₆) *δ*: 9.03 (d, J = 7.2 Hz, 1H), 8.76-8.76 (m, 1H), 8.68 (s, 1H), 7.89 (s, 1H), 7.46-7.42 (m, 4H), 7.40-7.32 (m, 3H), 7.27-7.16 (m, 4H), 7.06 (d, J= 2.4 Hz, 1H) 5.15-4.51 (br.s, 2H), 3.87-3.84 (m, 4H), 3.54 (t, J= 5.8 Hz, 2H), 2.71 (t, J= 5.8Hz, 2H), 2.03 (s, 3H), 1.94 (s, 3H), LC-MS calculated for C35H33N7O4 (M+H)+: m/z = 615.6; found: 616.3.

### Example 84: 2-((4-(7-(3'-((3-(((2-hydroxyethyl)amino)methyl)-1,7-naphthyridin-8-yl)amino)-2,2'-dimethyl-[1,1'-biphenyl]-3-yl)-[1,2,4]triazolo[4,3-a]pyridin-3-yl)benzyl)amino)ethan-1-ol

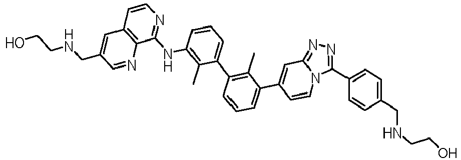

The compound of Example 84 was synthesized via the route shown in the scheme below,

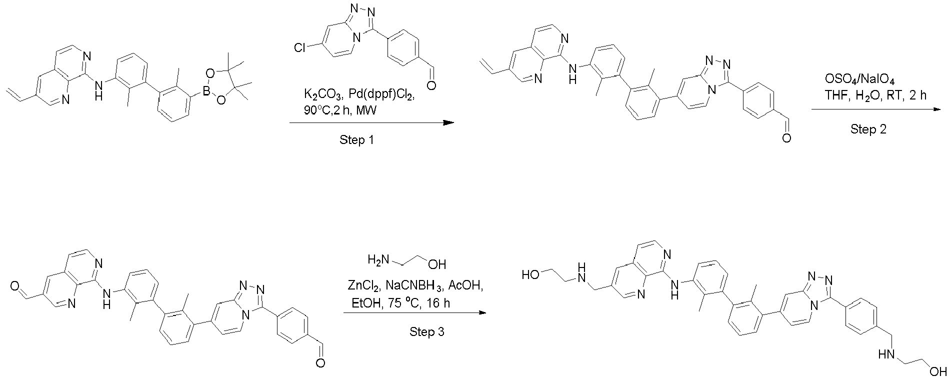

### Step-1: Synthesis of 4-(7-(2,2'-dimethyl-3'-((3-vinyl-1,7-naphthyridin-8-yl)amino)-[1,1'-biphenyl]-3-yl)-[1,2,4]triazolo[4,3-a]pyridin-3-yl)benzaldehyde

The title compound was prepared in a similar way as Example 1 to give the title compound (60 mg, 50 %) as a pale-yellow solid. LC-MS calculated for C37H28N6O (M+H)+: m/z = 572.67, found:573.40.

### Step-2: Synthesis of 8-((3'-(3-(4-formylphenyl)-[1,2,4]triazolo[4,3-a]pyridin-7-yl)-2,2'-dimethyl-[1,1'-biphenyl]-3-yl)amino)-1,7-naphthyridine-3-carbaldehyde

The title compound was prepared in a similar way as Example 79 to give the title compound (10 mg, 12 %) as a pale-yellow solid. LC-MS calculated for C36H26N6O2 (M+H)+: m/z = 574.64, found:575.35.

### Step-3: Synthesis of 2-((4-(7-(3'-((3-(((2-hydroxyethyl)amino)methyl)-1,7-naphthyridin-8-yl)amino)-2,2'-dimethyl-[1,1'-biphenyl]-3-yl)-[1,2,4]triazolo[4,3-a]pyridin-3-yl)benzyl)amino)ethan-1-ol

The title compound was prepared in a similar way as Example 1 to give rude which was purified by prep-HPLC (X-Bridge C18 column (250 x 19) mm, 5.0 µm; mobile phase (A: B), A = 0.1% FA in H₂O, B = ACN 100%.) to give the title compound (60 mg, 12.6 %) as a yellow solid. 1H NMR (400 MHz, DMSO-d6) δ:9.32 (s, 1H), 8.90 (s, 1H), 8.59 (d, J = 6.8Hz, 1H), 8.46 (d, J = 8 Hz, 1H), 8.2(s, 1H), 8.05(d, J = 5.6 Hz, 1H), 7.9(d, J = 8Hz, 2H), 7.86(s, 1H), 7.6(d ,J = 7.6Hz, 2H), 7.32-7.42 (m, 3H), 7.24 (d, J = 6.8 Hz, 1H), 7.16(d, J = 6 Hz, 1H), 7.1 (d, J = 7.2 Hz, 1H), 6.94(d, J = 7.6 Hz, 1H) , 4.51(s, 2H), 3.96 (s, 2H), 3.86 (s, 2H), 3.5 (s, 4H), 2.5-2.69 (m, 4H), 2.14 (s, 3H), 2.04 (s, 3H). LC-MS calculated for C40H40N8O2 (M+H)+: m/z = 664.81; found: 665.4.

### Example 85: 2-(((3"-(3-((2-hydroxyethyl)amino)-[1,2,4]triazolo[4,3-a]pyridin-7-yl)-2',2"-dimethyl-[1,1':3',1"-terphenyl]-4-yl)methyl)amino)ethan-1-ol

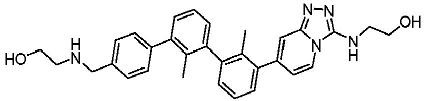

The compound of Example 85 was synthesized via the route shown in the scheme below.

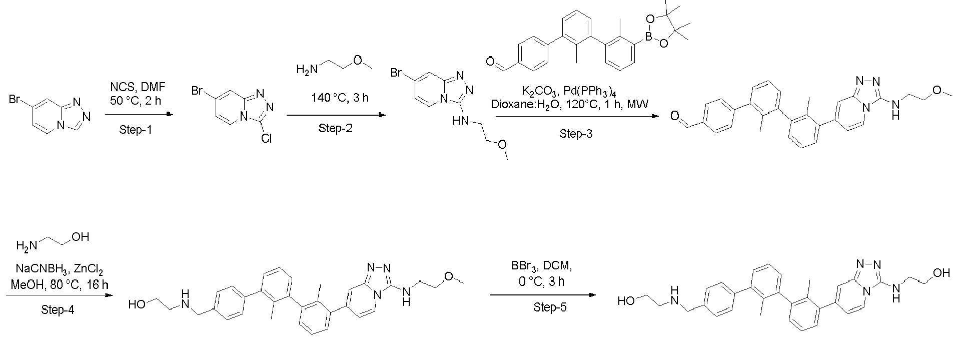

### Step-1: Synthesis of 7-bromo-3-chloro-[1,2,4]triazolo[4,3-a]pyridine

To a stirred solution of 7-bromo-[1,2,4]triazolo[4,3-*a*]pyridine (500 mg, 2.55 mmol) in DMF (10 mL) was added NCS (509 mg, 3.82 mmol) at room temperature under N₂ atmosphere. The resulted solution was stirred at 50 °C for 2 h. The reaction mixture was cooled to room temperature and diluted with water (20 mL) and extracted with ethyl acetate (2 x 10 mL). The combined organic layer was washed with water (2 x 10 mL), brine solution (10 mL), dried over anhydrous Na₂SO₄, filtered and evaporated under reduced vacuum to afford the title compound (520 mg, 88%) as a pale-yellow solid. LC-MS calculated for C40H40N8O2 (M+H)+: m/z = 232.4; found: 234.1.

### Step-2: Synthesis of 7-bromo-N-(2-methoxyethyl)-[1,2,4]triazolo[4,3-a]pyridin-3-amine

A mixture of 7-bromo-3-chloro-[1,2,4]triazolo[4,3-*a*]pyridine (500 mg, 2.15 mmol) and 2-methoxyethan-1-amine (1.0 ml) was stirred at 140 °C for 3 h in a sealed vessel. The reaction mixture was cooled to room temperature and diluted with DCM (10 ml), adsorbed to silica gel and purified by combi flash column chromatography using (50-60%) Ethyl acetate in hexane to give the title compound (130 mg, 22%) as an off-white. LC-MS calculated for C9H11BrN4O (M+H)+: m/z = 271.12; found: 271.3.

### Step-3: Synthesis of 3"-(3-((2-methoxyethyl)amino)-[1,2,4]triazolo[4,3-a]pyridin-7-yl)-2',2"-dimethyl-[1,1':3',1"-terphenyl]-4-carbaldehyde

The title compound was prepared in a similar way as example 1 to give the title compound (100 mg, 44%) as pale-yellow solid. LC-MS calculated for C30H28N4O2 (M+H)+: m/z = 476.5; found: 477.2.

### Step-4: Synthesis of 2-(((3"-(3-((2-methoxyethyl)amino)-[1,2,4]triazolo[4,3-a]pyridin-7-yl)-2',2"-dimethyl-[1,1':3',1"-terphenyl]-4-yl)methyl)amino)ethan-1-ol

The title compound was prepared in a similar way as in example 1 to give the title compound (100 mg, 60%) as white solid. LC-MS calculated for C32H35N5O2 (M+H)+: m/z = 521.6; found: 522.5.

### Step-5: Synthesis of 2-(((3"-(3-((2-hydroxyethyl)amino)-[1,2,4]triazolo[4,3-a]pyridin-7-yl)-2',2"-dimethyl-[1,1':3',1"-terphenyl]-4-yl)methyl)amino)ethan-1-ol

BBr₃ (0.036 mL, 0.383 mmol), was added to a mixture of 2-(((3"-(3-((2-methoxyethyl)amino)-[1,2,4]triazolo[4,3-*a*]pyridin-7-yl)-2',2"-dimethyl-[1,1':3',1"-terphenyl]-4-yl)methyl)amino) ethan-1-ol (100 mg, 0.191 mmol) in DCM (10 ml) at 0 °C and stirred the reaction mixture at room temperature for 2 h. quenched the reaction mixture with NaHCO₃ (10 mL),extracted with DCM (10 mL X 2), combined organic layer was washed with brine, dried over Na₂SO₄, filtered and concentrated the solvent under reduced pressure to obtain the title compound (90 mg, crude).. The crude material was purified by prep-HPLC (X-Bridge C18 column (250 x 19) mm, 5.0 µm; mobile phase (A: B), A = 0.1% FA in H₂O, B = ACN 100%.) to obtain the title compound (7.0 mg, 7.5 %) as off-white solid.

1H NMR (400 MHz, DMSO-d6) δ: 8.60 (d, J= 7.2 Hz, 1H), 7.40- 7.30 (m, 8 H), 7.21 (d, J= 7.2 Hz, 2H), 7.14 (d, J= 7.2 Hz, 1H), 6.90 (dd, J=6.8 Hz, 1.9 Hz, 1H), 6.43-6.41 (m, 1H), 4.63 (t, J= 5.4 Hz, 1H), 4.44 (t, J= 5.4 Hz, 1H), 3.75 (s, 2H), 3.56 (q, 6.0 Hz, 2H), 3.48 (q, 6.0 Hz, 2H), 3.34-3.29 (m, 2H), 2.61 (t, J= 5.8 Hz, 2H), 1.98 (s, 3H), 1.93 (s, 3H). LC-MS calculated for C31H33N5O2 (M+H)+: m/z = 507.64; found: 508.5.

### Example 86: (S)-5-((((7-(4"-(((2-hydroxyethyl)amino)methyl)-2,2'-dimethyl-[1,1':3',1"-terphenyl]-3-yl)-[1,2,4]triazolo[4,3-a]pyridin-3-yl)methyl)amino)methyl)pyrrolidin-2-one

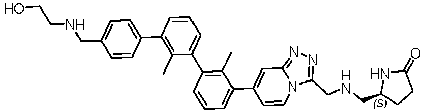

The compound of Example 86 was synthesized via the route shown in the scheme below,

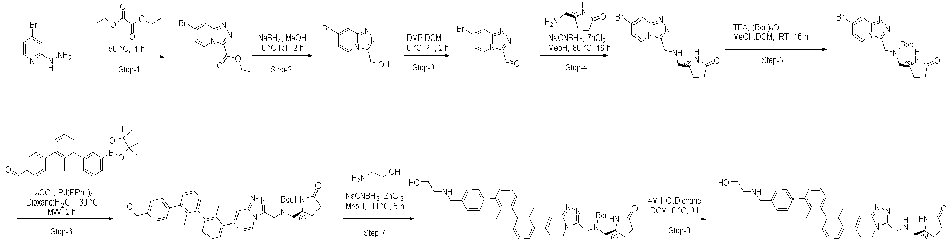

### Step-1: Synthesis of ethyl 7-bromo-[1,2,4]triazolo[4,3-a]pyridine-3-carboxylate

A mixture of 4-bromo-2-hydrazineylpyridine (6 g, 31.9 mmol) and diethyl oxalate (7.0 ml, 51.3 mmol) was stirred at 150 °C for 1 h in a sealed tube. The reaction mixture was cooled to room temperature, diluted with water (50 ml) and stirred for 30 min at room temperature. The solids formed were filtered and dried under vacuum to afford the title compound (4.0 g, 45.9%) as a yellow solid. LC-MS calculated for C9H8BrN3O2 (M+H)+: m/z = 270.09; found: 271.9.

### Step-2: Synthesis of (7-bromo-[1,2,4]triazolo[4,3-a]pyridin-3-yl)methanol

The title compound was prepared in a similar way as example 68 to give the title compound (2.5 g, 74.1%) as a yellow solid.

### Step-3: Synthesis of 7-bromo-[1,2,4]triazolo[4,3-a]pyridine-3-carbaldehyde

The title compound was prepared in a similar way as example 68 to give the title compound (1.5 g, 61%) as a yellow solid. LC-MS calculated for C7H4BrN3O (M)+: m/z = 226.03; found: 226.15.

### Step-4: Synthesis of (S)-5-((((7-bromo-[1,2,4]triazolo[4,3-a]pyridin-3-yl)methyl)amino)methyl)pyrrolidin-2-one

The title compound was prepared in a similar way as example 1 to give the title compound (950 mg, 68%) as a white solid. LC-MS calculated for C12H14BrN5O (M+H)+: m/z = 323.04; found: 324.

### Step-5: Synthesis of tert-butyl (S)-((7-bromo-[1,2,4]triazolo[4,3-a]pyridin-3-yl)methyl)((5-oxopyrrolidin-2-yl)methyl)carbamate

To a stirred solution of (*S*)-5-((((7-bromo-[1,2,4]triazolo[4,3-*a*]pyridin-3-yl)methyl)amino)methyl)pyrrolidin-2-one (950 mg, 2.93 mmol) in MeOH: DCM (5%)(30 ml) was added triethylamine (900 mg, 8.9 mmol) at room temperature followed by di-tert-butyl decarbonate (0.826 mg, 3.8 mmol) at room temperature under N₂ atmosphere, stirred for 16 h. The reaction mixture was diluted with water (30 ml) and extracted using DCM (3 x 30 ml). The combined organic layer was washed with water (20 ml), brine solution (10 ml), dried over sodium sulphate, filtered, and concentrated under reduced vacuum. The crude was purified by combi flash column chromatography using (0-5%) MeOH:DCM to afford title compound (290.0 mg , 23.3 %) as a pale yellow gummy solid. LC-MS calculated for C17H22BrN5O3 (M+H)+: m/z = 424.3; found: 424.2.

### Step-6: Synthesis of tert-butyl (S)-((7-(4"-formyl-2,2'-dimethyl-[1,1':3',1"-terphenyl]-3-yl)-[1,2,4]triazolo[4,3-a]pyridin-3-yl)methyl)((5-oxopyrrolidin-2-yl)methyl)carbamate

The title compound was prepared in a similar way as example 1 to give the title compound (90 mg, 43.4%) as a yellow gummy liquid. LC-MS calculated for C38H39BrN5O4 (M+H)+: m/z = 629.76; found: 630.2.

### Step-7: Synthesis of tert-butyl (S)-((7-(4"-(((2-hydroxyethyl)amino)methyl)-2,2'-dimethyl-[1,1':3',1"-terphenyl]-3-yl)-[1,2,4]triazolo[4,3-a]pyridin-3-yl)methyl)((5-oxopyrrolidin-2-yl)methyl)carbamate

The title compound was prepared in a similar way as example 1 to give the title compound (90 mg, 93.3%) as a white solid. LC-MS calculated for C40H46N6O4 (M+H)+: m/z = 674.85; found: 675.4

### Step-8: Synthesis of (S)-5-((((7-(4"-(((2-hydroxyethyl)amino)methyl)-2,2'-dimethyl-[1,1':3',1"-terphenyl]-3-yl)-[1,2,4]triazolo[4,3-a]pyridin-3-yl)methyl)amino)methyl) pyrrolidin-2-one

The title compound was prepared in a similar way as example 39 to give the crude which was purified by prep-HPLC (X-Bridge C18 column (250 x 19) mm, 5.0 µm; mobile phase (A: B), A = 0.1% FA in H₂O, B = ACN 100%.) to give the title compound (22 mg, 28.7%) as a white solid.

1H NMR (400 MHz, DMSO-d6) δ: 8.58 (d, J= 7.2 Hz, 1H), 7.71 (s, 1H), 7.64 (s, 1H), 7.41-7.31 (m, 7H), 7.25-7.21 (m, 2H), 7.15 (d, J= 7.6 Hz, 1H), 7.05 (d, J= 6.4 Hz, 1H), 4.48 (t, J= 5.4 Hz, 1H), 4.29 (s, 2H), 3.76 (s, 2H), 3.60- 3.58 (m, 1H), 3.49 (q, J= 5.6 Hz, 2H), 2.61 (t, J= 5.8 Hz, 2H), 2.56-2.50 (m, 2H), 2.11-2.03 (m, 6H), 2.00 (s, 3H), 1.94 (s, 3H), 1.64-1.63 (m, 1H). LC-MS calculated for C35H38N6O2 (M+H)+: m/z = 574.73; found: 575.3.

### Example 87: (R)-1-((7-(4"-(((2-hydroxyethyl)amino)methyl)-2,2'-dimethyl-[1,1':3',1"-terphenyl]-3-yl)-[1,2,4]triazolo[4,3-a]pyridin-3-yl)methyl)pyrrolidine-3-carboxylic acid

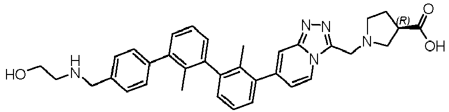

The compound of Example 87 was synthesized via the route shown in the scheme below,

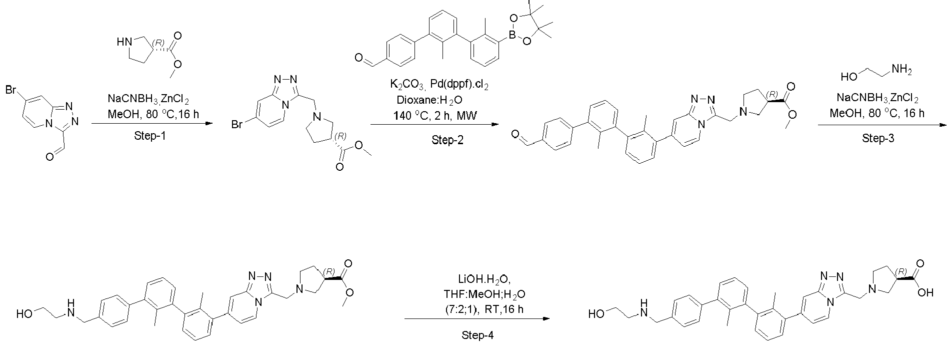

### Step-1: Synthesis of methyl (R)-1-((7-bromo-[1,2,4]triazolo[4,3-a]pyridin-3-yl)methyl)pyrrolidine-3-carboxylate

The title compound was prepared in a similar way as example 1 to give the title compound (76 mg, 17%) as a white solid. LC-MS calculated for C44H45N7O2 (M+H)+: m/z = 574.64, found:575.35.

### Step-2: Synthesis of methyl (R)-1-((7-(4"-formyl-2,2'-dimethyl-[1,1':3',1"-terphenyl]-3-yl)-[1,2,4]triazolo[4,3-a]pyridin-3-yl)methyl)pyrrolidine-3-carboxylate

The title compound was prepared in a similar way as example 1 to give the title compound (96.4 mg, 73%) as a pale-yellow semi solid. LC-MS calculated for C34H32N4O3 (M+H)+: m/z = 544.66; found: 545.30.

### Step-3: Synthesis of methyl (R)-1-((7-(4"-(((2-hydroxyethyl)amino)methyl)-2,2'-dimethyl-[1,1':3',1"-terphenyl]-3-yl)-[1,2,4]triazolo[4,3-a]pyridin-3-yl)methyl)pyrrolidine-3-carboxylate

The title compound was prepared in a similar way as example 1 to give the title compound (140 mg) as a crude, which has taken for next step without purification.

### Step-4: Synthesis of (R)-1-((7-(4"-(((2-hydroxyethyl)amino)methyl)-2,2'-dimethyl-[1,1':3',1"-terphenyl]-3-yl)-[1,2,4]triazolo[4,3-a]pyridin-3-yl)methyl)pyrrolidine-3-carboxylic acid

The title compound was prepared in a similar way as example 41 to give the crude which was purified by prep-HPLC (X-Bridge C18 column (250 x 19) mm, 5.0 µm; mobile phase (A: B), A = 0.1% FA in H₂O, B = ACN 100%.) to give the title compound (35 mg, 37%) as an off-white solid.

1H NMR (400 MHz, DMSO-d6) δ: 8.528 (d, J= 7.2 Hz, 1H), 7.726 (s, 1H), 7.318-7.421 (m, 7H), 7.235 (t, J = 6.4 Hz, 2H), 7.15 (d, J = 7.6 Hz, 1H), 7.063 (d, J= 7.2 Hz, 1H), 4.5 (s, br, 1H), 4.18 (s, 2H), 3.77 (s, 2H), 3.5 (t, J = 5.2 Hz, 2H), 2.9- 2.95 (m, 1H), 2.8 (t, J= 9.2 Hz, 1H), 2.502-2.722 (m, 6H), 1.944-2.0 (m, 7H), LC-MS calculated for C35H37N5O3 (M+H)+: m/z = 575.71; found: 576.7.

Following example as shown in Table 16 was prepared according to similar procedure described for Example 87 with appropriate amines:

**Table 16:**

| **Ex.No** | **Structure IUPAC Name** | **LC-MS(ESI)m/z: (M+1)** |
|---|---|---|
| **88** | 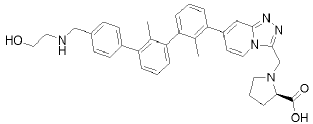 **((7-(4"-(((2-hydroxyethyl)amino)methyl)-2,2'-dimethyl-[1,1':3',1"-terphenyl]-3-yl)-[1,2,4]triazolo[4,3-*a*]pyridin-3-yl)methyl)-*D*-proline** | LC-MS calculated for C35H37N5O3 (M+H)+: m/z = 575.71; found: 576.2 |

### Example 89: 2,2'-((((2,2'-dimethyl-[1,1'-biphenyl]-3,3'-diyl)bis([1,2,4]triazolo[4,3-a]pyridine-7,3-diyl))bis(methylene))bis(azanediyl))bis(ethan-1-ol)

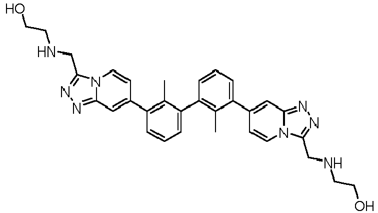

The compound of Example 89 was synthesized via the route shown in the scheme below,

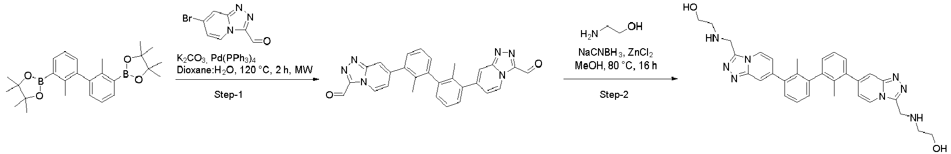

### Step-1: Synthesis of 7,7'-(2,2'-dimethyl-[1,1'-biphenyl]-3,3'-diyl)bis([1,2,4]triazolo[4,3-a]pyridine-3-carbaldehyde)

The title compound was prepared in a similar way as example 1 to give the title compound (55 mg, 34%) aa a white solid. LC-MS calculated for C28H20N6O2 (M+H)+: m/z = 472.51; found: 473.5.

### Step-2: Synthesis of 2,2'-((((2,2'-dimethyl-[1,1'-biphenyl]-3,3'-diyl)bis([1,2,4]triazolo[4,3-a]pyridine-7,3-diyl))bis(methylene))bis(azanediyl))bis(ethan-1-ol)

The title compound was prepared in a similar way as example 1 to give the crude which was purified by prep-HPLC (X-Bridge C18 column (250 x 19) mm, 5.0 µm; mobile phase (A: B), A = 0.1% FA in H₂O, B = ACN 100%.) to give the title compound (1.4 mg, 2.5 %) as an off-white solid.

**¹H NMR (400 MHz, DMSO-*d₆*) *δ***: 8.58 (d, J= 6.8 Hz, 1H), 7.71-7.64 (m, 3H), 7.42-7.31 (m, 7H), 7.23 (t, J= 6.8 Hz, 2H), 7.15 (d, J= 7.2 Hz, 1H), 7.05 (d, J= 6.4 Hz, 1H), 4.29 (s, 2H), 3.76-3.75 (m, 2H), 3.62-3.59 (m, 2H), 2.54-2.50 (m, 2H), 2.13-2.05(m, 7H), 2.00 (s, 3H), 1.94 (s, 3H), 1.70-1.63 (m, 3H).

LC-MS calculated for C32H34N8O2 (M+H)+: m/z = 562.68; found: 563.3.

### Example 90: (S)-5-((((2',2"-dimethyl-3"-(3-(((((S)-5-oxopyrrolidin-2-yl)methyl)amino)methyl)-[1,2,4]triazolo[4,3-a]pyridin-7-yl)-[1,1':3',1"-terphenyl]-4-yl)methyl)amino)methyl)pyrrolidin-2-one

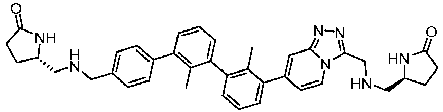

The compound of Example 90 was synthesized via the route shown in the scheme below,

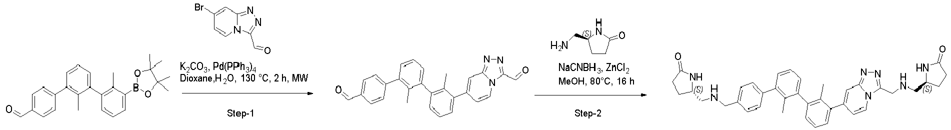

### Step-1: Synthesis of 7-(4"-formyl-2,2'-dimethyl-[1,1':3',1"-terphenyl]-3-yl)-[1,2,4]triazolo[4,3-a]pyridine-3-carbaldehyde

The title compound was prepared in a similar way as example 1 to give the title compound (110 mg, 42%) as an off-white solid. LC-MS calculated for C28H21N3O2 (M+H)+: m/z = 431.51; found: 432.30.

### Step-2: Synthesis of (S)-5-((((2',2"-dimethyl-3"-(3-(((((S)-5-oxopyrrolidin-2-yl)methyl)amino)methyl)-[1,2,4]triazolo[4,3-a]pyridin-7-yl)-[1,1':3',1"-terphenyl]-4-yl)methyl)amino)methyl)pyrrolidin-2-one

The title compound was prepared in a similar way as example 1 to give the crude which was purified by prep-HPLC (X-Bridge C18 column (250 x 19) mm, 5.0 µm; mobile phase (A: B), A = 0.1% FA in H₂O, B = ACN 100%.) to give the title compound (3 mg, 3.7%) as an off-white solid.

**¹H NMR (400 MHz, DMSO-*d₆*) *δ*:** 8.58 (d, J= 6.8 Hz, 1H), 7.71-7.64 (m, 3H), 7.42-7.31 (m, 7H), 7.23 (t, J= 6.8 Hz, 2H), 7.15 (d, J= 7.2 Hz, 1H), 7.05 (d, J= 6.4 Hz, 1H), 4.29 (s, 2H), 3.76-3.75 (m, 2H), 3.62-3.59 (m, 2H), 2.54-2.50 (m, 2H), 2.13-2.05(m, 7H), 2.00 (s, 3H), 1.94 (s, 3H), 1.70-1.63 (m, 3H). LC-MS calculated for C38H41N7O2 (M+H)+: m/z = 627.79; found: 628.3.

Following example as shown in Table 17 was prepared according to similar procedure described for Example 90 with appropriate amines:

**Table 17:**

| **Ex.No** | **Structure IUPAC Name** | **LC-MS(ESI)m/z:** |
|---|---|---|
| **91** | 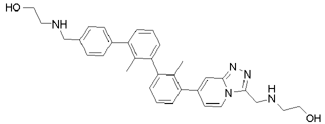 2-(((7-(4"-(((2-hydroxyethyl)amino)methyl)-2,2'-dimethyl-[1,1':3',1"-terphenyl]-3-yl)-[1,2,4]triazolo[4,3-*a*]pyridin-3-yl)methyl)amino)ethan-1-ol | LC-MS calculated for C32H35N5O2 (M+H)+: m/z = 521.67; found: 522.25 |

### Example 92: 5-(((2-hydroxyethyl)amino)methyl)-N-(3'-(3-(((2-hydroxyethyl)amino)methyl)-[1,2,4]triazolo[4,3-a]pyridin-7-yl)-2,2'-dimethyl-[1,1'-biphenyl]-3-yl)picolinamide

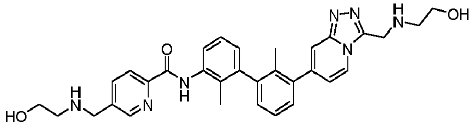

The compound of Example 92 was synthesized via the route shown in the scheme below,

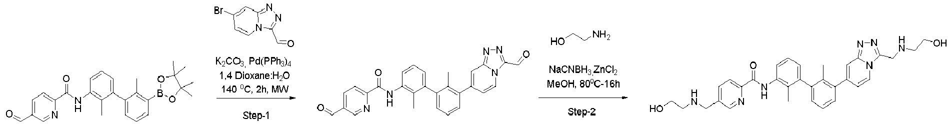

### Step-1: Synthesis of 5-formyl-N-(3'-(3-formyl-[1,2,4]triazolo[4,3-0]pyridin-7-yl)-2,2'-dimethyl-[1,1'-biphenyl]-3-yl)picolinamide

The title compound was prepared in a similar way as Example 1 to give the title compound (60 mg, 57%) as a white solid. LC-MS calculated for C28H21N5O3 (M+H)+: m/z = 475.51; found: 476.35 (M+1).

### Step-2: Synthesis of 5-(((2-hydroxyethyl)amino)methyl)-N-(3'-(3-(((2-hydroxyethyl)amino)methyl)-[1,2,4]triazolo[4,3-a]pyridin-7-yl)-2,2'-dimethyl-[1,1'-biphenyl]-3-yl)picolinamide

The title compound was prepared in a similar way as in Example 1 to give the title compound (15 mg, 16%) as an off-white solid.

**1H NMR (400 MHz, DMSO-d6)** 10.34 (s, 1H), 8.67 (d, J = 5.2 Hz, 2H), 8.59 (d, J = 7.2 Hz, 1H), 8.13 (d, J = 7.6 Hz, 1H), 8.01 (d, J = 8 Hz, 1H), 7.89 (d, J = 8 Hz, 1H), 7.71 (s, 1H), 7.49-7.39 (m, 3H), 7.2 (d, J = 7.2 Hz, 1H), 7.05 (d, J =6.4 Hz, 2H),4.52-4.47 (m, 2H), 4.39 (s, 2H), 3.85 (s, 2H), 3.49-3.44(m, 4H), 2.62-2.58(m, 4H), 2.07(d, J = 7.6 Hz, 3H), 1.98(s, 3H). LC-MS calculated for C33H36N6O3 (M+H)+: m/z = 564.69; found: 565.5

Following example as shown in Table 18 was prepared according to similar procedure described for Example 92 with appropriate amines:

**Table 18:**

| **Ex.No** | **Structure IUPAC Name** | **LC-MS(ESI)m/z:** |
|---|---|---|
| **93** | 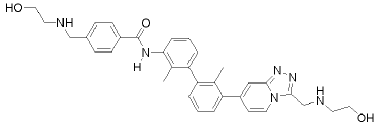 4-(((2-hydroxyethyl)amino)methyl)-*N*-(3'-(3-(((2-hydroxyethyl)amino)methyl)-[1,2,4]triazolo[4,3-*a*]pyridin-7-yl)-2,2'-dimethyl-[1,1'-biphenyl]-3-yl)benzamide | LC-MS calculated for C33H36N6O3 (M+H)+: m/z = 564.69; found: 565.5 |

### Example 94: (S)-5-((((5-(2,2'-dimethyl-3'-(3-(((((S)-5-oxopyrrolidin-2-yl)methyl)amino)methyl)-[1,2,4]triazolo[4,3-a]pyridin-6-yl)-[1,1'-biphenyl]-3-yl)-3-methoxypyrazin-2-yl)methyl)amino)methyl)pyrrolidin-2-one

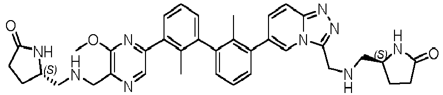

The compound of Example 94 was synthesized via the route shown in the scheme below,

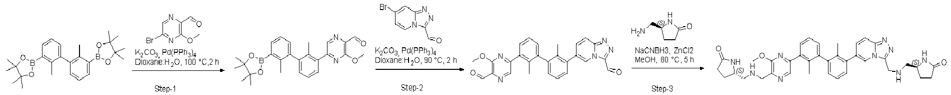

### Step-1: Synthesis of 5-(2,2'-dimethyl-3'-(4,4,5,5-tetramethyl-1,3,2-dioxaborolan-2-yl)-[1,1'-biphenyl]-3-yl)-3-methoxypyrazine-2-carbaldehyde

The title compound was prepared in a similar way as Example 1 to give the title compound (210 mg, 43 %) as a yellow gummy liquid. LC-MS calculated for C26H29BN2O4 (M+H)+: m/z = 444.34; found: 445.35.

### Step-2: Synthesis of 6-(3'-(5-formyl-6-methoxypyrazin-2-yl)-2,2'-dimethyl-[1,1'-biphenyl]-3-yl)-[1,2,4]triazolo[4,3-a]pyridine-3-carbaldehyde

The title compound was prepared in a similar way as Example 1 to give the title compound (150 mg, 69 %) as a yellow solid. LC-MS calculated for C27H21N5O3 (M+H)+: m/z = 463.5; found: 464.35.

### Step-3: Synthesis of (S)-5-((((5-(2,2'-dimethyl-3'-(3-(((((S)-5-oxopyrrolidin-2-yl)methyl) amino)methyl)-[1,2,4]triazolo[4,3-a]pyridin-6-yl)-[1,1'-biphenyl]-3-yl)-3-methoxy pyrazin-2-yl)methyl)amino)methyl)pyrrolidin-2-one

The title compound was prepared in a similar way as Example 1 to give the title compound (27 mg, 12.6 %) as a yellow solid.

¹H NMR (400 MHz, DMSO-*d*₆) *δ*: 8.60-8.28 (m, 1H), 8.34 (s, 1H), 8.16 (m, 1H), 7.72-7.64 (m, 3H), 7.51-7.49 (m, 1H), 7.43-7.38 (m, 3H), 7.28-7.24 (m, 2H), 7.06-7.04 (d, J= 6.8 Hz, 1H), 4.29 (s, 2H), 3.96 (s, 3H), 3.89 (s, 2H), 3.62-3.59 (m, 2H), 2.66-2.60 (m, 2H), 2.59-2.50 (m, 2H), 2.13-2.04 (m, 9H), 2.00 (s, 3H), 1.70-1.63 (m, 2H). LC-MS calculated for C37H41N9O3 (M+H)+: m/z = 659.8; found: 660.4.

### Example 95: (S)-N-(2,2'-dimethyl-3'-(3-((((5-oxopyrrolidin-2-yl)methyl)amino)methyl)-[1,2,4]triazolo[4,3-a]pyridin-7-yl)-[1,1'-biphenyl]-3-yl)-5-(((2-hydroxyethyl)amino)methyl)picolinamide

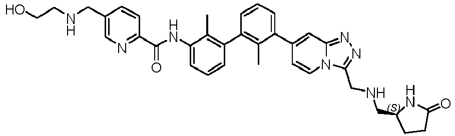

The compound of Example 95 was synthesized via the route shown in the scheme below,

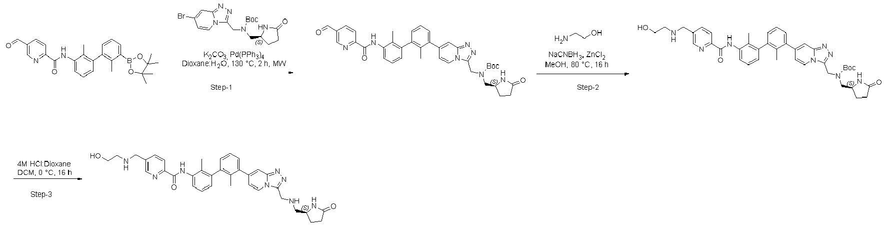

### Step-1: Synthesis of tert-butyl (S)-((7-(3'-(5-formylpicolinamido)-2,2'-dimethyl-[1,1'-biphenyl]-3-yl)-[1,2,4]triazolo[4,3-a]pyridin-3-yl)methyl)((5-oxopyrrolidin-2-yl)methyl)carbamate

The title compound was prepared in a similar way as Example 1 to give the title compound (90 mg, 41 %) as a pale yellow gummy solid.

LC-MS calculated for C38H39N7O5 (M-H): m/z = 673.77; found: 672.4.

### Step-2: Synthesis of tert-butyl (S)-((7-(3'-(5-(((2-hydroxyethyl)amino)methyl)picolinamido) -2,2'-dimethyl-[1,1'-biphenyl]-3-yl)-[1,2,4]triazolo[4,3-a]pyridin-3-yl)methyl)((5-oxopyrrolidin-2-yl)methyl)carbamate

The title compound was prepared in a similar way as Example 1 to give the title compound (850 mg, 91.3 %) as a white solid. LC-MS calculated for C40H46N8O5 (M+H)+: m/z = 718.86; found: 719.2.

### Step-3: Synthesis of (S)-N-(2,2'-dimethyl-3'-(3-((((5-oxopyrrolidin-2-yl)methyl)amino)methyl)-[1,2,4]triazolo[4,3-a]pyridin-7-yl)-[1,1'-biphenyl]-3-yl)-5-(((2-hydroxyethyl)amino)methyl)picolinamide

The title compound was prepared in a similar way as Example 39 to give rude which was purified by prep-HPLC (X-Bridge C18 column (250 x 19) mm, 5.0 µm; mobile phase (A: B), A = 0.1% FA in H₂O, B = ACN 100%.) to give the title compound (21 mg, 28.7 %) as a white solid. ¹H NMR (400 MHz, DMSO-*d*₆) *δ*:10.34 (s, 1H), 8.67 (s, 1H), 8.59 (d, J= 6.8 Hz, 1H), 8.13 (d, J= 7.6 Hz, 1H), 8.01 (d, J= 8.0 Hz, 1H), 7.88 (d, J= 8.4 Hz, 1H), 7.72 (s, 1H), 7.64 (s, 1H), 7.40-7.31 (m, 3H), 7.20 (d, J= 7.2 Hz, 1H), 7.05 (t, J= 6.2 Hz, 2H), 4.49 (t, J= 5.0 Hz, 1H), 4.29 (s, 2H), 3.85 (s, 2H), 3.60-3.58 (m, 1H), 3.47 (q, J= 5.4 Hz, 2H), 2.57-2.50 (m, 4H), 2.09-2.01 (m, 6H), 1.98 (s, 3H), 1.64-1.62 (m, 1H).

LC-MS calculated for C35H38N8O3 (M+H)+: m/z = 618.74; found: 619.2

### Example 96: 2-(((2',2"-dichloro-3"-(3-(((2-hydroxyethyl)amino)methyl)-[1,2,4]triazolo[4,3-a]pyridin-7-yl)-[1,1':3',1"-terphenyl]-4-yl)methyl)amino)ethan-1-ol

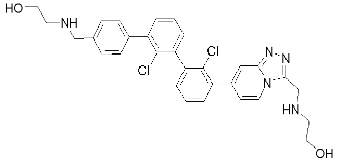

The compound of Example 96 was synthesized via the route shown in the scheme below,

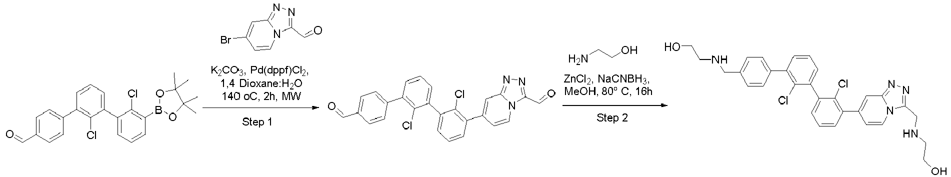

### Step-1: Synthesis of 7-(2,2'-dichloro-4"-formyl-[1,1':3',1"-terphenyl]-3-yl)-[1,2,4]triazolo[4,3-a]pyridine-3-carbaldehyde

A mixture of 2',2"-dichloro-3"-(4,4,5,5-tetramethyl-1,3,2-dioxaborolan-2-yl)-[1,1':3',1"-terphenyl]-4-carbaldehyde (200 mg, 0.44 mmol), 7-bromo-[1,2,4]triazolo[4,3-*a*]pyridine-3-carbaldehyde (99.74 mg, 0.44 mmol), in 1,4-dioxane (8 mL) was added K₂CO₃ (182.9 mg, 1.32 mmol) in water (0.8 mL), Pd(dppf)Cl₂ (17 mg, 0.022 mmol) and the mixture was degassed with N₂ for 30 minutes then heated the reaction in MW at 140 °C for 2 h. Diluted the reaction mixture with ethyl acetate (10 mL) and water (10 mL), organic layer was separated , aqueous layer was extracted with ethyl acetate (3 x 30 mL). The combined organic layer was washed with brine, a dried over Na₂SO₄, filtered and evaporated. Crude material was purified by Biotage flash column chromatography using (0-10%) methanol in DCM to obtain the title compound (45 mg, 22%) as a pale-yellow solid. LC-MS calculated for C26H15Cl2N3O2 (M+H)+: m/z = 472.33; found: 472.20.

### Step-2: Synthesis of 2-(((2',2"-dichloro-3"-(3-(((2-hydroxyethyl)amino)methyl)-[1,2,4]triazolo[4,3-a]pyridin-7-yl)-[1,1':3',1"-terphenyl]-4-yl)methyl)amino)ethan-1-ol

The title compound was prepared in a similar way as in Example 1, to give the title compound (8 mg, 16%) as off-white solid. The crude material was purified by prep-HPLC (X-Bridge C18 column (250 x 19) mm, 5.0 µm; mobile phase (A: B), A = 0.1% FA in H₂O, B = ACN 100%.) to obtain the title compound (20 mg, 42 %) as off-white solid.

1H NMR (400 MHz, DMSO-d6): δ: 8.62 (d, J = 7.6 Hz, 1H), 8.2 (s, 1H), 7.81 (s, 1H), 7.68-7.4 ( m, 10H), 7.08 ( d, J = 7.2 Hz, 1H), 4.50 ( s, 2H), 4.3 (s, 2H), 3.84 ( s, 2H), 3.52 (t, J= 5.6Hz, 2H), 3.45 (t, j = 6 Hz, 2H), 2.67-(t, J = 5.6 Hz, 2H), 2.6 ( t, J = 6 Hz, 2H). LC-MS calculated for C30H29Cl2N5O2 (M+H)+: m/z = 562.5; found: 564.

Following example as shown in Table 19 was prepared according to similar procedure described for Example 96 with appropriate amines:

**Table 19:**

| **Ex.No** | **Structure IUPAC Name** | **LC-MS(ESI)m/z:** |
|---|---|---|
| **97** | 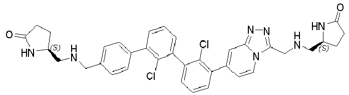 (S)-5-((((2',2"-dichloro-3"-(3-(((((S)-5-oxopyrrolidin-2-yl)methyl)amino)methyl)-[1,2,4]triazolo[4,3-a]pyridin-7-yl)-[1,1':3',1"-terphenyl]-4-yl)methyl)amino)methyl)pyrrolid in-2-one | LC-MS calculated for C36H35Cl2N7O2 (M+H)+: m/z = 667.2; found: 668.2 |

### Example 98: 2-(((7-(4"-(((2-hydroxyethyl)amino)methyl)-2,2'-dimethyl-[1,1':3',1"-terphenyl]-3-yl)-[1,2,4]triazolo[1,5-a]pyridin-2-yl)methyl)amino)ethan-1-ol

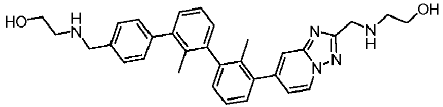

The compound of Example 98 was synthesized via the route shown in the scheme below,

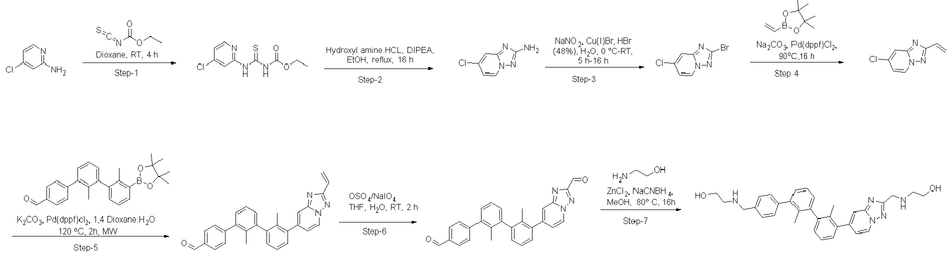

### Step-1: Synthesis of ethyl N-[(4-chloropyridin-2-yl)carbamothioyl]carbamate

A mixture of 4-Chloropyridin-2-amine (2 g, 11.5 mmol) and O-ethyl carbonisothiocyanatidate (1.4 mL, 11.5 mmol) in dioxane (40 mL) was stirred at room temperature for 4 h. Reaction mixture was diluted with water and extracted with ethyl acetate (3 x 10 mL). Combined organic layer was washed with brine, dried over Na₂SO₄, filtered, and evaporated the solvent under reduced pressure to obtain the title compound (3.2 g, 92%) which was taken for next step without purification. LC-MS calculated for C9H10CIN3O2S (M+H)+: m/z = 259.71; found: 260.20.

### Step-2: Synthesis of 7-chloro-[1,2,4]triazolo[1,5-a]pyridin-2-amine

7-chloro-[1,2,4]triazolo[1,5-a]pyridin-2-amine (0.74 g, 0.0024 mmol) was added to a stirred solution of hydroxylamine HCl (0.85 g, 0.0121 mmol) in EtOH (20 mL) and DIPEA (1.3 mL, 0.0072 mmol) at room temperature. The reaction mixture heated at 90 °C for 16 h. Organic volatiles were removed under reduced pressure, and the residue dissolved in water to obtain the solid, which was filtered, and washed with water and dried under vacuum to obtain the title compound (0.35 g, 67%). Crude material was taken for next step without further purification. LC-MS calculated for C6H5CIN4 (M+H)+: m/z =168.58; found: 169.

### Step-3: Synthesis of 7-bromo-2-iodo-[1,2,4]triazolo[1,5-a]pyridine

NaNO₂ (0.13 g, 0.0017 mmol) in water (0.5 mL) was added to a stirred solution of 2-bromo-7-chloro-[1,2,4]triazolo[1,5-*a*]pyridine (0.2 g, 0.0011 mmol) in 48% HBr in water (2 mL) at 0 °C. Stirred the reaction at 0 °C for 5 h. then, Copper (I) bromide (0.21 g, 0.0014 mmol) in 48% HBr in water (0.5 mL) was added. Stirred the reaction at room temperature for 16 h. Reaction mixture was poured into ice cold water and solid separated, filtered, washed with water and dried under vacuum to obtain the title compound (0.27 g, 51%). Crude material was taken for next step without further purification. LC-MS calculated for C6H3BClN3 (M+H)+: m/z = 232.47; found: 233.9.

### Step-4: Synthesis of 7-Chloro -2-vinyl-[1,2,4]triazolo[1,5-a]pyridine

A mixture of 2-bromo-7-chloro-[1,2,4]triazolo[1,5-*a*]pyridine (0.3 g, 9 mmol), 4,4,5,5-tetramethyl-2-vinyl-1,3,2-dioxaborolane (0.1 g, 6 mmol), in 1,4-dioxane (7 mL) was added Na₂CO₃ (0.2 g, 1.8 mmol) in water (3.0 mL), Pd(dppf)Cl₂ (35 mg, 0.04 mmol) and degassed with N₂ for 30 minutes then heated the reaction at 110 °C for 16 h. Then reaction cooled to RT and diluted with ethyl acetate (50 mL) and water (50 mL). The organic layer was separated, aqueous layer was extracted with ethyl acetate (3 x 30 mL). The combined organic layer was dried over Na₂SO₄, filtered, and evaporated. Crude material was purified by Biotage flash column chromatography using (0-10%) methanol in DCM to obtain the title compound (0.3 g, 45%) as a colorless liquid. LC-MS calculated for C8H6CIN3 (M+H)+: m/z = 179.61; found: 180.05.

### Step-5: Synthesis of 2',2"-dimethyl-3"-(2-vinyl-[1,2,4]triazolo[1,5-a]pyridin-7-yl)-[1,1':3',1"-terphenyl]-4-carbaldehyde

The title compound was prepared in a similar way as example 1 to give the title compound (300 mg, 45%) as a colourless liquid. LC-MS calculated for C29H23N3O (M+H)+: m/z = 429.52; found: 430.15.

### Step-6: Synthesis of 2',2"-dimethyl-3"-(2-vinyl-[1,2,4]triazolo[1,5-a]pyridin-7-yl)-[1,1':3',1"-terphenyl]-4-carbaldehyde

OsO₄ (0.55 mL, 8.72 mmol) was added to a stirred mixture of 2',2"-dimethyl-3"-(2-vinyl-[1,2,4]triazolo[1,5-a]pyridin-7-yl)-[1,1':3',1"-terphenyl]-4-carbaldehyde (100 mg, 23.3 mmol), 2, 6 Lutidine (0.05 mL, 41.95 mmol) in THF (8 ml) and water (2 mL). After 10 min NalO4 (90 mg, 41.95 mmol) was added and stirred the reaction at room temperature for 1 h. Reaction mixture was filtered, washed with mixture of 10% MeOH in DCM. The filtrate was concentrated under reduced pressure to obtain the crude. Crude material was purified by Biotage flash column chromatography using (0-10%) Ethyl acetate in Hexane to obtain the title compound (90 mg, 99%) as an off-white solid. LC-MS calculated for C28H21N3O2 (M+H)+: m/z = 431.5; found: 432.15.

### Step-7: Synthesis of 2-(((7-(4"-(((2-hydroxyethyl)amino)methyl)-2,2'-dimethyl-[1,1':3',1"-terphenyl]-3-yl)-[1,2,4]triazolo[1,5-a]pyridin-2-yl)methyl)amino)ethan-1-ol

The title compound was prepared in a similar way as in example 1, to get crude. The crude material was purified by prep-HPLC (X-Bridge C18 column (250 x 19) mm, 5.0 µm; mobile phase (A: B), A = 0.1% FA in H₂O, B = ACN 100%.) to obtain the title compound (10 mg, 3 %) as an off-white solid.

1H NMR (400 MHz, DMSO-d6): δ: 8.59 (d, J= 7.2 Hz, 1H), 7.7 (s, 1H), 7.41-7.31 (m, 7H), 7.24 (t, J = 5.6 Hz, 2H), 7.15 9d, J = 7.2 Hz, 1H), 7.05 (d, J = 7.2 Hz, 1H), 4.51 (d, J = 4.4 Hz, 2H), 4.29 (s, 2H), 3.77 9s, 2H), 3.32-3.49(m, 4H), 2.59-2.63 9m, 4H), 2.00 9s, 3H), 1.94 (s, 3H).

LC-MS calculated for C32H35N5O2 (M+H)+: m/z = 521.67; found: 522.25.

Following example as shown in Table 20 was prepared according to similar procedure described for Example 98 with appropriate amines:

**Table 20:**

| **Ex.No** | **Structure IUPAC Name** | **LC-MS(ESI)m/z:** |
|---|---|---|
| **99** | 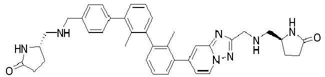 (*S*)-5-((((2',2"-dimethyl-3"-(2-(((((S)-5-oxopyrrolidin-2-yl)methyl)amino)methyl)-[1,2,4]triazolo[1,5-*a*]pyridin-7-yl)-[1,1':3',1"-terphenyl]-4-yl)methyl)amino)methyl)pyrrolid in-2-one | LC-MS calculated for C38H41N7O2 (M+H)+: m/z = 627.7; found: 628.1 |

### Example 100: 5-(((2-hydroxyethyl)amino)methyl)-N-(3'-(2-(((2-hydroxyethyl)amino)methyl)-[1,2,4]triazolo[1,5-a]pyridin-7-yl)-2,2'-dimethyl-[1,1'-biphenyl]-3-yl)picolinamide

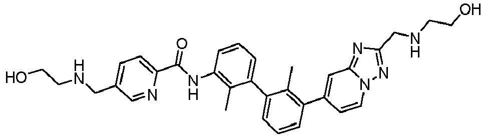

The compound of Example 100 was synthesized via the route shown in the scheme below,

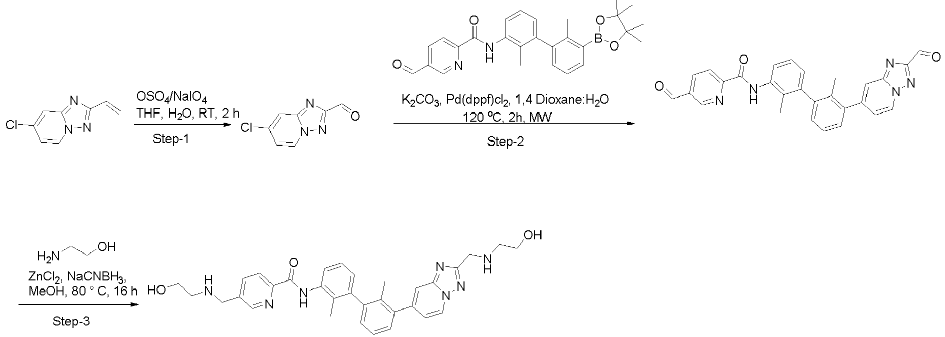

### Step-1: Synthesis of 7-chloro-[1,2,4]triazolo[1,5-a]pyridine-2-carbaldehyde

The title compound was prepared in a similar way as in Example 79 to give the title compound (40 mg, 42%) as a pale yellow solid. LC-MS calculated for C7H4CIN3O (M+H)+: m/z = 181.58, found:182.10.

### Step-2: Synthesis of 5-formyl-N-(3'-(2-formyl-[1,2,4]triazolo[1,5-a]pyridin-7-yl)-2,2'-dimethyl-[1,1'-biphenyl]-3-yl)picolinamide

The title compound was prepared in a similar way as in Example 1 to give the title compound (55 mg, 58%) as a pale yellow sticky mass. LC-MS calculated for C28H21N5O3 (M+H)+: m/z = 475.51, found:476.10.

### Step-3: Synthesis of 5-(((2-hydroxyethyl)amino)methyl)-N-(3'-(2-(((2-hydroxyethyl)amino)methyl)-[1,2,4]triazolo[1,5-a]pyridin-7-yl)-2,2'-dimethyl-[1,1'-biphenyl]-3-yl)picolinamide

The title compound was prepared in a similar way as in Example 1 to give the crude which was purified by prep-HPLC (X-Bridge C18 column (250 x 19) mm, 5.0 µm; mobile phase (A: B), A = 0.1% FA in H₂O, B = ACN 100%.) to get the title compound (5 mg, 10%) as an off-white solid.

1H NMR (400 MHz, DMSO-d6) : 8.929 (d, J = 6.8 Hz, 1H), 8.678 (s, 1H), 8.205 (s, 1H), 8.13 (d, *J* = 7.6 Hz, 1H), 8.02 (d, J = 8 Hz, 1H), 7.88 (d, J = 7.6 Hz, 1H), 7.76 (s, 1H), 7.31-7.41 (m, 3H), 7.21 (d, J =6.8 Hz, 2H), 7.04 (d, J = 6.8 Hz, 1H), 4.57 (s, 2H), 3.95 (s, 2H), 3.86(s, 2H), 3.48(s, 4H), 2.68(d, J = 7.6 Hz, 2H), 2.59(s, 2H). 2.06 (s, 3H), 1.97 (s, 3H), LC-MS calculated for C32H35N7O3 (M+H)+: m/z = 565.2; found: 565.5

Following examples as shown in Table 21 was prepared according to similar procedure described for Example 100 with appropriate amines:

**Table 21:**

| **Ex.No** | **Structure IUPAC Name** | **LC-MS(ESI)m/z:** |
|---|---|---|
| **101** | 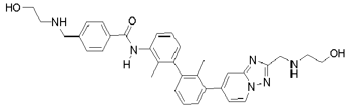 | LC-MS calculated for C33H36N6O3 (M+H)+: m/z = 564.6; found: 565.3 |
| | 4-(((2-hydroxyethyl)amino)methyl)-*N-*(3'-(2-(((2-hydroxyethyl)amino)methyl)-[1,2,4]triazolo[1,5-*a*]pyridin-7-yl)-2,2'-dimethyl-[1,1'-biphenyl]-3-yl)benzamide | |

### Example 102: (S)-5-((((7-(4"-(((2-hydroxyethyl)amino)methyl)-2,2'-dimethyl-[1,1':3',1"-terphenyl]-3-yl)-[1,2,4]triazolo[1,5-a]pyridin-2-yl)methyl) amino) methyl) pyrrolidin-2-one

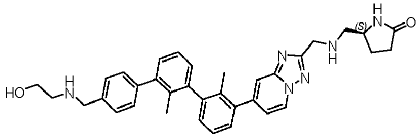

The compound of Example 102 was synthesized via the route shown in the scheme below,

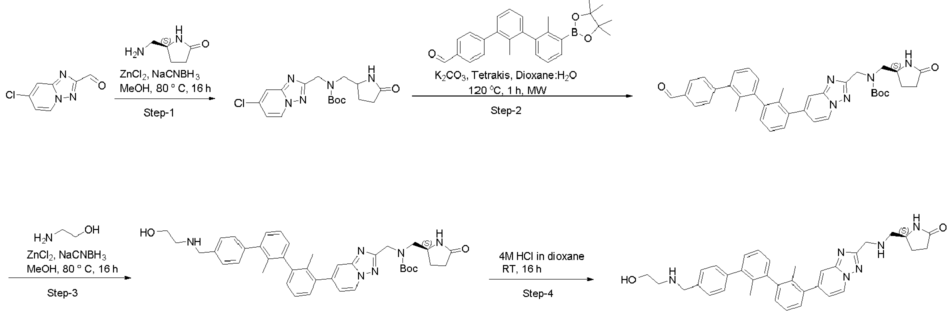

### Step-1 : Synthesis of tert-butyl ((7-chloro-[1,2,4]triazolo[1,5-a]pyridin-2-yl)methyl)((5-oxopyrrolidin-2-yl)methyl)carbamate

The title compound was prepared in a similar way as Example 1 to give the title compound (400 mg, 42 %) as a pale-yellow sticky mass.

LC-MS calculated for C17H22CIN5O3 (M+H)+: m/z = 379.85, found:380.10.

### Step-2 : Synthesis of tert-butyl (S)-((7-(4"-formyl-2,2'-dimethyl-[1,1':3',1"-terphenyl]-3-yl)-[1,2,4]triazolo[1,5-a]pyridin-2-yl)methyl)((5-oxopyrrolidin-2-yl)methyl)carbamate

The title compound was prepared in a similar way as Example 1 to give the title compound (230 mg, 100 %) as a pale-yellow solid.

LC-MS calculated for C38H39N5O4 (M+H)+: m/z = 629.76, found:630.25.

### Step-3 : Synthesis of tert-butyl (S)-((7-(4"-(((2-hydroxyethyl)amino)methyl)-2,2'-dimethyl-[1,1':3',1"-terphenyl]-3-yl)-[1,2,4]triazolo[1,5-a]pyridin-2-yl)methyl)((5-oxopyrrolidin-2-yl)methyl)carbamate

The title compound was prepared in a similar way as Example 1 to give the title compound (140 mg, 99 %) as a pale-yellow solid.

LC-MS calculated for C40H46N6O4 (M+H)+: m/z = 674.85, found:675.35.

### Step-4 : Synthesis of (S)-5-((((7-(4"-(((2-hydroxyethyl)amino)methyl)-2,2'-dimethyl-[1,1':3',1"-terphenyl]-3-yl)-[1,2,4]triazolo[1,5-a]pyridin-2-yl)methyl) amino) methyl) pyrrolidin-2-one

The title compound was prepared in a similar way as Example 39 to give rude which was purified by prep-HPLC (X-Bridge C18 column (250 x 19) mm, 5.0 µm; mobile phase (A: B), A = 0.1% FA in H₂O, B = ACN 100%.) to give the title compound (55 mg, 42 %) as an off-white solid.

1H NMR (400 MHz, DMSO-d6) δ:8.92 (d, J =7.2 Hz 1H), 8.21 (s, 2H), 7.75 (s, 1H), 7.66 (s, 1H), 7.46(d, J = 7.6Hz, 2H), 7.32-7.4 (m, 5H), 7.16-7.25(m, 4H), 3.916-3.989(m, 4H), 3.619(s, 1H), 3.559 (s, 2H), 2.74 (s, 2H), 2.61-2.67(s, 2H), 2.09(s, 3H), 1.97 (d, J = 20 Hz, 6H) ,1.69 (s,1H) , LC-MS calculated for C35H38N6O2 (M+H)+: m/z = 574.73; found: 575.7.

### Example 103: (S)-N-(2,2'-dimethyl-3'-(2-((((5-oxopyrrolidin-2-yl)methyl)amino)methyl)-[1,2,4]triazolo[1,5-a]pyridin-7-yl)-[1,1'-biphenyl]-3-yl)-4-(((2-hydroxyethyl)amino)methyl)benzamide

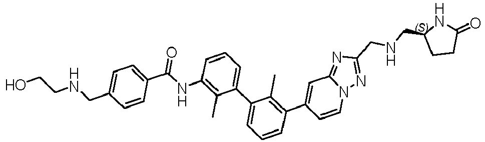

The compound of Example 103 was synthesized via the route shown in the scheme below,

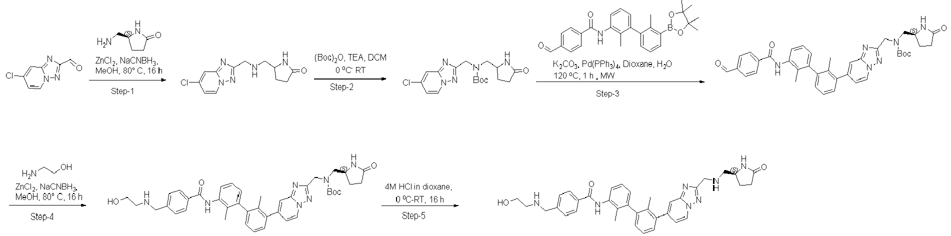

### Step-1: Synthesis of 5-((((7-chloro-[1,2,4]triazolo[1,5-a]pyridin-2-yl)methyl)amino)methyl)pyrrolidin-2-one

The title compound was prepared in a similar way as in example 1 to give the title compound (850 mg, 90%) as an off-white solid. LC-MS calculated for C12H14CIN5O (M+H)+: m/z = 279.73, found:280.05.

### Step-2: tert-butyl ((7-chloro-[1,2,4]triazolo[1,5-a]pyridin-2-yl)methyl)((5-oxopyrrolidin-2-yl)methyl)carbamate

Boc Anhydride (860 mg, 39 mmol) was added to a stirred mixture of 5-((((7-chloro-[1,2,4]triazolo[1,5-*a*]pyridin-2-yl)methyl)amino)methyl)pyrrolidin-2-one (850 mg, 30 mmol), TEA (1.3 mL, 91 mmol) in methanol (1 mL) and DCM (9 mL) at 0 °C. Stirred the reaction at room temperature for 5 h. Organic volatiles were removed under reduce pressure and the residue dissolved in water and extracted with DCM (3 x 10 mL). Combined organic layer was dried over Na₂SO₄, filtered, and evaporated the solvent under reduced pressure to obtain the crude. The crude compound was purified by combi flash column chromatography using 0-30 % ethyl acetate in hexane as a mobile phase to obtain the title compound 3 (850 mg, 74%) as an off-white solid. LC-MS calculated for C17H22CIN5O3 (M+H)+: m/z = 379.85, found:380.10.

### Step-3: Synthesis of tert-butyl (S)-((7-(3'-(4-formylbenzamido)-2,2'-dimethyl-[1,1'-biphenyl]-3-yl)-[1,2,4]triazolo[1,5-a]pyridin-2-yl)methyl)((5-oxopyrrolidin-2-yl)methyl)carbamate

The title compound was prepared in a similar way as in example 1 to give the title compound (200 mg, 79%) as an off-white solid. LC-MS calculated for C39H40N6O5 (M+H)+: m/z = 672.79 found:673.30.

### Step-4: Synthesis of tert-butyl (S)-((7-(3'-(4-(((2-hydroxyethyl)amino)methyl)benzamido)-2,2'-dimethyl-[1,1'-biphenyl]-3-yl)-[1,2,4]triazolo[1,5-a]pyridin-2-yl)methyl)((5-oxopyrrolidin-2-yl) methyl) carbamate

The title compound was prepared in a similar way as in example 1 to give the title compound (100 mg, 79.4%) as an off-white solid. LC-MS calculated for C41H47N7O5 (M-H)+: m/z = 717.87 found:716.25.

### Step-5: Synthesis of (S)-N-(2,2'-dimethyl-3'-(2-((((5-oxopyrrolidin-2-yl)methyl)amino)methyl)-[1,2,4]triazolo[1,5-a]pyridin-7-yl)-[1,1'-biphenyl]-3-yl)-4-(((2-hydroxyethyl)amino)methyl)benzamide

The title compound was prepared in a similar way as example 39 to give crude which was purified by prep-HPLC (X-Bridge C18 column (250 x 19) mm, 5.0 µm; mobile phase (A: B), A = 0.1% FA in H₂O, B = ACN 100%.) to obtain the title compound (25 mg, 17 %) as white solid. 1H NMR (400 MHz, DMSO-d6) δ:8.92 (d, J =6.8 Hz 1H), 8.19 (s, 2H), 7.97 (d, J = 7.6Hz, 2H), 7.76 (s, 1H), 7.65(s, 1H), 7.52 (d, J = 7.6 Hz, 2H), 7.297-7.29 (m, 4H), 7.212 (s, 2H), 7.09 (d , J = 7.2 Hz,, 1H), 3.91-3.99 (m, 4H), 3.62 (s, 1H), 3.53 (s, 2H), 3.53(s, 2H), 2.68 (s, 2H), 1.977 (d, J = 4.8 Hz, 6H), 1.69 (s, 1H). LC-MS calculated for C36H39N7O3 (M+H)+: m/z = 617.75; found: 618.3.

### Example 104: 2-(((2',2"-dichloro-3"-(2-(((2-hydroxyethyl)amino)methyl)-[1,2,4]triazolo[ 1,5-a]pyridin-7-yl)-[1,1':3',1"-terphenyl]-4-yl)methyl)amino)ethan-1-ol

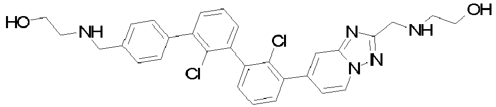

The compound of Example 104 was synthesized via the route shown in the scheme below.

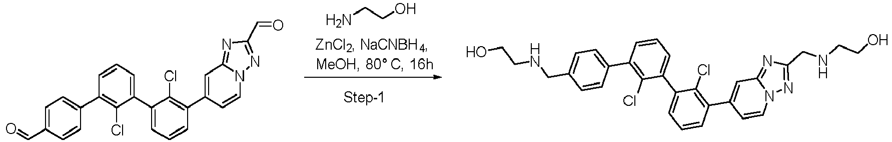

### Step-1: Synthesis of 2-(((2',2"-dichloro-3"-(2-(((2-hydroxyethyl)amino)methyl)-[1,2,4]triazolo[1,5-a]pyridin-7-yl)-[1,1':3',1"-terphenyl]-4-yl)methyl)amino)ethan-1-ol

The title compound was prepared in a similar way as in Example 1 to get crude. The crude material was purified by prep-HPLC (X-Bridge C18 column (250 x 19) mm, 5.0 µm; mobile phase (A: B), A = 0.1% FA in H₂O, B = ACN 100%.) to obtain the title compound (10 mg, 32 %) as a white solid.

1H NMR (400 MHz, DMSO-d6): δ: 8.96 (d, J= 7.2 Hz, 1H), 8.189 (s, 1H), 7.856 (s, 1H), 7.447-7.611 (m, 9H), 7.25 (d, J = 6.8 Hz, 1H), 4.5 (s, br, 2H), 3.957 (s, 2H), 3.83 (s, 2H), 3.504 (t, J = 6 Hz, 4H), 2.67 (d, J = 4.8 Hz, 4H), LC-MS calculated for C30H29Cl2N5O2 (M+H)+: m/z = 562.5; found: 563.2.

Following example as shown in Table 22 was prepared according to similar procedure described for Example 104 with appropriate amine:

**Table 22:**

| **Ex.No** | **Structure IUPAC Name** | **LC-MS(ESI)m/z:** |
|---|---|---|
| **105** | 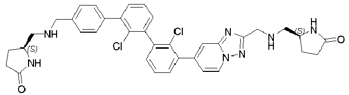 (*S*)-5-((((2',2"-dichloro-3"-(2-(((((S)-5-oxopyrrolidin-2-yl)methyl)amino)methyl)-[1,2,4]triazolo[1,5-*a*]pyridin-7-yl)-[1,1':3',1"-terphenyl]-4-yl)methyl)amino)methyl)pyrroli din-2-one | LC-MS calculated for C36H35Cl2N7O2 (M+H)+: m/z = 667.2; found: 668.2 |

### PD1-PDL1 HTRF assay

The CisBio HTRF assay kit, purchased from CisBio only, was employed to determine the IC₅₀ values of compounds. The PD1-PDL1 HTRF assay measures the interactions between the two proteins, PD1 and PDL1. The interaction is detected by using anti-Tag1 labeled with Europium (HTRF donor) which binds to PDL1 and anti-Tag2 labeled with XL665 (HTRF acceptor) which binds to PD1. When the two antibodies are in proximity, the excitation of the donor fluorophore results in FRET emission from the acceptor fluorophore. The presence of inhibitors for such an interaction result in reduction of HTRF signal.

The assay was conducted in 96-well white assay plates. All the reagents, including Tag1-PDL1, Tag2-PD1, Anti-Tag1-PDL1, Anti-Tag2-PD1 were thawed and diluted with PPI Europium Detection buffer to achieve the desired concentration as per the manufacturer's protocol. A 1000 µM stock solution of test inhibitors was prepared in DMSO. The stock solution was diluted in DMSO to prepare 8 concentrations (1000-0.001 µM). These stock solutions were further diluted in PPI Europium detection buffer to prepare 8 working stock solutions (100-0.0001 µM). To the 96-well plate, 2 µL of the test inhibitor working stock solutions were added followed by 4 µL of Tag1-PDL1 (5 nM) and 4 µL of Tag2-PD1 (50 nM). The plate was incubated at room temperature for 15 min. Subsequently, 10 µL of pre-mixed Anti-Tag1-PDL1 and Anti-Tag2-PD1 solution was added. The plate was sealed and incubated at room temperature for 1 h. The HTRF signal was read on Perkin Elmer Envision plate reader using excitation wavelength of 320 nm, emission wavelengths of 620 nm and 665 nm. Results were obtained by calculating the ratio of signal from acceptor at 665 nm to that of signal from donor at 620 nm multiplied by 104. The IC₅₀ values were determined by the Hill equation using GraphPad Prism software.

Compounds according to the invention as exemplified in the Examples, showed IC₅₀ values in the following ranges: +: IC₅₀ ≤10 nM; ++: 10 nM < IC₅₀ ≤100 nM; +++: 100 nM < IC₅₀<1000 nM

Data obtained for the Example compounds using the PD-1/PD-L1 homogenous time-resolved fluorescence (HTRF) binding assay described in Examples 1 to 105 is provided in Table 23.

**Table 23. PD-1/PD-L1 homogenous time-resolved fluorescence (HTRF) binding assay results for compounds of the invention.**

| **Example** | **HTRF (IC₅₀)** | **Example** | **HTRF (IC₅₀)** | **Example** | **HTRF (IC₅₀)** |
|---|---|---|---|---|---|
| Example 1 | +++ | Example 20 | +++ | Example 39 | +++ |
| Example 2 | ++ | Example 21 | +++ | Example 40 | +++ |
| Example 3 | +++ | Example 22 | +++ | Example 41 | +++ |
| Example 4 | +++ | Example 23 | +++ | Example 42 | +++ |
| Example 5 | +++ | Example 24 | +++ | Example 43 | +++ |
| Example 6 | +++ | Example 25 | +++ | Example 44 | +++ |
| Example 7 | +++ | Example 26 | +++ | Example 45 | +++ |
| Example 8 | +++ | Example 27 | +++ | Example 46 | +++ |
| Example 9 | +++ | Example 28 | +++ | Example 47 | +++ |
| Example 10 | +++ | Example 29 | +++ | Example 48 | ++ |
| Example 11 | +++ | Example 30 | +++ | Example 49 | ++ |
| Example 12 | +++ | Example 31 | +++ | Example 50 | +++ |
| Example 13 | +++ | Example 32 | +++ | Example 51 | +++ |
| Example 14 | +++ | Example 33 | +++ | Example 52 | +++ |
| Example 15 | +++ | Example 34 | +++ | Example 53 | +++ |
| Example 16 | +++ | Example 35 | +++ | Example 54 | +++ |
| Example 17 | +++ | Example 36 | +++ | Example 55 | +++ |
| Example 18 | +++ | Example 37 | +++ | Example 56 | ++ |
| Example 19 | +++ | Example 38 | +++ | | |

**Table 21 (contd)**

| | | | | | |
|---|---|---|---|---|---|
| Example 57 | ++ | Example 73 | ++ | Example 89 | +++ |
| Example 58 | ++ | Example 74 | ++ | Example 90 | + |
| Example 59 | +++ | Example 75 | ++ | Example 91 | ++ |
| Example 60 | +++ | Example 76 | ++ | Example 92 | + |
| Example 61 | +++ | Example 77 | + | Example 93 | +++ |
| Example 62 | + | Example 78 | + | Example 94 | + |
| Example 63 | ++ | Example 79 | + | Example 95 | + |
| Example 64 | + | Example 80 | + | Example 96 | + |
| Example 65 | ++ | Example 81 | + | Example 97 | + |
| Example 66 | ++ | Example 82 | ++ | Example 98 | ++ |
| Example 67 | + | Example 83 | ++ | Example 99 | + |
| Example 68 | + | Example 84 | + | Example 100 | + |
| Example 69 | + | Example 85 | +++ | Example 101 | + |
| Example 70 | + | Example 86 | ++ | Example 102 | ++ |
| Example 71 | ++ | Example 87 | ++ | Example 103 | +++ |
| Example 72 | + | Example 88 | ++ | Example 104 | + |
| | | | | Example 105 | + |

### Embodiments (Ea)

**E1a.** A compound of Formula (I) or a pharmaceutically acceptable salt or a stereoisomer thereof 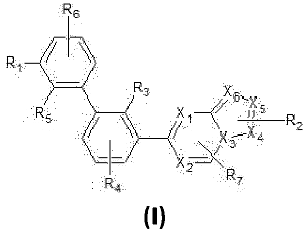 wherein:
   X₁-X₆ are each independently C or N, with the proviso that at least two and at most five of X₁-X₆ are N;
   R₃ is H, methyl, halogen, or CN;
   R₄, and R₅ are each independently H, methyl, halogen, or CN;
   R₇ is H, C₁-₆ alkyl, C₁-₆ alkoxy, halogen, or CN;
   R₁ is selected from H, C₁-₆ alkyl, C₂-₆ alkenyl, C₂-₆ alkynyl, C₁-₆ alkoxy, C₆-₁₀ aryl, 5-14 membered heteroaryl, C₆-₁₀ aryl- C₁-₄ alkyl-, C₃-₁₀ cycloalkyl-C₁-₄ alkyl-, (5-14 membered heteroaryl)-C₁-₄ alkyl-, (4-10 membered heterocycloalkyl)-C₁-₄ alkyl-, C₁-₆ alkyl-NHRₐ, NHCORₐ, NHRₐ, NHORₐ, C(O)Rₐ, CONHRₐ, NHSO₂Rₐ, SO₂NHRₐ, and OCH₂Rₐ, or R₁ and R₆ are linked together to form a 4-10 membered heterocycloalkyl, and wherein C₁-₆ alkyl, C₂-₆ alkynyl, C₁-₆ alkoxy, C₆-₁₀ aryl, 5-14 membered heteroaryl, C₆-₁₀ aryl- C₁-₄ alkyl-, C₃-₁₀ cycloalkyl-C₁-₄ alkyl-, (5-14 membered heteroaryl)-C₁-₄ alkyl-, (4-10 membered heterocycloalkyl)-C₁-₄ alkyl- of R₁ are each optionally substituted with 1, 2, or 3 independently selected R_{b} substituents;
   R₂ is selected from C₁-₆ alkyl, C₂-₆ alkenyl, C₂-₆ alkynyl, C₁-₆ alkoxy, C₆-₁₀ aryl, 5-14 membered heteroaryl, C₆-₁₀ aryl-C₁-₄ alkyl-, (5-14 membered heteroaryl)-C₁-₄ alkyl-, (4-10 membered heterocycloalkyl)-C₁-₄ alkyl-, C₁-₆ alkyl-NHRₐ, and NHRₐ, and wherein the C₁-₆ alkyl, C₂-₆ alkynyl, C₁-₆ alkoxy, C₆-₁₀ aryl, 5-14 membered heteroaryl, C₆-₁₀ aryl-C₁-₄ alkyl-, (5-14 membered heteroaryl)-C₁-₄ alkyl-, (4-10 membered heterocycloalkyl)-C₁-₄ alkyl-, of R₂ are each independently substituted with 1, 2, or 3 independently selected R_{b} substituents;
   R₆ is selected from H, C₁-₆ alkyl, halo, and OCH₂Rₐ or R₁ and R₆ are linked together to form a 4-10 membered heterocycloalkyl optionally substituted with 1, 2, 3, or 4 independently selected R_{b} substituents;
   each Rₐ is independently selected from H, C₁-₆ alkyl, C₁-₆ alkoxy, C₆-₁₀ aryl, 5-14 membered heteroaryl, 4-10 membered heterocycloalkyl, C₆-₁₀ aryl-C₁-₄ alkyl, (5-14 membered heteroaryl)-C₁-₄ alkyl-, (4-10 membered heterocycloalkyl)-C₁-₄ alkyl-, NHR_{c}, and OCH₂R_{c}, and wherein C₁-₆ alkyl, C₁-₆ alkoxy, C₆-₁₀ aryl, 5-14 membered heteroaryl, 4-10 membered heterocycloalkyl, C₆-₁₀ aryl-C₁-₄ alkyl, (5-14 membered heteroaryl)-C₁-₄ alkyl-, (4-10 membered heterocycloalkyl)-C₁-₄ alkyl- of Rₐ are each independently substituted with 1, or 2 independently selected R_{d} substituents;
   each R_{b} substituent is independently selected from C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, and wherein the C₁-₆ alkyl, C₁-₆ alkoxy, C₃₋₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, of R_{b} are each independently substituted with 1, 2, or 3 independently selected R_{d} substituents;
   each R_{c} is independently selected from H, C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl, C(O)R_{d}, C(O)NHR_{d}, and C(O)NR_{d}R_{d}, and wherein the C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl of R_{c} are each independently substituted with 1, 2, or 3 independently selected R_{d} substituents;
   each R_{d} is independently selected from C₁-₆ alkyl, C₁-₆ alkoxy, C₆-₁₀ aryl, C₃-₁₀ cycloalkyl, 5-14 membered heteroaryl, 4-10 membered heterocycloalkyl, C₃-C₆ cyclic amide, CN, NH₂, C(O)ORₑ, NHRₑ, NRₑRₑ, NRₑC(O)Rₑ, NRₑC(O)ORₑ, ORₑ, (=O), S(O)₂Rₑ and S(O)₂NRₑRₑ, and wherein the C₁-₆ alkyl, C₁-₆ alkoxy, C₆-₁₀aryl, C₃-₁₀ cycloalkyl, 5-14 membered heteroaryl, 4-10 membered heterocycloalkyl, C₃-C₆ cyclic amide of R_{d} are each optionally substituted with 1, 2, or 3 independently selected R_{f} substituents;
   each Rₑ is independently selected from H, OH, COOH, C(O)R_{f}, C₁-₆ alkyl, and 5-14 membered heteroaryl wherein the C₁-₆ alkyl, and 5-14 membered heteroaryl of Rₑ are each optionally substituted with 1, 2 or 3 independently selected R_{f} substituents;
   each R_{f} is independently selected from H, C₁-₄ alkyl, halo, CN, COOH, OH, (=O).
**E2a.** The compound according to E1a, having Formula (II) 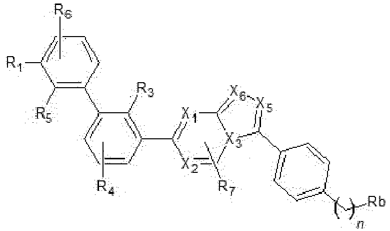 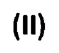 or a pharmaceutically acceptable salt or a stereoisomer thereof, wherein X₁-X₃, X₅ and X₆, R₁, R₃, R₄-R₇, and R_{b} have the same meaning as in E1a and the subscript n is 1, 2, or 3.
**E3a.** The compound according to E2a wherein n is 1.
**E4a.** The compound according to E1a, having Formula (III) 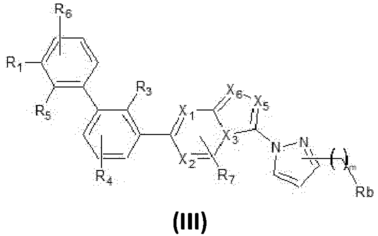 or a pharmaceutically acceptable salt or a stereoisomer thereof, wherein X₁-X₃, X₅ and X₆, R₁, R₃, R₄-R₇, and R_{b} have the same meaning as in E1a and the subscript m is 0, 1, 2, or 3.
**E5a.** The compound according to E1a, having Formula (IV) or (V) 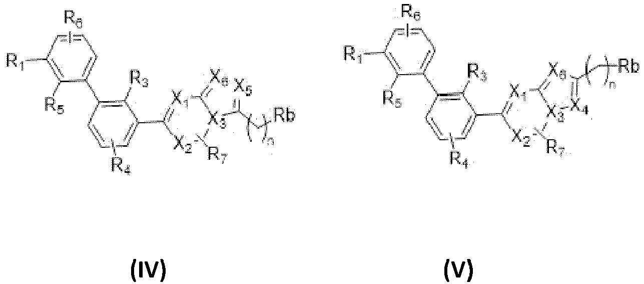 or a pharmaceutically acceptable salt or a stereoisomer thereof, wherein X₁-X₆, R₁, R₃, R₄-R₇, and R_{b} have the same meaning as in E1a and the subscript n is 1, 2, or 3.
**E6a.** The compound according to E1a, having Formula (VI) or (VII) 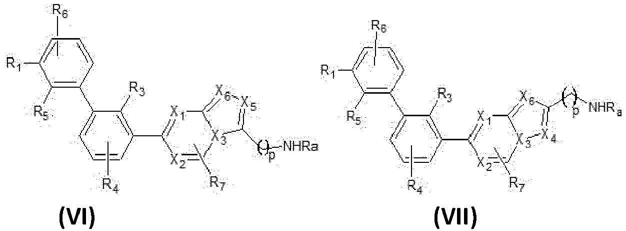 or a pharmaceutically acceptable salt or a stereoisomer thereof, wherein X₁-X₆, R₁, R₃, R₄-R₇, and Ra have the same meaning as in E1a and the subscript p is 0, or 1.
**E7a.** The compound according to any one of E1a- E6a, or a pharmaceutically acceptable salt or a stereoisomer thereof, wherein the heteroaryl group 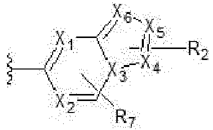 is selected from the group consisting of: 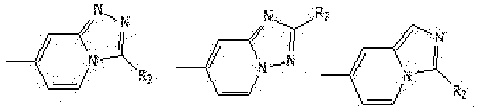 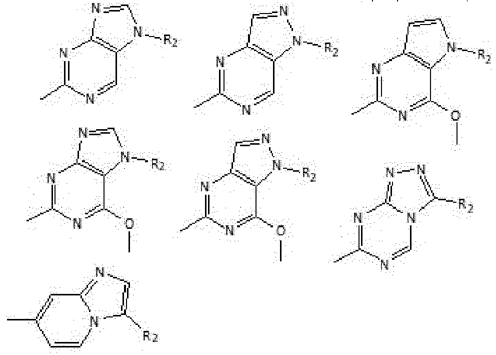
**E8a.** The compound of any one of E1a-E7a, wherein R₃ is methyl, CI, or CN, R₄, and R₇ are H, R₅ is H, methyl, or Cl.
**E9a.** The compound of any one of E1a-E7a, wherein R₃ is methyl, and R₄, R₅, and R₇ are H.
**E10a.** The compound of any one of E1a-E7a, wherein R₃ and R₅ are methyl, and R₄ and R₇ are H.
**E11a.** The compound of any one of E1a-E7a, wherein R₃ and R₅ are CI, and R₄ and R₇ are H.
**E12a.** The compound according to E1a wherein R₃ and R₅ are methyl or Cl, R₁, R₄, R₆ and R₇ are H, R₂ is C₆-₁₀ aryl-C₁-₄ alkyl- substituted with R_{b}, R_{b} is 4-10 membered heterocycloalkyl substituted with R_{d}, R_{d} is C(O)ORₑ, and Rₑ is H, and X₁-X₆ are each independently C or N, with the proviso that at least two and at most five of X₁-X₆ are N.
**E13a.** The compound according to E1a wherein R₃ is methyl or Cl, R₁, R₄, R₅, R₆ and R₇ are H, R₂ is C₆-₁₀ aryl-C₁-₄ alkyl- substituted with R_{b}, R_{b} is 4-10 membered heterocycloalkyl substituted with R_{d}, R_{d} is C(O)ORₑ, and Rₑ is H, and X₁-X₆ are each independently C or N, with the proviso that at least two and at most five of X₁-X₆ are N.
**E14a.** The compound according to E1a wherein R₃ is methyl, R₄, R₅, and R₇ are H, R₁ and R₆ are H, or are linked together to form a 4-10 membered heterocycloalkyl, R₂ is C₆-₁₀ aryl-C₁-₄ alkyl- substituted with R_{b}, R_{b} is NHR_{c}, R_{c} is C₁-₆ alkyl substituted with 1, or 2 R_{ds}, R_{d} is C(O)ORₑ, S(O)₂Rₑ, or ORₑ, and Rₑ is H, or OH, and wherein X₁-X₆ are each independently C or N, with the proviso that at least two and at most five of X₁-X₆ are N.
**E15a.** The compound according to E1a wherein R₃ is methyl or CI, R₄, R₅, and R₇ are H, R₁ and R₆ are linked together to form a 4-10 membered heterocycloalkyl, R₂ is C₆-₁₀ aryl-C₁-₄ alkylsubstituted with R_{b}, R_{b} is 4-10 membered heterocycloalkyl substituted with 1, or 2 R_{ds}, R_{d} is C(O)ORₑ, or ORₑ, and Rₑ is H, and wherein X₁-X₆ are each independently C or N, with the proviso that at least two and at most five of X₁-X₆ are N.
**E16a.** The compound according to E1a wherein R₃ is methyl, R₄, R₅, and R₇ are H, R₁ and R₆ are linked together to form a 4-10 membered heterocycloalkyl, R₂ is C₆-₁₀ aryl-C₁-₄ alkylsubstituted with 3 R_{bs}, R_{b1} is 4-10 membered heterocycloalkyl substituted with 1 or 2 R_{ds}, R_{d} is C(O)ORₑ, or ORₑ, and Rₑ is H, R_{b2} and R_{b3} are C₁-₆ alkoxy, and wherein X₁-X₆ are each independently C or N, with the proviso that at least two and at most five of X₁-X₆ are N.
**E17a.** The compound according to E1a wherein R₃ is CN, R₁, R₄, R₅, R₆ and R₇ are H, R₂ is C₆-₁₀ aryl-C₁-₄alkyl- substituted with R_{b}, R_{b} is 4-10 membered heterocycloalkyl substituted with R_{d}, R_{d} is C(O)ORₑ, and Rₑ is H, and wherein X₁-X₆ are each independently C or N, with the proviso that at least two and at most five of X₁-X₆ are N.
**E18a.** The compound according to E1a, wherein R₃ is methyl, R₁, R₄, R₅, R₆ and R₇ are H, R₂ is C₂-₆ alkynyl- substituted with R_{b}, R_{b} is 4-10 membered heterocycloalkyl substituted with R_{d}, R_{d} is C(O)ORₑ, and Rₑ is H, and wherein X₁-X₆ are each independently C or N, with the proviso that at least two and at most five of X₁-X₆ are N.
**E19a.** The compound according to E1a wherein R₃ is methyl, R₁, R₄, R₅, R₆ and R₇ are H, R₂ is 5-14 membered heteroaryl substituted with R_{b}, R_{b} is C(O)NHR_{c}, R_{c} is C₁-₆ alkyl substituted with R_{d}, R_{d} is C(O)ORₑ, and Rₑ is H, and wherein and X₁-X₆ are each independently C or N, with the proviso that at least two and at most five of X₁-X₆ are N.
**E20a.** The compound according to E1a, wherein R₃ is methyl, R₄, R₅, and R₇ are H, R₁ and R₆ are linked together to form a 4-10 membered heterocycloalkyl, R₂ is 5-14 membered heteroaryl substituted with R_{b}, R_{b} is C(O)NHR_{c}, R_{c} is C₁-₆ alkyl substituted with R_{d}, R_{d} is C(O)ORₑ, and Rₑ is H, and wherein X₁-X₆ are each independently C or N, with the proviso that at least two and at most five of X₁-X₆ are N.

### Embodiments (Eb)

**E1b.** A compound of Formula (I) or a pharmaceutically acceptable salt or a stereoisomer thereof 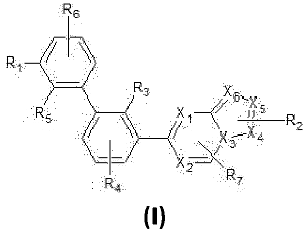 wherein:
   X₁-X₆ are each independently C or N, with the proviso that at least two and at most five of X₁-X₆ are N;
   R₂ is C₆-₁₀ aryl-C₁-₄ alkyl- substituted with R_{b}, R_{b} is 4-10 membered heterocycloalkyl substituted with R_{d}, R_{d} is C(O)ORₑ, and Rₑ is H;
   R₃ and R₅ are each independently methyl or CI;
   R₇ is H, C₁-₆ alkyl, C₁-₆ alkoxy, halogen, or CN;
   R₄ and R₆ are H
   and R₁ is selected from R₁₋ₐₐ, R_{1-bb}, R_{1-cc}, R_{1-dd}, R_{1-bc}, R_{1-bd}, R_{1-dd}, R_{1-de}, and R₁₋ᵢᵢ,
   wherein in R₁₋ₐₐ, R_{1-bb}, R_{1-cc}, R_{1-dd}, R_{1-bc}, R_{1-bd}, R_{1-dd}, R_{1-de}, and R₁₋ᵢᵢ, the subscripts r are each independently an integer of 0, 1, or 2, the subscripts q are each independently an integer of 0, 1, 2 or 3, Y₁ and Y₂ are each independently C or N;
   each R_{b} substituent is independently selected from C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, and wherein the C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, of R_{b} are each independently substituted with 1, 2, or 3 independently selected R_{d} substituents;
   each R_{c} is independently selected from H, C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl, C(O)R_{d}, C(O)NHR_{d}, and C(O)NR_{d}R_{d}, and wherein the C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl of R_{c} are each independently substituted with 1, 2, or 3 independently selected R_{d} substituents;
   each R_{d} is independently selected from C₁-₆ alkyl, C₁-₆ alkoxy, C₆-₁₀ aryl, C₃-₁₀ cycloalkyl, 5-14 membered heteroaryl, 4-10 membered heterocycloalkyl, C₃-C₆ cyclic amide, CN, NH₂, C(O)ORₑ, NHRₑ, NRₑRₑ, NRₑC(O)Rₑ, NRₑC(O)ORₑ, ORₑ, (=O), S(O)₂Rₑ and S(O)₂NRₑRₑ, and wherein the C₁-₆ alkyl, C₁-₆ alkoxy, C₆-₁₀aryl, C₃-₁₀ cycloalkyl, 5-14 membered heteroaryl, 4-10 membered heterocycloalkyl, C₃-C₆ cyclic amide of R_{d} are each optionally substituted with 1, 2, or 3 independently selected R_{f} substituents;
   each Rₑ is independently selected from H, OH, COOH, C(O)R_{f}, C₁-₆ alkyl, and 5-14 membered heteroaryl wherein the C₁-₆ alkyl, and 5-14 membered heteroaryl of Rₑ are each optionally substituted with 1, 2 or 3 independently selected R_{f} substituents;
   each R_{f} is independently selected from H, C₁-₄ alkyl, halogen, CN, COOH, OH, (=O). The other variables of Formula (I) are as defined in any embodiment disclosed herein.
**E2b.** The compound according to E1b, wherein R₁ is selected from R₁₋ₐ, R_{1-b}, R1_{-c}, R₁₋ₐ, R₁₋ₑ, R_{1-f}, R_{1-g}, R₁₋ₕ, and R₁₋ᵢ,
   wherein in R₁₋ₐ, R_{1-b}, R1_{-c}, R₁₋ₐ, R₁₋ₑ, R_{1-f}, R_{1-g}, R₁₋ₕ, and R₁₋ᵢ each R_{b} substituent is independently selected from C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, and wherein the C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, of R_{b} are each independently substituted with 1, 2, or 3 independently selected R_{d} substituents;
   each R_{c} is independently selected from H, C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl, C(O)R_{d}, C(O)NHR_{d}, and C(O)NR_{d}R_{d}, wherein the C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl of R_{c} are each independently substituted with 1, 2, or 3 independently selected R_{d} substituents;
   each R_{d} is independently selected from C₁-₆ alkyl, C₁-₆ alkoxy, C₆-₁₀ aryl, C₃-₁₀ cycloalkyl, 5-14 membered heteroaryl, 4-10 membered heterocycloalkyl, C₃-C₆ cyclic amide, CN, NH₂, C(O)ORₑ, NHRₑ, NRₑRₑ, NRₑC(O)Rₑ, NRₑC(O)ORₑ, ORₑ, (=O), S(O)₂Rₑ and S(O)₂NRₑRₑ, and wherein the C₁-₆ alkyl, C₁-₆ alkoxy, C₆-₁₀aryl, C₃-₁₀ cycloalkyl, 5-14 membered heteroaryl, 4-10 membered heterocycloalkyl, C₃-C₆ cyclic amide of R_{d} are each optionally substituted with 1, 2, or 3 independently selected R_{f} substituents;
   each Rₑ is independently selected from H, OH, COOH, C(O)R_{f}, C₁-₆ alkyl, and 5-14 membered heteroaryl, and wherein the C₁-₆ alkyl, and 5-14 membered heteroaryl of Rₑ are each optionally substituted with 1, 2 or 3 independently selected R_{f} substituents;
   each R_{f} is independently selected from H, C₁-₄ alkyl, halogen, CN, COOH, OH, (=O). The other variables of Formula (I) are as defined in any embodiment disclosed herein.
**E3b.** The compound according to E1b, wherein R₁ is selected from R₁-₁, R₁-₂, R₁-₃, R₁₋ₐ, R₁-₅, R-₆, R₁-₇, R₁-₈, R₁-₉, R₁-₁₀, R₁-₁₁, R₁-₁₂, R₁-₁₃, and R₁-₁₄.
**E4b.** The compound according to any one of E1b-E3b, wherein R₃ and R₅ are both CI.
**E5b.** The compound according to any one of E1b-E3b, wherein R₃ and R₅ are both methyl.
**E6b.** A compound of Formula (I) or a pharmaceutically acceptable salt or a stereoisomer thereof, wherein R₃ and R₅ are each independently methyl or CI, R₄, R₆ and R₇ are H, R₂ is C₆-₁₀ aryl-C₁-₄ alkyl- substituted with R_{b}, R_{b} is NHR_{c}, R_{c} is C₁-₆ alkyl substituted with R_{d}, R_{d} is ORₑ, Rₑ is H or C₁-₆ alkyl, and wherein X₁-X₆ are each independently C or N, with the proviso that at least two and at most five of X₁-X₆ are N, and wherein R₁ is as defined in E1b.
**E7b.** The compound according to E6b, wherein R₁ is wherein R₁ is selected from R₁₋ₐ, R_{1-b}, R_{1-c} R_{1-d}, R₁₋ₑ, R_{1-f}, R_{1-g}, R₁₋ₕ, and R₁₋ₗ as defined in E2b.
**E8b.** The compound according to E6b, wherein R₁ is wherein R₁ is selected from R₁-₁, R₁-₂, R₁-₃, R₁-₄, R₁-₅, R-₆, R₁-₇, R₁-₅, R₁-₉, R₁-₁₀, R₁-₁₁, R₁-₁₂, R₁-₁₃, and R₁-₁₄.
**E9b.** The compound according to any one of E6b-E8b, wherein R₃ and R₅ are both CI.
**E10b.** The compound according to any one of E6b-E8b, wherein R₃ and R₅ are both methyl.
**E11b.** A compound of Formula (I) or a pharmaceutically acceptable salt or a stereoisomer thereof, wherein R₃ and R₅ are each independently methyl or CI, R₄, R₆ and R₇ are H, R₂ is C₁-₆ alkyl-NHRₐ, Rₐ is C₁-₆ alkyl substituted with R_{d}, R_{d} is ORₑ or C₃-C₆ cyclic amide, Rₑ is H or C₁-₆ alkyl, and wherein X₁-X₆ are each independently C or N, with the proviso that at least two and at most five of X₁-X₆ are N, and wherein R₁ is as defined in E1b.
**E12b.** The compound according to E11b, wherein R₁ is selected from R₁₋ₐ, R_{1-b}, R1_{-c}, R_{1-d}, R₁₋ₑ, R_{1-f}, R_{1-g}, R₁₋ₕ, and R₁₋ₗ as defined in E2b.
**E13b.** The compound according to E11b, wherein R₁ is wherein R₁ is selected from from R₁-₁, R₁-₂, R₁-₃, R₁-₄, R₁-₅, R-₆, R₁-₇, R₁-₅, R₁-₉, R₁-₁₀, R₁-₁₁, R₁-₁₂, R₁-₁₃, and R₁-₁₄.
**E14b.** The compound according to any one of E11b-E13b, wherein R₃ and R₅ are both CI.
**E15b.** The compound according to any one of E11b-E13b, wherein R₃ and R₅ are both methyl.
**E16b.** A compound of Formula (I) or a pharmaceutically acceptable salt or a stereoisomer thereof, wherein R₃ and R₅ are each independently methyl or CI, R₄, R₆ and R₇ are H, R₂ is C₁-₆ alkyl-NHRₐ, Rₐ is C₁-₆ alkyl substituted with R_{d}, R_{d} is ORₑ or C₃-C₆ cyclic amide, Rₑ is H or C₁-₆ alkyl, and wherein X₁-X₆ are each independently C or N, with the proviso that at least two and at most five of X₁-X₆ are N, and wherein R₁ is selected from:
   C₁-₆ alkoxy, C₆-₁₀ aryl, 5-14 membered heteroaryl, C₆-₁₀ aryl- C₁-₄ alkyl-, (5-14 membered heteroaryl)-C₁-₄ alkyl-, NHORₐ, C(O)Rₐ, NHCORₐ, NHRₐ, CONHRₐ, NHSO₂Rₐ, and SO₂NHRₐ, and wherein the C₁-₆ alkoxy, C₆-₁₀ aryl, 5-14 membered heteroaryl, C₆-₁₀ aryl- C₁-₄ alkyl-, (5-14 membered heteroaryl)-C₁-₄alkyl-are substituted with 1, or 2 R_{b} substituents; in R₁:
   each Rₐ is independently selected from C₆-₁₀ aryl, 5-14 membered heteroaryl, C₆-₁₀ aryl-C₁-₄ alkyl, and (5-14 membered heteroaryl)-C₁-₄ alkyl- each independently substituted with R_{d};
   each R_{b} substituent is independently selected from C₆-₁₀ aryl, C₆-₁₀ aryl-C₁-₄ alkyl, NHC(O)R_{c}, C(O)NHR_{c}, NHR_{c}, and OMe, and wherein the C₆-₁₀ aryl and C₆-₁₀ aryl-C₁-₄ alkyl are each independently substituted with R_{d};
   each R_{c} is independently selected from C₁-₆ alkyl, C₁-₆ alkyl substituted with OH, 4-10 membered heterocycloalkyl, and (4-10 membered heterocycloalkyl)-C₁-₄ alkyl-, and wherein the C₁-₆ alkyl, 4-10 membered heterocycloalkyl, and (4-10 membered heterocycloalkyl)-C₁-₄ alkyl- are each independently substituted with (=O), (OH), or COOH;
   each R_{d} is independently selected from NHRₑ, 4-10 membered heterocycloalkyl, and (4-10 membered heterocycloalkyl)-C₁-₄ alkyl-, and wherein the 4-10 membered heterocycloalkyl, and (4-10 membered heterocycloalkyl)-C₁-₄ alkyl- are each independently substituted with (=O), (OH), or COOH;
   each Rₑ is independently selected from C₁-₆ alkyl substituted with OH, 4-10 membered heterocycloalkyl, and (4-10 membered heterocycloalkyl)-C₁-₄ alkyl-, and wherein the 4-10 membered heterocycloalkyl, and (4-10 membered heterocycloalkyl)-C₁-₄ alkyl- are each independently substituted with (=O), (OH), or COOH;
**E17b.** A compound of Formula (I) or a pharmaceutically acceptable salt or a stereoisomer thereof wherein;
   X₁-X₆ are each independently C or N, with the proviso that at least two and at most five of X₁-X₆ are N;
   R₂ is C₆-₁₀ aryl-C₁-₄ alkyl- substituted with R_{b}, R_{b} is 4-10 membered heterocycloalkyl substituted with R_{d}, R_{d} is C(O)ORₑ, and Rₑ is H;
   R₃ and R₅ are each independently methyl or CI;
   R₇ is H, C₁-₆ alkyl, C₁-₆ alkoxy, halogen, or CN;
   R₄ and R₆ are H; and wherein R₁ is as defined in E16b.
**E18b.** A compound of Formula (I) or a pharmaceutically acceptable salt or a stereoisomer thereof, wherein R₃ and R₅ are each independently methyl or CI, R₄, R₆ and R₇ are H, R₂ is C₆-₁₀ aryl-C₁-₄ alkyl- substituted with R_{b}, R_{b} is NHR_{c}, R_{c} is C₁-₆ alkyl substituted with R_{d}, R_{d} is ORₑ, Rₑ is H or C₁-₆ alkyl, and wherein X₁-X₆ are each independently C or N, with the proviso that at least two and at most five of X₁-X₆ are N, and wherein R₁ is as defined in E16b.

## Claims

1. A compound of Formula (I) or a pharmaceutically acceptable salt or a stereoisomer thereof 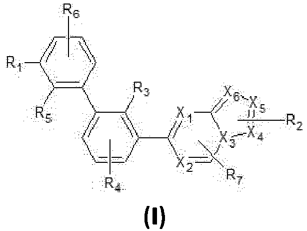 wherein:
X₁-X₆ are each independently C or N, with the proviso that at least two and at most five of X₁-X₆ are N;
R₃ is H, methyl, halogen, or CN;
R₄, and R₅ are each independently H, methyl, halogen, or CN;
R₇ is H, C₁-₆ alkyl, C₁-₆ alkoxy, halogen, or CN;
R₁ is selected from H, C₁-₆ alkyl, C₂-₆ alkynyl, C₁-₆ alkoxy, C₆-₁₀ aryl, 5-14 membered heteroaryl, C₆-₁₀ aryl- C₁-₄ alkyl-, C₃-₁₀ cycloalkyl-C₁-₄ alkyl-, (5-14 membered heteroaryl)-C₁-₄ alkyl-, (4-10 membered heterocycloalkyl)-C₁-₄ alkyl-, C₁-₆ alkyl-NHRₐ, NHORₐ, C(O)Rₐ, NHCORₐ, NHRₐ, CONHRₐ, NHSO₂Rₐ, SO₂NHRₐ, and OCH₂Rₐ, or R₁ and R₆ are linked together to form a 4-10 membered heterocycloalkyl, and wherein the C₁-₆ alkyl, C₂-₆ alkynyl, C₁-₆ alkoxy, C₆-₁₀ aryl, 5-14 membered heteroaryl, C₆-₁₀ aryl- C₁-₄ alkyl-, C₃-₁₀ cycloalkyl-C₁-₄ alkyl-, (5-14 membered heteroaryl)-C₁-₄ alkyl-, (4-10 membered heterocycloalkyl)-C₁-₄ alkyl- of R₁ are each optionally substituted with 1, 2, 3, or 4 independently selected R_{b} substituents;
R₂ is selected from C₁-₆ alkyl, C₂-₆ alkynyl, C₁-₆ alkoxy, C₆-₁₀ aryl, 5-14 membered heteroaryl, C₆-₁₀ aryl-C₁-₄ alkyl-, C₃-₁₀ cycloalkyl-C₁-₄ alkyl-, (5-14 membered heteroaryl)-C₁-₄ alkyl-, (4-10 membered heterocycloalkyl)-C₁-₄ alkyl-, C₃-C₆-cyclic-amide-C₁-₄ alkyl-, C₁-₆ alkyl-NHRₐ, - NHORₐ, C(O)Rₐ, NHCORₐ, CONHRₐ, NHSO₂Rₐ, SO₂NHRₐ, NHRₐ and OCH₂Rₐ, and wherein the C₁-₆ alkyl, C₂-₆ alkynyl, C₁-₆ alkoxy, C₆-₁₀ aryl, 5-14 membered heteroaryl, C₆-₁₀ aryl-C₁-₄alkyl-, C₃-₁₀ cycloalkyl-C₁-₄ alkyl-, (5-14 membered heteroaryl)-C₁-₄ alkyl-, (4-10 membered heterocycloalkyl)-C₁-₄ alkyl-, C₃-C₆-cyclic-amide-C₁-₄ alkyl- of R₂ are each independently substituted with 1, 2, 3, or 4 independently selected R_{b} substituents;
R₆ is selected from H, C₁-₆ alkyl, C₂-₆ alkynyl, halogen, and OCH₂Rₐ or R₁ and R₆ are linked together to form a 4-10 membered heterocycloalkyl optionally substituted with 1, 2, 3, or 4 independently selected R_{b} substituents;
each Rₐ is independently selected from H, C₁-₆ alkyl, C₁-₆ alkoxy, C₆-₁₀ aryl, C₃-₁₀ cycloalkyl, 5-14 membered heteroaryl, 4-10 membered heterocycloalkyl, C₆-₁₀ aryl-C₁-₄ alkyl, C₃-₁₀ cycloalkyl-C₁-₄ alkyl-, (5-14 membered heteroaryl)-C₁-₄ alkyl-, (4-10 membered heterocycloalkyl)-C₁-₄ alkyl-, C₁-₆ alkyl-NHR_{c}, NHOR_{c}, NHCOR_{c}, NHR_{c}, and OCH₂R_{c}, and wherein the C₁-₆ alkyl, C₁-₆ alkoxy, C₆-₁₀ aryl, C₃-₁₀ cycloalkyl, 5-14 membered heteroaryl, 4-10 membered heterocycloalkyl, C₆-₁₀ aryl-C₁-₄ alkyl, C₃-₁₀ cycloalkyl-C₁-₄ alkyl-, (5-14 membered heteroaryl)-C₁-₄ alkyl-, (4-10 membered heterocycloalkyl)-C₁-₄ alkyl- of Rₐ are each independently substituted with 1, 2, or 3 independently selected R_{d} substituents;
each R_{b} substituent is independently selected from C₁-₆ alkyl, C₁-₆ alkoxy, C₆-₁₀ aryl, C₃-₁₀ cycloalkyl, 5-14 membered heteroaryl, 4-10 membered heterocycloalkyl, C₆-₁₀ aryl-C₁-₄ alkyl, C₃-₁₀ cycloalkyl-C₁-₄ alkyl-, (5-14 membered heteroaryl)-C₁-₄ alkyl-, (4-10 membered heterocycloalkyl)-C₁-₄ alkyl-, C₃-C₆ cyclic amide C₁-₄ alkyl-, OH, (=O), NH₂, NHOR_{c}, NHC(O)R_{c}, OR_{c}, C(O)R_{c}, C(O)NHR_{c}, C(O)NR_{c}R_{c}, C(O)OR_{c}, OC(O)R_{c}, C₁-₆ alkyl-NHR_{c}, NHR_{c}, NR_{c}R_{c}, S(O)₂NR_{c}R_{c}, and wherein the C₁-₆ alkyl, C₁-₆ alkoxy, C₆-₁₀ aryl, C₃-₁₀ cycloalkyl, 5-14 membered heteroaryl, 4-10 membered heterocycloalkyl, C₆-₁₀ aryl-C₁-₄ alkyl, C₃-₁₀ cycloalkyl-C₁-₄ alkyl-, (5-14 membered heteroaryl)-C₁-₄ alkyl-, (4-10 membered heterocycloalkyl)-C₁-₄ alkyl-, C₃-C₆ cyclic amide C₁-₄ alkyl- of R_{b} are each independently substituted with 1, 2, or 3 independently selected R_{d} substituents;
each R_{c} is independently selected from H, C₁-₆ alkyl, C₂-₆ alkenyl, C₁-₆ alkoxy, C₆-₁₀ aryl, C₃-₁₀ cycloalkyl, 5-14 membered heteroaryl, 4-10 membered heterocycloalkyl, C₆-₁₀ aryl-C₁-₄ alkyl, C₃-₁₀ cycloalkyl-C₁-₄ alkyl-, (5-14 membered heteroaryl)-C₁-₄ alkyl-, (4-10 membered heterocycloalkyl)-C₁-₄ alkyl-, C₃-C₆ cyclic amide C₁-₄ alkyl, C(O)R_{d}, C(O)NHR_{d}, and C(O)NR_{d}R_{d}, and wherein the C₁-₆ alkyl, C₂-₆ alkenyl, C₁-₆ alkoxy, C₆-₁₀ aryl, C₃-₁₀ cycloalkyl, 5-14 membered heteroaryl, 4-10 membered heterocycloalkyl, C₆-₁₀ aryl-C₁-₄ alkyl, C₃-₁₀ cycloalkyl-C₁-₄ alkyl-, (5-14 membered heteroaryl)-C₁-₄ alkyl-, (4-10 membered heterocycloalkyl)-C₁-₄ alkyl-, C₃-C₆ cyclic amide C₁-₄ alkyl of R_{c} are each independently substituted with 1, 2, or 3 independently selected R_{d} substituents;
each R_{d} is independently selected from C₁-₆ alkyl, C₁-₆ alkoxy, C₆-₁₀ aryl, C₃-₁₀ cycloalkyl, 5-14 membered heteroaryl, 4-10 membered heterocycloalkyl, C₆-₁₀ aryl-C₁-₄ alkyl-, C₃-₁₀ cycloalkyl-C₁-₄ alkyl-, (5-14 membered heteroaryl)-C₁-₄ alkyl-, (4-10 membered heterocycloalkyl)-C₁-₄ alkyl-, C₃-C₆ cyclic amide, C₃-C₆ cyclic amide C₁-₄ alkyl-, CN, NH₂, NHORₑ, C(O)Rₑ, C(O)NRₑRₑ, C(O)ORₑ, OC(O)Rₑ, OC(O)NRₑRₑ, (=O), NHRₑ, NRₑRₑ, NRₑC(O)Rₑ, NRₑC(O)ORₑ, ORₑ, S(O)₂Rₑ and S(O)₂NRₑRₑ, and wherein the C₁-₆ alkyl, C₁-₆ alkoxy, C₆-₁₀ aryl, C₃-₁₀ cycloalkyl, 5-14 membered heteroaryl, 4-10 membered heterocycloalkyl, C₆-₁₀ aryl-C₁-₄ alkyl, C₃-₁₀ cycloalkyl-C₁-₄ alkyl-, (5-14 membered heteroaryl)-C₁-₄ alkyl-, (4-10 membered heterocycloalkyl)-C₁-₄ alkyl-, C₃-C₆ cyclic amide, C₃-C₆ cyclic amide C₁-₄ alkyl- of R_{d} are each optionally substituted with 1, 2, or 3 independently selected R_{f} substituents;
each Rₑ is independently selected from H, OH, COOH, C(O)R_{f}, C₁-₆ alkyl, C₆-₁₀ aryl, C₃-₁₀ cycloalkyl, 5-14 membered heteroaryl, 4-10 membered heterocycloalkyl, C₃-C₆ cyclic amide C₁-₄ alkyl-, and wherein the C₁-₆ alkyl, C₆-₁₀ aryl, C₃-₁₀ cycloalkyl, 5-14 membered heteroaryl, 4-10 membered heterocycloalkyl, C₃-C₆ cyclic amide C₁-₄ alkyl of Rₑ are each optionally substituted with 1, 2 or 3 independently selected R_{f} substituents;
each R_{f} is independently selected from H, C₁-₄ alkyl, C₆-₁₀ aryl, C₃-₁₀ cycloalkyl, 5-14 membered heteroaryl, 4-10 membered heterocycloalkyl, C₆-₁₀ aryl-C₁-₄ alkyl-, C₃-₁₀ cycloalkyl-C₁-₄ alkyl-, (5-14 membered heteroaryl)-C₁-₄ alkyl-, (4-10 membered heterocycloalkyl)-C₁-₄ alkyl-, C₃-C₆ cyclic amide, halogen, CN, COOH, OH, (=O).

2. A compound according to claim 1, or a pharmaceutically acceptable salt or a stereoisomer thereof, wherein:
X₁-X₆ are each independently C or N, with the proviso that at least two and at most five of X₁-X₆ are N;
R₃ is H, methyl, halogen, or CN;
R₄, and R₅ are each independently H, methyl, halogen, or CN;
R₇ is H, C₁-₆ alkyl, C₁-₆ alkoxy, halogen, or CN;
R₁ is selected from H, C₁-₆ alkyl, C₂-₆ alkynyl, C₁-₆ alkoxy, C₆-₁₀ aryl, 5-14 membered heteroaryl, C₆-₁₀ aryl- C₁-₄ alkyl-, C₃-₁₀ cycloalkyl-C₁-₄ alkyl-, (5-14 membered heteroaryl)-C₁-₄ alkyl-, (4-10 membered heterocycloalkyl)-C₁-₄ alkyl-, C₁-₆ alkyl-NHRₐ, NHCORₐ, NHRₐ, NHORₐ, C(O)Rₐ, CONHRₐ, NHSO₂Rₐ, SO₂NHRₐ, and OCH₂Rₐ, or R₁ and R₆ are linked together to form a 4-10 membered heterocycloalkyl, and wherein C₁-₆ alkyl, C₂-₆ alkynyl, C₁-₆ alkoxy, C₆-₁₀ aryl, 5-14 membered heteroaryl, C₆-₁₀ aryl- C₁-₄ alkyl-, C₃-₁₀ cycloalkyl-C₁-₄ alkyl-, (5-14 membered heteroaryl)-C₁-₄ alkyl-, (4-10 membered heterocycloalkyl)-C₁-₄ alkyl- of R₁ are each optionally substituted with 1, 2, or 3 independently selected R_{b} substituents;
R₂ is selected from C₁-₆ alkyl, C₂-₆ alkynyl, C₁-₆ alkoxy, C₆-₁₀ aryl, 5-14 membered heteroaryl, C₆-₁₀ aryl-C₁-₄ alkyl-, (5-14 membered heteroaryl)-C₁-₄ alkyl-, (4-10 membered heterocycloalkyl)-C₁-₄ alkyl-, C₁-₆ alkyl-NHRₐ, and NHRₐ and wherein the C₁-₆ alkyl, C₂-₆ alkynyl, C₁-₆ alkoxy, C₆-₁₀ aryl, 5-14 membered heteroaryl, C₆-₁₀ aryl-C₁-₄ alkyl-, (5-14 membered heteroaryl)-C₁-₄ alkyl-, (4-10 membered heterocycloalkyl)-C₁-₄ alkyl-, of R₂ are each independently substituted with 1, 2, or 3 independently selected R_{b} substituents;
R₆ is selected from H, C₁-₆ alkyl, halogen, and OCH₂Rₐ or R₁ and R₆ are linked together to form a 4-10 membered heterocycloalkyl optionally substituted with 1, 2, 3, or 4 independently selected R_{b} substituents;
each Rₐ is independently selected from H, C₁-₆ alkyl, C₁-₆ alkoxy, C₆-₁₀ aryl, 5-14 membered heteroaryl, 4-10 membered heterocycloalkyl, C₆-₁₀ aryl-C₁-₄ alkyl, (5-14 membered heteroaryl)-C₁-₄ alkyl-, (4-10 membered heterocycloalkyl)-C₁-₄ alkyl-, NHR_{c}, and OCH₂R_{c} and wherein C₁-₆ alkyl, C₁-₆ alkoxy, C₆-₁₀ aryl, 5-14 membered heteroaryl, 4-10 membered heterocycloalkyl, C₆-₁₀ aryl-C₁-₄ alkyl, (5-14 membered heteroaryl)-C₁-₄ alkyl-, (4-10 membered heterocycloalkyl)-C₁-₄ alkyl- of Rₐ are each independently substituted with 1, or 2 independently selected R_{d} substituents;
each R_{b} substituent is independently selected from C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, and wherein the C₁-₆ alkyl, C₁-₆ alkoxy, C₃-₁₀ cycloalkyl, 4-10 membered heterocycloalkyl, C₆-₁₀ aryl-C₁-₄ alkyl, OH, (=O), NH₂, NHOR_{c}, NHC(O)R_{c}, OR_{c}, C(O)R_{c}, C(O)NHR_{c}, C(O)OR_{c}, C₁-₆ alkyl-NHR_{c}, NHR_{c}, of R_{b} are each independently substituted with 1, 2, or 3 independently selected R_{d} substituents;
each R_{c} is independently selected from H, C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl, C(O)R_{d}, C(O)NHR_{d}, and C(O)NR_{d}R_{d}, and wherein the C₁-₆ alkyl, C₁-₆ alkoxy, C₃-₁₀ cycloalkyl, 5-14 membered heteroaryl, 4-10 membered heterocycloalkyl, C₆-₁₀ aryl-C₁-₄ alkyl, C₃-C₆ cyclic amide C₁-₄ alkyl of R_{c} are each independently substituted with 1, 2, or 3 independently selected R_{d} substituents;
each R_{d} is independently selected from C₁-₆ alkyl, C₁-₆ alkoxy, C₆-₁₀ aryl, C₃-₁₀ cycloalkyl, 5-14 membered heteroaryl, 4-10 membered heterocycloalkyl, C₃-C₆ cyclic amide, CN, NH₂, C(O)ORₑ, NHRₑ, NRₑRₑ, NRₑC(O)Rₑ, NRₑC(O)ORₑ, ORₑ, (=O), S(O)₂Rₑ and S(O)₂NRₑRₑ, and wherein the C₁-₆ alkyl, C₁-₆ alkoxy, C₆-₁₀aryl, C₃-₁₀ cycloalkyl, 5-14 membered heteroaryl, 4-10 membered heterocycloalkyl, C₃-C₆ cyclic amide of R_{d} are each optionally substituted with 1, 2, or 3 independently selected R_{f} substituents;
each Rₑ is independently selected from H, OH, COOH, C(O)R_{f}, C₁-₆ alkyl, and 5-14 membered heteroaryl wherein the C₁-₆ alkyl, and 5-14 membered heteroaryl of Rₑ are each optionally substituted with 1, 2 or 3 independently selected R_{f} substituents;
each R_{f} is independently selected from H, C₁-₄ alkyl, halogen, CN, COOH, OH, (=O).

3. A compound according to claim 1 or 2, having Formula (II) 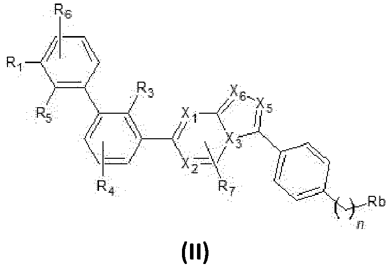 or a pharmaceutically acceptable salt or a stereoisomer thereof, wherein X₁-X₃, X₅ and X₆, R₁, R₃, R₄-R₇, and R_{b} have the same meaning as in claim 1 or 2 and the subscript n is 1, 2, or 3.

4. A compound according to claim 3 wherein n is 1.

5. A compound according to claim 1 or 2, having Formula (IV) or (V) 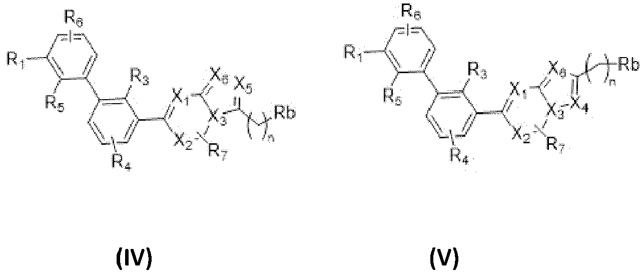
or a pharmaceutically acceptable salt or a stereoisomer thereof, wherein in Formula (IV) X₁-X₃, X₅ and X₆, R₁, R₃, R₄-R₇, and R_{b} have the same meaning as in claim 1 or 2 and the subscript n is 1, 2, or 3;
wherein in Formula (V), X₁-X₄, and X₆, R₁, R₃, R₄-R₇, and R_{b} have the same meaning as in claim 1 or 2 and the subscript n is 1, 2, or 3.

6. A compound according to claim 1 or 2, having Formula (VI) or (VII) 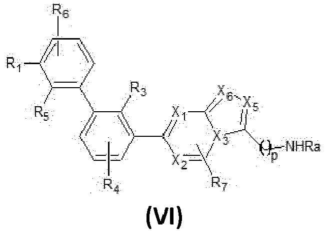 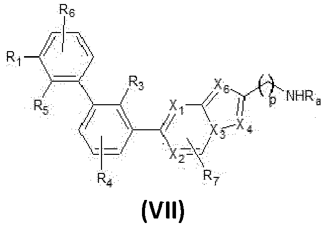 or a pharmaceutically acceptable salt or a stereoisomer thereof, wherein in Formula (VI) X₁-X₃, X₅ and X₆, R₁, R₃, R₄-R₇, and Rₐ have the same meaning as in claim 1 or 2 and the subscript p is 0, or 1; and wherein in Formula (VII) X₁-X₄, and X₆ R₁, R₃, R₄-R₇, and Rₐ have the same meaning as in claim 1 or 2 and the subscript p is 0, or 1.

7. A compound of any one of claims 1-6, wherein R₃ is methyl, CI, or CN, R₄, and R₇ are H, R₅ is H, methyl, or CI, or wherein R₃ is methyl, and R₄, R₅, and R₇ are H, or wherein R₃ and R₅ are methyl, and R₄ and R₇ are H, or wherein R₃ and R₅ are CI, and R₄ and R₇ are H.

8. A compound of any one of claims 1-7 wherein R₁ is selected from: 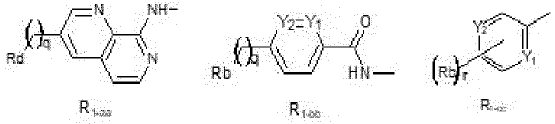 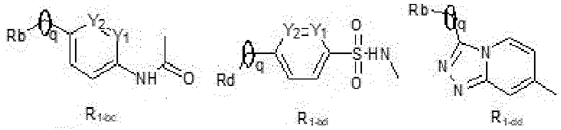 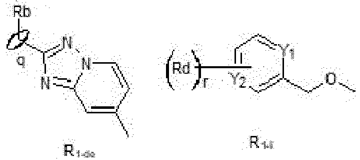 wherein, the subscripts r are each independently an integer of 0, 1, or 2, the subscripts q are each independently an integer of 0, 1, 2 or 3, Y₁ and Y₂ are each independently C or N, R_{b} and R_{d} are as defined in any of the preceding claims.

9. A compound of any one of claims 1 or 2 wherein R₃ and R₅ are methyl or CI, R₄, R₆ and R₇ are H, R₂ is C₆-₁₀ aryl-C₁-₄ alkyl- substituted with R_{b}, R_{b} is 4-10 membered heterocycloalkyl substituted with R_{d}, R_{d} is C(O)ORₑ, and Rₑ is H, and R₁ is selected from:

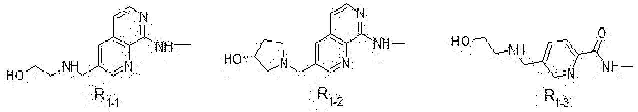

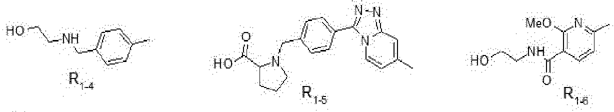

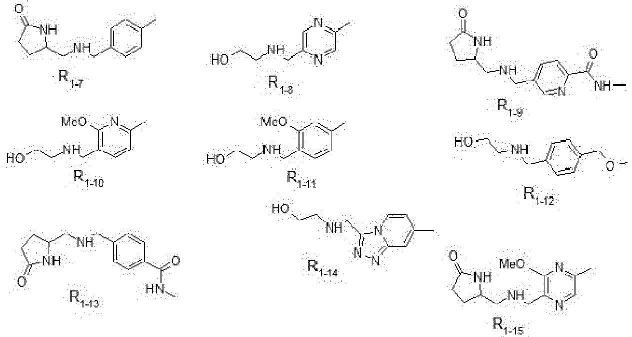

10. A compound of any one of claims 1 or 2 wherein R₃ and R₅ are methyl or CI, R₄, R₆ and R₇ are H, R₂ is C₆-₁₀ aryl-C₁-₄alkyl- substituted with R_{b}, R_{b} is NHR_{c}, R_{c} is C₁-₆ alkyl substituted with R_{d}, R_{d} is ORₑ, Rₑ is H or C₁-₆ alkyl, and R₁ is selected from the group consisting of R₁-₁, R₁-₂, R₁-₃, R₁-₄, R₁-₅, R₁-₆, R₁-₇, R₁-₈, R₁-₉, R₁-₁₀, R₁-₁₁, R₁-₁₂, R₁-₁₃, and R₁-₁₄, and R₁-₁₅.

11. A compound of any one of claims 1, or 2 wherein R₃ and R₅ are methyl or CI, R₄, R₆ and R₇ are H, R₂ is C₁-₆ alkyl-NHRₐ, Rₐ is C₁-₆ alkyl substituted with R_{d}, R_{d} is ORₑ or C₃-C₆ cyclic amide, Rₑ is H or C₁-₆ alkyl, and R₁ is selected from the group consisting of R₁-₁, R₁-₂, R₁-₃, R₁-₄, R₁-₅, R₁-₆, R₁-₇, R₁-₈, R₁-₉, R₁-₁₀, R₁-₁₁, R₁-₁₂, R₁-₁₃, R₁-₁₄, and R₁-₁₅.

12. A compound of claim 1 selected from:
1-(4-(7-(2-methyl-[1,1'-biphenyl]-3-yl)imidazo[1,2-a]pyridin-3-yl)benzyl)piperidine-2-carboxylic acid;
(4-(7-(2-methyl-[1,1'-biphenyl]-3-yl)imidazo[1,2-o]pyridin-3-yl)benzyl)proline;
3-((4-(7-(2-methyl-[1,1'-biphenyl]-3-yl)imidazo[1,2-a]pyridin-3-yl)benzyl)amino)oxetane-3-carboxylic acid;
(2*S*,4*R*)-4-hydroxy-1-(4-(7-(2-methyl-[1,1'-biphenyl]-3-yl)imidazo[1,2-*a*]pyridin-3-yl)benzyl)pyrrolidine-2-carboxylic acid;
1-((4-(7-(2-methyl-[1,1'-biphenyl]-3-yl)imidazo[1,2-*a*]pyridin-3-yl)benzyl)amino)cyclobutane-1-carboxylic acid;
4-(4-(7-(2-methyl-[1,1'-biphenyl]-3-yl)imidazo[1,2-*a*]pyridin-3-yl)benzyl)morpholine-3-carboxylic acid;
1-(2,6-dimethoxy-4-(7-(2-methyl-[1,1'-biphenyl]-3-yl)imidazo[1,2-*a*]pyridin-3-yl)benzyl)piperidine-2-carboxylic acid;
1-(4-(7-(2-methyl-[1,1'-biphenyl]-3-yl)imidazo[1,2-*a*]pyridin-3-yl)benzyl)azetidine-3-carboxylic acid;
*N*-(2-hydroxyethyl)-1-(7-(2-methyl-[1,1'-biphenyl]-3-yl)imidazo[1,2-*a*]pyridin-3-yl)-1H-pyrazole-3-carboxamide ;
1-(4-(7-(2-methyl-[1,1'-biphenyl]-3-yl)-[1,2,4]triazolo[4,3-*a*]pyridin-3-yl)benzyl)piperidine-2-carboxylic acid;
1-(4-(7-(3-(2,3-dihydrobenzo[*b*][1,4]dioxin-6-yl)-2-methylphenyl)imidazo[1,2-a]pyridin-3-yl)benzyl)piperidine-2-carboxylic acid;
(4-(7-(3-(2,3-dihydrobenzo[*b*][1,4]dioxin-6-yl)-2-methylphenyl)imidazo[1,2-*a*]pyridin-3-yl)benzyl)proline;
3-((4-(7-(2-methyl-[1,1'-biphenyl]-3-yl)imidazo[1,2-*a*]pyridin-3-yl)benzyl)amino)propan-1-ol;
(2,6-dimethoxy-4-(7-(2-methyl-[1,1'-biphenyl]-3-yl)imidazo[1,2-*a*]pyridin-3-yl)benzyl)proline;
(2,6-dimethoxy-4-(7-(2-methyl-[1,1'-biphenyl]-3-yl)imidazo[1,2-*a*]pyridin-3-yl)benzyl)proline;
(4-(7-(2-methyl-[1,1'-biphenyl]-3-yl)imidazo[1,2-*a*]pyridin-3-yl)benzyl)valine;
(4-(7-(2-methyl-[1,1'-biphenyl]-3-yl)imidazo[1,2-*a*]pyridin-3-yl)benzyl)-*L*-proline;
(4-(7-(2-methyl-[1,1'-biphenyl]-3-yl)imidazo[1,2-*a*]pyridin-3-yl)benzyl)-D-proline;
(4-(7-(2-methyl-[1,1'-biphenyl]-3-yl)imidazo[1,2-*a*]pyridin-3-yl)benzyl)glycine;
(*S*)-1-(4-(7-(2-methyl-[1,1'-biphenyl]-3-yl)imidazo[1,2-*a*]pyridin-3-yl)benzyl)azetidine-2-carboxylic acid;
(4-(7-(2-methyl-[1,1'-biphenyl]-3-yl)-[1,2,4]triazolo[4,3-*a*]pyridin-3-yl)benzyl)proline;
3-hydroxy-4-((4-(7-(2-methyl-[1,1'-biphenyl]-3-yl)imidazo[1,2-*a*]pyridin-3-yl)benzyl)amino)butanoic acid ;
(1-(7-(2-methyl-[1,1'-biphenyl]-3-yl)imidazo[1,2-*a*]pyridin-3-yl)-1*H*-pyrazole-3-carbonyl)glycine;
4-(4-(7-(3-(2,3-dihydrobenzo[*b*][1,4]dioxin-6-yl)-2-methylphenyl)imidazo[1,2-a]pyridin-3-yl)benzyl)morpholine-3-carboxylic acid;
2-((4-(7-(2-methyl-[1,1'-biphenyl]-3-yl)-[1,2,4]triazolo[4,3-*a*]pyridin-3-yl)benzyl)amino)ethan-1-ol;
(3*S*,5*R*)-1-(4-(7-(3-(2,3-dihydrobenzo[*b*][1,4]dioxin-6-yl)-2-methylphenyl)imidazo[1,2-a]pyridin-3-yl)benzyl)-5-hydroxypyrrolidine-3-carboxylic acid;
3-((4-(7-(2-methyl-[1,1'-biphenyl]-3-yl)-[1,2,4]triazolo[4,3-*a*]pyridin-3-yl)benzyl)amino)propan-1-ol;
1-(4-(7-(3-(2,3-dihydrobenzo[*b*][1,4]dioxin-6-yl)-2-methylphenyl)imidazo[1,2-o]pyridin-3-yl)benzyl)azetidine-3-carboxylic acid;
(*S*)-1-(4-(7-(3-(2,3-dihydrobenzo[*b*][1,4]dioxin-6-yl)-2-methylphenyl)imidazo[1,2-o]pyridin-3-yl)benzyl)azetidine-2-carboxylic acid;
((4-(7-(3-(2,3-dihydrobenzo[*b*][1,4]dioxin-6-yl)-2-methylphenyl)imidazo[1,2-*a*]pyridin-3-yl)-2,6-dimethoxybenzyl)-*D*-proline;
(4-(7-(3-(2,3-dihydrobenzo[*b*][1,4]dioxin-6-yl)-2-methylphenyl)imidazo[1,2-*a*]pyridin-3-yl)-2,6-dimethoxybenzyl)-*L*-proline;
(4-(7-(3-(2,3-dihydrobenzo[*b*][1,4]dioxin-6-yl)-2-methylphenyl)imidazo[1,2-*a*]pyridin-3-yl)benzyl)-*D*-proline;
(4-(7-(3-(2,3-dihydrobenzo[*b*][1,4]dioxin-6-yl)-2-methylphenyl)imidazo[1,2-*a*]pyridin-3-yl)benzyl)-*L*-proline;
1-(1-(1-(4-(7-(2-methyl-[1,1'-biphenyl]-3-yl)imidazo[1,2-*a*]pyridin-3-yl)phenyl)ethyl)azetidin-3-yl)ethan-1-one;
2-methyl-2-((4-(7-(2-methyl-[1,1'-biphenyl]-3-yl)imidazo[1,2-*a*]pyridin-3-yl)benzyl)amino)propan-1-ol;
3-hydroxy-4-((4-(7-(2-methyl-[1,1'-biphenyl]-3-yl)-[1,2,4]triazolo[4,3-*a*]pyridin-3-yl)benzyl)amino)butanoic acid;
(3-(7-(2-methyl-[1,1'-biphenyl]-3-yl)imidazo[1,2-*a*]pyridin-3-yl)prop-2-yn-1-yl)-*D-*proline;
(3-(7-(2-methyl-[1,1'-biphenyl]-3-yl)imidazo[1,2-*a*]pyridin-3-yl)prop-2-yn-1-yl)-*L*-proline
(*R*)-(1-(4-(7-(2-methyl-[1,1'-biphenyl]-3-yl)imidazo[1,2-*a*]pyridin-3-yl)benzyl)pyrrolidin-2-yl)methanol;
(*S*)-(1-(4-(7-(2-methyl-[1,1'-biphenyl]-3-yl)imidazo[1,2-*a*]pyridin-3-yl)benzyl)pyrrolidin-2-yl)methanol;
4-((4-(7-(3-(2,3-dihydrobenzo[*b*][1,4]dioxin-6-yl)-2-methylphenyl)imidazo[1,2-*a*]pyridin-3-yl)benzyl)amino)-3-hydroxybutanoic acid;
(*R*)-3-((4-(7-(3-(2,3-dihydrobenzo[*b*][1,4]dioxin-6-yl)-2-methylphenyl)imidazo[1,2-*a*]pyridin-3-yl)benzyl)amino)propane-1,2-diol;
(*S*)-3-((4-(7-(3-(2,3-dihydrobenzo[*b*][1,4]dioxin-6-yl)-2-methylphenyl)imidazo[1,2-*a*]pyridin-3-yl)benzyl)amino)propane-1,2-diol;
(4-(7-(3-(2,3-dihydrobenzo[*b*][1,4]dioxin-6-yl)-2-methylphenyl)-[1,2,4]triazolo[4,3-a]pyridin-3-yl)benzyl)-*D*-proline;
(4-(7-(3-(2,3-dihydrobenzo[*b*][1,4]dioxin-6-yl)-2-methylphenyl)-[1,2,4]triazolo[4,3-*a*]pyridin-3-yl)benzyl)-*L*-proline;
(4-(7-(3-(2,3-dihydrobenzo[*b*][1,4]dioxin-6-yl)-2-methylphenyl)imidazo[1,2-*a*]pyridin-3-yl)benzoyl)glycine;
*N*-(2-acetamidoethyl)-1-(7-(2-methyl-[1,1'-biphenyl]-3-yl)imidazo[1,2-*a*]pyridin-3-yl)-1*H*-pyrazole-3-carboxamide;
(4-(7-(3-(2,3-dihydrobenzo[*b*][1,4]dioxin-6-yl)-2-methylphenyl)imidazo[1,2-*a*]pyridin-3-yl)benzoyl)-*D*-proline;
(4-(7-(3-(2,3-dihydrobenzo[*b*][1,4]dioxin-6-yl)-2-methylphenyl)imidazo[1,2-*a*]pyridin-3-yl)benzoyl)-*L*-proline;
3-((4-(7-(2-methyl-[1,1'-biphenyl]-3-yl)imidazo[1,2-*a*]pyridin-3-yl)benzyl)amino)propanoic acid;
3-(1-(7-(2-methyl-[1,1'-biphenyl]-3-yl)imidazo[1,2-*a*]pyridin-3-yl)-1*H*-pyrazole-3-carboxamido)propanoic acid;
(1-(7-(3-(2,3-dihydrobenzo[*b*][1,4]dioxin-6-yl)-2-methylphenyl)imidazo[1,2-*a*]pyridin-3-yl)-1H-pyrazole-3-carbonyl)glycine;
(4-(7-(2-methyl-[1,1'-biphenyl]-3-yl)-[1,2,4]triazolo[4,3-*a*]pyridin-3-yl)benzyl)-*D-*proline;
(4-(7-(2-methyl-[1,1'-biphenyl]-3-yl)-[1,2,4]triazolo[4,3-*a*]pyridin-3-yl)benzyl)-*L-*proline;
2-((4-(7-(2-methyl-[1,1'-biphenyl]-3-yl)imidazo[1,2-*a*]pyridin-3-yl)benzyl)amino)ethane-1-sulfonic acid;
(2*S*,4*R*)-1-(4-(7-(3-(2,3-dihydrobenzo[b][1,4]dioxin-6-yl)-2-methylphenyl)-[1,2,4]triazolo[4,3-*a*]pyridin-3-yl)benzyl)-4-hydroxypyrrolidine-2-carboxylic acid;
3-((4-(7-(3-(2,3-dihydrobenzo[*b*][1,4]dioxin-6-yl)-2-methylphenyl)-[1,2,4]triazolo[4,3-*a*]pyridin-3-yl)benzyl)amino)propanoic acid;
(1-(7-(3-(2,3-dihydrobenzo[*b*][1,4]dioxin-6-yl)-2-methylphenyl)-[1,2,4]triazolo[4,3-*a*]pyridin-3-yl)-1*H*-pyrazole-3-carbonyl)glycine;
(4-(7-(3'-(5-(((2-hydroxyethyl)amino)methyl)picolinamido)-2,2'-dimethyl-[1,1'-biphenyl]-3-yl)-[1,2,4]triazolo[4,3-*a*]pyridin-3-yl)benzyl)-*D*-proline;
(2'*R*)-((((2,2'-dimethyl-[1,1'-biphenyl]-3,3'-diyl)bis([1,2,4]triazolo[4,3-*a*]pyridine-7,3-diyl))bis(4,1-phenylene))bis(methylene))di-*D*-proline;
(4-(7-(4"-(((2-hydroxyethyl)amino)methyl)-2,2'-dimethyl-[1,1':3',1"-terphenyl]-3-yl)-[1,2,4]triazolo[4,3-a]pyridin-3-yl)benzyl)-*D*-proline;
tert-butyl(4-(7-(3'-(5-((2-hydroxyethyl)carbamoyl)-6-methoxypyridin-2-yl)-2,2'-dimethyl-[1,1'-biphenyl]-3-yl)-[1,2,4]triazolo[4,3-*a*]pyridin-3-yl)benzyl)-*D*-prolinate;
(4-(7-(3'-(5-(((2-hydroxyethyl)amino)methyl)pyrazin-2-yl)-2,2'-dimethyl-[1,1'-biphenyl]-3-yl)-[1,2,4]triazolo[4,3-*a*]pyridin-3-yl)benzyl)-*D*-proline;
5-(((2-hydroxyethyl)amino)methyl)-*N*-(3'-(3-(4-(((2-hydroxyethyl)amino)methyl)phenyl)-[1,2,4]triazolo[4,3-*a*]pyridin-7-yl)-2,2'-dimethyl-[1,1'-biphenyl]-3-yl)picolinamide;
(4-(7-(3'-(5-(((2-hydroxyethyl)amino)methyl)picolinamido)-2,2'-dimethyl-[1,1'-biphenyl]-3-yl)-[1,2,4]triazolo[4,3-*a*]pyridin-3-yl)benzyl)-*L*-proline;
2-((4-(7-(4"-(((2-hydroxyethyl)amino)methyl)-2,2'-dimethyl-[1,1':3',1"-terphenyl]-3-yl)-[1,2,4]triazolo[4,3-*a*]pyridin-3-yl)benzyl)amino)ethan-1-ol;
*N*-(2,2'-dimethyl-3'-(3-(4-(((((*R*)-5-oxopyrrolidin-2-yl)methyl)amino)methyl)phenyl)-[1,2,4]triazolo[4,3-*a*]pyridin-7-yl)-[1,1'-biphenyl]-3-yl)-5-(((((*R*)-5-oxopyrrolidin-2-yl)methyl)amino)methyl)picolinamide;
(4-(7-(4"-(((2-hydroxyethyl)amino)methyl)-2,2'-dimethyl-[1,1':3',1"-terphenyl]-3-yl)-[1,2,4]triazolo[4,3-*a*]pyridin-3-yl)benzyl)-*L*-proline;
(*R*)-5-((((2',2"-dimethyl-3"-(3-(4-(((((*R*)-5-oxopyrrolidin-2-yl)methyl)amino)methyl)phenyl)-[1,2,4]triazolo[4,3-*a*]pyridin-7-yl)-[1,1':3',1"-terphenyl]-4-yl)methyl)amino)methyl)pyrrolidin-2-one;
2-((4-(7-(4"-(((2-hydroxyethyl)amino)methyl)-3"-methoxy-2,2'-dimethyl-[1,1':3',1"-terphenyl]-3-yl)-[1,2,4]triazolo[4,3-*a*]pyridin-3-yl)benzyl)amino)ethan-1-ol;
2-((4-(7-(3'-(5-(((2-hydroxyethyl)amino)methyl)-6-methoxypyridin-2-yl)-2,2'-dimethyl-[1,1'-biphenyl]-3-yl)-[1,2,4]triazolo[4,3-*a*]pyridin-3-yl)benzyl) amino)ethan-1-ol;
2-((4-(7-(3'-((3-(((2-hydroxyethyl)amino)methyl)-1,7-naphthyridin-8-yl)amino)-2,2'-dimethyl-[1,1'-biphenyl]-3-yl)-[1,2,4]triazolo[4,3-a]pyridin-3-yl)benzyl)amino)ethan-1-ol;
5-(((2-hydroxyethyl)amino)methyl)-N-(3'-(3-(4-(((2-methoxyethyl)amino)methyl)phenyl)-[1,2,4]triazolo[4,3-a]pyridin-7-yl)-2,2'-dimethyl-[1,1'-biphenyl]-3-yl)picolinamide;
(4-(7-(3'-((3-(((R)-3-hydroxypyrrolidin-1-yl)methyl)-1,7-naphthyridin-8-yl)amino)-2,2'-dimethyl-[1,1'-biphenyl]-3-yl)-[1,2,4]triazolo[4,3-o]pyridin-3-yl)benzyl)-*D*-proline;
ethyl (1-(7-(4"-(((2-hydroxyethyl)amino)methyl)-2,2'-dimethyl-[1,1':3',1"-terphenyl]-3-yl)-[1,2,4]triazolo[4,3-*a*]pyridin-3-yl)-1*H*-pyrazole-3-carbonyl)glycinate;
2-(((7-(4"-(((2-hydroxyethyl)amino)methyl)-2,2'-dimethyl-[1,1':3',1"-terphenyl]-3-yl)-[1,2,4]triazolo[1,5-*a*]pyridin-2-yl)methyl)amino)ethan-1-ol;
2-(((7-(4"-(((2-hydroxyethyl)amino)methyl)-2,2'-dimethyl-[1,1':3',1"-terphenyl]-3-yl)-[1,2,4]triazolo[4,3-*a*]pyridin-3-yl)methyl)amino)ethan-1-ol;
(*S*)-5-((((2',2"-dimethyl-3"-(3-(4-(((((*S*)-5-oxopyrrolidin-2-yl)methyl)amino)methyl)phenyl)-[1,2,4]triazolo[4,3-*a*]pyridin-7-yl)-[1,1':3',1"-terphenyl]-4-yl)methyl)amino)methyl)pyrrolidin-2-one;
2-(((3"-(3-((2-hydroxyethyl)amino)-[1,2,4]triazolo[4,3-a]pyridin-7-yl)-2',2"-dimethyl-[1,1':3',1"-terphenyl]-4-yl)methyl)amino)ethan-1-ol;
2-(((2',2"-dichloro-3"-(3-(4-(((2-hydroxyethyl)amino)methyl)phenyl)-[1,2,4]triazolo[4,3-a]pyridin-7-yl)-[1,1':3',1"-terphenyl]-4-yl)methyl)amino)ethan-1-ol;
(4-(7-(2,2'-dichloro-4"-(((2-hydroxyethyl)amino)methyl)-[1,1':3',1"-terphenyl]-3-yl)-[1,2,4]triazolo[4,3-a]pyridin-3-yl)benzyl)-*D*-proline;
2-(((2',2"-dichloro-3"-(3-(((2-hydroxyethyl)amino)methyl)-[1,2,4]triazolo[4,3-*a*]pyridin-7-yl)-[1,1':3',1"-terphenyl]-4-yl)methyl)amino)ethan-1-ol;
(*S*)-5-(((4-(7-(3'-((3-(((*R*)-3-hydroxypyrrolidin-1-yl)methyl)-1,7-naphthyridin-8-yl)amino)-2,2'-dimethyl-[1,1'-biphenyl]-3-yl)-[1,2,4]triazolo[4,3-*a*]pyridin-3-yl)benzyl) amino)methyl)pyrrolidin-2-one;
5-(((2-hydroxyethyl)amino)methyl)-N-(3'-(3-(((2-hydroxyethyl)amino)methyl)-[1,2,4]triazolo[4,3-*a*]pyridin-7-yl)-2,2'-dimethyl-[1,1'-biphenyl]-3-yl)picolinamide;
(*S*)-5-((((7-(4"-(((2-hydroxyethyl)amino)methyl)-2,2'-dimethyl-[1,1':3',1"-terphenyl]-3-yl)-[1,2,4]triazolo[4,3-*a*]pyridin-3-yl)methyl)amino)methyl)pyrrolidin-2-one;
(*S*)-*N*-(2,2'-dimethyl-3'-(3-((((5-oxopyrrolidin-2-yl)methyl)amino)methyl)-[1,2,4]triazolo[4,3-*a*]pyridin-7-yl)-[1,1'-biphenyl]-3-yl)-5-(((2-hydroxyethyl)amino)methyl)picolinamide;
(*S*)-5-((((2',2"-dimethyl-3"-(3-(((((*S*)-5-oxopyrrolidin-2-yl)methyl)amino)methyl)-[1,2,4]triazolo[4,3-*a*]pyridin-7-yl)-[1,1':3',1"-terphenyl]-4-yl)methyl)amino)methyl)pyrrolidin-2-one;
(*R*)-1-((8-((3'-(3-(4-(((2-hydroxyethyl)amino)methyl)phenyl)-[1,2,4]triazolo[4,3-*a*]pyridin-7-yl)-2,2'-dimethyl-[1,1'-biphenyl]-3-yl)amino)-1,7-naphthyridin-3-yl)methyl)pyrrolidin-3-ol;
(*R*)-1-(4-(7-(4"-(((2-hydroxyethyl)amino)methyl)-2,2'-dimethyl-[1,1':3',1"-terphenyl]-3-yl)-[1,2,4]triazolo[4,3-*a*]pyridin-3-yl)benzyl)pyrrolidine-3-carboxylic acid;
5-(((2-hydroxyethyl)amino)methyl)-*N*-(3'-(2-(((2-hydroxyethyl)amino)methyl)-[1,2,4]triazolo[1,5-*a*]pyridin-7-yl)-2,2'-dimethyl-[1,1'-biphenyl]-3-yl)picolinamide;
(*R*)-1-((7-(4"-(((2-hydroxyethyl)amino)methyl)-2,2'-dimethyl-[1,1':3',1"-terphenyl]-3-yl)-[1,2,4]triazolo[4,3-*a*]pyridin-3-yl)methyl)pyrrolidine-3-carboxylic acid;
(*S*)-*N*-(2,2'-dimethyl-3'-(2-((((5-oxopyrrolidin-2-yl)methyl)amino)methyl)-[1,2,4]triazolo[1,5-*a*]pyridin-7-yl)-[1,1'-biphenyl]-3-yl)-4-(((2-hydroxyethyl)amino)methyl)benzamide;
(*S*)-5-((((7-(4"-(((2-hydroxyethyl)amino)methyl)-2,2'-dimethyl-[1,1':3',1"-terphenyl]-3-yl)-[1,2,4]triazolo[1,5-*a*]pyridin-2-yl)methyl) amino) methyl) pyrrolidin-2-one;
4-(((2-hydroxyethyl)amino)methyl)-*N*-(3'-(3-(((2-hydroxyethyl)amino)methyl)-[1,2,4]triazolo[4,3-*a*]pyridin-7-yl)-2,2'-dimethyl-[1,1'-biphenyl]-3-yl)benzamide;
2,2'-((((2,2'-dimethyl-[1,1'-biphenyl]-3,3'-diyl)bis([1,2,4]triazolo[4,3-*a*]pyridine-7,3-diyl))bis(methylene))bis(azanediyl))bis(ethan-1-ol);
4-(((2-hydroxyethyl)amino)methyl)-*N*-(3'-(2-(((2-hydroxyethyl)amino)methyl)-[1,2,4]triazolo[1,5-*a*]pyridin-7-yl)-2,2'-dimethyl-[1,1'-biphenyl]-3-yl)benzamide;
(*S*)-5-((((2',2"-dimethyl-3"-(2-(((((*S*)-5-oxopyrrolidin-2-yl)methyl)amino)methyl)-[1,2,4]triazolo[1,5-*a*]pyridin-7-yl)-[1,1':3',1"-terphenyl]-4-yl)methyl)amino)methyl)pyrrolidin-2-one;
2-(((2',2"-dichloro-3"-(2-(((2-hydroxyethyl)amino)methyl)-[1,2,4]triazolo[1,5-*a*]pyridin-7-yl)-[1,1':3',1"-terphenyl]-4-yl)methyl)amino)ethan-1-ol;
(*S*)-5-((((5-(2,2'-dimethyl-3'-(3-(((((*S*)-5-oxopyrrolidin-2-yl)methyl)amino)methyl)-[1,2,4]triazolo[4,3-*a*]pyridin-6-yl)-[1,1'-biphenyl]-3-yl)-3-methoxypyrazin-2-yl)methyl)amino)methyl)pyrrolidin-2-one;
((7-(4"-(((2-hydroxyethyl)amino)methyl)-2,2'-dimethyl-[1,1':3',1"-terphenyl]-3-yl)-[1,2,4]triazolo[4,3-*a*]pyridin-3-yl)methyl)-*D*-proline.
(*S*)-5-((((2',2"-dichloro-3"-(2-(((((*S*)-5-oxopyrrolidin-2-yl)methyl)amino)methyl)-[1,2,4]triazolo[1,5-*a*]pyridin-7-yl)-[1,1':3',1"-terphenyl]-4-yl)methyl)amino)methyl)pyrrolidin-2-one;
(S)-5-((((2',2"-dichloro-3"-(3-(((((S)-5-oxopyrrolidin-2-yl)methyl)amino)methyl)-[1,2,4]triazolo[4,3-a]pyridin-7-yl)-[1,1':3',1"-terphenyl]-4-yl)methyl)amino)methyl)pyrrolidin-2-one.

13. A pharmaceutical composition comprising a compound of any one of claims 1-12, or a pharmaceutically acceptable salt or a stereoisomer thereof, and one or more pharmaceutically acceptable excipient or carrier.

14. A compound of any one of claims 1-12, or a pharmaceutically acceptable salt or a stereoisomer thereof, or a composition of claim 13, for use in a method of treating cancer.

15. A compound of any one of claims 1-12, or a pharmaceutically acceptable salt or a stereoisomer thereof, or a composition of claim 13, for use in a method of treating an infection, preferably wherein the infection is a viral, or bacterial or a fungal infection.
